(19) Europäisches Patentamt
European Patent Office
Office européen des brevets

(11) EP 1 308 513 A1

(12) **EUROPEAN PATENT APPLICATION**
published in accordance with Art. 158(3) EPC

(43) Date of publication:
**07.05.2003 Bulletin 2003/19**

(21) Application number: **01955656.2**

(22) Date of filing: **10.08.2001**

(51) Int Cl.[7]: **C12N 15/12**, C07K 14/47, G01N 33/50, G01N 33/15, A61K 31/711, A61K 38/00, A61K 45/00, A61K 48/00, A61P 25/00, A61P 25/18, A61P 25/20, A61P 25/22, A61P 25/24, A61P 43/00, A61K 31/4745, C07D 471/04, A61K 31/4375, A61K 31/55, C07D 223/16, A61K 31/4025, C07D 207/32

(86) International application number:
**PCT/JP01/06899**

(87) International publication number:
**WO 02/014513 (21.02.2002 Gazette 2002/08)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **10.08.2000 JP 2000247968**

(71) Applicant: **Takeda Chemical Industries, Ltd.**
**Osaka-shi, Osaka 541-0045 (JP)**

(72) Inventors:
- **MATSUMOTO, Yoshio**
  **Tsukuba-shi, Ibaraki 305-0035 (JP)**
- **WATANABE, Takuya**
  **Yodogawa-ku, Osaka-shi, Osaka 532-0033 (JP)**
- **TAKAHASHI, Hideki**
  **Suita-shi, Osaka 565-0836 (JP)**
- **MORI, Masaaki**
  **Tsukuba-shi, Ibaraki 305-0821 (JP)**

(74) Representative: **Lewin, John Harvey**
**Takeda Patent Office,**
**11-12 Charles II Street**
**London SW1Y 4QU (GB)**

(54) **USE OF POLYPEPTIDE**

(57) The present invention provides uses of a polypeptide having a ligand activity to a sensory epithelium neuropeptide-like receptor (SENR) which is a G protein-coupled receptor protein, and a DNA encoding the same. More specifically, the present invention provides an anti-attention-deficit-disorder or anti-narcolepsy agent, which comprises a polypeptide having a ligand activity for SENR or a salt thereof, as well as a method for screening compounds having an anti-attention-deficit-disorder or anti-narcolepsy activity or compounds having an anti-anxiety, anti-depression, anti-insomnia, anti-schizophrenia or anti-fear activity or salts thereof, which comprises using the above polypeptide or a precursor protein of the polypeptide or a salt thereof.

EP 1 308 513 A1

Sequence Listing

<110> Takeda Chemical Industries, Ltd.

<120> Polypeptide and Its Use

<130> 662731

<150> JP 2000-247968

<151> 2000-08-10

<160> 34

<210> 1

<211> 12

<212> PRT

<213> Pig

<223> The 6th cystein residue binds with the 11th cystein residue to form a intra-molecular disulfide-bond.

<400> 1

Gly Pro Thr Ser Glu Cys Phe Trp Lys Tyr Cys Val
1 5 10 12

<210> 2

<211> 12

<212> PRT

<213> Pig

<223> The 6th cystein residue binds with the 11th cystein residue to form a intra-molecular disulfide-bond.

<400> 2

Gly Pro Pro Ser Glu Cys Phe Trp Lys Tyr Cys Val
1 5 10 12

<210> 3
<211> 386
<212> PRT
<213> Rat

<400> 3

Met Ala Leu Ser Leu Glu Ser Thr Thr Ser Phe His Met Leu Thr Val
1 5 10 15
Ser Gly Ser Thr Val Thr Glu Leu Pro Glu Asp Ser Asn Val Ser Leu
20 25 30
Asn Ser Ser Trp Ser Gly Pro Thr Asp Pro Ser Ser Leu Lys Asp Leu
35 40 45
Val Ala Thr Gly Val Ile Gly Ala Val Leu Ser Ala Met Gly Val Val
50 55 60
Gly Met Val Gly Asn Val Tyr Thr Leu Val Val Met Cys Arg Phe Leu
65 70 75 80
Arg Ala Ser Ala Ser Met Tyr Val Tyr Val Val Asn Leu Ala Leu Ala
85 90 95
Asp Leu Leu Tyr Leu Leu Ser Ile Pro Phe Ile Ile Ala Thr Tyr Val
100 105 110
Thr Lys Asp Trp His Phe Gly Asp Val Gly Cys Arg Val Leu Phe Ser
115 120 125

Leu Asp Phe Leu Thr Met His Ala Ser Ile Phe Thr Leu Thr Ile Met
130 135 140
Ser Ser Glu Arg Tyr Ala Ala Val Leu Arg Pro Leu Asp Thr Val Gln
145 150 155 160
Arg Ser Lys Gly Tyr Arg Lys Leu Leu Val Leu Gly Thr Trp Leu Leu
165 170 175
Ala Leu Leu Leu Thr Leu Pro Met Met Leu Ala Ile Gln Leu Val Arg
180 185 190
Arg Gly Ser Lys Ser Leu Cys Leu Pro Ala Trp Gly Pro Arg Ala His
195 200 205
Arg Thr Tyr Leu Thr Leu Leu Phe Gly Thr Ser Ile Val Gly Pro Gly
210 215 220
Leu Val Ile Gly Leu Leu Tyr Val Arg Leu Ala Arg Ala Tyr Trp Leu
225 230 235 240
Ser Gln Gln Ala Ser Phe Lys Gln Thr Arg Arg Leu Pro Asn Pro Arg
245 250 255
Val Leu Tyr Leu Ile Leu Gly Ile Val Leu Leu Phe Trp Ala Cys Phe
260 265 270
Leu Pro Phe Trp Leu Trp Gln Leu Leu Ala Gln Tyr His Glu Ala Met
275 280 285
Pro Leu Thr Pro Glu Thr Ala Arg Ile Val Asn Tyr Leu Thr Thr Cys
290 295 300
Leu Thr Tyr Gly Asn Ser Cys Ile Asn Pro Leu Leu Tyr Thr Leu Leu
305 310 315 320
Thr Lys Asn Tyr Arg Glu Tyr Leu Arg Gly Arg Gln Arg Ser Leu Gly
325 330 335
Ser Ser Cys His Ser Pro Gly Ser Pro Gly Ser Phe Leu Pro Ser Arg
340 345 350
Val His Leu Gln Gln Asp Ser Gly Arg Ser Leu Ser Ser Ser Ser Gln

355 360 365

Gln Ala Thr Glu Thr Leu Met Leu Ser Pro Val Pro Arg Asn Gly Ala

370 375 380

Leu Leu

385

<210> 4

<211> 638

<212> DNA

<213> Pig

<220>

<223>

<400> 4

cggaccaaca gaagccagga aggaagtgtc ctgcctcctg ccagtcatgt ccaagctggt 60

cccctgcttg ctcctcctag gatgcttagg tctcctcttc gctcttcccg tccctgactc 120

caggaaagag cccctgccct tctcagcacc tgaagatgtc agatcagctt gggatgagct 180

ggaaagagcc tcccttcttc agatgctgcc agagacgcca ggtgcagagg caggagagga 240

tctcagggaa gcagatgccg gaatggacat tttttaccca agaggagaaa tgagaaaggc 300

tttctctgga caagatccta acatttttct gagtcacctt ttggccagaa tcaagaaacc 360

atacaagaaa cgtgggcccc cctctgaatg cttctggaaa tactgtgtct gaagtcacct 420

caacaacaac catcttagaa aatgtaaaaa aagtgcttga cttgacagca gtgcagatga 480

aaaaccaggc aaaccctact ctgttcacta ttatctggaa aataaaccct ttgtgtttgg 540

ccaaaaaaaa aaaaaaaaaa aaaaaaaaaa aaaaaaaaaa aaaaaaaaaa aaaaaaaaaa 600

aaaaaaaaaa aaaaaaaaaa aaaaaaaaaa aaaaaaaa 638

<210> 5

<211> 583

<212> DNA

<213> Pig

<400> 5

gaccaacaga agccaggaag gaagtgtcct gcctcctgcc agtcatgtcc aagctggtcc 60
cctgcttgct cctcctagga tgcttaggtc tcctcttcgc tcttcccgtc cctgactcca 120
ggaaagagcc cctgcccttc tcagcacctg aagatgtcag atcagcttgg gacgagctgg 180
aaagagcctc ccttcttcag atgctgccag agacgccagg tgcagaggca ggagaggatc 240
tcagggaagc agatgccgga atggacattt tttacccaag aggagaaatg agaaaggctt 300
tctctggaca agatcctaac atttttctga gtcacctttt ggccagaatc aagaaaccat 360
acaagaaacg tgggcccccc tctgaatgct tctggaaata ctgtgtctga agtcacctca 420
acaacaacca tcttagaaaa tgtaaaaaaa gtgcttgact tgacagcagt gcagatgaaa 480
aaccaggcaa accctactct gttcactatt atctggaaaa taaacccttt gtgtttggca 540
agttaaaaaa aaaaaaaaaa aaaaaaaaaa aaaaaaaaaa aaa 583

<210> 6

<211> 522

<212> DNA

<213> Pig

<400> 6

agttgaggct tcggaccaac agaagccagg aaggaagtgt cctgcctcct gccagtcatg 60
tccaagctgg tcccctgctt gctcctccta ggatgcttag gtctcctctt cgctcttccc 120
gtccctgact ccaggaaaga gcccctgccc ttctcagatg ccggaatgga catttttttac 180
ccaagaggag aaatgagaaa ggctttctct ggacaagatc ctaacatttt tctgagtcac 240
cttttggcca gaatcaagaa accatacaag aaacgtgggc ccccctctga atgcttctgg 300
aaatactgtg tctgaagtca cctcaacaac aaccatctta gaaaatgtaa aaaaagtgct 360
tgacttgaca gcagtgcaga tgaaaaacca ggcaaaccct actctgttca ctattatctg 420
gaaaataaac cctttgtgtt tggcaagtta aaaaaaaaaa aaaaaaaaaa aaaaaaaaaa 480

aaaaaaaaaa aaaaaaaaaa aaaaaaaaaa aaaaaaaaaa aa 522

<210> 7

<211> 121

<212> PRT

<213> Pig

<400> 7

Met Ser Lys Leu Val Pro Cys Leu Leu Leu Leu Gly Cys Leu Gly Leu
1 5 10 15

Leu Phe Ala Leu Pro Val Pro Asp Ser Arg Lys Glu Pro Leu Pro Phe
20 25 30

Ser Ala Pro Glu Asp Val Arg Ser Ala Trp Asp Glu Leu Glu Arg Ala
35 40 45

Ser Leu Leu Gln Met Leu Pro Glu Thr Pro Gly Ala Glu Ala Gly Glu
50 55 60

Asp Leu Arg Glu Ala Asp Ala Gly Met Asp Ile Phe Tyr Pro Arg Gly
65 70 75 80

Glu Met Arg Lys Ala Phe Ser Gly Gln Asp Pro Asn Ile Phe Leu Ser
85 90 95

His Leu Leu Ala Arg Ile Lys Lys Pro Tyr Lys Lys Arg Gly Pro Pro
100 105 110

Ser Glu Cys Phe Trp Lys Tyr Cys Val
115 120

<210> 8

<211> 85

<212> PRT

<213> Pig

<400> 8

Met Ser Lys Leu Val Pro Cys Leu Leu Leu Leu Gly Cys Leu Gly Leu
1 5 10 15

Leu Phe Ala Leu Pro Val Pro Asp Ser Arg Lys Glu Pro Leu Pro Phe
20 25 30

Ser Asp Ala Gly Met Asp Ile Phe Tyr Pro Arg Gly Glu Met Arg Lys
35 40 45

Ala Phe Ser Gly Gln Asp Pro Asn Ile Phe Leu Ser His Leu Leu Ala
50 55 60

Arg Ile Lys Lys Pro Tyr Lys Lys Arg Gly Pro Pro Ser Glu Cys Phe
65 70 75 80

Trp Lys Tyr Cys Val
85

<210> 9

<211> 12

<212> PRT

<213> Bovine

<223> The 6th cystein residue binds with the 11th cystein residue to form a intra-molecular disulfide-bond.

<400> 9

Gly Pro Ser Ser Glu Cys Phe Trp Lys Tyr Cys Val
1 5 10 12

<210> 10

<211> 11

<212> PRT

<213> Human

<223> The 5th cystein residue binds with the 10th cystein residue to form a intra-molecular disulfide-bond.

<400> 10

Glu Thr Pro Asp Cys Phe Trp Lys Tyr Cys Val
1 5 10 11

<210> 11
<211> 389
<212> PRT
<213> Human

<400> 11

Met Ala Leu Thr Pro Glu Ser Pro Ser Ser Phe Pro Gly Leu Ala Ala
1 5 10 15
Thr Gly Ser Ser Val Pro Glu Pro Pro Gly Gly Pro Asn Ala Thr Leu
20 25 30
Asn Ser Ser Trp Ala Ser Pro Thr Glu Pro Ser Ser Leu Glu Asp Leu
35 40 45
Val Ala Thr Gly Thr Ile Gly Thr Leu Leu Ser Ala Met Gly Val Val
50 55 60
Gly Val Val Gly Asn Ala Tyr Thr Leu Val Val Thr Cys Arg Ser Leu
65 70 75 80
Arg Ala Val Ala Ser Met Tyr Val Tyr Val Val Asn Leu Ala Leu Ala
85 90 95
Asp Leu Leu Tyr Leu Leu Ser Ile Pro Phe Ile Val Ala Thr Tyr Val
100 105 110

Thr Lys Glu Trp His Phe Gly Asp Val Gly Cys Arg Val Leu Phe Gly
115 120 125

Leu Asp Phe Leu Thr Met His Ala Ser Ile Phe Thr Leu Thr Val Met
130 135 140

Ser Ser Glu Arg Tyr Ala Ala Val Leu Arg Pro Leu Asp Thr Val Gln
145 150 155 160

Arg Pro Lys Gly Tyr Arg Lys Leu Leu Ala Leu Gly Thr Trp Leu Leu
165 170 175

Ala Leu Leu Leu Thr Leu Pro Val Met Leu Ala Met Arg Leu Val Arg
180 185 190

Arg Gly Pro Lys Ser Leu Cys Leu Pro Ala Trp Gly Pro Arg Ala His
195 200 205

Arg Ala Tyr Leu Thr Leu Leu Phe Ala Thr Ser Ile Ala Gly Pro Gly
210 215 220

Leu Leu Ile Gly Leu Leu Tyr Ala Arg Leu Ala Arg Ala Tyr Arg Arg
225 230 235 240

Ser Gln Arg Ala Ser Phe Lys Arg Ala Arg Arg Pro Gly Ala Arg Ala
245 250 255

Leu Arg Leu Val Leu Gly Ile Val Leu Leu Phe Trp Ala Cys Phe Leu
260 265 270

Pro Phe Trp Leu Trp Gln Leu Leu Ala Gln Tyr His Gln Ala Pro Leu
275 280 285

Ala Pro Arg Thr Ala Arg Ile Val Asn Tyr Leu Thr Thr Cys Leu Thr
290 295 300

Tyr Gly Asn Ser Cys Ala Asn Pro Phe Leu Tyr Thr Leu Leu Thr Arg
305 310 315 320

Asn Tyr Arg Asp His Leu Arg Gly Arg Val Arg Gly Pro Gly Ser Gly
325 330 335

Gly Gly Arg Gly Pro Val Pro Ser Leu Gln Pro Arg Ala Arg Phe Gln

340 345 350

Arg Cys Ser Gly Arg Ser Leu Ser Ser Cys Ser Pro Gln Pro Thr Asp

355 360 365

Ser Leu Val Leu Ala Pro Ala Ala Pro Ala Arg Pro Ala Pro Glu Gly

370 375 380

Pro Arg Ala Pro Ala

385

<210> 12

<211> 36

<212> DNA

<213> Pig

<400> 12

gggcccccct ctgaatgctt ctggaaatac tgtgtc 36

<210> 13

<211> 36

<212> DNA

<213> Bovine

<400> 13

ggaccttcct ctgaatgctt ctggaaatac tgtgtc 36

<210> 14

<211> 122

<212> PRT

<213> Bovine

<400> 14

Met Tyr Lys Leu Val Ser Cys Cys Leu Leu Phe Ile Gly Ser Leu Asn
1 5 10 15

Pro Leu Leu Ser Leu Pro Val Leu Asp Ser Arg Gln Glu Ser Leu Gln
20 25 30

Leu Leu Ala Pro Glu Asp Val Arg Ser Thr Leu Asp Glu Leu Glu Arg
35 40 45

Ala Ser Leu Leu Gln Met Leu Pro Glu Met Ser Gly Ala Glu Thr Gly
50 55 60

Glu Gly Leu Arg Asn Thr Asp Pro Ile Thr Asn Ile Phe Tyr Pro Arg
65 70 75 80

Gly Asn Met Arg Lys Ala Phe Ser Gly Gln Asp Pro Lys Leu Phe Leu
85 90 95

Ser Asp Leu Leu Ser Arg Ile Arg Lys Gln Ser Lys Lys Arg Gly Pro
100 105 110

Ser Ser Glu Cys Phe Trp Lys Tyr Cys Val
115 120

<210> 15

<211> 431

<212> DNA

<213> Bovine

<400> 15

atgtataagc tggtctcctg ctgtttgctt ttcataggat ccttaaatcc gctcctgtct 60

cttcctgtcc ttgactccag gcaagagtcc ctgcagctct tagcacctga agatgtcaga 120

tcaactctgg atgagctgga aagagcgtct cttctgcaga tgctgccaga gatgtcaggc 180

gcagagacag gagagggtct taggaacaca gatcccatta ccaacatttt ttacccaaga 240

ggaaacatga gaaaggcctt ctctgggcaa gatcctaagc ttttcctgag tgaccttttg 300

tccagaatta ggaaacaatc taagaaacgt ggaccttcct ctgaatgctt ctggaaatac 360
tgtgtctgaa gcaaaatgac cctctactag ttacctccaa gacgaccatc tgagaaaatg 420
taaaataaag a 431

<210> 16
<211> 405
<212> DNA
<213> Rat

<400> 16
tcttcccgtc gtcatggaca gggtgccctt ctgctgcctg ctcttcgtag gactcctgaa 60
tccactcctg tcttttcccg tcacggacac tggtgaaatg tctcttcagc ttccagtgct 120
tgaggaaaat gctcttcggg ctctggagga gctggagagg actgccctcc tgcagacgct 180
gcgccagacc gtgggcacag aagcagaggg aagccttggc caggcagatc ccagtgccga 240
gactcccact ccaaggggaa gcttgaggaa ggctctcact gggcaagatt ctaacactgt 300
actgagccgt cttttggcga gaaccaggaa acaacgtaag caacacggga ctgccccaga 360
atgcttctgg aagtactgca tttgaagaga gacgtctcct cagaa 405

<210> 17
<211> 123
<212> PRT
<213> Rat

<400> 17
Met Asp Arg Val Pro Phe Cys Cys Leu Leu Phe Val Gly Leu Leu Asn
1 5 10 15
Pro Leu Leu Ser Phe Pro Val Thr Asp Thr Gly Glu Met Ser Leu Gln
20 25 30
Leu Pro Val Leu Glu Glu Asn Ala Leu Arg Ala Leu Glu Glu Leu Glu

35 40 45

Arg Thr Ala Leu Leu Gln Thr Leu Arg Gln Thr Val Gly Thr Glu Ala

50 55 60

Glu Gly Ser Leu Gly Gln Ala Asp Pro Ser Ala Glu Thr Pro Thr Pro

65 70 75 80

Arg Gly Ser Leu Arg Lys Ala Leu Thr Gly Gln Asp Ser Asn Thr Val

85 90 95

Leu Ser Arg Leu Leu Ala Arg Thr Arg Lys Gln Arg Lys Gln His Gly

100 105 110

Thr Ala Pro Glu Cys Phe Trp Lys Tyr Cys Ile

115 120

<210> 18

<211> 17

<212> PRT

<213> Rat

<223> Xaa shows pyroglutamic acid or glutamine

<400> 18

Xaa Arg Lys Gln His Gly Thr Ala Pro Glu Cys Phe Trp Lys Tyr Cys Ile

1 5 10 15 17

<210> 19

<211> 14

<212> PRT

<213> Rat

<223> Xaa shows pyroglutamic acid or glutamine

<400> 19

Xaa His Gly Thr Ala Pro Glu Cys Phe Trp Lys Tyr Cys Ile

1 5 10 14

<210> 20

<211> 51

<212> DNA

<213> Rat

<400> 20

caacgtaagc aacacgggact gccccagaa tgcttctgga agtactgcat t 51

<210> 21

<211> 42

<212> DNA

<213> Rat

<400> 21

caacacggga ctgccccaga atgcttctgg aagtactgca tt 42

<210> 22

<211> 403

<212> DNA

<213> Mouse

<400> 22

atggacaggg tgcccttctg ctgcctgctc ttcataggac ttctgaatcc actgctgtcc 60

cttcccgtca cggacactgg tgagaggact cttcagcttc cagtgcttga ggaagacgct 120

cttcgggctc tggaggagct ggagaggatg gccctcctgc agaccctgcg tcagaccatg 180

ggcacggaag caggggagag ccctggagaa gcaggtccca gcactgagac tcccactcca 240

cggggaagca tgaggaaggc tttcgctggg caaaattcta acactgtact gagtcgtctc 300

ttggcaagaa ccaggaaaca acataagcaa cacggggctg ccccagagtg cttctggaaa 360

tactgcattt gaggagacac aagcgcccgt tggtctctca gaa 403

<210> 23

<211> 123

<212> PRT

<213> Mouse

<400> 23

Met Asp Arg Val Pro Phe Cys Cys Leu Leu Phe Ile Gly Leu Leu Asn
1 5 10 15

Pro Leu Leu Ser Leu Pro Val Thr Asp Thr Gly Glu Arg Thr Leu Gln
20 25 30

Leu Pro Val Leu Glu Glu Asp Ala Leu Arg Ala Leu Glu Glu Leu Glu
35 40 45

Arg Met Ala Leu Leu Gln Thr Leu Arg Gln Thr Met Gly Thr Glu Ala
50 55 60

Gly Glu Ser Pro Gly Glu Ala Gly Pro Ser Thr Glu Thr Pro Thr Pro
65 70 75 80

Arg Gly Ser Met Arg Lys Ala Phe Ala Gly Gln Asn Ser Asn Thr Val
85 90 95

Leu Ser Arg Leu Leu Ala Arg Thr Arg Lys Gln His Lys Gln His Gly
100 105 110

Ala Ala Pro Glu Cys Phe Trp Lys Tyr Cys Ile
115 120

<210> 24

<211> 17

<212> PRT

<213> Mouse

<223> Xaa shows pyroglutamic acid or glutamine

<400> 24

Xaa His Lys Gln His Gly Ala Ala Pro Glu Cys Phe Trp Lys Tyr Cys Ile
5 10 15 17

<210> 25

<211> 51

<212> DNA

<213> Mouse

<400> 25

caacataagc aacacggggc tgccccagag tgcttctgga aatactgcat t 51

<210> 26

<211> 20

<212> PRT

<213> Rat

<400> 26

Thr Arg Lys Gln Arg Lys Gln His Gly Thr Ala Pro Glu Cys Phe Trp
1 5 10 15

Lys Tyr Cys Ile
20

<210> 27

<211> 24

<212> PRT

<213> Rat

<400> 27

Leu Leu Ala Arg Thr Arg Lys Gln Arg Lys Gln His Gly Thr Ala Pro
1 5 10 15

Glu Cys Phe Trp Lys Tyr Cys Ile
20 24

<210> 28

<211> 20

<212> PRT

<213> Mouse

<400> 28

Thr Arg Lys Gln His Lys Gln His Gly Ala Ala Pro Glu Cys Phe Trp
1 5 10 15

Lys Tyr Cys Ile
20

<210> 29

<211> 24

<212> PRT

<213> Mouse

<400> 29

Leu Leu Ala Arg Thr Arg Lys Gln His Lys Gln His Gly Ala Ala Pro
1 5 10 15

Glu Cys Phe Trp Lys Tyr Cys Ile

20 24

<210> 30

<211> 60

<212> DNA

<213> Rat

<400> 30

accaggaaac aacgtaagca acacgggact gccccagaat gcttctggaa gtactgcatt 60

<210> 31

<211> 72

<212> DNA

<213> Rat

<400> 31

cttttggcga gaaccaggaa acaacgtaag caacacggga ctgccccaga atgcttctgg 60

aagtactgca tt 72

<210> 32

<211> 60

<212> DNA

<213> Mouse

<400> 32

accaggaaac aacataagca acacggggct gccccagagt gcttctggaa atactgcatt 60

<210> 33

<211> 72

<212> DNA

<213> Mouse

<400> 33

ctcttggcaa gaaccaggaa acaacataag caacacgggg ctgcccccaga gtgcttctgg 60

aaatactgca tt 72

<210> 34

<211> 33

<212> DNA

<213> Human

<400> 34

gagactcctg attgcttctg gaaatactgt gtc 33

## Description

### FIELD OF THE INVENTION

The present invention relates to uses of a polypeptide having a ligand activity for a G protein-coupled receptor protein GPR14. [sensory epithelium neuropeptide-like receptor (SENR)] and a DNA encoding the same.

### RELATED ART

A large number of hormones and neurotransmitters control body functions through specific receptors present on cell membranes. Since most of such receptors mediate intracellular signal transduction through activation of guanine nucleotide-binding proteins (hereinafter also referred to as G proteins) coupled to the receptors and also share a common structure with 7 transmembrane domains, they are collectively called G protein-coupled receptors or 7-transmembrane receptors.

SENR is one of the orphan G protein-coupled receptors reported previously (Tal, M. et al., Biochem. Biophys. Res. Commun., 209, 752-759, 1995). SENR shares a low homology with the somatostatin receptor (SSTR4), but its ligand has not yet been identified. GPR14 reported by Marchese, A. et al. (Marchese, A., Genomics, 29, 335-344, 1995) is a receptor identical with SENR Recently, some groups have reported that a ligand for this receptor is urotensin II (Davenport, A.P. and Maguire, J.J., Trends Pharmacol. Sci. 21, 80-82, 2000).

### DISCLOSURE OF THE INVENTION

(Technical Problems to be Solved by the Invention)

A ligand for a G protein-coupled receptor GPR14 (SENR) expressed in the central nerve system, circulatory system, genital system, immune system, digestive organs, urinary system organs, sensory organs or the like may be useful as a medicament. As to functions, such a ligand has been reported to have effects on the circulatory system (Ames, R.S., et al., Nature, 401, 282-286, 1999), but there is no report on other effects.

(Means Solving the Problems)

The inventors of the present invention administered a ligand for GPR14 (SENR) into the brain ventricles of rats and succeeded in clarifying effects and functions of a polypeptide recognized as a ligand by the receptor protein (GPR14 (SENR), based on measurements of spontaneous locomotion and rearings and in an elevated plus maze, etc.

Further, the inventors of the present invention found that the ligand identified as an active factor could be used to screen compounds having an anti-attention-deficit-disorder or anti-narcolepsy activity or compounds having an anti-anxiety, anti-depression, anti-insomnia, anti-schizophrenia or anti-fear activity.

Namely, the present invention is directed to the following embodiments:

(1) an anti-attention-deficit-disorder or anti-narcolepsy agent, which comprises a polypeptide containing the same or substantially the same amino acid sequence as shown in SEQ ID NO: 1 or an amide or ester of the polypeptide or a salt thereof;

(2) the anti-attention-deficit-disorder or anti-narcolepsy agent of (1) above,
wherein substantially the same amino acid sequence is the amino acid sequence shown in SEQ ID NO: 2, SEQ ID NO: 9, SEQ ID NO: 10, SEQ ID NO: 18, SEQ ID NO: 19, SEQ ID NO: 24, SEQ ID NO: 26, SEQ ID NO: 27, SEQ ID NO: 28 or SEQ ID NO: 29;

(3) an anti-attention-deficit-disorder or anti-narcolepsy agent, which comprises a DNA containing the nucleotide sequence encoding the polypeptide of (1) above;

(4) the anti-attention-deficit-disorder or anti-narcolepsy agent of (3) above, wherein the DNA contains the nucleotide sequence shown in SEQ ID NO: 12, SEQ ID NO: 13, SEQ ID NO: 34, SEQ ID NO: 20, SEQ ID NO: 21, SEQ ID NO: 25, SEQ ID NO: 30, SEQ ID NO: 31, SEQ ID NO: 32 or SEQ ID NO: 33;

(5) an anti-attention-deficit-disorder or anti-narcolepsy agent, which comprises a precursor protein of the polypeptide of (1) above or an amide or ester of the precursor protein or a salt thereof;

(6) the anti-attention-deficit-disorder or anti-narcolepsy agent of (5) above, wherein the agent comprises the same or substantially the same amino acid sequence as shown in SEQ ID NO: 7, SEQ ID NO: 8, SEQ ID NO: 14, SEQ ID NO: 17 or SEQ ID NO: 23;

(7) a method for screening compounds having an anti-attention-deficit-disorder or anti-narcolepsy activity or compounds having an anti-anxiety, anti-depression, anti-insomnia, anti-schizophrenia or anti-fear activity or salts of

thereof, which comprises using a polypeptide containing the same or substantially the same amino acid sequence as shown in SEQ ID NO: 1 or an amide or ester of the polypeptide or a salt thereof or using a precursor protein of the polypeptide or a salt thereof;

(8) a kit for screening compounds having an anti-attention-deficit-disorder or anti-narcolepsy activity or compounds having an anti-anxiety, anti-depression, anti-insomnia, anti-schizophrenia or anti-fear activity or salts thereof, which comprises a polypeptide containing the same or substantially the same amino acid sequence as shown in SEQ ID NO: 1 or an amide or ester of the polypeptide or a salt thereof or comprises a precursor protein of the polypeptide or a salt thereof;

(9) a compound having an anti-attention-deficit-disorder or anti-narcolepsy activity or a compound having an anti-anxiety, anti-depression, anti-insomnia, anti-schizophrenia or anti-fear activity or a salt thereof, which is obtainable using the method of (7) above or the kit of (8) above;

(10) an anti-attention-deficit-disorder or anti-narcolepsy agent or an anti-anxiety, anti-depression, anti-insomnia, anti-schizophrenia or anti-fear agent, which comprises the compound of (9) above or a salt thereof;

(11) a diagnostic method for attention deficit disorder or narcolepsy or for anxiety, depression, insomnia, schizophrenia or fear, which comprises using a polynucleotide containing part or all of a nucleotide sequence complementary to a DNA containing the nucleotide sequence encoding the polypeptide of (1) above;

(12) an anti-anxiety, anti-depression, anti-insomnia, anti-schizophrenia or anti-fear agent, which comprises an antibody against the polypeptide of (1) above or the precursor protein of (5) above or an amide or ester of the polypeptide or precursor protein or a salt thereof;

(13) an anti-attention-deficit-disorder or anti-narcolepsy agent or an anti-anxiety, anti-depression, anti-insomnia, anti-schizophrenia or anti-fear agent, which comprises an antibody against a protein containing the amino acid sequence shown in SEQ ID NO: 3 or SEQ ID NO: 11 or an amide or ester of the protein or a salt thereof;

(14) a diagnostic agent for anxiety, depression, insomnia, schizophrenia or fear, which comprises an antibody against the polypeptide of (1) above or the precursor protein of (5) above or an amide or ester of the polypeptide or precursor protein or a salt thereof;

(15) a diagnostic agent for attention deficit disorder or narcolepsy or for anxiety, depression, insomnia, schizophrenia or fear, which comprises an antibody against a protein containing the amino acid sequence shown in SEQ ID NO: 3 or SEQ ID NO: 11 or an amide or ester of the protein or a salt thereof;

(16) a diagnostic agent, which comprises single nucleotide polymorphisms (SNPs) of a DNA containing the nucleotide sequence shown in SEQ ID NO: 34;

(17) the diagnostic agent of (16) above, wherein the diagnostic agent is provided for diagnosis of attention deficit disorder or narcolepsy or diagnosis of anxiety, depression, insomnia, schizophrenia or fear;

(18) a diagnostic method for attention deficit disorder or narcolepsy or for anxiety, depression, insomnia, schizophrenia or fear, which comprises analyzing single nucleotide polymorphisms (SNPs) of a DNA containing the nucleotide sequence shown in SEQ ID NO: 34;

(19) an anti-attention-deficit-disorder or anti-narcolepsy agent, which comprises a GPR14 agonist;

(20) an anti-anxiety, anti-depression, anti-insomnia, anti-schizophrenia or anti-fear agent, which comprises a GPR14 antagonist;

(21) the anti-anxiety, anti-depression, anti-insomnia, anti-schizophrenia or anti-fear agent of (20) above, wherein the GPR14 antagonist is a compound of Formula (Ia):

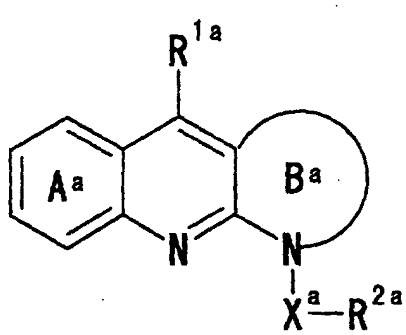

(Ia)

[wherein $A^a$ represents an optionally substituted benzene ring, $B^a$ represents an optionally substituted 5- to 8-membered ring, $X^a$ represents a divalent group containing 1 to 4 atoms in its linear chain moiety, $R^{1a}$ represents an optionally substituted amino group, and $R^{2a}$ represents an optionally substituted cyclic group] or a salt thereof;

(22) the anti-anxiety, anti-depression, anti-insomnia, anti-schizophrenia or anti-fear agent of (20) above, wherein

the GPR14 antagonist is a compound of Formula (IIa):

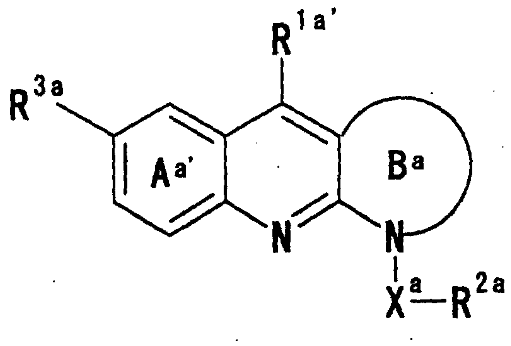

(IIa)

[wherein $A^{a'}$ represents a benzene ring which may have a substituent in addition to the substituent $R^{3a}$, $B^a$ represents an optionally substituted 5- to 8-membered ring, $X^a$ represents a divalent group containing 1 to 4 atoms in its linear chain moiety, $R^{1a'}$ represents a substituted amino group, $R^{2a}$ represents an optionally substituted cyclic group, and $R^{3a}$ represents an optionally substituted hydrocarbon group, an optionally substituted heterocyclic group, a nitro group, a halogen atom, an optionally substituted amino group or a group of the formula $R^{4a}$-$Y^a$- (wherein $Y^a$ represents an oxygen atom or an optionally oxidized sulfur atom, and $R^{4a}$ represents an optionally substituted hydrocarbon group or an optionally substituted heterocyclic group)] or a salt thereof;
(23) the anti-anxiety, anti-depression, anti-insomnia, anti-schizophrenia or anti-fear agent of (20) above, wherein the GPR14 antagonist is a compound of Formula (Ib):

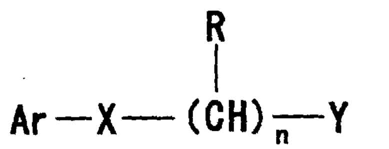

(Ib)

[wherein Ar represents an optionally substituted aryl group, X represents a spacer containing 1 to 4 atoms in its linear chain moiety, n represents an integer of 1 to 10, R represents a hydrogen atom or an optionally substituted hydrocarbon group which may be the same or different in n repeated units or R may form a ring together with Ar or a substituent on Ar, and Y represents an optionally substituted amino group or an optionally substituted nitrogen-containing heterocyclic group] or a salt thereof;
(24) the anti-anxiety, anti-depression, anti-insomnia, anti-schizophrenia or anti-fear agent of (20) above, wherein the GPR14 antagonist is a compound of Formula (IIb):

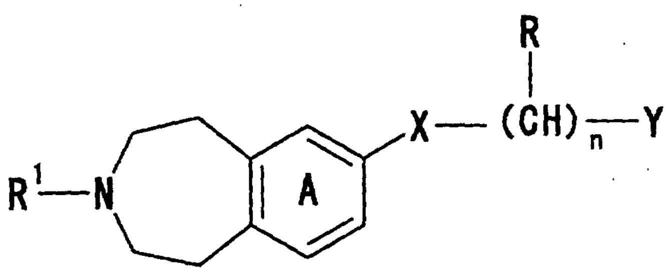

(IIb)

[wherein $R^1$ represents a hydrogen atom, an optionally substituted hydrocarbon group or an optionally substituted acyl group, the ring A represents a benzene ring which may have an additional substituent, X represents a spacer containing 1 to 4 atoms in its linear chain moiety, n represents an integer of 1 to 10, R represents a hydrogen atom or an optionally substituted hydrocarbon group which may be the same or different in n repeated units or R may form a ring together with the ring A or a substituent on the ring A, and Y represents an optionally substituted amino group or an optionally substituted nitrogen-containing heterocyclic group] or a salt thereof;
(25) the anti-anxiety, anti-depression, anti-insomnia, anti-schizophrenia or anti-fear agent of (20) above, wherein the GPR14 antagonist is a compound of Formula (Ic):

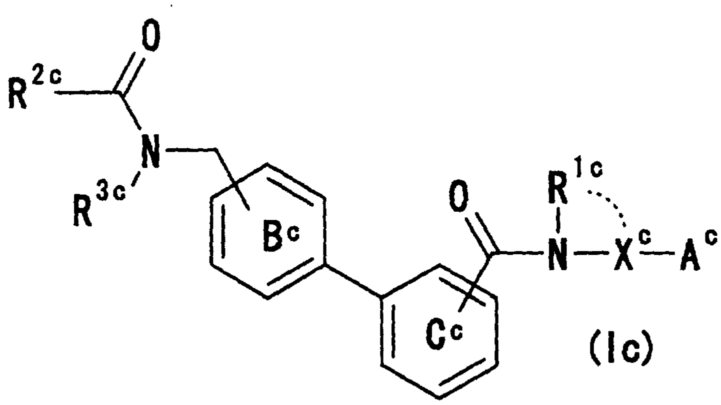

(Ic)

[wherein $R^{1c}$ represents a hydrogen atom or an optionally substituted hydrocarbon group, $X^c$ represents a spacer containing 1 to 12 atoms in its linear chain moiety, $R^{1c}$ and $X^c$ may together form a ring, $A^c$ represents an optionally substituted amino group or an optionally substituted nitrogen-containing heterocyclic group, $R^{2c}$ represents an optionally substituted hydrocarbon group or an optionally substituted amino group, $R^{3c}$ represents an optionally substituted hydrocarbon group, the rings $B^c$ and $C^c$ each represent a benzene ring which may further be substituted] or a salt thereof;

(26) a prophylactic or therapeutic method for attention deficit disorder or narcolepsy, which comprises administering an effective amount of a GPR14 agonist to a mammal;

(27) use of a GPR14 agonist for the preparation of a prophylactic or therapeutic agent for attention deficit disorder or narcolepsy;

(28) a prophylactic or therapeutic method for anxiety, depression, insomnia, schizophrenia or fear, which comprises administering an effective amount of a GPR14 antagonist to a mammal; and

(29) use of a GPR14 antagonist for the preparation of a prophylactic or therapeutic agent for anxiety, depression, insomnia, schizophrenia or fear.

## BRIEF DESCRIPTION OF DRAWINGS

Figure 1 shows changes in spontaneous locomotion induced by administration of the polypeptide shown in SEQ ID NO: 9 (10 nmol) into the lateral ventricle in Example 1. A) Changes in spontaneous locomotion, B) Changes in the number of rearings (PBS: n = 10; polypeptide of SEQ ID NO: 9: n = 10).

Figure 2 shows changes in spontaneous locomotion induced by administration of the polypeptide shown in SEQ ID NO: 9 (1 nmol) into the lateral ventricle in Example 2. A) Changes in spontaneous locomotion, B) Changes in the number of rearings. Each value is expressed as a mean ± SEM (PBS: n = 17; polypeptide of SEQ ID NO: 9: n = 10).

Figure 3 shows changes in spontaneous locomotion induced by administration of the polypeptide shown in SEQ ID NO: 9 (1 nmol or 10 nmol) into the lateral ventricle in Example 3. A) Changes in spontaneous locomotion, B) Changes in the number of rearings. Each value is expressed as a mean ± SEM (PBS: n = 27; polypeptide of SEQ ID NO: 9 (1 nmol): n = 9; polypeptide of SEQ ID NO: 9 (10 nmol): n = 10).

Figure 4 shows effects of diazepam (1 mg/kg) on administration of the polypeptide shown in SEQ ID NO: 9 (10 nmol) or PACAP38 (3 nmol) into the lateral ventricle in Example 4. A) Effects of diazepam (1 mg/kg) on changes in spontaneous locomotion induced by administration of the polypeptide shown in SEQ ID NO: 9 (10 nmol) into the lateral ventricle, B) Effects of diazepam (1 mg/kg) on cumulative locomotion induced by administration of the polypeptide shown in SEQ ID NO: 9 (10 nmol) or PACAP38 (3 nmol) into the lateral ventricle. Each value is expressed as a mean ± SEM (polypeptide of SEQ ID NO: 9: n = 10; diazepam + polypeptide of SEQ ID NO: 9: n = 10; PACAP38: n = 8; diazemap + PACAP38: n = 8). *p<0.05, Dunnett.

Figure 5 shows a schematic view of an elevated plus maze used in the test of Example 5.

Figure 6 shows the results of elevated plus maze test in Example 5 where the polypeptide shown in SEQ ID NO: 9 (10 nmol) was administered into the lateral ventricle [A) Number of closed arm entries, B) Number of open arm entries, C) Time on open arms]. Each value is expressed as a mean ± SEM (n = 9-10). *p<0.05, Dunnett.

Figure 7 shows the results of hole board test in Example 7 where the polypeptide shown in SEQ ID NO: 9 (0.1 nmol, 0.3 nmol or 3 nmol) was administered into the lateral ventricle. A) Cumulative spontaneous locomotion (5 minutes), B) Number of head dippings (5 minutes). Each value is expressed as a mean ± SEM (PBS: n = 18;

polypeptide of SEQ ID NO: 9 (0.1 nmol): n = 10; polypeptide of SEQ ID NO: 9 (0.3 nmol): n = 17; polypeptide of SEQ ID NO: 9 (3 nmol): n = 8). *p<0.05, **p<0.01, Dunnett.

Figure 8 shows effects of the polypeptide shown in SEQ ID NO: 9 (10 nmol) and CRF (1 nmol) on plasma ACTH levels (PBS: n = 8; polypeptide of SEQ ID NO: 9 (10 nmol): n = 7; CRF (1 nmol): n = 8). **p<0.01, Dunnett.

## PREFERRED EMODIMENT OF THE INVENTION

As used herein, the term "substantially the same" means that polypeptides or proteins share substantially the same activities, including binding activity between ligand and receptor (GPR14 (SENR)), physiological properties, etc. Amino acid substitution, deletion, addition or insertion often produces minor changes in physiological and/or chemical properties of polypeptides or proteins. In such cases, amino acid-substituted, -deleted, -added or -inserted polypeptides will be regarded as substantially identical with non-substituted, non-deleted, non-added or non-inserted polypeptides. Substantially the same substitutes for amino acids in the amino acid sequence may be selected, for example, among other amino acids belonging to the same class as the amino acids to be substituted. Examples of nonpolar (hydrophobic) amino acids include alanine, leucine, isoleucine, valine, proline, phenylalanine, tryptophan and methionine. Examples of polar (neutral) amino acids include glycine, serine, threonine, cysteine, tyrosine, asparagine and glutamine. Examples of positively-charged (basic) amino acids include arginine, lysine and histidine. Examples of negatively-charged (acidic) amino acids include aspartic acid and glutamic acid.

The polypeptide of the present invention or an amide or ester of the polypeptide or a salt thereof is a ligand for GRP14 (SENR). Specific examples include polypeptides containing the same or substantially the same amino acid sequence as shown in SEQ ID NO: 1 or amides or esters of the polypeptides or salts thereof. In the specification, the polypeptide of the present invention hereinafter refers to a polypeptide that is a ligand for GRP14 (SENR).

Preparation and uses of the polypeptide of the present invention or an amide or ester of the polypeptide or a salt thereof (hereinafter also simply referred to as the polypeptide of the present invention) will be described below in more detail.

The polypeptide of the present invention may be any polypeptide derived from every tissue (e.g., hypophysis, pancreas, brain, kidney, liver, genital gland, thyroid gland, gallbladder, bone marrow, suprarenal gland, skin, muscle, lung, digestive tract, blood vessel, heart) or every cell of homeotherms (e.g., human, guinea pig, rat, mouse, pig, sheep, cattle, monkey) as long as it contains the same or substantially the same amino acid sequence as shown in SEQ ID NO: 1. Examples of the polypeptide of the present invention include, in addition to polypeptides containing the amino acid sequence shown in SEQ ID NO: 1, polypeptides having activities of substantially the same type as found in the polypeptides containing the amino acid sequence shown in SEQ ID NO: 1 (e.g., polypeptides containing the amino acid sequence shown in SEQ ID NO: 2, 9, 10, 18, 19, 24, 26, 27, 28 or 29). Activities of substantially the same type include receptor-binding activity, signal transduction activity and the like. The term "substantially the same type" means that activities including receptor-binding activity are qualitatively identical among polypeptides. Thus, quantitative factors, such as strength of receptor-binding activity and molecular weights of polypeptides, may vary from polypeptide to polypeptide.

Examples of substantially the same amino acid sequence as shown in SEQ ID NO: 1 include amino acid sequences sharing a homology of at least about 50%, preferably at least about 60%, more preferably at least about 70%, even more preferably at least about 80%, particularly preferably at least about 90%, most preferably at least about 95% with the amino acid sequence shown in SEQ ID NO: 1.

Other examples of substantially the same amino acid sequence as shown in SEQ ID NO: 1 include proteins containing (i) an amino acid sequence with deletion of one or more amino acids (preferably around 1 to 5, more preferably around 1 to 3, even more preferably 1 or 2 amino acids) in the amino acid sequence shown in SEQ ID NO: 1; (ii) an amino acid sequence with addition of one or more amino acids (preferably around 1 to 20, more preferably around 1 to 12, even more preferably several (1 to 5) amino acids) in the amino acid sequence shown in SEQ ID NO: 1; (iii) an amino acid sequence with substitution of one or more amino acids (preferably around 1 to 5, more preferably around 1 to 3, even more preferably 1 or 2 amino acids) in the amino acid sequence shown in SEQ ID NO: 1; or (iv) an amino acid sequence with any combination of the deletion, addition and substitution mentioned above.

Specific examples of a polypeptide containing substantially the same amino acid sequence as shown in SEQ ID NO: 1 include polypeptides containing an amino acid sequence with substitution at the third amino acid (Thr) from the N-terminal in the amino acid sequence shown in SEQ ID NO: 1, in which the third amino acid (Thr) is replaced by other amino acid (e.g., Ala, Leu, Ile, Val, Pro, Phe, Trp, Met, Gly, Ser, Cys, Tyr, Asn, Gln, Arg, Lys, His, Asp, Glu). Above all, preferred examples include polypeptides containing an amino acid sequence (SEQ ID NO: 2) with Thr-to-Pro substitution at the third amino acid from the N-terminal in the amino acid sequence shown in SEQ ID NO: 1 and polypeptides containing an amino acid sequence (SEQ ID NO: 9) with Thr-to-Ser substitution at the third amino acid from the N-terminal in the amino acid sequence shown in SEQ ID NO: 1.

Other preferred examples of a polypeptide containing substantially the same amino acid sequence as shown

in SEQ ID NO: 1 include (1) polypeptides (i) having a glutamine residue or a pyroglutamine residue at the N-terminal, (ii) containing an amino acid sequence covering amino acids 8 (Ala) to 17 (Ile) from the N-terminal of the amino acid sequence shown in SEQ ID NO: 18 and (iii) being composed of 14 to 17 amino acid residues, and (2) polypeptides (i) having a glutamine residue or a pyroglutamine residue at the N-terminal, (ii) containing an amino acid sequence covering amino acids 8 (Ala) to 17 (Ile) from the N-terminal of the amino acid sequence shown in SEQ ID NO: 18, (iii) being composed of 14 to 17 amino acid residues, and (iv) having additional 3 to 10 amino acid residues at the N-terminal.

In the specification, polypeptides are shown in conventional notation, that is, the N-terminal (amino terminal) is placed at the left side and the C-terminal (carboxyl terminal) is placed at the right side. Polypeptides containing, for example, (1) the amino acid sequence shown in SEQ ID NO: 1, (2) the amino acid sequence shown in SEQ ID NO: 2, (3) the amino acid sequence shown in SEQ ID NO: 9, (4) the amino acid sequence shown in SEQ ID NO: 10, (5) the amino acid sequence shown in SEQ ID NO: 18, (6) the amino acid sequence shown in SEQ ID NO: 19, (7) the amino acid sequence shown in SEQ ID NO: 24, (8) the amino acid sequence shown in SEQ ID NO: 26, (9) the amino acid sequence shown SEQ ID NO: 27, (10) the amino acid sequence shown in SEQ ID NO: 28 or (11) the amino acid sequence shown in SEQ ID NO: 29 may have any one of a carboxyl group (-COOH), a carboxylate group ($-COO^-$), an amide group ($-CONH_2$) and an ester group (-COOR) at the C-terminal. Examples of R in the ester group include a $C_{1-6}$ allyl group such as methyl, ethyl, n-propyl, isopropyl or n-butyl, a $C_{3-8}$ cycloalkyl group such as cyclopentyl or cyclohexyl, a $C_{6-12}$ aryl group such as phenyl or $\alpha$-naphthyl, or a $C_{7-14}$ aralkyl group such as a phenyl-$C_{1-2}$ alkyl including benzyl, phenethyl or benzhydryl or an $\alpha$-naphthyl-$C_{1-2}$ alkyl including $\alpha$-naphthylmethyl, as well as a pivaloyloxymethyl group commonly used as an ester for oral administration.

Salts of the polypeptide of the present invention may be salts with physiologically acceptable bases (e.g., alkali metals) or acids (e.g., organic or inorganic acids); physiologically acceptable acid addition salts are particularly preferred for use. Examples of such salts include salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The polypeptide of the present invention may be prepared as described in, e.g., WO 00/32627, WO 00/31265, WO 99/35266 or Japanese Patent Application No. 2000-211996.

More specifically, the polypeptide of the present invention may be prepared by purification techniques for polypeptides from tissues or cells of homeotherms or according to the synthesis technique for polypeptides described below. Alternatively, it may also be prepared by culturing transformants carrying the DNA encoding the polypeptide, as described below.

When the polypeptide is prepared from tissues or cells of homeotherms, tissues or cells of homeotherms are homogenized and then extracted with an acid, an organic solvent and the like. The resulting extract is subjected to salt precipitation and dialysis in combination with chromatography such as gel filtration, reverse phase chromatography, ion exchange chromatography or affinity chromatography to give a purified and isolated polypeptide.

As described above, the polypeptide of the present invention may be prepared according to well-known synthesis techniques for polypeptides or by cleaving a polypeptide containing the polypeptide of the present invention with an appropriate peptidase. Synthesis techniques for polypeptides may be based on either solid phase or liquid phase. Namely, partial peptides or amino acids capable of constituting the polypeptide of the present invention are condensed with the remainder and a protecting group(s), if any, is removed from the product to give a peptide of interest. Examples of known techniques for condensation and removal of protecting groups include those which are found in (I) to (V) listed below:

(I) M. Bodanszky and M.A. Ondetti, Peptide Synthesis, Interscience Publishers, New York (1966);
(II) Schroeder and Luebke, The Peptide, Academic Press, New York (1965);
(III) Nobuo Izumiya et al., Principle and Experiments of Peptide Synthesis, Maruzen Co., Ltd. (1975);
(IV) Haruaki Yajima and Shunpei Sakakibara, Biochemical Experiment Course vol. 1, Protein Chemistry IV, 205 (1977); and
(V) Haruaki Yajima ed., Continued Pharmaceutical Development, vol. 14, Peptide Synthesis, Hirokawa Publishing Co.

After the reaction, the polypeptide of the present invention may be purified and isolated using standard purification procedures in combination, such as solvent extraction, distillation, column chromatography, liquid chromatography and recrystallization. If the polypeptide thus prepared is in free form, it may be converted into an appropriate salt in a known manner. If the polypeptide thus prepared is in salt form, on the other hand, it may be converted into a free form in a known manner.

An amide form of the polypeptide may be obtained using any commercially available peptide synthesis resin suitable for amidation. Examples of such a resin include chloromethyl resin, hydroxymethyl resin, benzhydrylamine

resin, aminomethyl resin, 4-benzyloxybenzyl alcohol resin, 4-methylbenzhydrylamine resin, PAM resin, 4-hydroxymethylmethylphenylacetamidemethyl resin, polyacrylamide resin, 4-(2',4'-dimethoxyphenyl-hydroxymethyl)phenoxy resin and 4-(2',4'-dimethoxyphenyl-Fmoc aminoethyl)phenoxy resin. Using such a resin, amino acids having appropriately protected $\alpha$-amino and side chain functional groups are condensed on the resin, as sequenced in the peptide sequence of interest, in accordance with various well-known condensation techniques. At the end of the reaction, the resulting peptide is cleaved form the resin simultaneously with removal of various protecting groups, followed by intramolecular disulfide bridging in a highly-diluted solution, if necessary, to obtain a polypeptide of interest.

To condense the protected amino acids mentioned above, various activating reagents available for use in peptide synthesis, particularly carbodiimides, may be used. Examples of carbodiimides include DCC, N,N'-diisopropylcarbodiimide and N-ethyl-N'-(3-dimethylaminopropyl)carbodiimide. When activated by these reagents, protected amino acids may be directly added to the resin in the presence of a racemization-inhibiting additive (e.g., HOBt, HOOBt) or may be added to the resin after activation of amino acids pre-protected as the corresponding acid anhydride or HOBt ester or HOOBt ester. A solvent used for the activation of protected amino acids and/or the condensation with the resin may be selected as appropriate from solvents known to be available for peptide condensation. Examples include acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethyl sulfoxide, tertiary amines such as pyridine, ethers such as dioxane and tetrahydrofuran, nitriles such as acetonitrile and propionitrile, esters such as methyl acetate and ethyl acetate, and appropriate combinations thereof. The reaction temperature may be selected as appropriate from the range known to be available for peptide linking, usually the range of about -20°C to 50°C. Activated amino acid derivatives are usually used in 1.5- to 4-fold excess. If condensation is insufficient, as tested by ninhydrin reaction, the condensation reaction may further be repeated without removal of protecting groups to achieve sufficient condensation. If condensation cannot succeed by repeating the reaction, acetic anhydride or acetylimidazole may be used to acetylate unreacted amino acids, thus eliminating influences on the subsequent reactions.

Examples of protecting groups for amino groups in starting amino acids include Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl and Fmoc. Examples of protecting groups for carboxyl groups include the $C_{1-6}$ alkyl, $C_{3-8}$ cycloalkyl and $C_{7-14}$ aralkyl groups mentioned above for R, as well as 2-adamantyl, 4-nitrobenzyl, 4-methoxybenzyl, 4-chlorobenzyl, phenacyl and benzyloxycarbonyl hydrazide, tert-butoxycarbonyl hydrazide and trityl hydrazide.

Hydroxyl groups in serine and threonine may be protected, for example, by esterification or etherification. Examples of groups suitable for this esterification include lower alkanoyl groups such as acetyl, aroyl groups such as benzoyl, and carbon-derived groups such as benzyloxycarbonyl and ethoxycarbonyl. Likewise, examples of groups suitable for etherification include benzyl, tetrahydropyranyl, and tert-butyl.

Examples of a protecting group for a phenolic hydroxyl group in tyrosine include Bzl, $Cl_2$-Bzl, 2-nitrobenzyl, Br-Z and tert-butyl.

Examples of a protecting group for an imidazole ring in histidine include Tos, 4-methoxy-2,3,6-trimethylbenzenesulfonyl, DNP, benzyloxymethyl, Bum, Boc, Trt and Fmoc.

Examples of activated forms of carboxyl groups in starting materials include the corresponding acid anhydrides, azides and active esters [esters with alcohols (e.g., pentachlorophenol, 2,4,5-trichlorophenol, 2,4-dinitrophenol, cyanomethyl alcohol, paranitrophenol, HONB, N-hydroxysuccinimide, N-hydroxyphthalimide, HOBt)]. Examples of activated forms of amino groups in starting materials include the corresponding phosphate amides.

Removal of protecting groups may be accomplished by some techniques including catalytic reduction in a hydrogen stream in the presence of a catalyst such as Pd black or Pd-carbon, treatment with an acid such as anhydrous hydrogen fluoride, methanesulfonic acid, trifluoromethanesulfonic acid, trifluoroacetic acid or any mixture thereof, treatment with a base such as diisopropylethylamine, triethylamine, piperidine or piperazine, and reduction with sodium in liquid ammonia. Removal by the above acid treatment is usually carried out at a temperature of -20°C to 40°C and the acid treatment system is advantageously supplemented with a cation-capturing agent such as anisole, phenol, thioanisole, metacresol, paracresol, dimethyl sulfide, 1,4-butanedithiol or 1,2-ethanedithiol. Likewise, a 2,4-dinitrophenyl group used as a protecting group for an imidazole ring in histidine is removed by thiophenol treatment, while a formyl group used as a protecting group for an indole ring in tryptophan is removed by the above-mentioned acid treatment in the presence of 1,2-ethanedithiol, 1,4-butanedithiol, etc., as well as by alkaline treatment with dilute sodium hydroxide, dilute ammonia, etc.

Protection of functional groups in starting materials, which should not be involved in the reaction, protecting groups for these functional groups, removal of these protecting groups, and activation of functional groups involved in the reaction may be selected as appropriate from known groups or known means.

Another technique for obtaining an amide form of the polypeptide involves first amidating an $\alpha$-carboxyl group in the carboxyl-terminal amino acid, extending a peptide chain toward the amino-terminal to give a desired chain length,

preparing a peptide lacking only a protecting group for the N-terminal α-amino group of said peptide chain and a peptide (or an amino acid) lacking only a protecting group for the C-terminal carboxyl group, and then condensing these two peptides in a mixed solvent as mentioned above. Details about condensation are as described above. After purification of the protected peptide obtained by condensation, all of the remaining protecting groups are removed as described above to give a crude polypeptide of interest. This crude polypeptide may be purified using various known purification means, followed by lyophillization of main fractions to give a desired amide form of the polypeptide.

To obtain an ester form of the polypeptide, an α-carboxyl group in the carboxyl-terminal amino acid may be condensed with a desired alcohol to form an amino acid ester, which is then treated as in the case of the amide form to give a desired ester form of the polypeptide.

The polypeptide of the present invention may be any polypeptide as long as it contains the same or substantially the same amino acid sequence as shown in SEQ ID NO: 1 and has the same activities as the polypeptide, such as anti-attention-deficit-disorder and/or anti-narcolepsy activities. Examples of such a polypeptide may include a peptide having the amino acid sequence shown in SEQ ID NO: 2, 9, 10, 18, 19, 24, 26, 27, 28 or 29.

The DNA encoding the polypeptide of the present invention may be any DNA as long as it contains a DNA encoding a polypeptide containing the same or substantially the same amino acid sequence as shown in SEQ ID NO: 1. Also, it may be any one of a genomic DNA, a genomic DNA library, a cDNA derived from the above-mentioned tissues or cells, a cDNA library derived from the above-mentioned tissues or cells and a synthetic DNA. Any vector may be used for these libraries, including bacteriophages, plasmids, cosmids, phagemids, etc. Alternatively, the DNA may be directly amplified in the Reverse Transcriptase Polymerase Chain Reaction (hereinafter simply referred to as RT-PCR) using an RNA fraction prepared from the above-mentioned tissues or cells.

As stated above, examples of a polypeptide containing the same or substantially the same amino acid sequence as shown in SEQ ID NO: 1 include the amino acid sequence shown in SEQ ID NO: 2, 9, 10, 18, 19, 24, 26, 27, 28 or 29. Examples of a DNA containing a DNA encoding a polypeptide containing the amino acid sequence shown in SEQ ID NO: 2 include DNAs containing the nucleotide sequence shown in SEQ ID NO: 12, examples of a DNA containing a DNA encoding a polypeptide containing the amino acid sequence shown in SEQ ID NO: 9 include DNAs containing the nucleotide sequence shown in SEQ ID NO: 13, examples of a DNA containing a DNA encoding a polypeptide containing the amino acid sequence shown in SEQ ID NO: 10 include DNAs containing the nucleotide sequence shown in SEQ ID NO: 34, examples of a DNA containing a DNA encoding a polypeptide containing the amino acid sequence shown in SEQ ID NO: 18 include DNAs containing the nucleotide sequence shown in SEQ ID NO: 20, examples of a DNA containing a DNA encoding a polypeptide containing the amino acid sequence shown in SEQ ID NO: 19 include DNAs containing the nucleotide sequence shown in SEQ ID NO: 21, examples of a DNA containing a DNA encoding a polypeptide containing the amino acid sequence shown in SEQ ID NO: 24 include DNAs containing the nucleotide sequence shown in SEQ ID NO: 25, examples of a DNA containing a DNA encoding a polypeptide containing the amino acid sequence shown in SEQ ID NO: 26 include DNAs containing the nucleotide sequence shown in SEQ ID NO: 30, examples of a DNA containing a DNA encoding a polypeptide containing the amino acid sequence shown in SEQ ID NO: 27 include DNAs containing the nucleotide sequence shown in SEQ ID NO: 31, examples of a DNA containing a DNA encoding a polypeptide containing the amino acid sequence shown in SEQ ID NO: 28 include DNAs containing the nucleotide sequence shown in SEQ ID NO: 32, and examples of a DNA containing a DNA encoding a polypeptide containing the amino acid sequence shown in SEQ ID NO: 29 include DNAs containing the nucleotide sequence shown in SEQ ID NO: 33.

Examples of a DNA containing a DNA encoding a polypeptide containing substantially the same amino acid sequence as shown in SEQ ID NO: 1 include DNAs containing a nucleotide sequence sharing a homology of at least about 80%, preferably at least about 90%, more preferably at least about 95%, even more preferably at least about 98% with the nucleotide sequence shown in SEQ ID NO: 12, 13, 34, 20, 21, 25, 30, 31, 32 or 33.

Other examples of a DNA containing a DNA encoding a polypeptide containing substantially the same amino acid sequence as shown in SEQ ID NO: 1 include DNAs containing (i) a nucleotide sequence with deletion of one or more nucleotides (preferably around 1 to 30, more preferably around 1 to 10, even more preferably 1 or 2 nucleotides) in the nucleotide sequence shown in SEQ ID NO: 12, 13, 34, 20, 21, 25, 30, 31, 32 or 33; (ii) a nucleotide sequence with addition of one or more nucleotides (preferably around 1 to 30, more preferably around 1 to 10, even more preferably 1 or 2 nucleotides) in the nucleotide sequence shown in SEQ ID NO: 12, 13, 34, 20, 21, 25, 30, 31, 32 or 33; (iii) a nucleotide sequence with insertion of one or more nucleotides (preferably around 1 to 30, more preferably around 1 to 10, even more preferably 1 or 2 nucleotides) in the nucleotide sequence shown in SEQ ID NO: 12, 13, 34, 20, 21, 25, 30, 31, 32 or 33; (iv) a nucleotide sequence with substitution of one or more nucleotides (preferably around 1 to 30, more preferably around 1 to 10, even more preferably 1 or 2 nucleotides) in the nucleotide sequence shown in SEQ ID NO: 12, 13, 34, 20, 21, 25, 30, 31, 32 or 33; or (v) a nucleotide sequence with any combination of the deletion, addition, insertion and substitution mentioned above. More specific examples include (1) mammalian-derived DNAs that hybridize under stringent conditions with a DNA containing a DNA capable of binding to a DNA encoding a polypeptide containing the same or substantially the same amino acid sequence as shown in SEQ ID NO: 1 and (2) DNAs that

form no hybrid neither with a DNA containing a DNA capable of binding to a DNA encoding a polypeptide containing the same or substantially the same amino acid sequence as shown in SEQ ID NO: 1 nor with the DNAs defined in (1) due to degeneracy of the genetic code, but that encode a polypeptide having the same amino acid sequence. Hybridization may be carried out according to well-known procedures or equivalents thereof. The stringent conditions mentioned above are set at 42°C in 50% formamide, 4 × SSPE (1 × SSPE = 150 mM NaCl, 10 mM $NaH_2PO_4 \cdot H_2O$, 1 mM EDTA pH7.4), 5 × Denhart's solution and 0.1% SDS, by way of example.

Examples of DNAs that hybridize with a DNA containing a DNA encoding a polypeptide containing the same or substantially the same amino acid sequence as shown in SEQ ID NO: 1 include DNAs containing a nucleotide sequence sharing a homology of at least about 70%, preferably at least about 80%, more preferably at least about 90%, most preferably at least about 95% with the nucleotide sequence shown in SEQ ID NO: 12, 13, 34, 20, 21, 25, 30, 31, 32 or 33.

The DNA encoding the polypeptide of the present invention may also be prepared by the genetic engineering procedures described below.

The DNA encoding the entire polypeptide of the present invention may be cloned, for example, by amplifying a DNA of interest from the above-mentioned DNA libraries in well-known PCR using synthetic primers having partial nucleotide sequences from the nucleotide sequence of the DNA encoding the polypeptide of the present invention or by selecting a DNA of interest with the aid of hybridization between the DNA integrated into an appropriate vector and a probe labeled with a DNA fragment or synthetic DNA having part or all of the region encoding the polypeptide of the present invention. Hybridization may be carried out, for example, as described in Molecular Cloning (2nd ed., J. Sambrook et al., Cold Spring Harbor Lab. Press, 1989). In the case of using commercially available libraries, hybridization may be carried out according to the attached instruction manual.

The cloned DNA encoding the polypeptide of the present invention may be used as such or may further be subjected to digestion with a restriction enzyme(s) or addition of a linker, depending on the intended purposes. The DNA may have ATG as an initiation codon at the 5'-terminal side and TAA, TGA or TAG as a stop codon at the 3'-terminal side. These initiation and stop codons may be attached using an appropriate synthetic DNA adaptor.

An expression vector for the polypeptide of the present invention may be prepared, for example, by (a) cleaving a DNA fragment of interest from the DNA encoding the polypeptide of the present invention and (b) allowing the DNA fragment to be ligated downstream of a promoter in an appropriate expression vector.

Examples of a vector available for use include *E. coli*-derived plasmids (e.g., pBR322, pBR325, pUC12, pUC13), *Bacillus subtilis*-derived plasmids (e.g., pUB110, pTP5, pC194), yeast-derived plasmids (e.g., pSH19, pSH15), bacteriophages such as phage λ, and animal viruses such as retrovirus, vaccinia virus and baculovirus.

Any promoter may be used in the present invention as long as it is suitable for a host used in gene expression.

When transformation is performed in animal cell hosts, examples of a promoter available for use include SV40-derived promoter, retrovirus promoter, metallothionein promoter, heat shock promoter, cytomegalovirus promoter and SRα promoter. When transformation is performed in other hosts, preferred promoters include trp promoter, T7 promoter, lac promoter, recA promoter, λPL promoter and lpp promoter for microorganism hosts belonging to Escherichia, SPO1 promoter, SPO2 promoter and penP promoter for microorganism hosts belonging to Bacillus, and PHO5 promoter, PGK promoter, GAP promoter, ADH1 promoter and GAL promoter for yeast cell hosts. For insect cell hosts, polyhedrin promoter and P10 promoter are preferred.

An expression vector used here may further comprise, in addition to the foregoing, an enhancer, a splicing signal, a poly(A) signal, a selective marker, an SV40 replication origin (hereinafter also simply referred to as SV40ori) and other elements, as needed. Examples of a selective marker include the dihydrofolate reductase (hereinafter also simply referred to as dhfr) gene [methotrexate (MTX) resistance], the ampicillin resistance gene (hereinafter also simply referred to as Ampr) and the neomycin resistance gene (hereinafter also simply referred to as Neo; G418 resistance). In particular, when the DHFR gene is used as a selective marker for expression in CHO ($dhfr^-$) cells, selection may also be accomplished in a thymidine-free medium.

Also, if necessary, a signal sequence suitable for a host may be attached to the N-terminal side of the polypeptide or a partial peptide thereof. Examples of a signal sequence available for use include phoA signal sequence and OmpA signal sequence for microorganism hosts belonging to Escherichia, α-amylase signal sequence and subtilisin signal sequence for microorganism hosts belonging to Bacillus, mating factor α (MFα) signal sequence and invertase signal sequence for yeast cell hosts, and insulin signal sequence, α-interferon signal sequence and antibody molecule signal sequence for animal cell hosts.

The vector thus constructed to carry the DNA encoding the polypeptide may be used to prepare transformants.

Examples of a host available for use include microorganisms belonging to Escherichia and Bacillus, yeast cells, insects or insect cells, and animal cells.

Examples of microorganisms belonging to Escherichia include *Escherichia coli* K12-DH1 [Proc. Natl, Acad. Sci. USA, vol. 60, 160 (1968)], JM103 [Nucleic Acids Research, vol. 9, 309 (1981)], JA221 [Journal of Molecular Biology, vol. 120, 517 (1978)], HB101 [Journal of Molecular Biology, vol. 41, 459 (1969)] and C600 [Genetics, vol. 39, 440

(1954)].

Examples of microorganisms belonging to Bacillus include *Bacillus subtilis* MI114 [Gene, vol. 24, 255 (1983)] and 207-21 [Journal of Biochemistry, vol. 95, 87 (1984)].

Examples of yeast cells include *Saccharomyces cerevisiae* AH22, AH22R$^-$, NA87-11A, DKD-5D and 20B-12.

Examples of insects include silk worm larvae [Maeda et al., Nature, vol. 315 592 (1985)].

Examples of insect cells include armyworm-derived established cells (*Spodoptera frugiperda* cells; Sf cells), *Trichoplusia ni* mesogaster-derived MG1 cells, *Trichoplusia ni* egg-derived High Five™ cells, *Mamestra brassicae*-derived cells or *Estigmena acrea*-derived cells for AcNPV virus and silk worm-derived established cells (*Bombyx mori* N; BmN cells) for BmNPV virus. Examples of such Sf cells include Sf9 cells (ATCC CRL1711) and Sf21 cells [Vaughn, J.L. et al., in Vitro, vol. 13, 213-217 (1977)].

Examples of animal cells include monkey COS-7 cells, Vero cells, Chinese hamster cells CHO, DHFR gene-deficient Chinese hamster cells CHO (dhfr$^-$ CHO cells), mouse L cells, mouse 3T3 cells, mouse myeloma cells, human HEK293 cells, human FL cells, 293 cells, C127 cells, BALB3T3 cells and Sp-2/O cells.

To transform microorganisms belonging to Escherichia, transformation may be performed as described in, e. g., Proc. Natl. Acad. Sci. USA, vol. 69, 2110 (1972) or Gene, vol. 17, 107 (1982).

To transform microorganisms belonging to Bacillus, transformation may be performed as described in, e.g., Molecular & General Genetics, vol. 168, 111 (1979).

To transform yeast cells, transformation may be performed as described in, e.g., Proc. Natl. Acad. Sci. USA, vol. 75, 1929 (1978).

To transform insects or insect cells, transformation may be performed as described in, e.g., Bio/Technology, vol. 6, 47-55 (1988).

To transform animal cells, transformation may be performed as described in, e.g., Virology, vol. 52, 456 (1973).

Introduction of an expression vector into cells may be accomplished, for example, by the lipofection method [Felgner, P.L. et al., Proceedings of the National Academy of Sciences of the United States of America, vol. 84, 7413 (1987)], the calcium phosphate method [Graham, F.L. and van der Eb, A.J., Virology, vol. 52, 456-467 (1973)] or electroporation [Nuemann, E. et al., EMBO J., vol. 1, 841-845 (1982)].

In this way, transformants transformed with an expression vector carrying the DNA encoding the polypeptide of the present invention can be obtained.

Meanwhile, stable expression of the polypeptide of the present invention in animal cells may be accomplished by clonal selection to select cells in which an expression vector introduced into the cells is integrated into the cellular chromosomes. More specifically, the above-mentioned selective markers are used as indicators for transfectant selection. Further, clonal selection may be repeated on the animal cells obtained using such selective markers to give a stable animal cell line capable of highly expressing the polypeptide of the present invention. Also, in the case of using the dhfr gene as a selective marker, animal cells are cultured at gradually increasing MTX concentrations to select resistant cells, so that the DNA encoding the polypeptide of the present invention or a partial peptide thereof is amplified in the cells together with the dhfr gene to give an animal cell line capable of higher-level expression.

The polypeptide of the present invention may be obtained by culturing the above transformants under conditions allowing expression of the DNA encoding the polypeptide of the present invention to produce and accumulate the polypeptide of the present invention.

In the case of culturing transformants obtained from microorganism hosts belonging to Escherichia or Bacillus, a liquid medium is suitable for culturing the transformants and it is supplemented with a carbon source, a nitrogen source, minerals and other ingredients, which are necessary for growth of the transformants. Examples of a carbon source include glucose, dextrin, soluble starch and sucrose. Examples of a nitrogen source include inorganic or organic substances such as ammonium salts, nitrate salts, corn steep liquor, peptone, casein, meat extracts, soy bean cake and potato extracts. Examples of minerals include calcium chloride, sodium dihydrogen phosphate and magnesium chloride. The medium may also be supplemented with yeast extracts, vitamins, growth-stimulating factors, etc. The medium desirably has a pH of about 5 to 8.

A preferred medium for culturing microorganisms belonging to Escherichia is M9 medium containing glucose and casamino acid [Miller, Journal of Experiments in Molecular Genetics, 431-433, Cold Spring Harbor Laboratory, New York (1972)]. When needed, this medium may be supplemented with an agent such as 3β-indolylacrylic acid for the purpose of efficient operation of a promoter.

For microorganism hosts belonging to Escherichia, the culturing is usually carried out at about 15°C to 43°C for about 3 to 24 hours and, if necessary, under aerobic and/or stirring conditions.

For microorganism hosts belonging to Bacillus, the culturing is usually carried out at about 30°C to 40°C for about 6 to 24 hours and, if necessary, under aerobic and/or stirring conditions.

In the case of culturing transformants obtained from yeast cell hosts, examples of a medium available for use include Burkholder minimal medium [Bostian, K.L. et al., Proc. Natl. Acad. Sci. USA, vol. 77, 4505 (1980)] and SD medium containing 0.5% casamino acid [Bitter, G.A. et al., Proc. Natl. Acad. Sci. USA, vol. 81, 5330 (1984)]. The

medium is preferably adjusted to about pH5 to pH8. The culturing is usually carried out at about 20°C to 35°C for about 24 to 72 hours and, if necessary, under aerobic and/or stirring conditions.

In the case of culturing transformants obtained from insect cell hosts, examples of a medium available for use include Grace's Insect Medium [Grace, T.C.C., Nature, 195, 788 (1962)] supplemented with additives such as 10% inactivated bovine serum. The medium is preferably adjusted to about pH6.2 to pH6.4. The culturing is usually carried out at about 27°C for about 3 to 5 days and, if necessary, under aerobic and/or stirring conditions.

In the case of culturing transformants obtained from animal cell hosts, examples of a medium available for use include MEM medium containing about 5-20% fetal bovine serum [Science, vol. 122, 501 (1952)], DMEM medium [Virology, vol. 8, 396 (1959)], RPMI 1640 medium [The Journal of the American Medical Association, vol. 199, 519 (1967)] and 199 medium [Proceeding of the Society for the Biological Medicine, vol. 73, 1 (1950)]. pH is preferably about 6 to 8. The culturing is usually carried out at about 30°C to 40°C for about 15 to 60 hours and, if necessary, under aerobic and/or stirring conditions.

In particular, DMEM medium containing dialyzed fetal bovine serum and substantially free of thymidine is preferably used for a combination of CHO ($dhfr^-$) cells and the dhfr gene as a selective marker.

The polypeptide of the present invention may be isolated and purified from the above culture as follows.

To extract the polypeptide of the present invention from the cultured microorganisms or cells, any procedure may be used as appropriate. For example, the microorganisms or cells are collected in a known manner after culturing, suspended in an appropriate buffer, subjected to ultrasonic treatment, lysozyme treatment and/or freezing-thawing to disrupt the microorganisms or cells, followed by centrifugation and/or filtration to give a crude extract of the polypeptide. The buffer may be supplemented with a protein-denaturing agent such as urea or guanidine hydrochloride and/or a surfactant such as Triton X-100 (trade mark; hereinafter also simply referred to as TM).

If the polypeptide is secreted into the culture solution, after the completion of culturing, the microorganisms or cells and the supernatant are separated from each other in a well-known manner to collect the supernatant.

The polypeptide of the present invention contained in the culture supernatant or extract thus prepared may be purified by well-known isolation and purification techniques in combination as appropriate. Examples of these known isolation and purification techniques include techniques based on solubility (e.g., salt precipitation, solvent precipitation), techniques mainly based on differences in molecular weights (e.g., dialysis, ultrafiltration, gel filtration, SDS-polyacrylamide gel electrophoresis), techniques based on differences in electric charges (e.g., ion exchange chromatography), techniques based on specific affinity (e.g., affinity chromatography), techniques based on differences in hydrophobicity (e.g., reverse phase high performance liquid chromatography) and techniques based on differences in isoelectric points (e.g., electro-focusing electrophoresis, chromatofocusing).

If the polypeptide of the present invention thus obtained is in free form, it may be converted into a salt according to well-known procedures or equivalents thereof. If the polypeptide thus obtained is in salt form, on the other hand, it may be converted into a free form or other salt according to well-known procedures or equivalents thereof.

Meanwhile, the polypeptide of the present invention recombinantly produced may be treated with an appropriate protein-modifying enzyme before or after purification such that the polypeptide is optionally modified or partially removed. Examples of a protein-modifying enzyme include trypsin, chymotrypsin, arginyl endopeptidase, protein kinase and glycosidase.

The presence of the thus produced polypeptide of the present invention can be detected by an enzyme immunoassay using a specific antibody or other assays.

The DNA encoding the polypeptide of the present invention or the polypeptide of the present invention can be used for development of medicaments including an anti-attention-deficit-disorder or anti-narcolepsy agent or an anti-anxiety, anti-depression, anti-insomnia, anti-schizophrenia or anti-fear agent, development of a receptor-binding assay system using a recombinant receptor protein expression system, screening of candidate compounds for medicaments, gene therapy, etc.

In particular, the receptor-binding assay system using the recombinant GPR14 (SENR) expression system illustrated later achieves screening of GPR14 (SENR) agonists or antagonists specific to homeotherms including human. Such agonists or antagonists can be used as prophylactic and/or therapeutic agents for various diseases, by way of example.

Further, the polypeptide of the present invention or the DNA encoding the same can be recognized as a ligand by GPR14 (SENR) expressed in the central nerve system, circulatory system, heart, kidneys, urinary system, sensory organs or the like; it is therefore useful as a safer and less toxic medicament. The polypeptide of the present invention or the DNA encoding the same can be used as a therapeutic and/or prophylactic agent for attention deficit disorder or narcolepsy, by way of example.

In a case where the polypeptide of the present invention or the DNA encoding the same is used as the medicament mentioned above, it may be formulated in a routine manner. For example, it may be used for oral administration in dosage forms of optionally sugar-coated or enteric-coated tablets, capsules, elixirs, microcapsules and the like or may be used for parenteral administration in dosage forms of injections such as sterile solutions or suspensions in

water or other pharmaceutically acceptable fluids. For example, these formulations may be prepared by incorporating the compound or a salt thereof in unit dosage forms required in generally-accepted pharmaceutical practice in combination with physiologically acceptable carriers, flavors, excipients, vehicles, antiseptics, stabilizers, binders and the like. The amount of an active ingredient in these formulations is intended to give an appropriate dose within the indicated range.

When the DNA of the present invention is used, the DNA may be used alone or inserted into an appropriate vector such as a retrovirus vector, an adenovirus vector or an adenovirus-associated virus vector, followed by formulation in a routine manner.

Examples of additives, which can be incorporated into tablets, capsules and the like, include binders such as gelatin, corn starch, gum tragacanth or gum arabic; excipients such as crystalline cellulose; swelling agents such as corn starch, gelatin or alginic acid; lubricants such as magnesium stearate; edulcorants such as sucrose, lactose or saccharin; and flavors such as peppermint, *Gaultheria adenothrix* oil or cherry. In a case where the unit dosage form is a capsule, it may further contain liquid carriers such as fats and oils, in addition to the above types of materials. Sterile compositions for injection may be formulated according to general pharmaceutical practice, for example, by dissolving or suspending an active substance, a naturally-occurring vegetable oil(s) (e.g., sesame oil, coconut oil) and other ingredients, if any, into a vehicle such as water for injection.

Examples of aqueous fluids for injection include physiological saline and isotonic solutions containing glucose and/or other auxiliaries (e.g., D-sorbitol, D-mannitol, sodium chloride), which may be used in combination with appropriate solubilizers such as alcohols (e.g., ethanol), polyalcohols (e.g., propylene glycol, polyethylene glycol) and nonionic surfactants (e.g., Polysorbate 80™, HCO-50). Examples of oil fluids include sesame oil and soy bean oil, which may be used in combination with solubilizers such as benzyl benzoate and benzyl alcohol.

In addition, these fluids may be blended with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), antioxidants, etc. The prepared solutions for injection are usually filled into appropriate ampules.

Since the formulations thus prepared are safer and less toxic, they can be administered to, for example, mammals (e.g., human, mouse, rat, guinea pig, rabbit, sheep, pig, cattle, cat, dog, monkey).

The DNA encoding the polypeptide of the present invention or the polypeptide of the present invention can be recognized as a ligand by GPR14 (SENR) expressed in the central nerve system, circulatory system, heart, kidneys, urinary system, sensory organs or the like; it is therefore useful as a safer and less toxic medicament. The DNA encoding the polypeptide of the present invention or the polypeptide of the present invention can be used as a therapeutic and/or prophylactic agent for attention deficit disorder or narcolepsy, by way of example.

The dose of the polypeptide of the present invention or the DNA encoding the same will vary depending on symptoms etc. For oral administration, it is usually administered to an adult patient with attention deficit disorder (body weight: 60 kg) in an amount of about 0.1 to 100 mg/day, preferably about 1.0 to 50 mg/day, more preferably about 1.0 to 20 mg/day. For parenteral administration, the single dose will vary depending on subjects to be administered, target organs, symptoms, the intended route of administration, etc. In a dosage form of injection, for example, it is advantageously administered to an adult patient with attention deficit disorder (body weight: 60 kg) in an amount of about 0.01 to 30 mg/day, preferably about 0.1 to 20 mg/day, more preferably about 0.1 to 10 mg/day, by intravenous injection. Other animals may be administered with the polypeptide or DNA in an amount calculated per 60 kg.

A precursor protein for the polypeptide of the present invention or a salt thereof, as well as its preparation and uses will be described below in more detail.

Examples of a precursor protein for the polypeptide of the present invention or a salt thereof (hereinafter also referred to as the precursor protein of the present invention) include proteins or salts thereof, which have one or more, preferably around 1 to 200, more preferably around 1 to 120, even more preferably around 50 to 120 amino acids attached at the N-terminal or/and C-terminal of the polypeptide of the present invention.

Specific examples of the precursor protein of the present invention available for use include a protein having the same or substantially the same amino acid sequence as shown in SEQ ID NO: 7, 8, 14, 17 or 23.

Also, the precursor protein of the present invention may be any protein derived from every tissue (e.g., hypophysis, pancreas, brain, kidney, liver, genital gland, thyroid gland, gallbladder, bone marrow, suprarenal gland, skin, muscle, lung, digestive tract, blood vessel, heart) or every cell of homeotherms (e.g., human, guinea pig, rat, mouse, pig, sheep, cattle, monkey), as long as it contains the same or substantially the same amino acid sequence as shown in SEQ ID NO: 7, 8, 14, 17 or 23. Activities of substantially the same type include receptor-binding activity, signal transduction activity and the like. The term "substantially the same type" means that activities including receptor-binding activity are qualitatively identical among proteins. Thus, quantitative factors, such as strength of receptor-binding activity and molecular weights of proteins, may vary from protein to protein.

Substantially the same amino acid sequence as shown in SEQ ID NO: 7, 8, 14, 17 or 23 specifically refers to an amino acid sequence sharing a homology of at least about 50%, preferably at least about 60%, more preferably

at least about 70%, even more preferably at least about 80%, particularly preferably at least about 90%, most preferably at least about 95% with the amino acid sequence shown in SEQ ID NO: 7, 8, 14, 17 or 23.

Other examples of the precursor protein of the present invention include proteins containing (i) an amino acid sequence with deletion of one or more amino acids (preferably around 1 to 30, more preferably around 1 to 10, even more preferably 1 or 2 amino acids) in the amino acid sequence shown in SEQ ID NO: 7, 8, 14, 17 or 23; (ii) an amino acid sequence with addition of one or more amino acids (preferably around 1 to 30, more preferably around 1 to 10, even more preferably 1 or 2 amino acids) in the amino acid sequence shown in SEQ ID NO: 7, 8, 14, 17 or 23; (iii) an amino acid sequence with insertion of one or more amino acids (preferably around 1 to 30, more preferably around 1 to 10, even more preferably 1 or 2 amino acids) in the amino acid sequence shown in SEQ ID NO: 7, 8, 14, 17 or 23; (iv) an amino acid sequence with substitution of one or more amino acids (preferably around 1 to 30, more preferably around 1 to 10, even more preferably 1 or 2 amino acids) in the amino acid sequence shown in SEQ ID NO: 7, 8, 14, 17 or 23; or (v) an amino acid sequence with any combination of the deletion, addition, insertion and substitution mentioned above.

Specific examples of a precursor protein for the polypeptide of the present invention containing the amino acid sequence shown in SEQ ID NO: 2 include proteins containing the amino acid sequence shown in SEQ ID NO: 7 or 8, while specific examples of a precursor protein for the polypeptide of the present invention containing the amino acid sequence shown in SEQ ID NO: 9 include proteins containing the amino acid sequence shown in SEQ ID NO: 14.

Specific examples of a precursor protein for the polypeptide of the present invention containing the amino acid sequence shown in SEQ ID NO: 10 include precursor proteins disclosed in WO 99/35266.

Specific examples of a precursor protein for the polypeptide of the present invention containing the amino acid sequence shown in SEQ ID NO: 18, 19, 26 or 27 include proteins containing the amino acid sequence shown in SEQ ID NO: 17.

Specific examples of a precursor protein for the polypeptide of the present invention containing the amino acid sequence shown in SEQ ID NO: 24, 28 or 29 include proteins containing the amino acid sequence shown in SEQ ID NO: 23.

In the specification, precursor proteins are shown in conventional notation, that is, the N-terminal (amino terminal) is placed at the left side and the C-terminal (carboxyl terminal) is placed at the right side. The precursor protein of the present invention containing, for example, the amino acid sequence shown in SEQ ID NO: 7, 8, 14, 17 or 23 may have any one of a carboxyl group (-COOH), a carboxylate group ($-COO^-$), an amide group ($-CONH_2$) and an ester group (-COOR) at the C-terminal. Examples of R in the ester group include a $C_{1-6}$ alkyl group such as methyl, ethyl, n-propyl, isopropyl or n-butyl, a $C_{3-8}$ cycloalkyl group such as cyclopentyl or cyclohexyl, a $C_{6-12}$ aryl group such as phenyl or α-naphthyl, or a $C_{7-14}$ aralkyl group such as a phenyl-$C_{1-2}$ alkyl including benzyl, phenethyl or benzhydryl or an α-naphthyl-$C_{1-2}$ alkyl including α-naphthylmethyl, as well as a pivaloyloxymethyl group commonly used as an ester for oral administration.

Salts of the precursor protein of the present invention may be the same salts as illustrated above for the polypeptide of the present invention.

The precursor protein of the present invention may be prepared as described in, e.g., WO 00/32627, WO 00/31265, WO 99/35266 or Japanese Patent Application No. 2000-211996. Also, the precursor protein of the present invention may be prepared by purification techniques for proteins from tissues or cells of homeotherms, as in the case of the polypeptide of the present invention, or may be prepared according to synthesis techniques for proteins. Alternatively, it may also be prepared by culturing transformants carrying the DNA encoding the precursor protein of the present invention, as in the case of the polypeptide of the present invention.

When the precursor protein is prepared from tissues or cells of homeotherms, tissues or cells of homeotherms are homogenized and then extracted with an acid, an organic solvent and the like. The resulting extract is subjected to salt precipitation and dialysis in combination with chromatography such as gel filtration, reverse phase chromatography, ion exchange chromatography or affinity chromatography to give a purified and isolated precursor protein.

An amide form of the precursor protein of the present invention may be obtained using any commercially available peptide synthesis resin suitable for amidation. Examples of such a resin include the above-listed resins for use in peptide synthesis. Using such a resin, amino acids having appropriately protected α-amino and side chain functional groups are condensed on the resin, as sequenced in the peptide sequence of interest, in accordance with various well-known condensation techniques. At the end of the reaction, the resulting peptide is cleaved form the resin simultaneously with removal of various protecting groups, followed by intramolecular disulfide bridging in a highly-diluted solution, if necessary, to obtain the target precursor protein of the present invention.

The precursor protein of the present invention per se may contain the same or substantially the same amino acid sequence as shown in SEQ ID NO: 7, 8, 14, 17 or 23 and may have the same activities as the polypeptide of the present invention, such as anti-attention-deficit-disorder and/or anti-narcolepsy activities.

The DNA encoding the precursor protein of the present invention may be any DNA as long as it contains a DNA encoding a protein containing the same or substantially the same amino acid sequence as shown in SEQ ID NO:

7, 8, 14, 17 or 23. Also, it may be any one of a genomic DNA, a genomic DNA library, a cDNA derived from the above-mentioned tissues or cells, a cDNA library derived from the above-mentioned tissues or cells and a synthetic DNA. Any vector may be used for these libraries, including bacteriophages, plasmids, cosmids, phagemids, etc. Alternatively, the DNA may be directly amplified in the Reverse Transcriptase Polymerase Chain Reaction (hereinafter simply referred to as RT-PCR) using an RNA fraction prepared from the above-mentioned tissues or cells.

Examples of a DNA containing a DNA encoding a protein containing the same or substantially the same amino acid sequence as shown in SEQ ID NO: 7, 8, 14, 17 or 23 include DNAs containing the nucleotide sequence shown in SEQ ID NO: 4, 5, 6, 15, 16 or 22, as well as DNAs containing a nucleotide sequence sharing a homology of at least about 50%, preferably at least about 60%, more preferably at least about 70%, even more preferably at least about 80%, particularly preferably at least about 90%, most preferably at least about 95% with the nucleotide sequence shown in SEQ ID NO: 4, 5, 6, 15, 16 or 22.

Other examples of a DNA containing a DNA encoding a protein containing the same or substantially the same amino acid sequence as shown in SEQ ID NO: 7, 8, 14, 17 or 23 include DNAs containing (i) a nucleotide sequence with deletion of one or more nucleotides (preferably around 1 to 30, more preferably around 1 to 10, even more preferably 1 or 2 nucleotides) in the nucleotide sequence shown in SEQ ID NO: 4, 5, 6, 15, 16 or 22; (ii) a nucleotide sequence with addition of one or more nucleotides (preferably around 1 to 30, more preferably around 1 to 10, even more preferably 1 or 2 nucleotides) in the nucleotide sequence shown in SEQ ID NO: 4, 5, 6, 15, 16 or 22; (iii) a nucleotide sequence with insertion of one or more nucleotides (preferably around I to 30, more preferably around 1 to 10, even more preferably 1 or 2 nucleotides) in the nucleotide sequence shown in SEQ ID NO: 4, 5, 6, 15, 16 or 22; (iv) an amino acid sequence with substitution of one or more nucleotides (preferably around 1 to 30, more preferably around 1 to 10, even more preferably 1 or 2 nucleotides) in the nucleotide sequence shown in SEQ ID NO: 4, 5, 6, 15, 16 or 22; or (v) a nucleotide sequence with any combination of the deletion, addition, insertion and substitution mentioned above.

More specific examples include (1) mammalian-derived DNAs that hybridize under stringent conditions with a DNA containing a DNA encoding a protein containing the same or substantially the same amino acid sequence as shown in SEQ ID NO: 7, 8, 14, 17 or 23 and (2) DNAs that form no hybrid neither with a DNA containing a DNA encoding a protein containing the same or substantially the same amino acid sequence as shown in SEQ ID NO: 7, 8, 14, 17 or 23 nor with the DNAs defined in (1) due to degeneracy of the genetic code, but that encodes a protein having the same amine acid sequence. Hybridization may be carried out according to well-known procedures or equivalents thereof. The stringent conditions mentioned above are set at 42°C in 50% formamide, 4 × SSPE (1 × SSPE = 150 mM NaCl, 10 mM $NaH_2PO_4 \cdot H_2O$, 1 mM EDTA pH7.4), 5 × Denhart's solution and 0.1% SDS, by way of example.

Examples of DNAs that hybridize with a DNA containing a DNA encoding a protein containing the same or substantially the same amino acid sequence as shown in SEQ ID NO: 7, 8, 14, 17 or 23 include DNAs containing a nucleotide sequence sharing a homology of at least about 70%, preferably at least about 80%, more preferably at least about 90%, most preferably at least about 95% with the nucleotide sequence shown in SEQ ID NO: 4, 5, 6, 15, 16 or 22.

The DNA encoding the precursor protein of the present invention may also be prepared by the genetic engineering procedures in the same manner as described above for the polypeptide of the present invention.

The DNA encoding the precursor protein of the present invention or the precursor protein of the present invention can be used for development of medicaments including an anti-attention-deficit-disorder or anti-narcolepsy agent or an anti-anxiety, anti-depression, anti-insomnia, anti-schizophrenia or anti-fear agent, development of a receptor-binding assay system using a recombinant receptor protein expression system, screening of candidate compounds for medicaments, gene therapy, etc.

In particular, the receptor-binding assay system using the recombinant GPR14 (SENR) expression system illustrated later achieves screening of GPR14 (SENR) agonists or antagonists specific to homeotherms including human. Such agonists or antagonists can be used as prophylactic and/or therapeutic agents for various diseases, by way of example.

Further, the precursor protein of the present invention or the DNA encoding the same can be recognized as a ligand by GPR14 (SENR) expressed in the central nerve system, circulatory system, heart, kidneys, urinary system, sensory organs or the like; it is therefore useful as a safer and less toxic medicament. The precursor protein of the present invention or the DNA encoding the same can be used as a therapeutic and/or prophylactic agent for attention deficit disorder or narcolepsy, by way of example.

In a case where the precursor protein of the present invention or the DNA encoding the same is used as the medicament mentioned above, it may be formulated in a routine manner. For example, it may be used for oral administration in dosage forms of optionally sugar-coated or enteric-coated tablets, capsules, elixirs, microcapsules and the like or may be used for parenteral administration in dosage forms of injections such as sterile solutions or suspensions in water or other pharmaceutically acceptable fluids. For example, these formulations may be prepared by incorporating the compound or a salt thereof in unit dosage forms required for generally-accepted formulations in combination with physiologically acceptable carriers, flavors, excipients, vehicles, antiseptics, stabilizers, binders and the like. The

amount of an active ingredient in these formulations is intended to give an appropriate dose within the indicated range.

Additives, which can be incorporated into tablets, capsules and the like, may be the same additives as illustrated above.

Examples of aqueous fluids for injection include physiological saline and isotonic solutions containing glucose and/or other auxiliaries (e.g., D-sorbitol, D-mannitol, sodium chloride), which may be used in combination with appropriate solubilizers such as alcohols (e.g., ethanol), polyalcohols (e.g., propylene glycol, polyethylene glycol) and nonionic surfactants (e.g., Polysorbate 80™, HCO-50). Examples of oil fluids include sesame oil and soy bean oil, which may be used in combination with solubilizers such as benzyl benzoate and benzyl alcohol.

In addition, these fluids may be blended with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), antioxidants, etc. The prepared solutions for injection are usually filled into appropriate ampules.

Since the formulations thus prepared are safer and less toxic, they can be administered to, for example, mammals (e.g., human, mouse, rat, guinea pig, rabbit, sheep, pig, cattle, cat, dog, monkey).

The dose of the precursor protein of the present invention or the DNA encoding the same will vary depending on symptoms etc. For oral administration, it is usually administered to an adult patient with attention deficit disorder (body weight: 60 kg) in an amount of about 0.1 to 100 mg/day, preferably about 1.0 to 50 mg/day, more preferably about 1.0 to 20 mg/day. For parenteral administration, the single dose will vary depending on subjects to be administered, target organs, symptoms, the intended route of administration, etc. In a dosage form of injection, for example, it is advantageously administered to an adult patient with attention deficit disorder (body weight: 60 kg) in an amount of about 0.01 to 30 mg/day, preferably about 0.1 to 20 mg/day, more preferably about 0.1 to 10 mg/day, by intravenous injection. Other animals may be administered with the precursor protein or DNA in an amount calculated per 60 kg.

Examples of GPR14 (SENR) according to the present invention include not only those described in Tal, M. et al., Biochem. Biophys. Res. Commun., 209, 752-759, 1995, Marchese, A., Genomics, 29, 335-344, 1995 and EP 859052, as stated above, but also GPR14 (SENR) or a salt thereof, which contains the same or substantially the same amino acid sequence as shown in SEQ ID NO: 3 or SEQ ID NO: 11, and a modified GPR14 (SENR) or a salt thereof, which contains an amino acid sequence with deletion of 1 to 30 amino acids, preferably 1 to 10 amino acids in the amino acid sequence shown in SEQ ID NO: 3 or SEQ ID NO: 11, an amino acid sequence with addition (or insertion) of 1 to 30 amino acids, preferably 1 to 10 amino acids in the amino acid sequence shown in SEQ ID NO: 3 or SEQ ID NO: 11 or an amino acid sequence with substitution of 1 to 30 amino acids, preferably 1 to 10 amino acids in the amino acid sequence shown in SEQ ID NO: 3 or SEQ ID NO: 11.

A partial peptide of GPR14 (SENR) used herein may be any peptide as long as it is a partial peptide derived from the above-mentioned GPR14 (SENR) according to the present invention. Examples include partial peptides that are derived from extracellular domains of the GPR14 (SENR) protein molecule according to the present invention and that are capable of binding to the polypeptide of the present invention.

GPR14 (SENR) used herein or a partial peptide thereof may be prepared in the same or equivalent manner as described in Tal, M. et al., Biochem. Biophys. Res. Commun., 209, 752-759, 1995, Marchese, A., Genomics, 29, 335-344, 1995 and EP 859052. Alternatively, it may also be prepared in the same manner as described above for the polypeptide of the present invention.

Salts of GPR14 (SENR) or a partial peptide thereof may be the same salts as illustrated above for the polypeptide of the present invention.

The DNA encoding GPR14 (SENR) used herein or a partial peptide thereof may be any DNA as long as it contains a DNA encoding the above-mentioned GPR14 (SENR) or a partial peptide thereof. Also, it may be any one of a genomic DNA, a genomic DNA library, a cDNA derived from the above-mentioned tissues or cells, a cDNA library derived from the above-mentioned tissues or cells and a synthetic DNA. Any vector may be used for these libraries, including bacteriophages, plasmids, cosmids, phagemids, etc. Alternatively, the DNA may be directly amplified in RT-PCR using an RNA fraction prepared from the above-mentioned tissues or cells. The DNA encoding GPR14 (SENR) used herein or a partial peptide thereof may also be prepared in the same or equivalent manner as described in Tal, M. et al., Biochem. Biophys. Res. Commun., 209, 752-759, 1995, Marchese, A., Genomics, 29, 335-344, 1995 and EP 859052.

The term "polynucleotide containing part or all of a nucleotide sequence complementary to a DNA containing the nucleotide sequence encoding the polypeptide of the present invention" is intended to encompass both the DNA of the present invention and the corresponding RNA.

The polynucleotide (nucleic acid) is capable of hybridizing with an RNA for the polypeptide gene of the present invention to inhibit synthesis or functions of the RNA, or it is capable of interacting with an RNA related to the polypeptide of the present invention to regulate and control gene expression of the polypeptide of the present invention. A polynucleotide complementary to the selected sequence of an RNA related to the polypeptide of the present invention and a polynucleotide capable of specific hybridization with an RNA related to the polypeptide of the present invention are

useful in regulating and controlling *in vivo* and *in vitro* gene expression of the polypeptide of the present invention. For example, they are useful for treatment and diagnosis of diseases such as attention deficit disorder, narcolepsy, anxiety, depression, insomnia, schizophrenia or fear.

In terms of relation between a nucleic acid of interest and a polynucleotide complementary to at least part of the target region, the relation between the nucleic acid and a polynucleotide capable of hybridizing with the same can be expressed as "antisense." Examples of an antisense polynucleotide include a polydeoxynucleotide containing 2-deoxy-D-riboses, a polydeoxynucleotide containing D-riboses, other types of polynucleotides containing N-glycosides of purine or pyrimidine bases, or other polymers having a non-nucleotide backbone (e.g., commercially available protein/nucleic acids and synthetic sequence-specific nucleic acid polymers) or other polymers containing special linkages (provided that said polymers contain nucleotides allowing base paring and nucleotide attachment found in DNA or RNA). Such an antisense polynucleotide may be a double-stranded DNA, a single-stranded DNA, a double-stranded RNA, a single-stranded RNA or a DNA:RNA hybrid. In addition, it may be a non-modified polynucleotide (or non-modified oligonucleotide) or a modified polynucleotide with modification known in the art, for example, labeled, capped, methylated, substituted with one or more nucleotide analogs, or subjected to intramolecular nucleotide modification, for example, to have non-charged linkages (e.g., methylphosphonate, phosphotriester, phosphoramidate, carbamate), to have charged linkages or sulfur-containing linkages (e.g., phosphorothioate, phosphorodithioate), to have side chain groups such as proteins (nuclease, nuclease inhibitor, toxin, antibody, signal peptide, poly-L-lysine) or sugars (e.g., monosaccharide), to have intercalated compounds (e.g., acridine, psoralen), to contain chelated compounds (e.g., metal, radioactive metal, boron, oxidizing metal), to contain alkylating agents, or to have modified linkages (e.g., α-anomeric nucleic acid). As used here, "nucleoside", "nucleotide" and "nucleic acid" may contain not only purine and pyrimidine bases, but also other modified heterocyclic bases. Such modified nucleoside, nucleotide and nucleic acid may be those containing methylated purine and pyrimidine, acylated purine and pyrimidine, or other heterocyclic rings. Such modified nucleoside and nucleotide may receive modification in their sugar moieties where one or more hydroxyl groups may be substituted with a halogen atom, an aliphatic group, etc., or converted into a functional group(s) such as ether or amine.

The antisense polynucleotide (nucleic acid) is an RNA, a DNA or a modified nucleic acid (RNA, DNA). Specific examples of a modified nucleic acid include, but are not limited to, sulfur derivatives or thiophosphate derivatives of nucleic acids as well as those resistant to degradation of polynucleoside amide and oligonucleoside amide. The antisense nucleic acid may preferably be designed on the basis of the following strategies. Namely, it may be designed to improve its stability in cells, to enhance its permeability across cells, to increase its affinity for the target sense chain, and to reduce its toxicity, if any.

These modifications are widely known in the art and disclosed in, e.g., J. Kawakami et al., Pharm Tech Japan, Vol. 8, pp.247, 1992; Vol. 8, pp.395, 1992 and S. T. Crooke et al. ed., Antisense Research and Applications, CRC Press, 1993.

The antisense nucleic acid may be altered or may contain modified sugars, bases and linkages. It may be provided in special forms such as liposomes and microspheres, applied via gene therapy, or given in adduct form. Materials used in adduct form include polycations such as polylysine serving to neutralize charges on the phosphate backbone and hydrophobic materials such as lipids (e.g., phospholipid, cholesterol) serving to enhance interaction with cellular membranes and/or uptake of the nucleic acid. Examples of lipids preferred for this purpose include cholesterol and derivatives thereof (e.g., cholesteryl chloroformate, cholic acid). Such material may be attached to the nucleic acid at the 3'- or 5'-terminal via a base, a sugar and/or an intramolecular nucleoside linkage. Other groups used for this purpose include a capping group specifically located at the 3'- or 5'-terminal of the nucleic acid, preventing degradation caused by a nuclease such as exonuclease and RNase. Examples of such a capping group include, but are not limited to, protecting groups for a hydroxyl group known in the art, e.g., glycols such as polyethylene glycol and tetraethylene glycol.

Preparation of an antibody against the polypeptide of the present invention or a precursor protein thereof or an amide or ester of the polypeptide or precursor protein or a salt thereof will be described below.

An antibody against the polypeptide of the present invention or a precursor protein thereof or an amide or ester of the polypeptide or precursor protein or a salt thereof may be either polyclonal or monoclonal as long as it can recognize the polypeptide of the present invention or a precursor protein thereof or an amide or ester of the polypeptide or precursor protein or a salt thereof.

An antibody against the polypeptide of the present invention or a precursor protein thereof or an amide or ester of the polypeptide or precursor protein or a salt thereof (hereinafter also simply referred to as the polypeptide or its analog of the present invention) may be prepared according to well-known antibody or antiserum preparation procedures using the polypeptide or its analog of the present invention as an antigen.

[Preparation of monoclonal antibodies]

(a) Preparation of monoclonal antibody-producing cells

The polypeptide or its analog of the present invention is administered, alone or in combination with a carrier or diluent, to a mammal at a site where antibody production is inducible by administration. At the time of administration, Freund's complete adjuvant or Freund's incomplete adjuvant may be administered to enhance the antibody-producing capacity. Administration is usually carried out once every 2 to 6 weeks and repeated about 2 to 10 times in total. Examples of a mammal available for use include monkey, rabbit, dog, guinea pig, mouse, rat, sheep and goat, with mouse and rat being preferred for use.

In preparing monoclonal antibody-producing cells, individuals showing a detectable antibody titer are selected among homeotherms (e.g., mice) immunized with the antigen. Their spleens or lymph nodes are collected 2 to 5 days after the last immunization and antibody-producing cells contained in the collected tissues are fused with myeloma cells to prepare monoclonal antibody-producing hybridomas. The antibody titer in antiserum may be determined, for example, by reacting the labeled polypeptide or its analog stated later with antiserum and then assaying the activity of the label bound to antibody molecules. Fusion manipulation may be accomplished in a known manner, e.g., the Keller & Milstein method [Nature, vol. 256, p. 495 (1975)]. Examples of a fusion-stimulating agent include polyethylene glycol (PEG) and Sendai virus, with PEG being preferred for use.

Examples of myeloma cells include NS-1, P3U1 and SP2/0, with P3U1 being preferred for use. A preferred ratio between antibody-producing cells (spleen cells) and myeloma cells ranges from about 1:1 to 20:1 and efficient cell fusion may be accomplished by incubation at about 20°C to 40°C, preferably about 30°C to 37°C, for about 1 to 10 minutes in the presence of PEG (preferably PEG1000 to PEG6000) at a concentration of about 10% to 80%.

Monoclonal antibody-producing hybridomas may be screened by various techniques, for example, by adding the culture supernatant of each hybridoma to a solid phase (e.g., a microplate) on which the polypeptide or its analog as an antigen is adsorbed directly or together with a carrier and then adding an anti-immunoglobulin antibody (an anti-mouse immunoglobulin antibody being used when cells used for cell fusion are of mouse origin) or protein A labeled with a radioactive substance or an enzyme to detect monoclonal antibody molecules bound to the solid phase, or by adding the culture supernatant of each hybridoma to a solid phase on which an anti-immunoglobulin antibody or protein A is adsorbed and then adding the polypeptide or its analog labeled with a radioactive substance or an enzyme to detect monoclonal antibody molecules bound to the solid phase.

Selection of monoclonal antibodies may be carried out according to well-known procedures or equivalents thereof, usually in a medium for animal cells supplemented with HAT (hypoxanthine, aminopterin, thymidine), etc. Any medium may be used as a selective and culture medium as long as hybridomas can grow in the medium. Examples include RPMI 1640 medium supplemented with 1% to 20% (preferably 10% to 20%) fetal bovine serum, GIT medium (Wako Pure Chemical Industries, Ltd.) supplemented with 1% to 10% fetal bovine serum, and a serum-free medium for hybridoma culture (SFM-101, Nissui Pharmaceuticals, Co.). The culture temperature is usually 20°C to 40°C, preferably about 37°C. The culture period is usually 5 days to 3 weeks, preferably 1 week to 2 weeks. The culturing may usually be carried out under 5% carbon dioxide atmosphere. The antibody titer in the hybridoma culture supernatant may be determined in the same manner as described for the antibody titer in antiserum.

(b) Purification of monoclonal antibodies

Isolation and purification of monoclonal antibodies may be carried out, as in the case of standard isolation and purification of polyclonal antibodies, according to isolation and purification procedures for immunoglobulins [e.g., salt precipitation, alcohol precipitation, isoelectric precipitation, electrophoresis, adsorption-desorption on an ion exchanger (e.g., DEAE), ultracentrifugation, gel filtration, or a specific purification procedure in which antibody molecules are collected alone on an antigen-immobilized solid phase or an active adsorbent such as protein A or protein G and then released from the solid phase or adsorbent by dissociation].

[Preparation of polyclonal antibodies]

The polyclonal antibodies of the present invention may be prepared according to well-known procedures or equivalents thereof. For example, they may be prepared by creating a conjugate between an immunogen (the polypeptide or its analog of the present invention as an antigen) and a carrier protein, immunizing a mammal in the same manner as described above for preparation of monoclonal antibodies, collecting from the immunized mammal a product containing antibodies against the receptor protein or its analog of the present invention, and then isolating and purifying the antibodies.

In the immunogen-carrier protein conjugate used for immunization of a mammal, any type of carrier protein

and any mixing ratio between carrier and hapten may be used for conjugation as long as antibodies can be efficiently produced against the carrier-conjugated hapten used for immunization. For example, the hapten may be capsulated with bovine serum albumin, bovine thyroglobulin, keyhole limpet hemocyanin, etc., at a weight ratio of about 0.1 to 20, preferably about 1 to 5, relative to the hapten (=1).

In addition, various condensing agents may be used for coupling between hapten and carrier, including glutaraldehyde, carbodiimide, a maleimide active ester, and an active ester reagent containing a thiol group or a dithiopyridyl group.

The condensed product is administered, alone or in combination with a carrier or diluent, to a homeotherm at a site where antibody production is inducible. At the time of administration, Freund's complete adjuvant or Freund's incomplete adjuvant may be administered to enhance the antibody-producing capacity. Administration may usually be carried out once every 2 to 6 weeks and repeated about 3 to 10 times in total.

The polyclonal antibodies may be collected from blood, ascites fluid or the like, preferably blood, of the mammal thus immunized.

The polyclonal antibody titer in antiserum may be determined in the same manner as described above for the antibody titer in serum. Isolation and purification of polyclonal antibodies may be carried out according to the same isolation and purification procedures for immunoglobulins as described above for isolation and purification of monoclonal antibodies.

Preparation of an antibody against a protein containing GPR14 (SENR) (e.g., the amino acid sequence shown in SEQ ID NO: 3 or SEQ ID NO: 11 of the present invention) or an amide or ester of the protein or a salt thereof will be described below.

An antibody against a protein containing GPR14 (SENR) (e.g., the amino acid sequence shown in SEQ ID NO: 3 or SEQ ID NO: 11 of the present invention) or an amide or ester of the protein or a salt thereof may be either polyclonal or monoclonal as long as it can recognize a protein containing GPR14 (SENR) (e.g., the amino acid sequence shown in SEQ ID NO: 3 or SEQ ID NO: 11 of the present invention) or an amide or ester of the protein or a salt thereof.

An antibody against a protein containing GPR14 (SENR) (e.g., the amino acid sequence shown in SEQ ID NO: 3 or SEQ ID NO: 11 of the present invention) or an amide or ester of the protein or a salt thereof (hereinafter also simply referred to as GPR14 (SENR) or its analog) may be prepared according to well-known antibody or antiserum preparation procedures using GPR14 (SENR) or its analog as an antigen.

[Preparation of monoclonal antibodies]

(a) Preparation of monoclonal antibody-producing cells

GPR14 (SENR) or its analog is administered, alone or in combination with a carrier or diluent, to a mammal at a site where antibody production is inducible by administration. At the time of administration, Freund's complete adjuvant or Freund's incomplete adjuvant may be administered to enhance the antibody-producing capacity. Administration is usually carried out once every 2 to 6 weeks and repeated about 2 to 10 times in total. Examples of a mammal available for use include monkey, rabbit, dog, guinea pig, mouse, rat, sheep and goat, with mouse and rat being preferred for use.

In preparing monoclonal antibody-producing cells, individuals showing a detectable antibody titer are selected among homeotherms (e.g., mice) immunized with the antigen. Their spleens or lymph nodes are collected 2 to 5 days after the last immunization and antibody-producing cells contained in the collected tissues are fused with myeloma cells to prepare monoclonal antibody-producing hybridomas. The antibody titer in antiserum may be determined, for example, by reacting labeled GPR14 (SENR) or its analog with antiserum and then assaying the activity of the label bound to antibody molecules. Fusion manipulation may be accomplished in a known manner, e.g., the Keller & Milstein method [Nature, vol. 256, p. 495 (1975)]. Examples of a fusion-stimulating agent include polyethylene glycol (PEG) and Sendai virus, with PEG being preferred for use.

Examples of myeloma cells include NS-1, P3U1 and SP2/0, with P3U1 being preferred for use. A preferred ratio between antibody-producing cells (spleen cells) and myeloma cells ranges from about 1:1 to 20:1 and efficient cell fusion may be accomplished by incubation at about 20°C to 40°C, preferably about 30°C to 37°C, for about 1 to 10 minutes in the presence of PEG (preferably PEG1000 to PEG6000) at a concentration of about 10% to 80%.

Monoclonal antibody-producing hybridomas may be screened by various techniques, for example, by adding the culture supernatant of each hybridoma to a solid phase (e.g., a microplate) on which GPR14 (SENR) or its analog as an antigen is adsorbed directly or together with a carrier and then adding an anti-immunoglobulin antibody (an anti-mouse immunoglobulin antibody being used when cells used for cell fusion are of mouse origin) or protein A labeled with a radioactive substance or an enzyme to detect monoclonal antibody molecules bound to the solid phase, or by adding the culture supernatant of each hybridoma to a solid phase on which an anti-immunoglobulin antibody or protein A is adsorbed and then adding the polypeptide or its analog labeled with a radioactive substance or an enzyme to

detect monoclonal antibody molecules bound to the solid phase.

Selection of monoclonal antibodies may be carried out according to well-known procedures or equivalents thereof, usually in a medium for animal cells supplemented with HAT (hypoxanthine, aminopterin, thymidine), etc. Any medium may be used as a selective and culture medium as long as hybridomas can grow in the medium. Examples include RPMI 1640 medium supplemented with 1% to 20% (preferably 10% to 20%) fetal bovine serum, GIT medium (Wako Pure Chemical Industries, Ltd.) supplemented with 1% to 10% fetal bovine serum, and a serum-free medium for hybridoma culture (SFM-101, Nissui Pharmaceuticals, Co.). The culture temperature is usually 20°C to 40°C, preferably about 37°C. The culture period is usually 5 days to 3 weeks, preferably 1 week to 2 weeks. The culturing may usually be carried out under 5% carbon dioxide atmosphere. The antibody titer in the hybridoma culture supernatant may be determined in the same manner as described for the antibody titer in antiserum.

(b) Purification of monoclonal antibodies

Isolation and purification of monoclonal antibodies may be carried out, as in the case of standard isolation and purification of polyclonal antibodies, according to isolation and purification procedures for immunoglobulins [e.g., salt precipitation, alcohol precipitation, isoelectric precipitation, electrophoresis, adsorption-desorption on an ion exchanger (e.g., DEAE), ultracentrifugation, gel filtration, or a specific purification procedure in which antibody molecules are collected alone on an antigen-immobilized solid phase or an active adsorbent such as protein A or protein G and then released from the solid phase or adsorbent by dissociation].

[Preparation of polyclonal antibodies]

The polyclonal antibodies of the present invention may be prepared according to well-known procedures or equivalents thereof. For example, they may be prepared by creating a conjugate between an immunogen (the polypeptide or its analog of the present invention as an antigen) and a carrier protein, immunizing a mammal in the same manner as described above for preparation of monoclonal antibodies, collecting from the immunized mammal a product containing antibodies against the receptor protein or its analog of the present invention, and then isolating and purifying the antibodies.

In the immunogen-carrier protein conjugate used for immunization of a mammal, any type of carrier protein and any mixing ratio between carrier and hapten may be used for conjugation as long as antibodies can be efficiently produced against the carrier-conjugated hapten used for immunization. For example, the hapten may be capsulated with bovine serum albumin, bovine thyroglobulin, keyhole limpet hemocyanin, etc., at a weight ratio of about 0.1 to 20, preferably about 1 to 5, relative to the hapten (= 1).

In addition, various condensing agents may be used for coupling between hapten and carrier, including glutaraldehyde, carbodiimide, a maleimide active ester, and an active ester reagent containing a thiol group or a dithiopyridyl group.

The condensed product is administered, alone or in combination with a carrier or diluent, to a homeotherm at a site where antibody production is inducible. At the time of administration, Freund's complete adjuvant or Freund's incomplete adjuvant may be administered to enhance the antibody-producing capacity. Administration may usually be carried out once every 2 to 6 weeks and repeated about 3 to 10 times in total.

The polyclonal antibodies may be collected from blood, ascites fluid or the like, preferably blood, of the mammal thus immunized.

The polyclonal antibody titer in antiserum may be determined in the same manner as described above for the antibody titer in serum. Isolation and purification of polyclonal antibodies may be carried out according to the same isolation and purification procedures for immunoglobulins as described above for isolation and purification of monoclonal antibodies.

The following methods will be described below in more detail: (1) a method for screening compounds (agonists, antagonists) capable of changing the binding property between GPR14 (SENR) and the polypeptide of the present invention or a precursor protein thereof, which uses the polypeptide of the present invention or a precursor protein thereof, the DNA encoding the polypeptide or precursor protein, etc.; (2) a diagnostic method using a polynucleotide containing part or all of a nucleotide sequence complementary to a DNA containing the nucleotide sequence encoding the polypeptide of the present invention; (3) a diagnostic method using an antibody against the polypeptide of the present invention or a precursor protein thereof or an amide or ester of the polypeptide or precursor protein or a salt thereof; (4) a diagnostic method using an antibody against GPR14 (SENR) or a salt thereof; (5) a gene diagnostic method associated with the polypeptide of the present invention; and (6) a gene diagnostic method associated with GPR14 (SENR).

(1) Method for screening compounds (agonists, antagonists) capable of changing the binding property between GPR14 (SENR) and the polypeptide of the present invention or a precursor protein thereof, which uses the polypeptide of the present invention or a precursor protein thereof, the DNA encoding the polypeptide or precursor protein, etc.

Compounds (e.g., peptides, proteins, non-peptide compounds, synthetic compounds, fermentation products) or salts thereof, which are capable of changing the binding property between GPR14 (SENR) and the polypeptide of the present invention or a precursor protein thereof, can be screened by using GPR14 (SENR) or a salt thereof or a partial peptide of GPR14 (SENR) or a salt thereof or by using a receptor-binding assay system using an expression system constructed for recombinant GPR14 (SENR). Such compounds include compounds with GPR14 (SENR)-mediated cell-stimulating activities (e.g., promotional or inhibitory activities on arachidonate release, acetylcholine release, intracellular $Ca^{2+}$ release, intracellular cAMP production, intracellular cGMP production, inositol phosphate production, variation in cell membrane potential, intracellular protein phosphorylation, c-fos activation, pH decrease, etc.) (i.e., GPR14 (SENR) agonists) and compound without these cell-stimulating activities (i.e., GPR14 (SENR) antagonists). "Changing the binding property with ligands" is intended to encompass both inhibition and promotion of binding to the ligands.

The present invention provides a method for screening compounds or salts thereof capable of changing the binding property between GPR14 (SENR) and the polypeptide of the present invention or a precursor protein thereof, which comprises performing a comparison between (i) when the polypeptide of the present invention or a precursor protein thereof is contacted with GPR14 (SENR) or a salt thereof or a partial peptide of GPR14 (SENR) or a salt thereof and (ii) when the polypeptide of the present invention or a precursor protein thereof and a test compound are contacted with GPR14 (SENR) or a salt thereof or a partial peptide of GPR14 (SENR) or a salt thereof.

In the screening method of the present invention, for example, the amount of the ligand bound to the GPR14 (SENR) or the partial peptide of GPR14 (SENR), cell-stimulating activities or the like may be measured and compared between (i) when the polypeptide of the present invention or a precursor protein thereof is contacted with the GPR14 (SENR) or the partial peptide of GPR14 (SENR) and (ii) when the polypeptide of the present invention or a precursor protein thereof and a test compound are contacted with the GPR14 (SENR) or the partial peptide of GPR14 (SENR).

More specifically, the screening method of the present invention encompasses:

(I) a method for screening compounds or salts thereof capable of changing the binding property between GPR14 (SENR) and the polypeptide of the present invention or a precursor protein thereof, wherein the amount of the labeled polypeptide of the present invention or a labeled precursor protein thereof bound to GPR14 (SENR) or a salt thereof or a partial peptide of GPR14 (SENR) or a salt thereof is measured and compared between when the labeled polypeptide or precursor protein thereof is contacted with the GPR14 (SENR) or salt thereof or the partial peptide of GPR14 (SENR) or salt thereof and when the labeled polypeptide or precursor protein thereof and a test compound are contacted with the GPR14 (SENR) or salt thereof or the partial peptide of GPR14 (SENR) or salt thereof;

(II) a method for screening compounds or salts thereof capable of changing the binding property between GPR14 (SENR) and the polypeptide of the present invention or a precursor protein thereof, wherein the amount of the labeled polypeptide of the present invention or a labeled precursor protein thereof bound to GPR14 (SENR)-containing cells or a cell membrane fraction thereof is measured and compared between when the labeled polypeptide or precursor protein thereof is contacted with the GPR14 (SENR)-containing cells or cell membrane fraction thereof and when the labeled polypeptide or precursor protein thereof and a test compound are contacted with the GPR14 (SENR)-containing cells or cell membrane fraction thereof;

(III) a method for screening compounds or salts thereof capable of changing the binding property between GPR14 (SENR) and the polypeptide of the present invention or a precursor protein thereof, wherein the amount of the labeled polypeptide of the present invention or a labeled precursor protein thereof bound to GPR14 (SENR) is measured and compared between when the labeled polypeptide or precursor protein thereof is contacted with GPR14 (SENR) expressed on cell membranes of cultured transformants carrying the DNA encoding GPR14 (SENR) and when the labeled polypeptide or precursor protein thereof and a test compound are contacted with GPR14 (SENR) expressed on cell membranes of cultured transformants carrying the DNA encoding GPR14 (SENR);

(IV) a method for screening compounds or salts thereof capable of changing the binding property between GPR14 (SENR) and the polypeptide of the present invention or a precursor protein thereof, wherein GPR14 (SENR)-mediated cell-stimulating activities (e.g., promotional or inhibitory activities on arachidonate release, acetylcholine release, intracellular $Ca^{2+}$ release, intracellular cAMP production, intracellular cGMP production, inositol phosphate production, variation in cell membrane potential, intracellular protein phosphorylation, c-fos activation, pH decrease, etc.) are measured and compared between when a compound capable of activating GPR14 (SENR) (e.g., the polypeptide of the present invention or a precursor protein thereof) is contacted with GPR14 (SENR)-containing cells and when the compound capable of activating GPR14 (SENR) and a test compound are contacted with GPR14 (SENR)-containing cells; and

(V) a method for screening compounds or salts thereof capable of changing the binding property between GPR14 (SENR) and the polypeptide of the present invention or a precursor protein thereof, wherein GPR14 (SENR)-mediated cell-stimulating activities (e.g., promotional or inhibitory activities on arachidonate release, acetylcholine release, intracellular $Ca^{2+}$ release, intracellular cAMP production, intracellular cGMP production, inositol phosphate production, variation in cell membrane potential, intracellular protein phosphorylation, c-fos activation, pH decrease, etc.) are measured and compared between when a compound capable of activating GPR14 (SENR) (e.g., the polypeptide of the present invention or a precursor protein thereof) is contacted with GPR14 (SENR) expressed on cell membranes of cultured transformants carrying the DNA encoding GPR14 (SENR) and when the compound capable of activating GPR14 (SENR) and a test compound are contacted with GPR14 (SENR) expressed on cell membranes of cultured transformants carrying the DNA encoding GPR14 (SENR).

The screening method of the present invention will be described below in more detail.

First, GPR14 (SENR) used in the screening method of the present invention may be the above-mentioned GPR14 (SENR) or any fraction containing a partial peptide of GPR14 (SENR). Preferred examples include membrane fractions derived from homeotherm's organs. However, since human-derived organs are particularly quite difficult to obtain, GPR14 (SENR) recombinantly expressed at a high level is suitable for use in the screening, by way of example.

For production of GPR14 (SENR), the above-mentioned procedure or the like is used.

In a case where GPR14 (SENR)-containing cells or a cell membrane fraction thereof is used in the screening method of the present invention, they may be prepared as stated below.

In a case where GPR14 (SENR)-containing cells are used, the cells may be fixed in glutaraldehyde, formalin, etc. Fixation may be accomplished according to well-known procedures.

GPR14 (SENR)-containing cells are intended to mean host cells expressing GPR14 (SENR) thereon. Examples of the host cells include *Escherichia coli* cells, *Bacillus subtilis* cells, yeast cells, insect cells and animal cells as mentioned above.

A cell membrane fraction is intended to mean a fraction rich in cell membranes obtained in a well-known manner after cell disruption. Techniques for cell disruption include cell crushing with a Potter-Elvehjem homogenizer, disruption in a Waring blender or a Polytron (manufactured by Kinematica), ultrasonic disruption, and cell crushing through a narrow nozzle while applying pressure with a French press or the like. Centrifugal fractionation, including differential centrifugation and density gradient centrifugation, is predominantly used for fractionation of cell membranes. For example, a disrupted cell solution is centrifuged at a low speed (500 rpm to 3000 rpm) for a short time (usually about 1 to 10 minutes) and the supernatant is further centrifuged at a high speed (15000 rpm to 30000 rpm) and usually for 30 minutes to 2 hours to give a precipitate, which is used as a cell membrane fraction. This membrane fraction is rich in expressed GPR14 (SENR) and membrane components such as cellular phospholipids and membrane proteins.

The amount of GPR14 (SENR) in the GPR14 (SENR)-containing cells or the cell membrane fraction is preferably $10^3$ to $10^8$ molecules per cell, particularly $10^5$ to $10^7$ molecules per cell. A higher expression level results in a higher ligand-binding activity (specific activity) per membrane fraction, thus enabling not only construction of a high-sensitive screening system, but also measurement of large samples in a single lot.

In order to perform the above methods (I) to (III) for screening compounds capable of changing the binding property between GPR14 (SENR) and the polypeptide of the present invention or a precursor protein thereof, an appropriate GPR14 (SENR) fraction and the labeled polypeptide of the present invention or a labeled precursor protein thereof are used. For example, such a GPR14 (SENR) fraction is desirably a native GPR14 (SENR) fraction or a recombinant GPR14 (SENR) fraction having an equal activity to the native fraction. As used here, an equal activity refers to an equal ligand-binding activity etc. As a labeled ligand, for example, a labeled ligand or a labeled ligand analog compound is used. Examples include a ligand labeled with a radioactive isotope such as [$^3$H], [$^{125}$I], [$^{14}$C] or [$^{35}$S].

More specifically, in screening compounds capable of changing the binding property between GPR14 (SENR) and the polypeptide of the present invention or a precursor protein thereof, GPR14 (SENR)-containing cells or a cell membrane fraction thereof is first suspended in a buffer suitable for screening to prepare a receptor specimen. Any buffer of pH4 to pH10 (desirably pH6 to pH8) may be used as long as it dos not inhibit the binding between ligand and receptor, including phosphate buffer, Tris-HC1 buffer, etc. In addition, the buffer may be supplemented with a surfactant such as CHAPS, Tween-80™ (Kao Atlas, Co.), digitonin or deoxycholate for the purpose of reducing non-specific binding. Further, the buffer may be supplemented with a protease inhibitor such as PMSF, leupeptin, E-64 (prepared by Peptide Institute, Inc.) or pepstatin for the purpose of inhibiting protease-induced degradation of the receptor and the polypeptide of the present invention. An aliquot (5000 cpm to 500000 cpm) of the labeled polypeptide of the present invention is added to the receptor solution (0.01 ml to 10 ml) in the presence of a test compound at a concentration of $10^{-10}$ to $10^{-7}$ M. A reaction tube containing a large excess amount of the unlabeled polypeptide of the present invention is also provided in order to determine the level of non-specific binding (NSB). The reaction is carried out at 0°C to

50°C, desirably at 4°C to 37°C, for 20 minutes to 24 hours, desirably for 30 minutes to 3 hours. After the reaction, the reaction system is filtered on a glass fiber filter or the like and then washed with an appropriate volume of the same buffer, followed by measuring radioactivity remaining on the glass fiber filter using a liquid scintillation counter or a γ-counter. Assuming that a count ($B_0$-NSB) calculated by subtracting the count of non-specific binding (NSB) from the count in the absence of an antagonistic agent ($B_0$) is set to 100%, a compound showing a specific binding level (B-NSB) of, for example, 50% or below can be selected as a candidate substance capable of antagonistic inhibition.

In order to perform the above methods (IV) to (V) for screening compounds capable of changing the binding property between GPR14 (SENR) and the polypeptide of the present invention or a precursor protein thereof, GPR14 (SENR)-mediated cell-stimulating activities (e.g., promotional or inhibitory activities on arachidonate release, acetylcholine release, intracellular $Ca^{2+}$ release, intracellular cAMP production, intracellular cGMP production, inositol phosphate production, variation in cell membrane potential, intracellular protein phosphorylation, c-fos activation, pH decrease, etc.) may be measured in a known manner or using a commercially available assay kit. More specifically, GPR14 (SENR)-containing cells are first cultured in multi-well plates etc. Before starting the screening, the medium is replaced by fresh medium or an appropriate buffer which is not toxic to the cells. The cells are then incubated for a given period of time in the presence of a test compound and others, followed by extraction of the cells or collection of the supernatant to quantify the resulting product in a manner suitable for each case. If production of substances (e.g., arachidonate) used as indicators for cell-stimulating activities is difficult to assay due to the action of catabolic enzymes present in the cells, assays may be carried out in the presence of inhibitors against the catabolic enzymes. Likewise, activities including an inhibitory activity on cAMP production may be detected as inhibitory effects on production in cells pre-treated with forskolin or the like to enhance basic production in the cells.

To perform the screening based on assays of cell-stimulating activities, there is a need to use appropriate GPR14 (SENR)-expressing cells. Desirably, such GPR14 (SENR)-expressing cells used in the present invention may be the above-mentioned recombinant GPR14 (SENR)-expressing cell lines.

Examples of a test compound include peptides, proteins, non-peptide compounds, synthetic compounds, fermentation products, cell extracts, plant extracts and animal tissue extracts.

A kit for screening compounds or salts thereof capable of changing the binding property between GPR14 (SENR) and the polypeptide of the present invention or a precursor protein thereof comprises the polypeptide of the present invention or a precursor protein thereof and GPR14 (SENR) or a salt thereof, a partial peptide of GPR14 (SENR) or a salt thereof, GPR14 (SENR)-containing cells, or a cell membrane fraction of the GPR14 (SENR)-containing cells.

An example of the screening kit of the present invention will be presented below.

1. Screening reagents

(I) Assay buffer and washing buffer

Hanks' Balanced Salt Solution (Gibco) supplemented with 0.05% bovine serum albumin (Sigma). Hanks' Balanced Salt Solution (Gibco) supplemented with 0.05% bovine serum albumin (Sigma)

This buffer may be sterilized by filtration through a filter with a pore size of 0.45 μm and stored at 4°C until use, or it may be prepared *in situ.*

(II) GPR14 (SENR) specimen

GPR14 (SENR)-expressing CHO cells subcultured in 12-well plates at a density of $5 \times 10^5$ cells/well and incubated at 37°C under 5% $CO_2$ and 95% air for 2 days before use.

(III) Labeled ligand

The polypeptide of the present invention or a precursor protein thereof, labeled with a radioactive isotope such as [$^3H$], [$^{125}I$], [$^{14}C$] or [$^{35}S$].

The labeled ligand dissolved in an appropriate solvent or buffer is stored at 4°C or -20°C and diluted to 1 μM in the assay buffer before use.

(IV) Ligand standard solution

The polypeptide of the present invention or a precursor protein thereof is dissolved in PBS containing 0.1% bovine serum albumin (Sigma) to give a concentration of 1 mM and then stored at -20°C until use.

2. Assay procedures

(I) The GPR14 (SENR)-expressing cells cultured in 12-well tissue culture plates are washed twice with the assay buffer (1 ml), followed by addition of the assay buffer (490 μl) to each well.
(II) After addition of a test compound solution ($10^{-3}$ to $10^{-10}$ M; 5 μl), the labeled polypeptide of the present invention or precursor protein thereof (5 μl) is added and reacted at room temperature for 1 hour. In order to determine the level of non-specific binding, the polypeptide of the present invention or a precursor protein thereof ($10^{-3}$ M; 5 μl) is added instead of the test compound.
(III) The reaction solution is removed and the wells are washed three times with the washing buffer (1 ml). The labeled ligand molecules bound to the cells are solubilized with 0.2 N NaOH-1% SDS and mixed with 4 ml liquid scintillator A (Wako Pure Chemical Industries, Ltd.).
(IV) Radioactivity is measured using a liquid scintillation counter (manufactured by Beckman) and Percent Maximum Binding (PMB) is determined according to the following equation:
Equation

$$PMB = [(B-NSB)/(B_0-NSB)] \times 100$$

wherein

| | |
|---|---|
| PMB | Percent maximum binding |
| B | Count in the presence of an analyte |
| NSB | Non-specific binding (count for non-specific binding) |
| $B_0$ | Count for maximum binding. |

Compounds or salts thereof obtained by the screening method or screening kit of the present invention are capable of changing the binding property (i.e., inhibiting or promoting the binding) between GPR14 (SENR) and the polypeptide of the present invention or a precursor protein thereof. More specifically, they are compounds or salts thereof with GPR14 (SENR)-mediated cell-stimulating activities (so-called GPR14 (SENR) agonists) or compounds without such cell-stimulating activities (so-called GPR14 (SENR) antagonists). Examples of such compounds include peptides, proteins, non-peptide compounds, synthetic compounds and fermentation products, which may be novel or known.
Detailed procedures for evaluating whether a test compound is a member of the above GPR14 (SENR) agonists or antagonists may be as shown in (i) or (ii) below.

(i) Any one of binding assays shown in the above screening methods (I) to (III) is performed to obtain a compound capable of changing the binding property (particularly, inhibiting the binding) between GPR14 (SENR) and the polypeptide of the present invention or a precursor protein thereof. The compound is then assayed for whether it has the above-mentioned GPR14 (SENR)-mediated cell-stimulating activities. A compound or a salt thereof with the cell-stimulating activities is regarded as a GPR14 (SENR) agonist, whereas a compound or a salt thereof without the activities is regarded as a GPR14 (SENR) antagonist.
(ii) (a) A test compound is contacted with GPR14 (SENR)-containing cells and assayed for the above-mentioned GPR14 (SENR)-mediated cell-stimulating activities. A compound or a salt thereof with the cell-stimulating activities is regarded as a GPR14 (SENR) agonist. (b) The GPR14 (SENR)-mediated cell-stimulating activities are measured and compared between when a compound capable of activating GPR14 (SENR) (e.g., the polypeptide of the present invention, a precursor protein thereof or a SENR agonist) is contacted with GPR14 (SENR)-containing cells and when the compound capable of activating GPR14 (SENR) and a test compound are contacted with GPR14 (SENR)-containing cells. A compound or a salt thereof which can reduce the cell-stimulating activities induced by the compound capable of activating GPR14 (SENR) is regarded as a GPR14 (SENR) antagonist.

The GPR14 (SENR) agonist has the same physiological activities on GPR14 (SENR) as the polypeptide of the present invention or a precursor protein thereof; it is therefore useful as a safer and less toxic medicament, as in the case of the polypeptide of the present invention or a precursor protein thereof.
In contrast, the GPR14 (SENR) antagonist can inhibit physiological activities on GPR14 (SENR) found in the

polypeptide of the present invention; it is therefore useful as a safer and less toxic medicament for inhibition of the receptor's activities.

Since the polypeptide of the present invention or a precursor protein thereof is associated with an effect resembling anxiety stimulation, the GPR14 (SENR) agonist can be used, for example, as a therapeutic and/or prophylactic agent for attention deficit disorder or narcolepsy, whereas the GPR14 (SENR) antagonist can be used, for example, as a therapeutic and/or prophylactic agent for anxiety, depression, insomnia, schizophrenia or fear.

Compounds useful as GPR14 (SENR) antagonists, obtained by the screening method or screening kit of the present invention, include compounds of Formula (Ia):

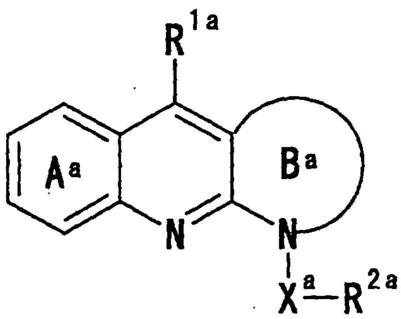

(Ia)

[wherein $A^a$ represents an optionally substituted benzene ring, $B^a$ represents an optionally substituted 5- to 8-membered ring, $X^a$ represents a divalent group containing 1 to 4 atoms in its linear chain moiety, $R^{1a}$ represents an optionally substituted amino group, and $R^{2a}$ represents an optionally substituted cyclic group] or salts thereof.

In the above formula, examples of a substituent which may be on the benzene ring in the "optionally substituted benzene ring" represented by $A^a$ include an optionally substituted hydrocarbon group, an optionally substituted heterocyclic group, a nitro group, a halogen atom, an optionally substituted amino group, a group of the formula $R^{4a}$-$Y^a$- (wherein $Y^a$ represents an oxygen atom or an optionally oxidized sulfur atom, and $R^{4a}$ represents an optionally substituted hydrocarbon group or an optionally substituted heterocyclic group), a cyano group, an optionally substituted acyl group, and a carboxyl group which may be esterified or amidated.

Examples of a "hydrocarbon group" in an "optionally substituted hydrocarbon group" as a substituent which may be on the benzene ring in the "optionally substituted benzene ring" represented by $A^a$ and a "hydrocarbon group" in the "optionally substituted hydrocarbon group" represented by $R^{4a}$ include:

(1) alkyls (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(2) cycloalkyls (e.g., $C_{3-8}$ cycloalkyls such as cyclopropyl, cyclobutyl, cyclopentyl, cyclohexyl and cycloheptyl), in which the cycloalkyl may be condensed with the benzene ring to form indan (e.g., indan-1-yl, indan-2-yl), tetrahydronaphthalene (e.g., tetrahydronaphthalen-5-yl, tetrahydronaphthalen-6-yl) or the like (preferably, e.g., indan) or the cycloalkyls may be bridged together via a linear atom chain containing 1 or 2 carbon atoms to form a bridged cyclic hydrocarbon residue such as bicyclo[2.2.1]heptyl, bicyclo[2.2.2]octyl, bicyclo[3.2.1]octyl or bicyclo[3.2.2]nonyl (preferably, e.g., cyclohexyls bridged together via a linear atom chain containing 1 or 2 carbon atoms, more preferably, e.g., bicyclo[2.2.1]heptyl);
(3) alkenyls (e.g., $C_{2-10}$ alkenyls such as vinyl, allyl, crotyl, 2-pentenyl and 3-hexenyl, preferably lower ($C_{2-6}$) alkenyls);
(4) cycloalkenyls (e.g., $C_{3-8}$ cycloalkenyls such as 2-cyclopentenyl, 2-cyclohexenyl, 2-cyclopentenylmethyl and 2-cyclohexenylmethyl);
(5) alkynyls (e.g., $C_{2-10}$ alkynyls such as ethynyl, 1-propynyl, 2-propynyl, 1-butynyl, 2-pentynyl and 3-hexynyl, preferably lower ($C_{2-6}$) alkynyls);
(6) aryls (e.g., $C_{6-14}$ aryls such as phenyl and naphthyl, preferably $C_{6-10}$ aryls, more preferably phenyl); and
(7) aralkyls (e.g., $C_{1-6}$ alkyls having 1 to 3 $C_{6-14}$ aryls, preferably phenyl-$C_{1-4}$ alkyls such as benzyl and phenethyl).
Above all, alkyls are preferred, $C_{1-4}$ alkyls such as methyl and ethyl are more preferred, and methyl is particularly preferred for use.

The hydrocarbon group may have a substituent(s). Examples of such a substituent include a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a mono-$C_{2-5}$ alkanoylamino, a 5- to 6-membered cyclic amino such as tetrahydropyrrole, piperazine, piperidine, morpholine, thiomorpholine, pyrrole or imidazole), a phenyl-lower ($C_{1-4}$) alkyl, a $C_{3-7}$ cycloalkyl, a carboxyl group which may be esterified or amidated (e.g., carboxyl, a $C_{1-4}$ alkoxy-carbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl) and a $C_{1-4}$ alkylsulfinyl (e.g., methanesulfinyl, ethanesulfinyl). The number of substituents is preferably 1 to 3.

Examples of a "heterocyclic group" in an "optionally substituted heterocyclic group" as a substituent which may be on the benzene ring in the "optionally substituted benzene ring" represented by $A^a$ and a "heterocyclic group" in the "optionally substituted heterocyclic group" represented by $R^{4a}$ include groups formed by removing one hydrogen atom from a 5- to 8-membered aromatic heterocyclic ring, a 5- to 8-membered saturated or unsaturated non-aromatic heterocyclic ring (aliphatic heterocyclic ring) or the like, each containing at least one heteroatom (preferably 1 to 4, more preferably 1 or 2 heteroatoms) selected from 1 to 3 kinds (preferably 1 or 2 kinds) of an oxygen atom, a sulfur atom, a nitrogen atom and the like.

Examples of the "aromatic heterocyclic ring" as used here include 5- to 8-membered (preferably 5- to 6-membered) aromatic monocyclic heterocyclic rings (e.g., furan, thiophene, pyrrole, oxazole, isooxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine). Examples of the "non-aromatic heterocyclic ring" as used here include 5- to 8-membered (preferably 5- to 6-membered) saturated or unsaturated monocyclic non-aromatic heterocyclic rings (aliphatic heterocyclic rings) such as pyrrolidine, tetrahydrofuran, tetrahydrothiophene, thiolane, dithiolane, oxathiolane, pyroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepine, thiepine and azepine, as well as 5- to 8-membered non-aromatic heterocyclic rings obtained by saturating part or all of double bonds in the above-mentioned aromatic monocyclic heterocyclic rings.

Alternatively, a "heterocyclic group" in an "optionally substituted heterocyclic group" as a substituent which may be on the benzene ring in the "optionally substituted benzene ring" represented by $A^a$ and a "heterocyclic group" in the "optionally substituted heterocyclic group" represented by $R^{4a}$ may each be a group formed by removing one hydrogen atom from a condensed ring formed by condensing 2 to 3 rings (preferably 2 rings) selected from the above-mentioned monocyclic heterocyclic rings (monocyclic aromatic heterocyclic rings and monocyclic non-aromatic heterocyclic rings) and 5- to 8-membered cyclic hydrocarbons (e.g., 5- to 8-membered (preferably 5-to 6-membered) saturated or unsaturated alicyclic hydrocarbons such as $C_{5-8}$ cycloalkanes, $C_{5-8}$ cycloalkenes and $C_{5-8}$ cycloalkadienes, 6-membered aromatic hydrocarbons such as benzene); such a condensed ring may be a saturated condensed ring, a partially unsaturated condensed ring or an aromatic condensed ring.

Preferred examples of such a condensed ring include a ring formed by condensing the same or different two heterocyclic rings (preferably one heterocyclic ring and one aromatic heterocyclic ring, more preferably the same or different two aromatic heterocyclic rings) and a ring formed by condensing one heterocyclic ring and one homocyclic ring (preferably one heterocyclic ring and one benzene ring, more preferably one aromatic heterocyclic ring and one benzene ring). Specific examples of such a condensed ring include indole, benzothiophene, benzofuran, benzimidazole, imidazo[1,2-a]pyridine, quinoline, isoquinoline and cinnoline.

A "heterocyclic group" in an "optionally substituted heterocyclic group" as a substituent which may be on the benzene ring in the "optionally substituted benzene ring" represented by $A^a$ and a "heterocyclic group" in the "optionally substituted heterocyclic group" represented by $R^{4a}$ may have a substituent(s). Examples of such a substituent include the same substituents as listed above for the "optionally substituted hydrocarbon group" as a substituent which may be on the benzene ring in the "optionally substituted benzene ring" represented by $A^a$.

Examples of a "halogen atom" as a substituent which may be on the benzene ring in the "optionally substituted benzene ring" represented by $A^a$ include fluorine, chlorine, bromine and iodine.

Examples of an "optionally substituted amino group" as a substituent which may be on the benzene ring in the "optionally substituted benzene ring" represented by $A^a$ include the same groups as listed below for the "optionally substituted amino group" represented by $R^{1a}$. Above all, preferred are amino groups which may have 1 or 2 substituents selected from an "optionally substituted hydrocarbon group" (e.g., the same groups as listed above for the "optionally substituted hydrocarbon group" as a substituent which may be on the benzene ring in the "optionally substituted benzene ring" represented by $A^a$), an "optionally substituted heterocyclic group" (e.g., the same groups as listed above for the "optionally substituted heterocyclic group" as a substituent which may be on the benzene ring in the "optionally substituted benzene ring" represented by $A^a$) and an "optionally substituted acyl group" (e.g., the same groups as listed below for the "optionally substituted acyl group" as a substituent which may be on the benzene ring in the "optionally substituted benzene ring" represented by $A^a$). Particularly preferred are amino groups which may have 1 or 2 optionally substituted alkyls [e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls, each of which

may have 1 to 3 substituents selected from a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a phenyl-lower ($C_{1-4}$) alkyl, a $C_{3-7}$ cycloalkyl, a carboxyl group which may be esterified or amidated (e.g., carboxyl, a $C_{1-4}$ alkoxycarbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl) and the like].

Alternatively, an "optionally substituted amino group" as a substituent which may be on the benzene ring in the "optionally substituted benzene ring" represented by $A^a$ may form a cyclic amino group through binding between substituents on the amino group (e.g., a cyclic amino group which is formed by removing one hydrogen atom from a ring-constituting nitrogen atom in a 5- to 6-membered ring such as tetrahydropyrrole, piperazine, piperidine, morpholine, thiomorpholine, pyrrole or imidazole and which has a binding hand on the nitrogen atom). The cyclic amino group may have a substituent(s). Examples of such a substituent include a halogen atom (e.g., fluorine, chlorine, bromine, iodine), nitro, cyano, a hydroxyl group, a thiol group, an amino group, a carboxyl group, an optionally halogenated $C_{1-4}$ alkyl (e.g., trifluoromethyl, methyl, ethyl), an optionally halogenated $C_{1-4}$ alkoxy (e.g., methoxy, ethoxy, propoxy, butoxy, trifluoromethoxy, trifluoroethoxy), formyl, a $C_{2-4}$ alkanoyl (e.g., acetyl, propionyl) and a $C_{1-4}$ alkylsulfonyl (e.g., methanesulfonyl, ethanesulfonyl). The number of substituents is preferably 1 to 3.

Examples of an "optionally substituted acyl group" as a substituent which may be on the benzene ring in the "optionally substituted benzene ring" represented by $A^a$ include a carbonyl or sulfonyl group attached to a hydrogen atom, an "optionally substituted hydrocarbon group" (e.g., the same groups as listed above for the "optionally substituted hydrocarbon group" as a substituent which may be on the benzene ring in the "optionally substituted benzene ring" represented by $A^a$), an "optionally substituted heterocyclic group" (e.g., the same groups as listed above for the "optionally substituted heterocyclic group" as a substituent which may be on the benzene ring in the "optionally substituted benzene ring" represented by $A^a$) or the like. Preferred examples include a carbonyl or sulfonyl group attached to:

(1) a hydrogen atom;
(2) an optionally substituted alkyl (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(3) an optionally substituted cycloalkyl (e.g., $C_{3-7}$ cycloalkyls such as cyclopropyl, cyclobutyl, cyclopentyl, cyclohexyl and cycloheptyl);
(4) an optionally substituted alkenyl (e.g., $C_{2-10}$ alkenyls such as allyl, crotyl, 2-pentenyl and 3-hexenyl, preferably lower ($C_{2-6}$) alkenyls);
(5) an optionally substituted cycloalkenyl (e.g., $C_{3-7}$ cycloalkenyls such as 2-cyclopentenyl, 2-cyclohexenyl, 2-cyclopentenylmethyl and 2-cyclohexenylmethyl);
(6) an optionally substituted 5- to 6-membered monocyclic aromatic group (e.g., phenyl, pyridyl) or the like (e.g., acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl). Examples of a substituent which may be on the above-mentioned (2) optionally substituted alkyl, (3) optionally substituted cycloalkyl, (4) optionally substituted alkenyl, (5) optionally substituted cycloalkenyl or (6) optionally substituted 5- to 6-membered monocyclic aromatic group include a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group which may be esterified or amidated (e.g., carboxyl, a $C_{1-4}$ alkoxycarbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), formyl, a $C_{2-4}$ alkanoyl (e.g., acetyl, propionyl), a $C_{1-4}$ alkylsulfonyl (e.g., methanesulfonyl, ethanesulfonyl) and a $C_{1-4}$ alkylsulfinyl (e.g., methanesulfinyl, ethanesulfinyl). The number of substituents is preferably 1 to 3.

Examples of a "carboxyl group which may be esterified" as a substituent which may be on the benzene ring in the "optionally substituted benzene ring" represented by $A^a$ include a carbonyloxy group attached to a hydrogen atom, an "optionally substituted hydrocarbon group" (e.g., the same groups as listed above for the "optionally substituted hydrocarbon group" as a substituent which may be on the benzene ring in the "optionally substituted benzene

ring" represented by $A^a$) or the like. Preferred examples include a carbonyloxy group attached to:

(1) a hydrogen atom;
(2) an optionally substituted alkyl (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(3) an optionally substituted cycloalkyl (e.g., $C_{3-7}$ cycloalkyls such as cyclopropyl, cyclobutyl, cyclopentyl, cyclohexyl and cycloheptyl);
(4) an optionally substituted alkenyl (e.g., $C_{2-10}$ alkenyls such as allyl, crotyl, 2-pentenyl and 3-hexenyl, preferably lower ($C_{2-6}$) alkenyls);
(5) an optionally substituted cycloalkenyl (e.g., $C_{3-7}$ cycloalkenyls such as 2-cyclopentenyl, 2-cyclohexenyl, 2-cyclopentenylmethyl and 2-cyclohexenylmethyl);
(6) an optionally substituted aryl (e.g., phenyl, naphthyl) or the like. More preferred examples include carboxyl, a lower ($C_{1-6}$) alkoxycarbonyl and an aryloxycarbonyl (e.g., methoxycarbonyl, ethoxycarbonyl, propoxycarbonyl, phenoxycarbonyl, naphthoxycarbonyl). Examples of a substituent which may be on the above-mentioned (2) optionally substituted alkyl, (3) optionally substituted cycloalkyl, (4) optionally substituted alkenyl, (5) optionally substituted cycloalkenyl or (6) optionally substituted aryl include a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group which may be esterified or amidated (e.g., carboxyl, a $C_{1-4}$ alkoxycarbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), formyl, a $C_{2-4}$ alkanoyl (e.g., acetyl, propionyl), a $C_{1-4}$ alkylsulfonyl (e.g., methanesulfonyl, ethanesulfonyl) and a $C_{1-4}$ alkylsulfinyl (e.g., methanesulfinyl, ethanesulfinyl). The number of substituents is preferably 1 to 3.

Examples of a "carboxyl group which may be amidated" as a substituent which may be on the benzene ring in the "optionally substituted benzene ring" represented by $A^a$ include a carbonyl group attached to:

(1) a hydroxyl group;
(2) an "optionally substituted amino group" (e.g., the same groups as listed above for the "optionally substituted amino group" as a substituent which may be on the benzene ring in the "optionally substituted benzene ring" represented by $A^a$) or the like.

The benzene ring in the "optionally substituted benzene ring" represented by $A^a$ may have the same or different 1 to 4 substituents (preferably 1 or 2 substituents) at any position on the ring. Also, in a case where the benzene ring in the "optionally substituted benzene ring" represented by $A^a$ has two or more substituents, two of these substituents may together form a lower ($C_{1-6}$) alkylene (e.g., trimethylene, tetramethylene), a lower ($C_{1-6}$) alkyleneoxy (e.g., $-CH_2-O-CH_2-$, $-O-CH_2-CH_2-$), a lower ($C_{1-6}$) alkylenedioxy (e.g., $-O-CH_2-O-$, $-O-CH_2-CH_2-O-$), a lower ($C_{2-6}$) alkenylene (e.g., $-CH_2-CH=CH-$, $-CH_2-CH_2-CH=CH-$, $-CH_2-CH=CH-CH_2-$), a lower ($C_{4-6}$) alkadienylene (e.g., $-CH=CH-CH=CH-$) or the like.

Preferred examples of a substituent which may be on the benzene ring in the "optionally substituted benzene ring" represented by $A^a$ include an optionally substituted hydrocarbon group, an optionally substituted heterocyclic group, a nitro group, a halogen atom, an optionally substituted amino group, and a group of the formula $R^{4a}$-$Y^a$- (wherein $Y^a$ represents an oxygen atom or an optionally oxidized sulfur atom, and $R^{4a}$ represents an optionally substituted hydrocarbon group or an optionally substituted heterocyclic group). More preferred examples include an optionally substituted hydrocarbon group, an optionally substituted heterocyclic group, a halogen atom, an optionally substituted amino group, and a group of the formula $R^{4a}$-$Y^a$-(wherein $Y^a$ represents an oxygen atom or an optionally oxidized sulfur atom, and $R^{4a}$ represents an optionally substituted hydrocarbon group or an optionally substituted heterocyclic group), with a lower ($C_{1-4}$) alkyl, a halogen atom and the like being particularly preferred.

Thus, the "optionally substituted benzene ring" represented by $A^a$ is preferably a benzene ring of the following formula:

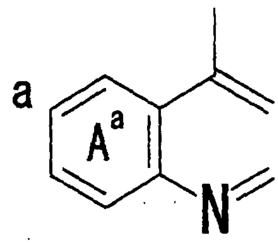

which has at least one substituent at the position "a" on the benzene ring, particularly preferably a benzene ring of the following formula:

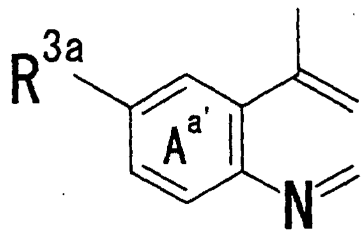

[wherein $A^{a,}$ represents a benzene ring which may have a substituent in addition to the substituent $R^{3a}$, and $R^{3a}$ represents an optionally substituted hydrocarbon group, an optionally substituted heterocyclic group, a nitro group, a halogen atom, an optionally substituted amino group or a group of the formula $R^{4a}$-$Y^{a}$- (wherein $Y^a$ represents an oxygen atom or an optionally oxidized sulfur atom, and $R^{4a}$ represents an optionally substituted hydrocarbon group or an optionally substituted heterocyclic group)], and most particularly preferably a benzene ring of the following formula:

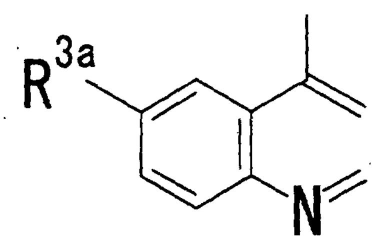

[wherein $R^{3a}$ is as defined above]. In the above formulae, preferred for $R^{3a}$ is an optionally substituted hydrocarbon group, an optionally substituted heterocyclic group, a halogen atom, an optionally substituted amino group, or a group of the formula $R^{4a}$-$Y^{a}$- (wherein $Y^a$ represents an oxygen atom or an optionally oxidized sulfur atom, and $R^{4a}$ represents an optionally substituted hydrocarbon group or an optionally substituted heterocyclic group). Above all, particularly preferred is an optionally substituted hydrocarbon group, an optionally substituted heterocyclic group, a halogen atom or the like, and most particularly preferred is an optionally substituted lower alkyl group or a halogen atom.

In the above formula, examples of the "optionally substituted 5- to 8-membered ring" represented by $B^a$ include a 5- to 8-membered saturated ring of the following formula:

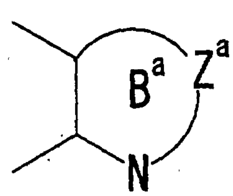

[wherein $Z^a$ represents a saturated divalent group such that the ring $B^a$ may form an optionally substituted 5- to 8-membered saturated ring], which may have a substituent(s) at any substitutable position. Such a 5- to 8-membered saturated ring may be partially unsaturated or may form an aromatic ring. The ring $B^a$ is preferably an optionally substituted 5-

to 8-membered saturated ring.

As used herein, a "5- to 8-membered saturated ring" in the "optionally substituted 5- to 8-membered saturated ring" as the ring $B^a$ is intended to mean a "5- to 8-membered ring in which all of the bonds constituting the ring $B^a$ are saturated single bonds, except for the double bond at the site of condensation between the ring $B^a$ and the quinoline ring." A "5- to 8-membered unsaturated ring" in the "optionally substituted 5- to 8-membered unsaturated ring" as the ring $B^a$ is intended to mean a "5-to 8-membered ring in which at least one of the bonds constituting the ring $B^a$ is an unsaturated bond, except for the double bond at the site of condensation between the ring $B^a$ and the quinoline ring."

In the above formula, the saturated divalent group represented by $Z^a$ may be any group as long as the ring $B^a$ may form an optionally substituted 5- to 8-membered saturated ring. Namely, $Z^a$ may be any saturated divalent group containing 2 to 5 atoms in its linear chain moiety (preferably, any saturated divalent hydrocarbon group containing 2 to 5 atoms in its linear chain moiety). Specific examples include:

(1) $-(CH_2)_{a1}-$ (wherein a1 represents an integer of 2 to 5);
(2) $-(CH_2)_{b1}-Z^{a1}-(CH_2)_{b2}-$ (wherein b1 and b2, which may be the same or different, each represent an integer of 0 to 4, provided that the sum of b1 and b2 is 1 to 4, and $Z^1$ represents NH, O, S, SO or $SO_2$);
(3) $-(CH_2)_{d1}-Z^{a1}-(CH_2)_{d2}-Z^{a2}-(CH_2)_{d3}-$ (wherein d1, d2 and d3, which may be the same or different, each represent an integer of 0 to 3, provided that the sum of d1, d2 and d3 is 0 to 3, and $Z^{a1}$ and $Z^{a2}$ each represent NH, O, S, SO or $SO_2$); and
(4) $-(CH_2)_{e1}-Z^{a1}-(CH_2)_{e2}-Z^{a2}-(CH_2)_{e3}-Z^{a3}-(CH_2)_{e4}-$ (wherein e1, e2, e3 and e4, which may be the same or different, each represent an integer of 0 to 2, provided that the sum of d1, d2 and d3 is 0 to 2, and $Z^{a1}$, $Z^{a2}$ and $Z^{a3}$ each represent NH, O, S, SO or $SO_2$); with $-(CH_2)_{a1}-$ (wherein al represents an integer of 2 to 5) being preferred. More specific examples include divalent groups such as $-O-(CH_2)_{k1}-$ (wherein k1 represents an integer of 1 to 4), $-(CH_2)_{k1}-O-$ (wherein k1 represents an integer of 1 to 4), $-S-(CH_2)_{k1}-$ (wherein k1 represents an integer of 1 to 4), $-(CH_2)_{k1}-S-$ (wherein k1 represents an integer of 1 to 4), $-NH-(CH_2)_{k1}-$ (wherein k1 represents an integer of 1 to 4), $-(CH_2)_{k1}-NH-$ (wherein k1 represents an integer of 1 to 4), $-(CH_2)_{k2}-$ (wherein k2 represents an integer of 2 to 5), -NH-NH-, $-CH_2-NH-NH-$, $-NH-NH-CH_2-$ and $-NH-CH_2-NH-$.

In the above formula, the "optionally substituted 5- to 8-membered ring" represented by $B^a$ may be not only an "optionally substituted 5- to 8-membered saturated ring" as illustrated above, but also an "optionally substituted 5- to 8-membered unsaturated ring" whose bonds are partially unsaturated or an "optionally substituted 5- to 8-membered aromatic ring." In such a case, $Z^a$ in the ring of the formula:

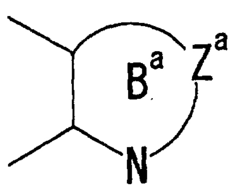

may represent a divalent group obtained by partial unsaturation of the "saturated divalent group containing 2 to 5 atoms in its linear chain moiety" as illustrated above.

In addition, the divalent group may have a substituent(s). Such a substituent may be any group as long as it can be attached to the divalent group, including the same substituents as listed above for the "optionally substituted benzene ring" represented by $A^a$ and an oxo group. The divalent group may have the same or different 1 to 4 substituents (preferably 1 or 2 substituents) at any position. Also, in a case where the divalent group has two or more substituents, two of these substituents may together form a lower ($C_{1-6}$) alkylene (e.g., trimethylene, tetramethylene), a lower ($C_{1-6}$) alkyleneoxy (e.g., $-CH_2-O-CH_2-$, $-O-CH_2-CH_2-$), a lower ($C_{1-6}$) alkylenedioxy (e.g., $-O-CH_2-O-$, $-O-CH_2-CH_2-O-$), a lower ($C_{2-6}$) alkenylene (e.g., $-CH_2-CH=CH-$, $-CH_2-CH_2-CH=CH-$, $-CH_2-CH=CH-CH_2-$), a lower ($C_{4-6}$) alkadienylene (e.g., -CH=CH-CH=CH-) or the like.

In the above formula, examples of the "divalent group containing 1 to 4 atoms in its linear chain moiety" represented by $X^a$ include saturated divalent groups such as:

(1) $-(CH_2)_n-$ (wherein f1 represents an integer of 1 to 4);
(2) $-(CH_2)_{g1}-X^{a1}-(CH_2)_{g2}-$ (wherein g1 and g2, which may be the same or different, each represent an integer of 0 to 3, provided that the sum of g1 and g2 is 1 to 3, and $X^{a1}$ represents NH, O, S, SO or $SO^2$); and
(3) $-(CH_2)_{h1}-X^{a1}-(CH_2)_{h2}-X^{a2}-(CH_2)_{h3}-$ (wherein h1, h2 and h3, which may be the same or different, each represent an integer of 0 to 2, provided that the sum of h1, h2 and h3 is 0 to 2, and $X^{a1}$ and $X^{a2}$ each represent NH, O, S,

SO or $SO_2$, provided that when h2 is 0, at least one of $X^{a1}$ and $X^{a2}$ preferably represents NH); as well as divalent groups obtained by partial unsaturation of these groups. Specific examples include divalent groups such as $-O-(CH_2)_{k3}-$ (wherein k3 represents an integer of 1 to 3), $-(CH_2)_{k3}-O-$ (wherein k3 represents an integer of 1 to 3), $-S-(CH_2)_{k3}-$ (wherein k3 $-NH-(CH_2)_{k3}-$ (wherein k3 represents an integer of 1 to 3), $-(CH_2)_{k3}-NH-$ (wherein k3 represents an integer of 1 to 3), $-(CH_2)_{k3}-S-$ (wherein k3 represents an integer of 1 to 3), represents an integer of 1 to 3), $-(CH_2)_{k4}-$ (wherein k4 represents an integer of 1 to 4), -CH=CH-, -C≡C-, -CO-NH- and $-SO_2-NH-$.

$X^a$ is preferably a divalent group excluding $-CO-O-CH_2-$, more preferably a divalent group containing 1 to 4 carbon atoms in its linear chain moiety, and particularly preferably a $C_{1-4}$ alkylene, a $C_{2-4}$ alkenylene, etc. Above all, a $C_{1-4}$ alkylene, especially methylene, is preferred for use.
The divalent group represented by $X^a$ may have a substituent(s) at any position (preferably, on its carbon atom(s)). Such a substituent may be any group as long as it can be attached to the divalent chain constituting the linear chain moiety, including the same substituents as listed above for the "optionally substituted benzene ring" represented by $A^a$ and an oxo group. The divalent group may have the same or different 1 to 4 substituents (preferably 1 or 2 substituents) at any position.
Examples of a preferred substituent which may be on the divalent group represented by $X^a$ include a lower ($C_{1-6}$) alkyl (e.g., methyl, ethyl, propyl, isopropyl, butyl, isobutyl, sec-butyl, tert-butyl, pentyl, isopentyl, neopentyl, hexyl), a lower ($C_{3-7}$) cycloalkyl (e.g., cyclopropyl, cyclobutyl, cyclopentyl, cyclohexyl, cycloheptyl), formyl, a lower ($C_{2-7}$) alkanoyl (e.g., acetyl, propionyl, butyryl), a lower ($C_{2-7}$) alkoxy-carbonyl, a lower ($C_{1-6}$) alkoxy, a hydroxyl group and oxo.
In the above formula, examples of the "optionally substituted amino group" represented by $R^{1a}$ include amino groups which may have 1 or 2 substituents selected from an "optionally substituted hydrocarbon group" (e.g., the same groups as listed above for the "optionally substituted hydrocarbon group" as a substituent which may be on the benzene ring in the "optionally substituted benzene ring" represented by $A^a$), an "optionally substituted heterocyclic group" (e.g., the same groups as listed above for the "optionally substituted heterocyclic group" as a substituent which may be on the benzene ring in the "optionally substituted benzene ring" represented by $A^a$) and an "optionally substituted acyl group" (e.g., the same groups as listed above for the "optionally substituted acyl group" as a substituent which may be on the benzene ring in the "optionally substituted benzene ring" represented by $A^a$). Alternatively, the "optionally substituted amino group" represented by $R^{1a}$ may form a cyclic amino group through binding between substituents on the amino group (e.g., a cyclic amino group which is formed by removing one hydrogen atom from a ring-constituting nitrogen atom in a 5- to 6-membered ring such as tetrahydropyrrole, piperazine, piperidine, morpholine, thiomorpholine, pyrrole or imidazole and which has a binding hand on the nitrogen atom). The cyclic amino group may have a substituent (s). Examples of such a substituent include a halogen atom (e.g., fluorine, chlorine, bromine, iodine), nitro, cyano, a hydroxyl group, a thiol group, an amino group, a carboxyl group, an optionally halogenated $C_{1-4}$ alkyl (e.g., trifluoromethyl, methyl, ethyl), an optionally halogenated $C_{1-4}$ alkoxy (e.g., methoxy, ethoxy, propoxy, butoxy, trifluoromethoxy, trifluoroethoxy), formyl, a $C_{2-4}$ alkanoyl (e.g., acetyl, propionyl) and a $C_{1-4}$ alkylsulfonyl (e.g., methanesulfonyl, ethanesulfonyl). The number of substituents is preferably 1 to 3.
Preferred examples of a substituent on the amino group in the "optionally substituted amino group" represented by $R^{1a}$ include:

(1) an optionally substituted alkyl (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(2) an optionally substituted cycloalkyl (e.g., $C_{3-8}$ cycloalkyls such as cyclopropyl, cyclobutyl, cyclopentyl, cyclohexyl, cycloheptyl and cyanooctyl), in which the cycloalkyl may be condensed with the benzene ring to form indan (e.g., indan-1-yl, indan-2-yl), tetrahydronaphthalene (e.g., tetrahydronaphthalen-5-yl, tetrahydronaphthalen-6-yl) or the like (preferably, e.g., indan) or the cycloalkyls may be bridged together via a linear atom chain containing 1 or 2 carbon atoms to form a bridged cyclic hydrocarbon residue such as bicyclo[2.2.1]heptyl, bicyclo[2.2.2]octyl, bicyclo[3.2.1]octyl or bicyclo[3.2.2]nonyl (preferably, e.g., cyclohexyls bridged together via a linear atom chain containing 1 or 2 carbon atoms, more preferably, e.g., bicyclo[2.2.1]heptyl);
(3) an optionally substituted alkenyl (e.g., $C_{2-10}$ alkenyls such as allyl, crotyl, 2-pentenyl and 3-hexenyl, preferably lower ($C_{2-6}$) alkenyls);
(4) an optionally substituted cycloalkenyl (e.g., $C_{3-7}$ cycloalkenyls such as 2-cyclopentenyl, 2-cyclohexenyl, 2-cyclopentenylmethyl and 2-cyclohexenylmethyl);
(5) an optionally substituted aralkyl (e.g., phenyl-$C_{1-4}$ alkyls such as benzyl and phenethyl);
(6) formyl or an optionally substituted acyl (e.g., $C_{2-4}$ alkanoyls such as acetyl, propionyl, butyryl and isobutyryl, $C_{1-4}$ alkylsulfonyls such as methanesulfonyl and ethanesulfonyl);
(7) an optionally substituted aryl (e.g., phenyl, naphthyl); and
(8) an optionally substituted heterocyclic group (e.g., a group formed by removing one hydrogen atom from a 5- to 6-membered aromatic heterocyclic ring containing 1 to 4 heteroatoms selected from 1 or 2 kinds of a nitrogen

atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine or triazole, a group formed by removing one hydrogen atom from a 5- to 6-membered non-aromatic heterocyclic ring containing 1 to 4 heteroatoms selected from 1 or 2 kinds of a nitrogen atom, a sulfur atom and an oxygen atom, such as tetrahydrofuran, tetrahydrothiophene, dithiolane, oxathiolane, pyrrolidine, pyroline, imidazolidine, imidazoline, pyrazolidine, pyrazoline, piperidine, piperazine, oxazine, oxadiazine, thiazine, thiadiazine, morpholine, thiomorpholine, pyran or tetrahydropyran).

Examples of a substituent which may be on the above-mentioned (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, (7) optionally substituted aryl or (8) optionally substituted heterocyclic group include a halogen atom (e.g., fluorine, chlorine, bromine, iodine), a $C_{1-4}$ alkyl which may be substituted with a halogen atom or a $C_{1-4}$ alkoxy, a $C_{1-4}$ alkoxy which may be substituted with a halogen atom or a $C_{1-4}$ alkoxy (e.g., methoxy, ethoxy, propoxy, butoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl), a phenyl-lower ($C_{1-4}$) alkyl, a $C_{3-7}$ cycloalkyl, cyano, nitro, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group, a lower ($C_{1-4}$) alkoxy-carbonyl, a lower ($C_{7-10}$) aralkyloxy-carbonyl, carbamoyl, a mono-$C_{1-4}$ alkylcarbamoyl and a di-$C_{1-4}$ alkylcarbamoyl (preferably, e.g., a halogen atom, an optionally halogenated lower ($C_{1-4}$) alkyl, an optionally halogenated lower ($C_{1-4}$) alkoxy, a phenyl-lower ($C_{1-4}$) alkyl, a $C_{3-7}$ cycloalkyl, cyano, a hydroxyl group). The number of substituents is preferably 1 to 3.

Above all, the "optionally substituted amino group" represented by $R^{1a}$ is preferably an amino group which may have 1 or 2 optionally substituted alkyls [e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls, each of which may have 1 to 3 substituents selected from a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group which may be esterified or amidated (e.g., carboxyl, a $C_{1-4}$ alkoxycarbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), a phenyl-lower ($C_{1-4}$) alkyl, a $C_{3-7}$ cycloalkyl, formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl) and the like].

In the above formula, examples of a "cyclic group" in the "optionally substituted cyclic group" represented by $R^{2a}$ include 5- to 8-membered (preferably 5- to 6-membered) saturated or unsaturated alicyclic monocyclic hydrocarbons such as $C_{5-8}$ cycloalkanes (e.g., cyclopentane, cyclohexane, cycloheptane), $C_{5-8}$ cycloalkenes (e.g., 1-cyclopentene, 2-cyclopentene, 3-cyclopentene, 2-cyclohexene, 3-cyclohexene), $C_{5-8}$ cycloalkadienes (e.g., 2,4-cyclopentadiene, 2,4-cyclohexadiene, 2,5-cyclohexadiene); 6-membered aromatic monocyclic hydrocarbons such as benzene; and 5- to 8-membered aromatic monocyclic heterocyclic rings, 5- to 8-membered saturated or unsaturated non-aromatic monocyclic heterocyclic rings (aliphatic heterocyclic rings) or the like, each containing at least one heteroatom (preferably 1 to 4, more preferably 1 or 2 heteroatoms) selected from 1 to 3 kinds (preferably 1 or 2 kinds) of an oxygen atom, a sulfur atom, a nitrogen atom and the like; as well as groups formed by removing one hydrogen atom from a condensed ring formed by condensing the same or different 2 to 3 rings selected from these monocyclic rings.

Examples of the "aromatic monocyclic heterocyclic ring" as used here include 5- to 8-membered (preferably 5- to 6-membered) aromatic monocyclic heterocyclic rings (e.g., furan, thiophene, pyrrole, oxazole, isooxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine). Examples of the "non-aromatic monocyclic heterocyclic ring" as used here include 5- to 8-membered (preferably 5- to 6-membered) saturated or unsaturated monocyclic non-aromatic heterocyclic rings (aliphatic heterocyclic rings) such as pyrrolidine, tetrahydrofuran, tetrahydrothiophene, thiolane, dithiolane, oxathiolane, pyroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepine, thiepine and azepine, as well as 5- to 8-membered non-aromatic heterocyclic rings obtained by saturating part or all of double bonds in the above-mentioned aromatic monocyclic heterocyclic rings.

Alternatively, a "cyclic group" in the "optionally substituted cyclic group" represented by $R^{2a}$ may be a group formed by removing one hydrogen atom from a condensed ring formed by condensing the same or different 2 to 3

rings (preferably 2 rings) selected from the monocyclic homocyclic or heterocyclic rings as listed above; such a condensed ring may be a saturated condensed ring, a partially unsaturated condensed ring or an aromatic condensed ring.

Preferred examples of such a condensed ring include a ring formed by condensing the same or different two heterocyclic rings (preferably one heterocyclic ring and one aromatic heterocyclic ring, more preferably the same or different two aromatic heterocyclic rings) and a ring formed by condensing one heterocyclic ring and one homocyclic ring (preferably one heterocyclic ring and one benzene ring, more preferably one aromatic heterocyclic ring and one benzene ring). Specific examples of such a condensed ring include indole, benzothiophene, benzofuran, benzimidazole, imidazo[1,2-a]pyridine, quinoline, isoquinoline and cinnoline.

Examples of a substituent which may be on the "cyclic group" in the "optionally substituted cyclic group" represented by $R^{2a}$ include the same substituents as listed above for the "optionally substituted hydrocarbon group" as a substituent which may be on the benzene ring in the "optionally substituted benzene ring" represented by $A^a$.

The "cyclic group" in the "optionally substituted cyclic group" represented by $R^{2a}$ is preferably a 5- to 6-membered cyclic group, more preferably a 5- to 6-membered aromatic ring group, even more preferably phenyl, furyl, thienyl, pyrrolyl, pyridyl (preferably, a 6-membered ring) or the like, and particularly preferably phenyl.

Among compounds of Formula (Ia) or salts thereof, preferred for use are a compound of Formula (IIa):

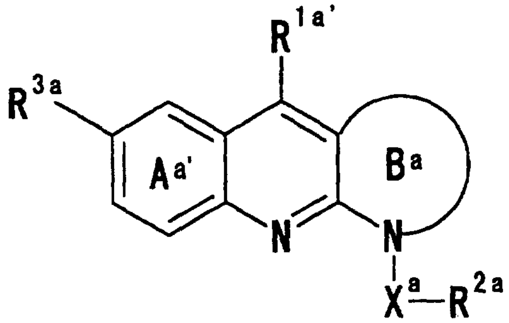

(IIa)

[wherein $A^{a'}$ represents a benzene ring which may have a substituent in addition to the substituent $R^{3a}$, $B^a$ represents an optionally substituted 5- to 8-membered ring, $X^a$ represents a divalent group containing 1 to 4 atoms in its linear chain moiety, $R^{1a'}$ represents an amino group substituted with 1 or 2 optionally substituted lower alkyl groups, $R^{2a}$ represents an optionally substituted cyclic group, and $R^{3a}$ represents an optionally substituted hydrocarbon group, an optionally substituted heterocyclic group, a nitro group, a halogen atom, an optionally substituted amino group or a group of the formula $R^{4a}$-$Y^a$- (wherein $Y^a$ represents an oxygen atom or an optionally oxidized sulfur atom, and $R^{4a}$ represents an optionally substituted hydrocarbon group or an optionally substituted heterocyclic group)] or a salt thereof and a compound of Formula (IIa'):

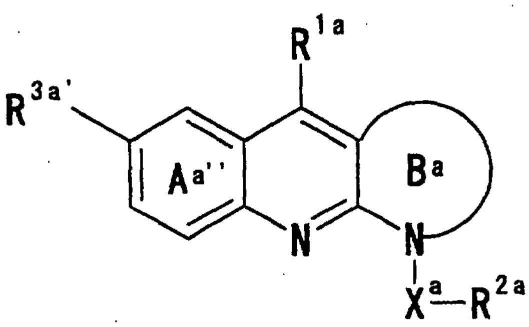

(IIa')

[wherein $A^{a''}$ represents a benzene ring which may have a substituent in addition to the substituent $R^{3a'}$, $B^a$ represents an optionally substituted 5- to 8-membered ring, $X^a$ represents a divalent group containing 1 to 4 atoms in its linear chain moiety, $R^{1a}$ represents an optionally substituted amino group, $R^{2a}$ represents an optionally substituted cyclic group, and $R^{3a'}$ represents an optionally substituted hydrocarbon group, an optionally substituted heterocyclic group, a halogen atom, an optionally substituted amino group or a group of the formula $R^{4a}$-$Y^a$- (wherein $Y^a$ represents an oxygen atom or an optionally oxidized sulfur atom, and $R^{4a}$ represents an optionally substituted hydrocarbon group or an optionally substituted heterocyclic group)] or a salt thereof.

In the above formulae, the "benzene ring" in the "benzene ring which may have a substituent in addition to the substituent $R^{3a}$" represented by $A^{a'}$ and the "benzene ring which may have a substituent in addition to the substituent $R^{3a'}$" represented by $A^{a''}$ may each have the same substituent(s) as listed above for the "optionally substituted benzene ring" represented by $A^a$, in addition to the substituent $R^{3a}$.

In the above formula, examples of the "substituted amino group" represented by $R^{1a'}$ include the amino groups (excluding unsubstituted one) listed above for the "optionally substituted amino group" represented by $R^{1a}$, i.e., amino groups having the same or different 1 or 2 substituents selected from the same substituents as listed above for the "optionally substituted amino group" represented by $R^{1a}$. Above all, an "amino group substituted with 1 or 2 optionally substituted lower alkyl groups" is preferred.

Examples of such an "amino group substituted with 1 or 2 optionally substituted lower alkyl groups" include amino groups substituted with 1 or 2 lower ($C_{1-6}$) alkyls (e.g., methyl, ethyl, propyl, isopropyl, butyl, isobutyl, sec-butyl, tert-butyl, pentyl, isopentyl, neopentyl, hexyl) which may have 1 to 3 substituents selected from:

(1) a halogen atom (e.g., fluorine, chlorine, bromine, iodine),
(2) nitro,
(3) cyano,
(4) a hydroxyl group,
(5) an optionally substituted thiol group (e.g., thiol, a $C_{1-4}$ alkylthio),
(6) an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole),
(7) a carboxyl group which may be esterified or amidated (e.g., carboxyl, a $C_{1-4}$ alkoxycarbonyl, a lower ($C_{7-10}$) aralkyloxy-carbonyl, carbamoyl, a mono-$C_{1-4}$ alkylcarbamoyl, a di-$C_{1-4}$ alkylcarbamoyl),
(8) a $C_{1-4}$ alkyl which may be substituted with a halogen atom or a $C_{1-4}$ alkoxy (e.g., trifluoromethyl, methyl, ethyl),
(9) a $C_{1-4}$ alkoxy which may be substituted with a halogen atom or a $C_{1-4}$ alkoxy (e.g., methoxy, ethoxy, trifluoromethoxy, trifluoroethoxy),
(10) a $C_{1-4}$ alkylenedioxy (e.g., $-O-CH_2-O-$, $-O-CH_2-CH_2-O-$),
(11) a phenyl-lower ($C_{1-4}$) alkyl,
(12) a $C_{3-7}$ cycloalkyl, formyl, a $C_{2-4}$ alkanoyl (e.g., acetyl, propionyl),
(13) a $C_{1-4}$ alkylsulfonyl (e.g., methanesulfonyl, ethanesulfonyl),
(14) a $C_{1-4}$ alkylsulfinyl (e.g., methanesulfinyl, ethanesulfinyl) and the like. If such an amino group has two substituents, they may be the same or different.

In the above formulae, examples of the "optionally substituted hydrocarbon group" represented by $R^{3a}$ and $R^{3a'}$ include the same groups as listed above for the "optionally substituted hydrocarbon group" as a substituent which may be on the benzene ring in the "optionally substituted benzene ring" represented by $A^a$.

In the above formulae, examples of the "optionally substituted heterocyclic group" represented by $R^{3a}$ and $R^{3a'}$ include the same groups as listed above for the "optionally substituted heterocyclic group" as a substituent which may be on the benzene ring in the "optionally substituted benzene ring" represented by $A^a$.

In the above formulae, examples of the "optionally substituted amino group" represented by $R^{3a}$ and $R^{3a'}$ include the same groups as listed above for the "optionally substituted amino group" as a substituent which may be on the benzene ring in the "optionally substituted benzene ring" represented by $A^a$.

In the above formulae, examples of the "optionally substituted hydrocarbon group" or "optionally substituted heterocyclic group" represented by $R^{4a}$ in the group of the formula $R^{4a}$-$Y^a$- include the same groups as listed above for the "optionally substituted hydrocarbon group" or "optionally substituted heterocyclic group" as a substituent which may be on the benzene ring in the "optionally substituted benzene ring" represented by $A^a$.

In the above formulae, examples of the "optionally oxidized sulfur atom" represented by $Y^a$ in the group of the formula $R^{4a}$-$Y^a$- include S, S(O) and $S(O)_2$.

A compound of Formula (Ia) or a salt thereof may be prepared in a well-known manner. Alternatively, a compound of Formula (Ia) or a salt thereof may be prepared, for example, according to the procedures shown below or described in, e.g., Tetrahedron Lett., vol. 40, pp. 5643-5646, JP-A-3-220189 or JP-B-48-30280 or equivalent procedures.

Compounds used in the respective procedures shown below may be in the same salt form as Compound (Ia) as long as they do not adversely affect the reaction.

Likewise, in each reaction shown below, when a starting compound has an amino group, a carboxyl group or a hydroxyl group as a substituent, it may have an introduced protecting group(s) such as those commonly used for these substituents in peptide chemistry or elsewhere. Such a protecting group(s) may be removed after the reaction, when needed, to give a compound of interest.

Protecting groups used for an amino group include, for example, an optionally substituted $C_{1-6}$ alkylcarbonyl (e.g., acetyl, propionyl), formyl, phenylcarbonyl, a $C_{1-6}$ alkyloxycarbonyl (e.g., methoxycarbonyl, ethoxycarbonyl, t-butoxycarbonyl), phenyloxycarbonyl (e.g., benzoxycarbonyl), a $C_{7-10}$ aralkyloxycarbonyl (e.g., benzyloxycarbonyl), trityl and phthaloyl. These protecting groups may have around 1 to 3 substituents such as a halogen atom (e.g., fluorine, chlorine, bromine, iodine), a $C_{1-6}$ alkylcarbonyl (e.g., acetyl, propionyl, butyryl) or a nitro group.

Protecting groups used for a carboxyl group include, for example, an optionally substituted $C_{1-6}$ alkyl (e.g., methyl, ethyl, propyl, isopropyl, butyl, tert-butyl), phenyl, trityl and silyl. These protecting groups may have around 1 to 3 substituents such as a halogen atom (e.g., fluorine, chlorine, bromine, iodine), a $C_{1-6}$ alkylcarbonyl (e.g., acetyl, propionyl, butyryl), formyl or a nitro group.

Protecting groups used for a hydroxyl group include, for example, an optionally substituted $C_{1-6}$ alkyl (e.g., methyl, ethyl, propyl, isopropyl, butyl, tert-butyl), phenyl, a $C_{7-10}$ aralkyl (e.g., benzyl), a $C_{1-6}$ alkylcarbonyl (e.g., acetyl, propionyl), formyl, phenyloxycarbonyl, a $C_{7-10}$ aralkyloxycarbonyl (e.g., benzyloxycarbonyl), pyranyl, furanyl and silyl. These protecting groups may have around 1 to 4 substituents such as a halogen atom (e.g., fluorine, chlorine, bromine, iodine), a $C_{1-6}$ alkyl, phenyl, a $C_{7-10}$ aralkyl or a nitro group.

Although introduction and removal of protecting groups may be carried out according to well-known procedures or equivalents thereof [see, e.g., Protective Groups in Organic Chemistry (J.F.W. McOmie et al., Prenum Press)], protecting groups may be removed by treatment with, e.g., an acid, a base, reduction, ultraviolet radiation, hydrazine, phenylhydrazine, sodium N-methyldithiocarbamate, tetrabutylammonium fluoride and/or palladium acetate.

Preparation procedures

Among compounds of Formula (Ia) or salts thereof, a compound of Formula (Iaa) having an unsubstituted amino group as $R^{1a}$ or a salt thereof may be prepared, for example, according to the following scheme:

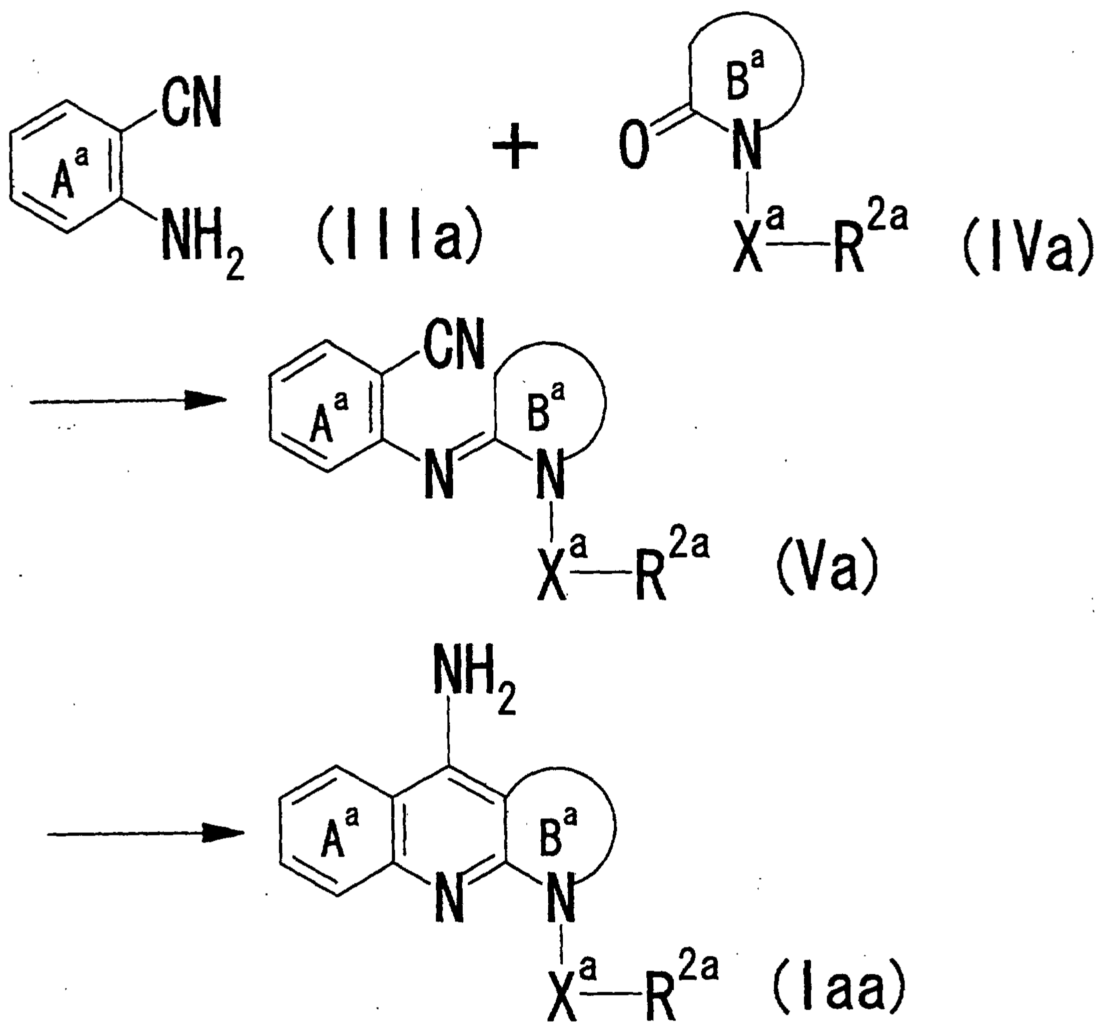

(IIIa)

(IVa)

(Va)

(Iaa)

[wherein each symbol is as defined above].

According to the procedures as described in JP-A-3-220189 or JP-B-48-30280 or equivalent procedures, a compound of Formula (IIIa) or a salt thereof is reacted with a compound of Formula (IVa) or a salt thereof to give a compound of Formula (Va) or a salt thereof, which in turn is subjected to cyclization to give the compound of Formula (Iaa) or salt thereof.

A compound of Formula (Ia) or a salt thereof may be prepared, for example, according to the following scheme:

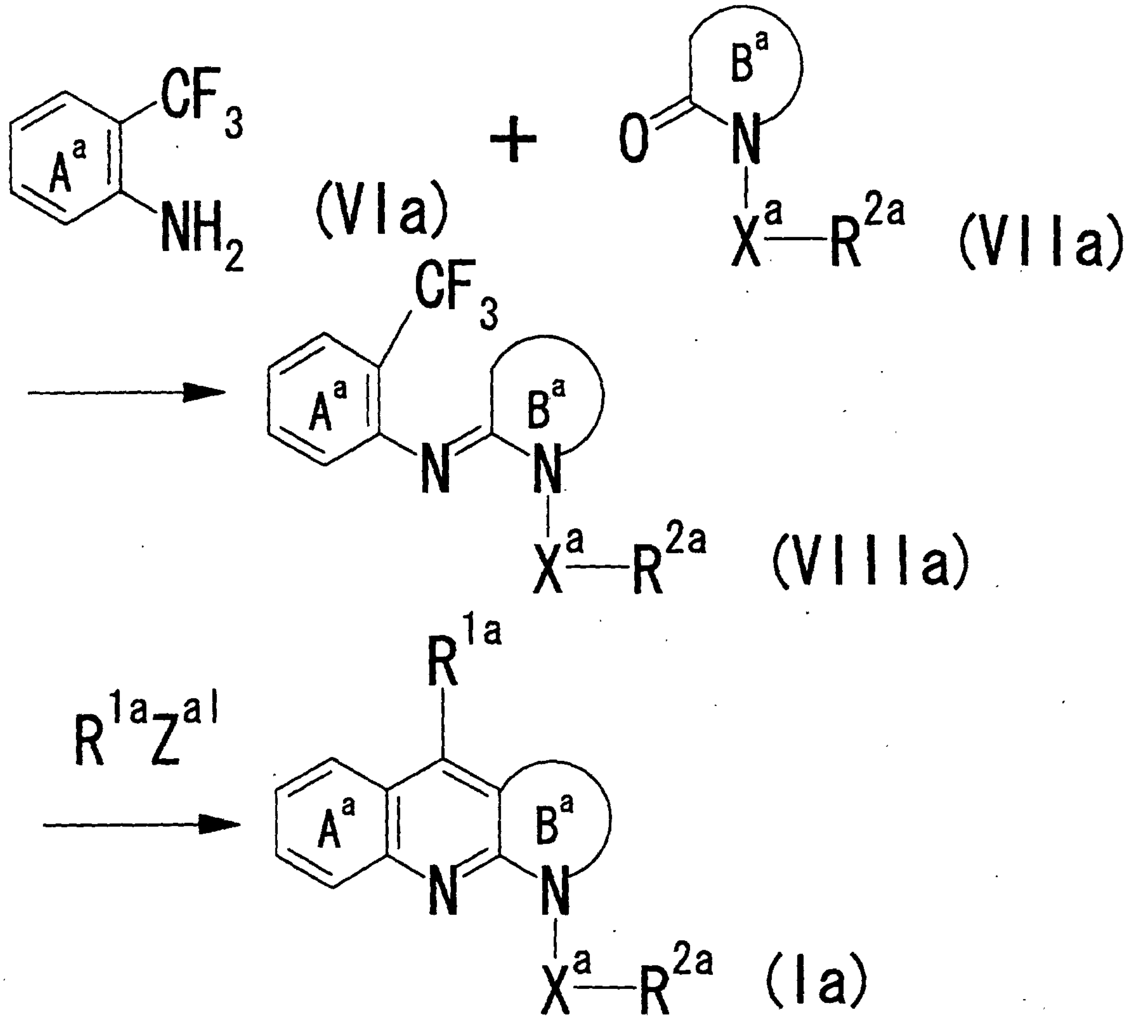


(VIa) (VIIa) (VIIIa) (Ia)

[wherein $Z^{a1}$ represents an alkali metal and the other symbols are as defined above].

According to the procedures as described in Tetrahedron Lett., vol. 40, pp. 5643-5646 or equivalent procedures, a compound of Formula (VIa) or a salt thereof is reacted with a compound of Formula (VIIa) or a salt thereof to give a compound of Formula (Villa) or a salt thereof, which in turn is reacted with a compound of the formula $R^{1a}Z^{a1}$ to give the compound of Formula (Ia) or a salt thereof.

Examples of the alkali metal represented by $Z^{a1}$ include lithium and sodium.

The reaction may be carried out with or without a solvent. Any solvent may be used as long as it does not affect the reaction, including ether solvents (e.g., diethyl ether, tetrahydrofuran, dioxane), halogenated solvents (e.g., dichloromethane, dichloroethane, chloroform, carbon tetrachloride), hydrocarbon solvents (e.g., benzene, toluene, hexane, heptane), amide solvents (e.g., dimethylformamide, dimethylacetamide, N-methylpyrrolidone), ester solvents (e.g., ethyl acetate, methyl acetate), acetonitrile and dimethyl sulfoxide. These solvents may also be used in combination.

The compound of the formula $R^{1a}Z^{a1}$ is used in an amount of about 0.5 to 20 molar equivalents, preferably about 0.8 to 10 molar equivalents, relative to the compound of Formula (VIIIa) or a salt thereof, and reacted at a temperature of about -80°C to 200°C, preferably about -80°C to 80°C, for about 0.1 to 96 hours, preferably about 0.5 to 72 hours.

Among compounds of Formula (Ia) or salts thereof, a compound wherein $R^{1a}$ is not an unsubstituted amino group or a salt thereof may be prepared in a known manner, for example, starting with the compound of Formula (Iaa) synthesized in the above scheme or a salt thereof, which receives various conversions according to the following scheme:

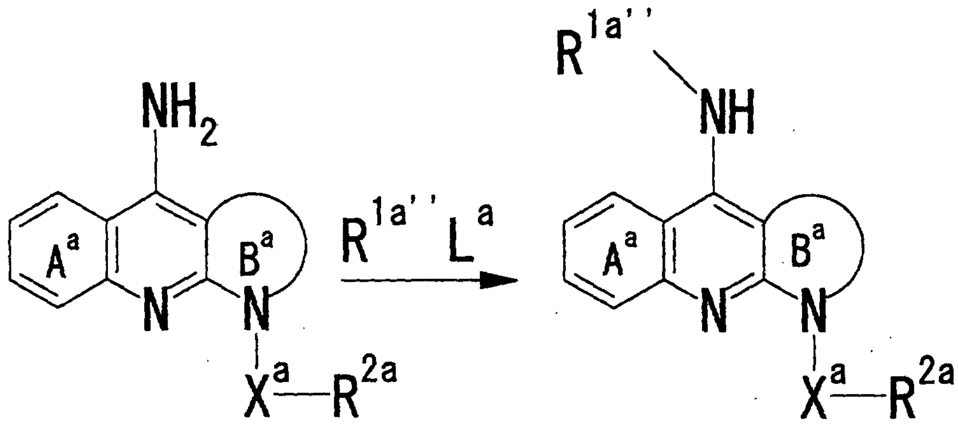


(Iaa) (Iab)

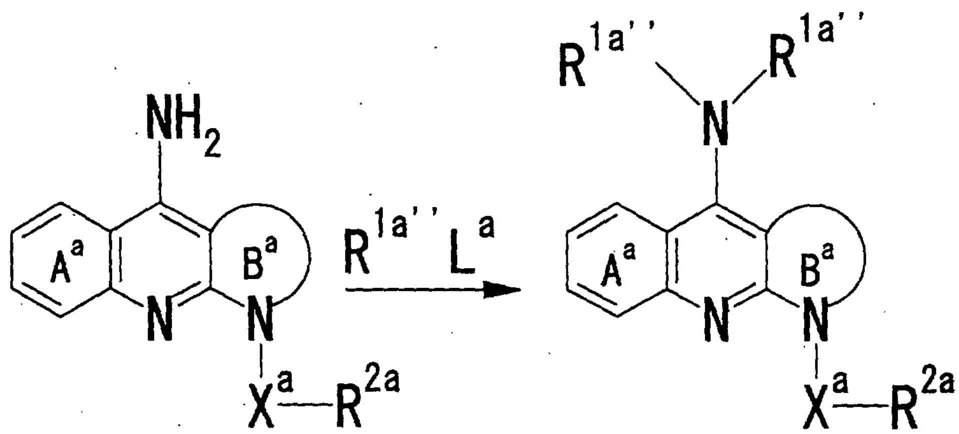


(Iaa) (Iac)

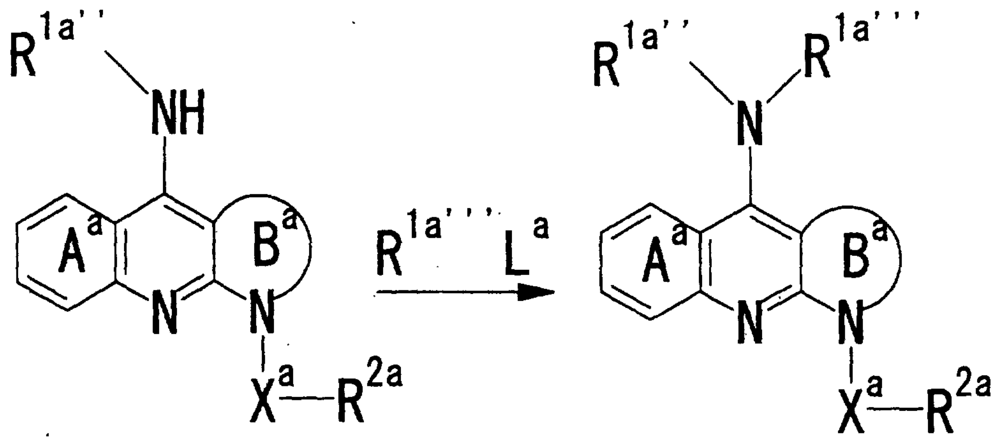

(Iab) (Iad)

[wherein $R^{1a''}$ and $R^{1a'''}$ each represent a substituent on the amino group (preferably, an optionally substituted lower alkyl group), and $L^a$ represents a leaving group].

Examples of the leaving group represented by $L^a$ include halogen atoms such as chlorine, bromine and iodine or sulfonic esters such as a methanesulfonyl group and a toluenesulfonyl group.

The reaction may be carried out with or without a solvent. Any solvent may be used as long as it does not affect the reaction, including ether solvents (e.g., diethyl ether, tetrahydrofuran, dioxane), halogenated solvents (e.g., dichloromethane, dichloroethane, chloroform, carbon tetrachloride), hydrocarbon solvents (e.g., benzene, toluene, hexane, heptane), amide solvents (e.g., dimethylformamide, dimethylacetamide, N-methylpyrrolidone), ester solvents (e.g., ethyl acetate, methyl acetate), acetonitrile and dimethyl sulfoxide. These solvents may also be used in combination. In some cases, the reaction may be carried out in the presence of a base (e.g., triethylamine, 4-(dimethylamino) pyridine,
2-tert-butylimino-2-diethylamino-1,3-dimethylperhydro-1,3,2-diazaphospholine, sodium bicarbonate, potassium bicarbonate, sodium carbonate, potassium carbonate, sodium hydride, potassium hydride) or in the presence of a phase-transfer catalyst (e.g., a quaternary ammonium salt such as tetrabutylammonium bromide or benzyltriethylammonium chloride, a crown ether such as 18-crown-6) or in the presence of a base and a phase-transfer catalyst.

The compound of the formula $R^{1a''}L^a$ and the compound of the formula $R^{1a'''}L^a$ are used in an amount of about 0.5 to 20 molar equivalents, preferably about 0.8 to 10 molar equivalents, relative to the compound of Formula (Iaa) or a salt thereof and the compound of Formula (Iab) or a salt thereof, respectively, and reacted at a temperature of about -20°C to 200°C, preferably about 20°C to 150°C, for about 0.1 to 96 hours, preferably about 0.5 to 72 hours. The base is usually used in an amount of about 0.5 to 10 molar equivalents, preferably about 1 to 5 molar equivalents, relative to the compound of Formula (Iaa) or (Iab).

Further, in a case where each of the compounds of Formulae (Iaa) to (Iad) or salts thereof has a halogen atom such as chlorine, bromine or iodine as a substituent on the ring $A^a$, the halogen atom may be readily converted into various functional groups (e.g., substituents which may be on the benzene ring represented by the ring $A^a$) through a known substitution reaction (e.g., the Suzuki coupling reaction, the Still reaction, the Heck reaction).

Compound (Ia) thus prepared may be isolated and purified by known isolation and purification means including concentration, vacuum concentration, solvent extraction, crystallization, recrystallization, phasic transfer and chromatography.

Other compounds useful as GPR14 (SENR) antagonists, obtained by the screening method or screening kit of the present invention, include compounds of

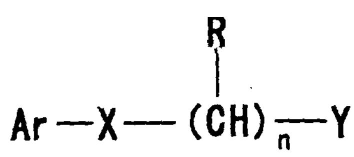 (Ib)

[wherein Ar represents an optionally substituted aryl group, X represents a spacer containing 1 to 4 atoms in its linear chain moiety, n represents an integer of 1 to 10, R represents a hydrogen atom or an optionally substituted hydrocarbon

group which may be the same or different in n repeated units or R may form a ring together with Ar or a substituent on Ar, and Y represents an optionally substituted amino group or an optionally substituted nitrogen-containing heterocyclic group] or salts thereof.

In the above formula, Ar represents an "optionally substituted aryl group."

Examples of a "substituent" on the "optionally substituted aryl group" include (i) an optionally halogenated lower alkyl group, (ii) a halogen atom (e.g., fluoro, chloro, bromo, iodo), (iii) a lower alkylenedioxy group (e.g., a $C_{1-3}$ alkylenedioxy group such as methylenedioxy or ethylenedioxy), (iv) a nitro group, (v) a cyano group, (vi) a hydroxyl group, (vii) an optionally halogenated lower alkoxy group, (viii) a lower cycloalkyl group (e.g., a $C_{3-6}$ cycloalkyl group such as cyclopropyl, cyclobutyl, cyclopentyl or cyclohexyl), (ix) an optionally halogenated lower alkylthio group, (x) an amino group, (xi) a mono-lower alkylamino group (e.g., a mono-$C_{1-6}$ alkylamino group such as methylamino, ethylamino or propylamino), (xii) a di-lower alkylamino group (e.g., a di-$C_{1-6}$ alkylamino group such as dimethylamino or diethylamino), (xiii) a 5- to 7-membered cyclic amino group which may contain, in addition to one nitrogen atom, 1 to 3 heteroatoms selected from a nitrogen atom, an oxygen atom, a sulfur atom and the like (e.g., pyrrolidino, piperidino, piperazino, morpholino, thiomorpholino), (xiv) a lower alkyl-carbonylamino group (e.g., a $C_{1-6}$ alkyl-carbonylamino group such as acetylamino, propionylamino or butyrylamino), (xv) an aminocarbonyloxy group, (xvi) a mono-lower alkylamino-carbonyloxy group (e.g., a mono-$C_{1-6}$ alkylamino-carbonyloxy group such as methylaminocarbonyloxy or ethylaminocarbonyloxy), (xvii) a di-lower alkylamino-carbonyloxy group (e.g., a di-$C_{1-6}$ alkylamino-carbonyloxy group such as dimethylaminocarbonyloxy or diethylaminocarbonyloxy), (xviii) a lower alkylsulfonylamino group (e.g., a $C_{1-6}$ alkylsulfonylamino group such as methylsulfonylamino, ethylsulfonylamino or propylsulfonylamino), (xix) a lower alkoxy-carbonyl group (e.g., a $C_{1-6}$ alkoxy-carbonyl group such as methoxycarbonyl, ethoxycarbonyl, propoxycarbonyl or isobutoxycarbonyl), (xx) a carboxyl group, (xxi) a lower alkyl-carbonyl group (e.g., a $C_{1-6}$ alkyl-carbonyl group such as methylcarbonyl, ethylcarbonyl or butylcarbonyl), (xxii) a lower cycloalkyl-carbonyl (e.g., a $C_{3-6}$ cycloalkyl-carbonyl group such as cyclopropylcarbonyl, cyclobutylcarbonyl, cyclopentylcarbonyl or cyclohexylcarbonyl), (xxiii) a carbamoyl group, (xxiv) a mono-lower alkyl-carbamoyl group (e.g., a mono-$C_{1-6}$ alkyl-carbamoyl group such as methylcarbamoyl, ethylcarbamoyl, propylcarbamoyl or butylcarbamoyl) (xxv) a di-lower alkyl-carbamoyl group (e.g., a di-$C_{1-6}$ alkyl-carbamoyl group such as diethylcarbamoyl or dibutylcarbamoyl), (xxvi) a lower alkylsulfonyl group (e.g., a $C_{1-6}$ alkylsulfonyl group such as methylsulfonyl, ethylsulfonyl or propylsulfonyl), (xxvii) a lower cycloalkylsulfonyl (e.g., a $C_{3-6}$ cycloalkylsulfonyl such as cyclopentylsulfonyl or cyclohexylsulfonyl), (xxviii) a phenyl group, (xxix) a naphthyl group, (xxx) a mono-phenyl-lower alkyl group (e.g., a mono-phenyl-$C_{1-6}$ alkyl group such as benzyl or phenylethyl), (xxxi) a di-phenyl-lower alkyl group (e.g., a di-phenyl-$C_{1-6}$ alkyl group such as diphenylmethyl or diphenylethyl), (xxxii) a mono-phenyl-lower alkyl-carbonyloxy group (e.g., a mono-phenyl-$C_{1-6}$ alkyl-carbonyloxy group such as phenylmethylcarbonyloxy or phenylethylcarbonyloxy), (xxxiii) a di-phenyl-lower alkyl-carbonyloxy group (e.g., a di-phenyl-$C_{1-6}$ alkyl-carbonyloxy group such as diphenylmethylcarbonyloxy or diphenylethylcarbonyloxy), (xxxiv) a phenoxy group, (xxxv) a mono-phenyl-lower alkyl-carbonyl group (e.g., a mono-phenyl-$C_{1-6}$ alkyl-carbonyl group such as phenylmethylcarbonyl or phenylethylcarbonyl), (xxxvi) a di-phenyl-lower alkyl-carbonyl group (e.g., a di-phenyl-$C_{1-6}$ alkyl-carbonyl group such as diphenylmethylcarbonyl or diphenylethylcarbonyl), (xxxvii) a benzoyl group, (xxxviii) a phenoxycarbonyl group, (xxxix) a phenyl-lower alkyl-carbamoyl group (e.g., a phenyl-$C_{1-6}$ alkyl-carbamoyl group such as phenyl-methylcarbamoyl or phenyl-ethylcarbamoyl), (xxxx) a phenylcarbamoyl group, (xxxxi) a phenyl-lower alkyl-carbonylamino group (e.g., a phenyl-$C_{1-6}$ alkyl-carbonylamino such as phenyl-methylcarbonylamino or phenyl-ethylcarbonylamino), (xxxxii) a phenyl-lower alkylamino (e.g., a phenyl-$C_{1-6}$ alkylamino such as phenyl-methylamino or phenyl-ethylamino), (xxxxiii) a phenyl-lower alkylsulfonyl group (e.g., a phenyl-$C_{1-6}$ alkylsulfonyl group such as phenyl-methylsulfonyl or phenyl-ethylsulfonyl), (xxxxiv) a phenylsulfonyl group, (xxxxv) a phenyl-lower alkylsulfinyl group (e.g., a phenyl-$C_{1-6}$ alkylsulfinyl group such as phenyl-methylsulfinyl or phenyl-ethylsulfinyl), (xxxxvi) a phenyl-lower alkylsulfonylamino group (e.g., a phenyl-$C_{1-6}$ alkylsulfonylamino group such as phenyl-methylsulfonylamino or phenyl-ethylsulfonylamino) and (xxxxvii) a phenylsulfonylamino group [said (xxviii) phenyl group, (xxix) naphthyl group, (xxx) mono-phenyl-lower alkyl group, (xxxi) di-phenyl-lower alkyl group, (xxxii) mono-phenyl-lower alkyl-carbonyloxy group, (xxxiii) di-phenyl-lower alkyl-carbonyloxy group, (xxxiv) phenoxy group, (xxxv) mono-phenyl-lower alkyl-carbonyl group, (xxxvi) di-phenyl-lower alkyl-carbonyl group, (xxxvii) benzoyl group, (xxxviii) phenoxycarbonyl group, (xxxix) phenyl-lower alkyl-carbamoyl group, (xxxx) phenylcarbamoyl group, (xxxxi) phenyl-lower alkyl-carbonylamino group, (xxxxii) phenyl-lower alkylamino, (xxxxiii) phenyl-lower alkylsulfonyl group, (xxxxiv) phenylsulfonyl group, (xxxxv) phenyl-lower alkylsulfinyl group, (xxxxvi) phenyl-lower alkylsulfonylamino group and (xxxxvii) phenylsulfonylamino group may further have 1 to 4 substituents selected from, for example, a lower alkyl (e.g., a $C_{1-6}$ alkyl such as methyl, ethyl, propyl, isopropyl, butyl, sec-butyl, tert-butyl, pentyl or hexyl), a lower alkoxy (e.g., a $C_{1-6}$ alkoxy such as methoxy, ethoxy, propoxy, isopropoxy, n-butoxy, isobutoxy, sec-butoxy or tert-butoxy), a halogen atom (e.g., chloro, bromo, iodo), hydroxy, benzyloxy, amino, a mono-lower alkylamino (e.g., a mono-$C_{1-6}$ alkylamino such as methylamino, ethylamino or propylamino), a di-lower alkylamino (e.g., a di-$C_{1-6}$ alkylamino such as dimethylamino or diethylamino), nitro, a lower alkyl-carbonyl (e.g., a $C_{1-6}$ alkyl-carbonyl such as methylcarbonyl, ethylcarbonyl or butylcarbonyl) and benzoyl].

Examples of the above-mentioned "optionally halogenated lower alkyl group" include lower alkyl groups (e.

g., $C_{1-6}$ alkyl groups such as methyl, ethyl, propyl, isopropyl, butyl, sec-butyl, tert-butyl, pentyl and hexyl) which may have 1 to 3 halogen atoms (e.g., chloro, bromo, iodo). Specific examples include methyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl.

Examples of the above-mentioned "optionally halogenated lower alkoxy group" include lower alkoxy groups (e.g., $C_{1-6}$ alkoxy groups such as methoxy, ethoxy, propoxy, isopropoxy, n-butoxy, isobutoxy, sec-butoxy and tert-butoxy) which may have 1 to 3 halogen atoms (e.g., chloro, bromo, iodo). Specific examples include methoxy, difluoromethoxy, trifluoromethoxy, ethoxy, 2,2,2-trifluoroethoxy, n-propoxy, isopropoxy, n-butoxy, 4,4,4-trifluorobutoxy, isobutoxy, sec-butoxy, pentyloxy and hexyloxy.

Examples of the above-mentioned "optionally halogenated lower alkylthio group" include lower alkylthio groups (e.g., $C_{1-6}$ alkylthio groups such as methylthio, ethylthio, n-propylthio, isopropylthio, n-butylthio, isobutylthio, sec-butylthio and tert-butylthio) which may have 1 to 3 halogen atoms (e.g., chloro, bromo, iodo). Specific examples include methylthio, difluoromethylthio, trifluoromethylthio, ethylthio, n-propylthio, isopropylthio, n-butylthio, 4,4,4-trifluorobutylthio, isobutylthio, sec-butylthio, tert-butylthio, pentylthio and hexylthio.

Preferred examples of a "substituent" on the "optionally substituted aryl group" include (i) an amino group, (ii) a mono-lower alkylamino group (e.g., a mono-$C_{1-6}$ alkylamino group such as methylamino, ethylamino or propylamino), (iii) a di-lower alkylamino group (e.g., a di-$C_{1-6}$ alkylamino group such as dimethylamino or diethylamino), (iv) a 5- to 7-membered cyclic amino group which may contain, in addition to one nitrogen atom, 1 to 3 heteroatoms selected from a nitrogen atom, an oxygen atom, a sulfur atom and the like (e.g., pyrrolidino, piperidino, piperazino, morpholino, thiomorpholino), (v) a lower alkyl-carbonylamino group (e.g., a $C_{1-6}$ alkyl-carbonylamino group such as acetylamino, propionylamino or butyrylamino), (vi) an aminocarbonyloxy group, (vii) a mono-lower alkylamino-carbonyloxy group (e.g., a mono-$C_{1-6}$ alkylamino-carbonyloxy group such as methylaminocarbonyloxy or ethylaminocarbonyloxy), (viii) a di-lower alkylamino-carbonyloxy group (e.g., a di-$C_{1-6}$ alkylamino-carbonyloxy group such as dimethylaminocarbonyloxy or diethylaminocarbonyloxy), (ix) a lower alkylsulfonylamino group (e.g., a $C_{1-6}$ alkylsulfonylamino group such as methylsulfonylamino, ethylsulfonylamino or propylsulfonylamino), (x) a phenyl-lower alkylamino (e.g., a phenyl-$C_{1-6}$ alkylamino such as phenyl-methylamino or phenyl-ethylamino), (xi) a phenyl-lower alkylsulfonylamino group (e.g., a phenyl-$C_{1-6}$ alkyl-sulfonylamino group such as phenyl-methylsulfonylamino or phenyl-ethylsulfonylamino), (xii) a phenylsulfonylamino group, (xiii) a halogen atom (e.g., fluoro, chloro), (xiv) an optionally halogenated lower (e.g., $C_{1-6}$) alkyl group (e.g., methyl, ethyl, isopropyl, tert-butyl, trifluoromethyl) and (xv) an optionally halogenated lower (e.g., $C_{1-6}$) alkoxy group (e.g., methoxy, ethoxy, isopropoxy, tert-butoxy, trifluoromethoxy). Particularly preferred examples include a 5- to 7-membered cyclic amino group which may contain, in addition to one nitrogen atom, 1 to 3 heteroatoms selected from a nitrogen atom, an oxygen atom, a sulfur atom and the like (e.g., pyrrolidino, piperidino, piperazino, morpholino, thiomorpholino).

In the above formula, examples of an "aryl group" in the "optionally substituted aryl group" represented by Ar include $C_{6-14}$ aryls such as phenyl and naphthyl, with $C_{6-10}$ aryls being preferred and with phenyl being more preferred. The "optionally substituted aryl group" as used here may form a condensed ring through binding between substituents on the "aryl group." The aryl (preferably phenyl) group as Ar may form a condensed ring together with, for example:

(1) an optionally substituted monocyclic heterocyclic ring;
(2) an optionally substituted bicyclic heterocyclic ring, or the same or different two monocyclic rings (at least one of which is a monocyclic heterocyclic ring); or
(3) an optionally substituted tricyclic heterocyclic ring.

Specific examples of those groups where the "aryl group" in the "optionally substituted aryl group" is condensed with an optionally substituted monocyclic heterocyclic ring include a group of the following formula:

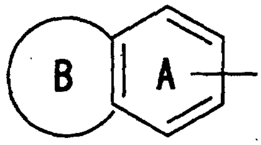

[wherein the ring B represents an optionally substituted heterocyclic ring, and the ring A represents an optionally substituted benzene ring].

Examples of a substituent on the ring A include the same substituents as listed above for the "optionally substituted aryl group."

Examples of a "heterocyclic ring" used in the "optionally substituted heterocyclic ring" represented by the ring B include 4- to 14-membered rings, preferably 5- to 9-membered rings, which may be either aromatic or non-aromatic. These rings may contain 1 to 3 or 4 heteroatoms selected from a nitrogen atom, an oxygen atom, a sulfur atom and the like. Specific examples include pyridine, pyrazine, pyrimidine, imidazole, furan, thiophene, dihydropyridine, azepine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, pyrrole, pyrazole, 1,2,3-triazole, oxazole, oxazolidine, thiazole, thiazolidine, isooxazole and imidazoline. Particularly preferred examples include 5- to 9-membered non-aromatic heterocyclic rings containing one heteroatom or the same or different two heteroatoms (e.g., pyrrolidine, piperidine, hexamethyleneimine, heptamethyleneimine, tetrahydrofuran, piperazine, homopiperazine, tetrahydrooxazepine, morpholine, thiomorpholine). In particular, frequently used are non-aromatic heterocyclic rings containing one heteroatom selected from a nitrogen atom, an oxygen atom and a sulfur atom as well as non-aromatic heterocyclic rings containing one nitrogen atom and one heteroatom selected from a nitrogen atom, an oxygen atom and a sulfur atom.

The "optionally substituted heterocyclic ring" represented by the ring B may have a substituent(s) on any carbon atom in the ring B. For example, 1 to 5 substituents selected from the following may be used for substitution on any carbon atom in the ring B: (i) a halogen atom (e.g., fluoro, chloro, bromo, iodo), (ii) a nitro group, (iii) a cyano group, (iv) an oxo group, (v) a hydroxyl group, (vi) a lower alkyl group (e.g., a $C_{1-6}$ alkyl group such as methyl, ethyl, propyl, isopropyl, butyl, isobutyl, tert-butyl or sec-butyl), (vii) a lower alkoxy group (e.g., a $C_{1-6}$ alkoxy group such as methoxy, ethoxy, n-propyloxy, i-propyloxy or n-butyloxy), (viii) a lower alkylthio group (e.g., a $C_{1-6}$ alkylthio group such as methylthio, ethylthio or propylthio), (ix) an amino group, (x) a mono-lower alkylamino group (e.g., a mono-$C_{1-6}$ alkylamino group such as methylamino, ethylamino or propylamino), (xi) a di-lower alkylamino group (e.g., a di-$C_{1-6}$ alkylamino group such as dimethylamino or diethylamino), (xii) a 5- to 7-membered cyclic amino group which may contain, in addition to carbon atoms and one nitrogen atom, 1 to 3 heteroatoms selected from a nitrogen atom, an oxygen atom, a sulfur atom and the like (e.g., pyrrolidino, piperidino, piperazino, morpholino, thiomorpholino), (xiii) a lower alkyl-carbonylamino group (e.g., a $C_{1-6}$ alkyl-carbonylamino group such as acetylamino, propionylamino or butyrylamino), (xiv) a lower alkylsulfonylamino group (e.g., a $C_{1-6}$ alkyl-carbonylamino group such as methylsulfonylamino or ethylsulfonylamino), (xv) a lower alkoxy-carbonyl group (e.g., a $C_{1-6}$ alkoxy-carbonyl group such as methoxycarbonyl, ethoxycarbonyl or propoxycarbonyl), (xvi) a carboxyl group, (xvii) a lower alkyl-carbonyl group (e.g., a $C_{1-6}$ alkyl-carbonyl group such as methylcarbonyl, ethylcarbonyl or propylcarbonyl), (xviii) a carbamoyl group, (xix) a mono-lower alkylcarbamoyl group (e.g., a mono-$C_{1-6}$ alkylcarbamoyl group such as methylcarbamoyl or ethylcarbamoyl), (xx) a di-lower alkylcarbamoyl group (e.g., a di-$C_{1-6}$ alkylcarbamoyl group such as dimethylcarbamoyl or diethylcarbamoyl) and (xxi) a lower alkylsulfonyl group (e.g., a $C_{1-6}$ alkylsulfonyl group such as methylsulfonyl, ethylsulfonyl or propylsulfonyl).

Above all, preferred are an oxo group, a lower alkyl group (e.g., a $C_{1-6}$ alkyl group such as methyl, ethyl, propyl, isopropyl, butyl, isobutyl, tert-butyl or sec-butyl) and the like. For example, an oxo group is commonly used.

Further, when the ring B has a nitrogen atom as its ring member, it may have a substituent on the nitrogen atom. Namely, the ring B may have the following moiety in its ring:

$$>N-R^1$$

[wherein $R^1$ represents a hydrogen atom, an optionally substituted hydrocarbon group, an optionally substituted acyl group or an optionally substituted heterocyclic group].

A "hydrocarbon group" in the "optionally substituted hydrocarbon group" represented by $R^1$ refers to a group formed by removing one hydrogen atom from a hydrocarbon compound, examples of which include open-chain or cyclic hydrocarbon groups such as an alkyl group, an alkenyl group, an alkynyl group, a cycloalkyl group, an aryl group and an aralkyl group. Among these, $C_{1-16}$ hydrocarbon groups are preferably used, which comprise either or both of open-chain and cyclic moieties.

Preferred examples of open-chain or cyclic hydrocarbon groups available for use include:

(1) linear or branched lower alkyl groups (e.g., $C_{1-6}$ alkyl groups such as methyl, ethyl, propyl, isopropyl, butyl, isobutyl, tert-butyl, sec-butyl, pentyl and hexyl);
(2) linear or branched lower alkenyl groups (e.g., $C_{2-6}$ alkenyl groups such as vinyl, allyl, isopropenyl, butenyl, isobutenyl and sec-butenyl);
(3) linear or branched lower alkynyl groups (e.g., $C_{2-6}$ alkynyl groups such as propargyl, ethynyl, butynyl and 1-hexynyl);
(4) monocyclic lower cycloalkyl groups (e.g., monocyclic $C_{3-6}$ cycloalkyl groups such as cyclopropyl, cyclobutyl, cyclopentyl and cyclohexyl);
(5) bridged cyclic lower saturated hydrocarbon groups (e.g., bridged cyclic $C_{8-14}$ saturated hydrocarbon groups

such as bicyclo[3.2.1]oct-2-yl, bicyclo[3.3.1]non-2-yl and adamantan-1-yl); and
(6) aryl groups (e.g., $C_{6-14}$ aryl groups such as phenyl, 1-naphthyl, 2-naphthyl, biphenyl, 2-indenyl and 2-anthryl, preferably a phenyl group).

Preferred examples of hydrocarbon groups comprising both open-chain and cyclic moieties available for use include:

(1) lower aralkyl groups (e.g., $C_{7-16}$ aralkyl groups such as phenyl-$C_{1-10}$ alkyls (e.g., benzyl, phenylethyl, phenylpropyl, phenylbutyl, phenylpentyl, phenylhexyl), naphthyl-$C_{1-6}$ alkyls (e.g., α-naphthylmethyl) or diphenyl-$C_{1-3}$ alkyls (e.g., diphenylmethyl, diphenylethyl));
(2) aryl-alkenyl groups (e.g., $C_{6-14}$ aryl-$C_{2-12}$ alkenyl groups such as phenyl-$C_{2-12}$ alkenyls including styryl, cinnamyl, 4-phenyl-2-butenyl and 4-phenyl-3-butenyl);
(3) aryl-$C_{2-12}$ alkynyl groups (e.g., $C_{6-14}$ aryl-$C_{2-12}$ alkynyl groups such as phenyl-$C_{2-12}$ alkynyls including phenylethynyl, 3-phenyl-2-propynyl and 3-phenyl-1-propynyl);
(4) lower cycloalkyl-lower alkyl groups (e.g., $C_{3-7}$ cycloalkyl-$C_{1-6}$ alkyl groups such as cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl and cyclohexylhexyl); and
(5) aryl-$C_{1-10}$ alkyl groups (e.g., biphenyl-$C_{1-10}$ alkyls such as biphenylmethyl and biphenylethyl).

A preferred "hydrocarbon group" frequently used in the "optionally substituted hydrocarbon group" represented by $R^1$ is, for example:

(1) a linear, branched or cyclic alkyl group, preferably a linear or branched $C_{1-6}$ alkyl group (e.g., a $C_{1-6}$ alkyl group such as methyl, ethyl, propyl, isopropyl, butyl, isobutyl, tert-butyl, sec-butyl, pentyl or hexyl), a cyclic $C_{3-8}$ alkyl group (e.g., cyclopropyl, cyclobutyl, cyclopentyl, cyclohexyl), or a $C_{4-12}$ alkyl group comprising any combination of linear, branched or cyclic moieties (e.g., cyclopropylmethyl, cyclopentylmethyl, cyclohexylmethyl, cyclohexylethyl, (4-methylcyclohexyl)methyl); or
(2) a $C_{7-16}$ aralkyl group (e.g., a phenyl-$C_{1-10}$ alkyl (e.g., benzyl, phenylethyl, phenylpropyl, phenylbutyl, phenylpentyl, phenylhexyl), a naphthyl-$C_{1-6}$ alkyl (e.g., α-naphthylmethyl) or a diphenyl-$C_{1-3}$ alkyl (e.g., diphenylmethyl, diphenylethyl)), more preferably a $C_{7-10}$ aralkyl group (e.g., a phenyl-$C_{1-4}$ alkyl such as benzyl, phenylethyl or phenylpropyl).

The "hydrocarbon group" represented by $R^1$ may have a substituent(s), for example, any appropriate substituent commonly used for substitution on a hydrocarbon group. More specifically, 1 to 5 substituents (preferably, 1 to 3 substituents) selected from the following may be used: (i) a halogen atom (e.g., fluoro, chloro, bromo, iodo), (ii) a nitro group, (iii) a cyano group, (iv) an oxo group, (v) a hydroxyl group, (vi) a lower alkyl group (e.g., a $C_{1-6}$ alkyl group such as methyl, ethyl, propyl, isopropyl, butyl, isobutyl, tert-butyl or sec-butyl) which may be substituted with a halogen atom or a phenyl group, (vii) a lower alkoxy group (e.g., a $C_{1-6}$ alkoxy group such as methoxy, ethoxy, n-propyloxy, i-propyloxy or n-butyloxy) which may be substituted with a halogen atom or a phenyl group, (viii) a lower alkylthio group (e.g., a $C_{1-6}$ alkylthio group such as methylthio, ethylthio or propylthio) which may be substituted with a halogen atom or a phenyl group, (ix) an amino group, (x) a mono-lower alkylamino group (e.g., a mono-$C_{1-6}$ alkylamino group such as methylamino, ethylamino or propylamino), (xi) a di-lower alkylamino group (e.g., a di-$C_{1-6}$ alkylamino group such as dimethylamino or diethylamino), (xii) a 5- to 7-membered cyclic amino group which may contain, in addition to carbon atoms and one nitrogen atom, 1 to 3 heteroatoms selected from a nitrogen atom, an oxygen atom, a sulfur atom and the like (e.g., pyrrolidino, piperidino, piperazino, morpholino, thiomorpholino), (xiii) a lower alkyl-carbonylamino group (e.g., a $C_{1-6}$ alkyl-carbonylamino group such as acetylamino, propionylamino or butyrylamino), (xiv) a lower alkylsulfonylamino group (e.g., a $C_{1-6}$ alkyl-sulfonylamino group such as methylsulfonylamino or ethylsulfonylamino), (xv) a lower alkoxy-carbonyl group (e.g., a $C_{1-6}$ alkoxy-carbonyl group such as methoxycarbonyl, ethoxycarbonyl or propoxycarbonyl), (xvi) a carboxyl group, (xvii) formyl, a lower alkyl-carbonyl group (e.g., a $C_{1-6}$ alkyl-carbonyl group such as methylcarbonyl, ethylcarbonyl or propylcarbonyl), (xviii) a carbamoyl group, (xix) a mono-lower alkyl-carbamoyl group (e.g., a mono-$C_{1-6}$ alkyl-carbamoyl group such as methylcarbamoyl or ethylcarbamoyl), (xx) a di-lower alkyl-carbamoyl group (e.g., a di-$C_{1-6}$ alkyl-carbamoyl group such as dimethylcarbamoyl or diethylcarbamoyl), (xxi) a lower alkylsulfonyl group (e.g., a $C_{1-6}$ alkylsulfonyl group such as methylsulfonyl, ethylsulfonyl or propylsulfonyl), (xxii) a lower alkoxy-carbonyl-lower alkyl group (e.g., a $C_{1-6}$ alkoxy-carbonyl-$C_{1-6}$ alkyl group such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl or tert-butoxycarbonyl(dimethyl)methyl), (xxiii) a carboxyl-lower alkyl group (e.g., a carboxyl-$C_{1-6}$ alkyl group such as carboxylmethyl, carboxylethyl or carboxyl(dimethyl)methyl), (xxiv) an optionally substituted heterocyclic group, (xxv) an optionally substituted alkyl group, (xxvi) an optionally substituted alkoxy group, (xxvii) an optionally substituted ureido group (e.g., ureido, 3-methylureido, 3-ethylureido, 3-phenylureido, 3-(4-fluorophenyl)ureido, 3-(2-methylphenyl)ureido, 3-(4-methoxyphenyl)ureido, 3-(2,4-difluorophenyl)ureido, 3-[3,5-bis(trifluoromethyl)phenyl]ureido, 3-benzylureido, 3-(1-naphthyl)ureido, 3-(2-biphenylyl)ureido), (xxviii) an optionally substituted thioureido group (e.g., thioureido, 3-methylthioureido, 3-ethylthioureido, 3-phenylthioureido, 3-(4-fluorophenyl)thioureido, 3-(4-methylphenyl)thioureido, 3-(4-methoxyphenyl)thioureido, 3-(2,4-dichlorophenyl) thioureido, 3-benzylthioureido, 3-(1-naphthyl)thioureido), (xxix) an optionally substituted amidino group (e.g., amidino, $N^1$-methylamidino, $N^1$-ethylamidino, $N^1$-phenylamidino, $N^1$,$N^1$-dimethylamidino, $N^1$,$N^2$-dimethylamidino, $N^1$-methyl-$N^1$-ethylamidino, $N^1$,$N^1$-diethylamidino, $N^1$-methyl-$N^1$-phenylamidino, $N^1$,$N^1$-di(4-nitrophenyl)amidino), (xxx) an optionally substituted guanidino group (e.g., guanidino, 3-methylguanidino, 3,3-dimethylguanidino, 3,3-diethylguanidino), (xxxi) an optionally substituted cyclic aminocarbonyl group (e.g., pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, thiomorpholinocarbonyl), (xxxii) an optionally substituted aminothiocarbonyl group (e.g., aminothiocarbonyl, methylaminothiocarbonyl, dimethylaminothiocarbonyl), (xxxiii) an optionally substituted aminosulfonyl (e.g., aminosulfonyl, methylaminosulfonyl, dimethylaminosulfonyl), (xxxiv) an optionally substituted phenylsulfonylamino (e.g., phenylsulfonylamino, (4-methylphenyl)sulfonylamino, (4-chlorophenyl)sulfonylamino, (2,5-dichlorophenyl)sulfonylamino, (4-methoxyphenyl)sulfonylamino, (4-acetylaminophenyl)sulfonylamino, (4-nitrophenyl)phenylsulfonylaniino), (xxxv) a sulfo group, (xxxvi) a sulfino group, (xxxvii) a sulfeno group, (xxxviii) a $C_{1-6}$ alkylsulfo group (e.g., methylsulfo, ethylsulfo, propylsulfo), (xxxix) a $C_{1-6}$ alkylsulfino group (e.g., methylsulfino, ethylsulfino, propylsulfino), (xxxx) a $C_{1-6}$ alkylsulfeno group (e.g., methylsulfeno, ethylsulfeno, propylsulfeno), (xxxxi) a phosphono group, (xxxxii) a di-$C_{1-6}$ alkoxyphosphoryl group (e.g., dimethoxyphosphoryl, diethoxyphosphoryl, dipropoxyphosphoryl), (xxxxiii) a $C_{1-4}$ alkylenedioxy (e.g., $-O-CH_2-O-$, $-O-CH_2-CH_2-O-$), (xxxxiv) a phenylthio group which may be substituted with a halogen atom and (xxxxv) a phenoxy group which may be substituted with a halogen atom.

Preferred examples of a "substituent" used for substitution on the "optionally substituted hydrocarbon group" represented by $R^1$ include a halogen atom, an optionally substituted alkyl group, an optionally substituted alkoxy group, a hydroxyl group, a nitro group, a cyano group, a carboxyl group, a $C_{1-6}$ alkoxycarbonyl group, a carbamoyl group, an aminothiocarbonyl group, a mono-lower alkyl-carbamoyl group, a di-lower alkyl-carbamoyl group, an optionally substituted cyclic aminocarbonyl group, an amino group, a mono-lower alkylamino group, a di-lower alkylamino group, a 5- to 7-membered cyclic amino group which may contain, in addition to carbon atoms and one nitrogen atom, 1 to 3 heteroatoms selected from a nitrogen atom, an oxygen atom, a sulfur atom and the like, a $C_{1-6}$ alkylcarbonylamino group, an optionally substituted phenylsulfonylamino group, a $C_{1-6}$ alkylsulfonylamino group, an optionally substituted amidino group, an optionally substituted ureido group and an optionally substituted heterocyclic group.

A "heterocyclic group" used in the "optionally substituted heterocyclic group" may be a group such as formed by removing one hydrogen atom from a monocyclic heterocyclic ring, a bicyclic heterocyclic ring or a polycyclic (e.g., tricyclic or tetracyclic) heterocyclic ring. The heterocyclic ring may be either aromatic or non-aromatic. It may contain 1 to 6 heteroatoms selected form a nitrogen atom, an oxygen atom, a sulfur atom and the like. More specifically, a monocyclic heterocyclic group may be a group such as formed by removing one hydrogen atom from the "heterocyclic ring" in the "optionally substituted heterocyclic ring" represented by the ring B. In addition to this, other groups may be used, such as those formed by removing one hydrogen atom from a monocyclic heterocyclic ring such as triazole, thiadiazole, oxadiazole, oxathiadiazole, triazine or tetrazole. A bicyclic heterocyclic group may be a group such as formed by removing one hydrogen atom from a bicyclic heterocyclic ring such as indole, dihydroindole, isoindole, dihydroisoindole, benzofuran, dihydrobenzofuran, benzimidazole, benzoxazole, benzisooxazole, benzothiazole, indazole, quinoline, tetrahydroquinoline, isoquinoline, tetrahydroisoquinoline, tetrahydro-1H-1-benzazepine, tetrahydro-1H-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxole, benzothiazine or imidazopyridine. A polycyclic (e.g., tricyclic or tetracyclic) heterocyclic group may be a group such as formed by removing one hydrogen atom from a polycyclic heterocyclic ring such as acridine, tetrahydroacridine, pyrroloquinoline, pyrroloindole, cyclopentoindole or isoindolobenzazepine.

In particular, a "heterocyclic group" frequently used in the "optionally substituted heterocyclic group" is a group such as formed by removing one hydrogen atom from the above-mentioned monocyclic heterocyclic ring or bicyclic heterocyclic ring.

In addition, examples of a "substituent" used for substitution on the "optionally substituted heterocyclic group" include the substituents (excluding an "optionally substituted heterocyclic group") listed above for the "optionally substituted heterocyclic ring" represented by the ring B.

Examples of a "substituent" used for substitution on the "optionally substituted alkyl (preferably, optionally substituted $C_{1-6}$ alkyl)" or "optionally substituted alkoxy (preferably, optionally substituted $C_{1-6}$ alkoxy)" include the substituents listed above in (i) to (xxiv) or (xxvii) to (xxxxii) for the "optionally substituted hydrocarbon group" represented by $R^1$.

Examples of a "substituent" used for substitution on the "optionally substituted ureido group", "optionally substituted thioureido group", "optionally substituted amidino group", "optionally substituted guanidino group", "optionally substituted cyclic aminocarbonyl group", "optionally substituted aminothiocarbonyl group", "optionally substituted aminosulfonyl" or "optionally substituted phenylsulfonylamino" include the substituents listed above in (i) to (xxvi) or (xxxv) to (xxxxii) for the "optionally substituted hydrocarbon group" represented by $R^1$, $C_{6-14}$ aryl groups (which may have a substituent(s) selected from a halogen atom, a $C_{1-6}$ alkyl group, a halo-$C_{1-6}$ alkyl group, a $C_{1-6}$ alkoxy group, a nitro group and the like) and $C_{7-16}$ aralkyl groups.

Preferred examples of the "optionally substituted hydrocarbon group" represented by $R^1$ include (i) $C_{1-6}$ alkyl groups and (ii) phenyl-$C_{1-6}$ alkyl groups which may have a substituent(s) such as a halogen atom, nitro, a $C_{1-6}$ alkyl or a $C_{1-6}$ alkoxy. More preferred examples include benzyl groups which may be substituted with a $C_{1-4}$ alkyl (e.g., methyl), a trihalogeno-$C_{1-4}$ alkyl (e.g., methyl), a halogen atom (e.g., fluoro, chloro), nitro, cyano, a $C_{1-4}$ alkoxy (e.g., methoxy), a trihalogeno-$C_{1-4}$ alkoxy (e.g., methoxy), hydroxy, carbamoyl, a (4-$C_{1-4}$ alkyl (e.g., methyl)-1-piperazinyl) carbonyl, aminothiocarbonyl, morpholinocarbonyl, carboxyl, a $C_{1-4}$ alkoxy (e.g., methoxy)-carbonyl, a $C_{1-4}$ alkoxy (e.g., ethoxy)-carbonyl-$C_{1-4}$ alkoxy (e.g., methoxy), a carboxyl-$C_{1-4}$ alkoxy (e.g., methoxy), a $C_{1-4}$ alkoxy (e.g., ethoxy)-carbonyl-$C_{1-6}$ alkyl (e.g., isopropyl), a carboxyl-$C_{1-6}$ alkyl (e.g., isopropyl), amino, acetylamino, a $C_{1-4}$ alkyl (e.g., methyl)-sulfonylamino, a (4-$C_{1-4}$ alkyl (e.g., methyl)-phenyl)sulfonylamino, ureido, a 3-$C_{1-4}$ alkyl (e.g., methyl)-ureido, amidino, dihydrothiazolyl or dihydroimidazolyl.

Above all, preferred for $R^1$ are benzyl groups which may be substituted with a $C_{1-4}$ alkyl (e.g., methyl), a trihalogeno (e.g., fluoro)-$C_{1-4}$ alkyl (e.g., methyl), a halogen atom (e.g., fluoro, chloro), nitro, cyano, carbamoyl, a $C_{1-4}$ alkoxy (e.g., methoxy)-carbonyl, a $C_{1-4}$ alkoxy (e.g., ethoxy)-carbonyl-$C_{1-4}$ alkoxy (e.g., methoxy), amino, acetylamino, a $C_{1-4}$ alkyl (e.g., methyl)-sulfonylamino, a 3-$C_{1-4}$ alkyl (e.g., methyl)-ureido, amidino or dihydroimidazolyl. Particularly preferred are benzyl groups which may be substituted with a $C_{1-4}$ alkyl, and most particularly preferred is a benzyl group which may be substituted with methyl.

Examples of the "optionally substituted acyl group" represented by $R^1$ include -(C=O)-$R^{2b}$, -$SO_2$-$R^{2b}$, -SO-$R^{2b}$, (C=O)N$R^{3b}R^{2b}$, -(C=O)O-$R^{2b}$, -(C=S)O-$R^{2b}$ and -(C=S)N$R^{3b}R^{2b}$ [wherein $R^{2b}$ and $R^{3b}$, which may be the same or different, each represent (i) a hydrogen atom, (ii) an optionally substituted hydrocarbon group or (iii) an optionally substituted heterocyclic group, or $R^{2c}$ and $R^{3c}$ may together form an optionally substituted nitrogen-containing saturated heterocyclic group together with their adjacent nitrogen atom].

Among these, preferred is -(C=O)-$R^{2b}$, -$SO_2$-$R^{2b}$, -SO-$R^{2b}$, -(C=O)N$R^{3b}R^{2b}$ or -(C=O)O-$R^{2b}$ (wherein $R^{2b}$ and $R^{3b}$ are as defined above). In particular, -(C=O)-$R^{2b}$ or -(C=O)N$R^{3b}R^{2b}$ (wherein $R^{2b}$ and $R^{3b}$ are as defined above) is commonly used.

A "hydrocarbon group" in the "optionally substituted hydrocarbon group" represented by $R^{2b}$ and $R^{3b}$ refers to a group formed by removing one hydrogen atom from a hydrocarbon compound, examples of which include open-chain or cyclic hydrocarbon groups such as an alkyl group, an alkenyl group, an alkynyl group, a cycloalkyl group, an aryl group and an aralkyl group. Specific examples include the same groups as listed above for the "hydrocarbon group" in the "optionally substituted hydrocarbon group" represented by $R^1$. Above all, preferred are open-chain or cyclic $C_{1-16}$ hydrocarbon groups, and particularly preferred is a lower ($C_{1-6}$) alkyl group, a lower ($C_{2-6}$) alkenyl group, a $C_{7-16}$ aralkyl group or a $C_{6-14}$ aryl group. Among these, a lower ($C_{1-6}$) alkyl group, a $C_{7-16}$ aralkyl group or a $C_{6-14}$ aryl group is commonly used.

A "heterocyclic group" used in the "optionally substituted heterocyclic group" represented by $R^{2b}$ and $R^{3b}$ may be a group such as formed by removing one hydrogen atom from a monocyclic heterocyclic ring, a bicyclic heterocyclic ring or a polycyclic (e.g., tricyclic or tetracyclic) heterocyclic ring. The heterocyclic ring may be either aromatic or non-aromatic. It may contain 1 to 6 heteroatoms selected form a nitrogen atom, an oxygen atom, a sulfur atom and the like. More specifically, a monocyclic heterocyclic group may be a group such as formed by removing one hydrogen atom from the "heterocyclic ring" in the "optionally substituted heterocyclic ring" represented by the ring B. In addition to this, other groups may be used, such as those formed by removing one hydrogen atom from a monocyclic heterocyclic ring such as triazole, thiadiazole, oxadiazole, oxathiadiazole, triazine or tetrazole. A bicyclic heterocyclic group may be a group such as formed by removing one hydrogen atom from a bicyclic heterocyclic ring such as indole, dihydroindole, isoindole, dihydroisoindole, benzofuran, dihydrobenzofuran, benzimidazole, benzoxazole, benzisooxazole, benzothiazole, indazole, quinoline, tetrahydroquinoline, isoquinoline, tetrahydroisoquinoline, tetrahydro-1H-1-benzazepine, tetrahydro-1H-2-benzazepine, tetrahydro-lH-3-benzazepine, tetrahydrobenzoxazepine, quinazoline, tetrahydroquinazoline, quinoxaline, tetrahydroquinoxaline, benzodioxane, benzodioxole, benzothiazine or imidazopyridine. A polycyclic (e.g., tricyclic or tetracyclic) heterocyclic group may be a group such as formed by removing one hydrogen atom from a polycyclic heterocyclic ring such as acridine, tetrahydroacridine, pyrroloquinoline, pyrroloindole, cyclopentoindole or

isoindolobenzazepine.

In particular, a "heterocyclic group" frequently used in the "optionally substituted heterocyclic group" is a group such as formed by removing one hydrogen atom from the above-mentioned monocyclic heterocyclic ring or bicyclic heterocyclic ring.

The "optionally substituted nitrogen-containing saturated heterocyclic group" formed by $R^{2b}$ and $R^{3b}$ together with their adjacent nitrogen atom may be a 5- to 9-membered nitrogen-containing saturated heterocyclic group which may contain, in addition to carbon atoms and one nitrogen atom, 1 to 3 heteroatoms selected from a nitrogen atom, an oxygen atom, a sulfur atom and the like. Such a nitrogen-containing saturated heterocyclic group preferably has a binding hand on a ring-constituting nitrogen atom. Such a group having a binding hand on a ring-constituting nitrogen atom may be represented, for example, by the following formula:

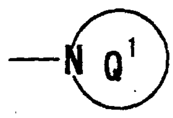

[wherein the ring $Q^1$ represents a 5- to 9-membered nitrogen-containing saturated heterocyclic group which may contain, in addition to carbon atoms and one nitrogen atom, 1 or 2 heteroatoms selected from a nitrogen atom, an oxygen atom, a sulfur atom and the like]. More specifically, the following group:

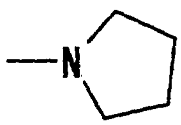 , 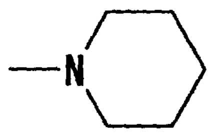 , 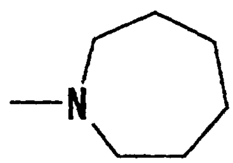 , 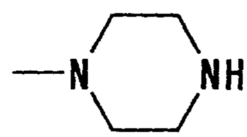 ,

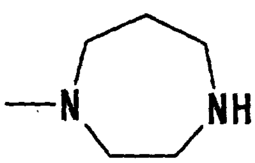 , 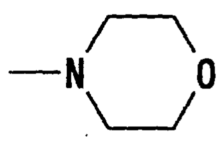 or 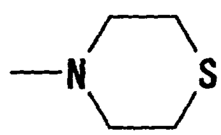

is frequently used, by way of example.

The "hydrocarbon group" or "heterocyclic group" represented by $R^{2b}$ and $R^{3b}$ and the "nitrogen-containing saturated heterocyclic group" represented by $NR^{3b}R^{2b}$ may each have 1 to 5 substituents (preferably, 1 to 3 substituents) which are preferably selected from (i) a halogen atom (e.g., fluoro, chloro, bromo, iodo), (ii) a nitro group, (iii) a cyano group, (iv) an oxo group, (v) a hydroxyl group, (vi) an optionally substituted hydrocarbon group, (vii) a lower alkoxy group (e.g., a $C_{1-6}$ alkoxy group such as methoxy, ethoxy, n-propyloxy, i-propyloxy or n-butyloxy) which may be substituted with a phenyl group, (viii) a lower alkylthio group (e.g., a $C_{1-6}$ alkylthio group such as methylthio, ethylthio or propylthio) which may be substituted with a phenyl group, (ix) an amino group, (x) a mono-lower alkylamino group (e.g., a mono-$C_{1-6}$ alkylamino group such as methylamino, ethylamino or propylamino), (xi) a di-lower alkylamino group (e.g., a di-$C_{1-6}$ alkylamino group such as dimethylamino or diethylamino), (xii) a 5- to 7-membered cyclic amino group which may contain, in addition to carbon atoms and one nitrogen atom, 1 to 3 heteroatoms selected from a nitrogen atom, an oxygen atom, a sulfur atom and the like (e.g., pyrrolidino, piperidino, piperazino, morpholino, thiomorpholino), (xiii) a lower alkyl-carbonylamino group (e.g., a $C_{1-6}$ alkyl-carbonylamino group such as acetylamino, propionylamino or butyrylamino), (xiv) a lower alkyl-sulfonylamino group (e.g., a $C_{1-6}$ alkyl-sulfonylamino group such as methylsulfonylamino or ethylsulfonylamino), (xv) a lower alkoxy-carbonyl group (e.g., a $C_{1-6}$ alkoxy-carbonyl group such as methoxycarbonyl, ethoxycarbonyl or propoxycarbonyl), (xvi) a carboxyl group, (xvii) a lower alkyl-carbonyl group (e.g., a $C_{1-6}$ alkyl-carbonyl group such as methylcarbonyl, ethylcarbonyl or propylcarbonyl), (xviii) a carbamoyl group, (xix) a mono-lower alkyl-carbamoyl group (e.g., a mono-$C_{1-6}$ alkyl-carbamoyl group such as methylcarbamoyl or ethylcarbamoyl), (xx) a di-lower alkyl-carbamoyl group (e.g., a di-$C_{1-6}$ alkyl-carbamoyl group such as dimethylcarbamoyl or diethylcarbamoyl), (xxi) a lower alkylsulfonyl group (e.g., a $C_{1-6}$ alkylsulfonyl group such as methylsulfonyl, ethylsulfonyl

or propylsulfonyl), (xxii) a lower alkoxy-carbonyl-lower alkyl group (e.g., a $C_{1-6}$ alkoxy-carbonyl-$C_{1-6}$ alkyl group such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl or tert-butoxycarbonyl(dimethyl)methyl), (xxiii) a carboxyl-lower alkyl group (e.g., a carboxyl-$C_{1-6}$ alkyl group such as carboxylmethyl, carboxylethyl or carboxyl(dimethyl)methyl), (xxiv) an optionally substituted heterocyclic group, (xxv) a phenylthio group which may be substituted with a halogen atom and (xxvi) a phenoxy group which may be substituted with a halogen atom.

Each of the "lower alkoxy group" and "lower alkylthio group" may further have a phenyl group as a substituent.

Examples of a "substituent" used for substitution on and a "hydrocarbon group" used in the "optionally substituted hydrocarbon group" include the substituents and hydrocarbon groups listed above for the "optionally substituted hydrocarbon group" represented by $R^1$.

A "heterocyclic group" used in the "optionally substituted heterocyclic group" may be a group such as formed by removing one hydrogen atom from the "heterocyclic ring" in the "optionally substituted heterocyclic ring" represented by the ring B.

In addition, examples of a "substituent" used for substitution on the "optionally substituted heterocyclic group" include the substituents (excluding an "optionally substituted heterocyclic group") listed above for the "optionally substituted heterocyclic ring" represented by the ring B.

Preferred examples of $R^{2b}$ and $R^{3b}$ include phenyl which may be substituted with a $C_{1-4}$ alkyl (e.g., methyl, ethyl) or a $C_{1-4}$ alkoxy (e.g., methoxy, ethoxy), a $C_{1-4}$ alkyl (e.g., methyl, ethyl), a halogeno (e.g., fluoro, chloro)-$C_{1-4}$ alkyl (e.g., methyl, ethyl), benzyl, naphthyl, pyridyl, thienyl, furyl and a hydrogen atom.

Preferred examples of the "optionally substituted acyl group" represented by $R^1$ include formyl, acetyl, a trihalogeno (e.g., fluoro)-acetyl, pyridylcarbonyl, thienylcarbonyl, furylcarbonyl, phenacyl, benzoyl, a $C_{1-4}$ alkyl (e.g., methyl)-benzoyl, a $C_{1-4}$ alkoxy (e.g., methoxy)-benzoyl, benzenesulfonyl, naphthylsulfonyl and thienylsulfonyl, with -(C=O)-$R^{2b}$ being more preferred [wherein $R^{2b}$ represents a $C_{1-6}$ alkyl group, a phenyl group which may be substituted with a $C_{1-6}$ alkoxy group, or a phenyl-$C_{1-6}$ alkyl group].

A "heterocyclic group" used in the "optionally substituted heterocyclic group" represented by $R^1$ may be a group such as formed by removing one hydrogen atom from a monocyclic heterocyclic ring, a bicyclic heterocyclic ring or a polycyclic (e.g., tricyclic or tetracyclic) heterocyclic ring. The heterocyclic ring may be either aromatic or non-aromatic. It may contain 1 to 6 heteroatoms selected form a nitrogen atom, an oxygen atom, a sulfur atom and the like. More specifically, a monocyclic heterocyclic group may be a group such as formed by removing one hydrogen atom from the "heterocyclic ring" in the "optionally substituted heterocyclic ring" represented by the ring B. In addition to this, other groups may be used, such as those formed by removing one hydrogen atom from a monocyclic heterocyclic ring such as triazole, thiadiazole, oxadiazole, oxathiadiazole, triazine or tetrazole. A bicyclic heterocyclic group may be a group such as formed by removing one hydrogen atom from a bicyclic heterocyclic ring such as indole, dihydroindole, isoindole, dihydroisoindole, benzofuran, dihydrobenzofuran, benzimidazole, benzoxazole, benzisooxazole, benzothiazole, indazole, quinoline, tetrahydroquinoline, isoquinoline, tetrahydroisoquinoline, tetrahydro-1H-1-benzazepine, tetrahydro-1H-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxole, benzothiazine or imidazopyridine. A polycyclic (e.g., tricyclic or tetracyclic) heterocyclic group may be a group such as formed by removing one hydrogen atom from a polycyclic heterocyclic ring such as acridine, tetrahydroacridine, pyrroloquinoline, pyrroloindole, cyclopentoindole or isoindolobenzazepine.

In particular, a "heterocyclic group" frequently used in the "optionally substituted heterocyclic group" is a group such as formed by removing one hydrogen atom from the above-mentioned monocyclic heterocyclic ring or bicyclic heterocyclic ring. Above all, a pyridyl group is preferred.

In addition, examples of a "substituent" used for substitution on the "optionally substituted heterocyclic group" include the substituents (excluding an "optionally substituted heterocyclic group") listed above for the "optionally substituted heterocyclic ring" represented by the ring B and the substituents listed above for the "optionally substituted hydrocarbon group" represented by $R^1$.

Preferred examples of $R^1$ include (i) a hydrogen atom, (ii) a $C_{1-6}$ alkyl group, (iii) a phenyl-$C_{1-6}$ alkyl group which may be substituted with a halogen atom, nitro, a $C_{1-6}$ alkyl or a $C_{1-6}$ alkoxy, and (iv) -(C=O)-$R^{2b}$ [wherein $R^{2b}$ represents a $C_{1-6}$ alkyl group, a phenyl group which may be substituted with a $C_{1-6}$ alkoxy group, or a phenyl-$C_{1-6}$ alkyl group].

More specific examples of those groups where the "aryl group" in the "optionally substituted aryl group" is condensed with an optionally substituted monocyclic heterocyclic ring include a phenyl group condensed with a monocyclic heterocyclic ring, represented by the following formula:

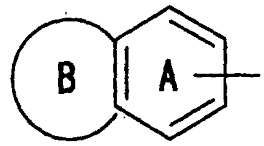

which may be formed by removing one hydrogen atom from a bicyclic condensed benzene ring such as 2,3-dihydrobenzofuran; 3,4-dihydro-2H-1-benzothiopyran; 2,3-dihydro-1H-indole; 1,2,3,4-tetrahydroquinoline; 2,3-dihydro-1H-isoindole; 1,2,3,4-tetrahydroisoquinoline; benzazepine (e.g., 2,3,4,5-tetrahydro-1H-1-benzazepine, 2,3,4,5-tetrahydro-1H-2-benzepine, 2,3,4,5-tetrahydro-1H-3-benzazepine); benzazocine (e.g., 1,2,3,4,5,6-hexahydro-1-benzazocine, 1,2,3,4,5,6-hexahydro-2-benzazocine, 1,2,3,4,5,6-hexahydro-3-benzazocine); benzazonine (e.g., 2,3,4,5,6,7-hexahydro-1H-1-benzazonine, 2,3,4,5,6,7-hexahydro-1H-2-benzazonine, 2,3,4,5,6,7-hexahydro-1H-3-benzazonine, 2,3,4,5,6,7-hexahydro-1H-4-benzazonine); benzoxazole (e.g., 2,3-dihydrobenzoxazole); benzothiazole (e.g., 2,3-dihydrobenzothiazole); benzimidazole (e.g., 2,3-dihydro-1H-benzimidazole); benzoxazine (e.g., 3,4-dihydro-1H-2,1-benzoxazine, 3,4-dihydro-1H-2,3-benzoxazine, 3,4-dihydro-2H-1,2-benzoxazine, 3,4-dihydro-2H-1,4-benzoxazine, 3,4-dihydro-2H-1,3-benzoxazine, 3,4-dihydro-2H-3,1-benzoxazine); benzothiazine (e.g., 3,4-dihydro-1H-2,1-benzothiazine, 3,4-dihydro-1H-2,3-benzothiazine, 3,4-dihydro-2H-1,2-benzothiazine, 3,4-dihydro-2H-1,4-benzothiazine, 3,4-dihydro-2H-1,3-benzothiazine, 3,4-dihydro-2H-3,1-benzothiazine); benzodiazine (e.g., 1,2,3,4-tetrdhydrocinnoline, 1,2,3,4-tetrahydrophthalazine, 1,2,3,4-tetrahydroquinazoline, 1,2,3,4-tetrahydroquinoxaline); benzoxathiine (e.g., 3,4-dihydro-1,2-benzoxathiine, 3,4-dihydro-2,1-benzoxathiine, 2,3-dihydro-1,4-benzoxathiine, 1,4-dihydro-2,3-benzoxathiine, 4H-1,3-benzoxathiine, 4H-3,1-benzoxathiine); benzodioxin (e.g., 3,4-dihydro-1,2-benzodioxin, 2,3-dihydro-1,4-benzodioxin, 1,4-dihydro-2,3-benzodioxin, 4H-1,3-benzodioxin); benzdithiine (e.g., 3,4-dihydro-1,2-benzdithiine, 2,3-dihydro-1,4-benzdithiine, 1,4-dihydro-2,3-benzdithiine, 4H-1,3-benzdithiine); benzoxazepine (e.g., 2,3,4,5-tetrahydro-1,2-benzoxazepine, 2,3,4,5-tetrahydro-1,3-benzoxazepine, 2,3,4,5-tetrahydro-1,4-benzoxazepine, 2,3,4,5-tetrahydro-1,5-benzoxazepine, 1,3,4,5-tetrahydro-2,1-behzoxazepine, 1,3,4,5-tetrahydro-2,3-benzoxazepine, 1,3,4,5-tetrahydro-2,4-benzoxazepine, 1,2,4,5-tetrahydro-3,1-benzoxazepine, 1,2,4,5-tetrahydro-3,2-benzoxazepine, 1,2,3,5-tetrahydro-4,1-benzoxazepine); benzothiazepine (e.g., 2,3,4,5-tetrahydro-1,2-benzothiazepine, 2,3,4,5-tetrahydro-1,4-benzothiazepine, 2,3,4,5-tetrahydro-1,5-benzothiazepine, 1,3,4,5-tetrahydro-2,1-benzothiazepine, 1,3,4,5-tetrahydro-2,4-benzothiazepine, 1,2,4,5-tetrahydro-3,1-benzothiazepine, 1,2,4,5-tetrahydro-3,2-benzothiazepine, 1,2,3,5-tetrahydro-4,1-benzothiazepine); benzodiazepine (e.g., 2,3,4,5-tetrahydro-1H-1,2-benzodiazepine, 2,3,4,5-tetrahydro-1H-1,3-benzodiazepine, 2,3,4,5-tetrahydro-1H-1,4-benzodiazepine, 2,3,4,5-tetrahydro-1H-1,5-benzodiazepine, 2,3,4,5-tetrahydro-1H-2,3-benzodiazepine, 2,3,4,5-tetrahydro-1H-2,4-benzodiazepine); benzodioxepine (e.g., 4,5-dihydro-1,3-benzodioxepine, 4,5-dihydro-3H-1,2-benzodioxepine, 2,3-dihydro-5H-1,4-benzodioxepine, 3,4-dihydro-2H-1,5-benzodioxepine, 4,5-dihydro- 1H-2,3-benzodioxepine, 1,5-dihydro-2,4-benzodioxepine); benzodithiepine (e.g., 4,5-dihydro-1H-2,3-benzothiepine, 1,5-dihydro-2,4-benzodithiepine, 3,4-dihydro-2H-1,5-benzodithiepine, 2,3-dihydro-5H-1,4-benzodithiepine); benzoxazocine (e.g., 3,4,5,6-tetrahydro-2H-1,5-benzoxazocine, 3,4,5,6-tetrahydro-2H-1,6-benzoxazocine); benzothiazocine (e.g., 3,4,5,6-tetrahydro-2H-1,5-benzothiazocine, 3,4,5,6-tetrahydro-2H-1,6-benzothiazocine); benzodiazocine (e.g., 1,2,3,4,5,6-hexahydro-1,6-benzodiazocine); benzoxathiocine (e.g., 2,3,4,5-tetrahydro-1,6-benzoxathiocine); benzodioxocine (e.g., 2,3,4,5-tetrahydro-1,6-benzodioxocine); benzotrioxepine (e.g., 1,3,5-benzotrioxepine, 5H-1,3,4-benzotrioxepine); benzoxathiazepine (e.g., 3,4-dihydro-1H-5,2,1-benzoxathiazepine, 3,4-dihydro-2H-5,1,2-benzoxathiazepine, 4,5-dihydro-3,1,4-benzoxathiazepine, 4,5-dihydro-3H-1,2,5-benzoxathiazepine); benzoxadiazepine (e.g., 2,3,4,5-tetrahydro-1,3,4-benzoxadiazepine); benzthiadiazepine (e.g., 2,3,4,5-tetrahydro-1,3,5-benzthiadiazepine); benzotriazepine (e.g., 2,3,4,5-tetrahydro-1H-1,2,5-benzotriazepine); 4,5-dihydro-1,3,2-benzooxathiepine, 4,5-dihydro-1H-2,3-benzoxathiepine, 3,4-dihydro-2H-1,5-benzoxathiepine, 4,5-dihydro-3H-1,2-benzoxathiepine, 4,5-dihydro-3H-2,1-benzoxathiepine, 2,3-dihydro-5H-1,4-benzoxathiepine or 2,3-dihydro-5H-4,1-benzoxathiepine; particularly 2,3,4,5-tetrahydro-1H-3-benzazepine, 2,3,4,5-tetrahydro-1H-2-benzazepine, 2,3-dihydro-1H-indole or 2,3,4,5-tetrahydro-1,4-benzoxazepine.

Preferred examples of those groups where the "aryl group" in the "optionally substituted aryl group" is condensed with an optionally substituted monocyclic heterocyclic ring include a group of the following formula:

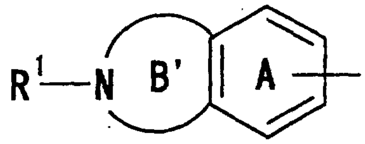

[wherein the ring B' represents a 5- to 9-membered nitrogen-containing heterocyclic ring which may be substituted with an oxo group, in addition to $R^1$, and the ring A and $R^1$ are as defined above].

Examples of a "5- to 9-membered nitrogen-containing heterocyclic ring" in the "5- to 9-membered nitrogen-containing heterocyclic ring which may be substituted with an oxo group" include a 5- to 9-membered nitrogen-containing heterocyclic group which may contain, in addition to carbon atoms and one nitrogen atom, 1 to 3 heteroatoms selected from a nitrogen atom, an oxygen atom, a sulfur atom and the like. Preferred for use is a 5- to 9-membered non-aromatic nitrogen-containing heterocyclic ring (e.g., pyrrolidine, piperidine, hexamethyleneimine, heptamethyleneimine, piperazine, homopiperazine, tetrahydrooxazepine, morpholine, thiomorpholine). More preferred examples of those groups where the "aryl group" in the "optionally substituted aryl group" is condensed with an optionally substituted monocyclic heterocyclic ring include a group of the following formula:

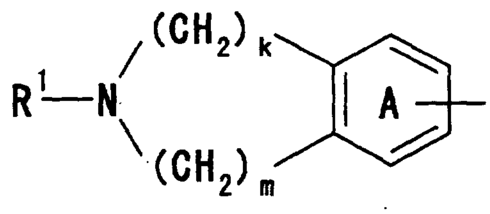

[wherein the ring A and $R^1$ are as defined above, k and m each independently represent an integer of 0 to 5, provided that 1<k+m<5], as well as a group of the following formula:

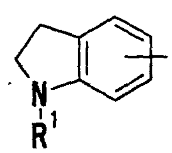 , 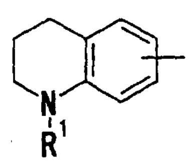 , 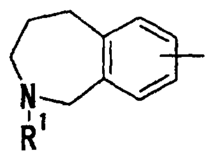 , 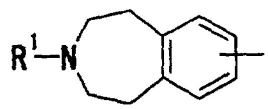

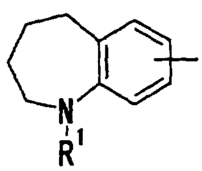 , 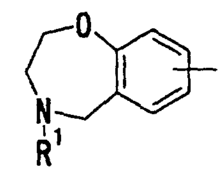 or 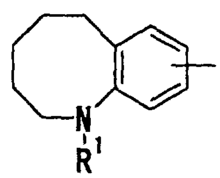

[wherein $R^1$ is as defined above]. Particularly preferred examples include a group of the following formula:

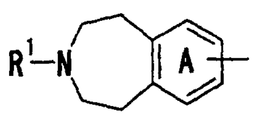

[wherein the ring A and $R^1$ are as defined above], as well as groups of the following formulae:

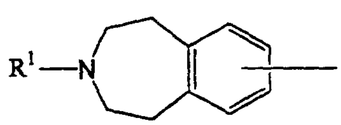 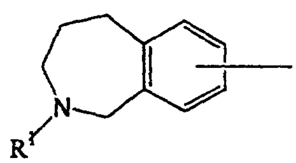 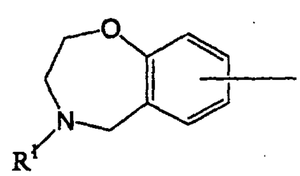

[wherein $R^1$ is as defined above].

Specific examples of those groups where the "aryl group" in the "optionally substituted aryl group" represented by Ar is condensed with an optionally substituted bicyclic heterocyclic ring or with the same or different two monocyclic rings (at least one of which is a monocyclic heterocyclic ring) include a group of the following formula:

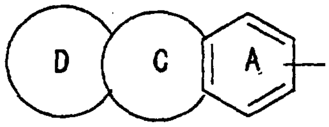
,
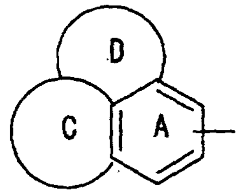

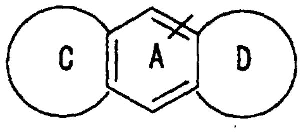
or
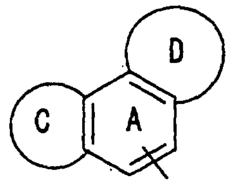

[wherein the ring A is as defined above, and one of the rings C and D represents an optionally substituted heterocyclic ring and the other represents an optionally substituted 5- to 9-membered ring which may contain a heteroatom(s)].

Examples of a "heterocyclic ring" used in the "optionally substituted heterocyclic ring" represented by the rings C and D include 4- to 14-membered heterocyclic rings, preferably 5- to 9-membered heterocyclic rings, which may be either aromatic or non-aromatic. These rings may contain 1 to 3 heteroatoms selected from a nitrogen atom, an oxygen atom, a sulfur atom and the like. Specific examples include pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine, and thiomorpholine.

A "substituent" on the "optionally substituted heterocyclic ring" has the same meaning as defined for the "substituent" on the "optionally substituted heterocyclic ring" represented by the ring B.

Examples of a "5- to 9-membered ring which may contain a heteroatom(s)" used in the "optionally substituted 5- to 9-membered ring which may contain a heteroatom(s)" represented by the rings C and D include 5- to 9-membered heterocyclic rings (e.g., 5- to 9-membered saturated or unsaturated heterocyclic rings such as pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine and thiomorpholine) and 5- to 9-membered carbocyclic rings. The "5- to 9-membered carbocyclic rings" may be saturated or unsaturated rings, including benzene, cyclopentane, cyclopentene, cyclohexane, cyclohexene, cyclohexadiene, cycloheptane, cycloheptene and cycloheptadiene. Among these, benzene or cyclohexane is preferred.

A "substituent" on the "optionally substituted 5- to 9-membered ring which may contain a heteroatom(s)" has the same meaning as the "substituent on any carbon atom in the ring B" defined for the "optionally substituted heterocyclic ring" represented by the ring B.

More specific examples of those groups where the "aryl group" in the "optionally substituted aryl group" represented by Ar is condensed with an optionally substituted bicyclic heterocyclic ring include:

(1) a phenyl group condensed with a bicyclic heterocyclic ring, represented by the following formula:

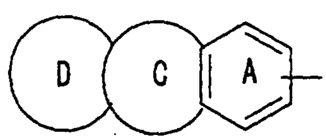

which may be formed by removing one hydrogen atom from a tricyclic condensed benzene ring such as carbazole, 1,2,3,4,4a,9a-hexahydrocarbazole, 9,10-dihydroacridine, 1,2,3,4-tetrahydroacridine, 10,11-dihydro-5H-dibenz[b,f]azepine, 5,6,7,12-tetrahydrodibenz[b,g]azocine, 6,11-dihydro-5H-dibenz[b,e]azepine, 6,7-dihydro-5H-dibenz[c,e]azepine, 5,6,11,12-tetrahydrodibenz[b,f]azocine, dibenzofuran, 9H-xanthene, 10,11-dihydrodibenz[b,f]oxepine, 6,11-dihydrodibenz[b,e]oxepine, 6,7-dihydro-5H-dibenz[b,g]oxocine, dibenzothiophene, 9H-thioxanthene, 10,11-dihydrodibenzo[b,f]thiepine, 6,11-dihydrodibenzo[b,e]thiepine, 6,7-dihydro-5H-dibenzo[b,g]thiocine, 10H-phenothiazine, 10H-phenoxazine, 5,10-dihydrophenazine, 10,11-dibenzo[b,f][1,4]thiazepine, 10,11-dihydrodibenz[b,f][1,4]oxazepine, 2,3,5,6,11,11a-hexahydro-1H-pyrrolo[2,1-b][3]benzazepine, 10,11-dihydro-5H-dibenzo[b,e][1,4]diazepine,

5,11-dihydrodibenz[b,e][1,4]oxazepine, 5,11-dihydrodibenzo[b,f][1,4]thiazepine, 10,11-dihydro-5H-dibenzo[b,e][1,4]diazepine or 1,2,3,3a,8,8a-hexahydropyrrolo[2,3-b]indole;

(2) a phenyl group condensed with a bicyclic heterocyclic ring, represented by the following formula:

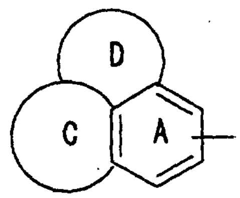

which may be formed by removing one hydrogen atom from a tricyclic condensed benzene ring such as 1H,3H-naphtho[1,8-cd][1,2]oxazine, naphtho[1,8-de]-1,3-oxazine, naphtho[1,8-de]-1,2-oxazine, 1,2,2a,3,4,5-hexahydrobenz[cd]indole, 2,3,3a,4,5,6-hexahydro-1H-benzo[de]quinoline, 4H-pyrrolo[3,2,1-ij]quinoline, 1,2,5,6-tetrahydro-4H-pyrrolo(3,2,1-ij]quinoline,

5,6-dihydro-4H-pyrrolo[3,2,1-ij]quinoline, 1H,5H-benzo[ij]quinolizine, azepino[3,2,1-hi]indole, 1,2,4,5,6,7-hexahydroazepino[3,2,1-hi]indole, 1H-pyrido[3,2,1-jk][1]benzazepine, 5,6,7,8-tetrahydro-1H-pyrido[3,2,1-jk][1]benzazepine, 1,2,5,6,7,8-hexahydro-1H-pyrido[3,2,1-jk][1]benzazepine, 2,3-dihydro-1H-benz[de]isoquinoline, 1,2,3,4,4a,5,6,7-octahydronaphtho[1,8-bc]azepine or 2,3,5,6,7,8-hexahydro-1H-pyrido[3,2,1-jk][1]benzazepine;

(3) a phenyl group condensed with the same or different two monocyclic rings (at least one of which is a monocyclic heterocyclic ring), represented by the following formula:

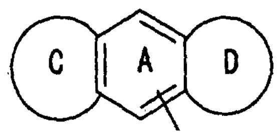

which may be formed by removing one hydrogen atom from a tricyclic condensed benzene ring such as 1,2,3,5,6,7-hexahydrobenzo[1,2-b:4,5-b']dipyrrole or 1,2,3,5,6,7-hexahydrocyclopento[f]indole; and

(4) a phenyl group condensed with the same or different two rings (at least one of which is a monocyclic heterocyclic ring), represented by the following formula:

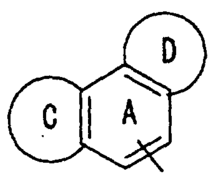

which may be formed by removing one hydrogen atom from a tricyclic condensed benzene ring such as 1,2,3,6,7,8-hexahydrocyclopento[e]indole or 2,3,4,7,8,9-hexahydro-1H-cyclopenta[f]quinoline.

Preferred examples of those groups where the "aryl group" in the "optionally substituted aryl group" represented by Ar is condensed with an optionally substituted bicyclic heterocyclic ring include a group of the following formula:

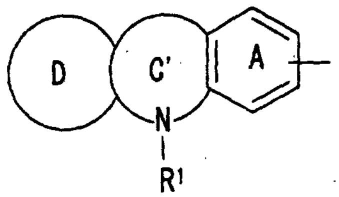,
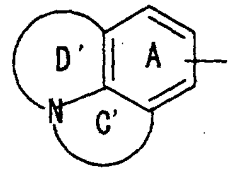

or
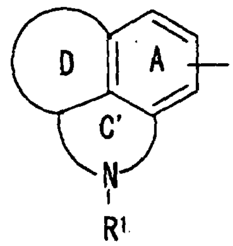

[wherein the rings C' and D' each represent a 5- to 9-membered nitrogen-containing heterocyclic ring which may be substituted with an oxo group, in addition to $R^1$, and the ring A, the ring D and $R^1$ are as defined above].

Examples of a "5- to 9-membered nitrogen-containing heterocyclic ring" in the "5- to 9-membered nitrogen-containing heterocyclic ring which may be substituted with an oxo group" include a 5- to 9-membered nitrogen-containing heterocyclic group which may contain, in addition to carbon atoms and one nitrogen atom, 1 to 3 heteroatoms selected from a nitrogen atom, an oxygen atom, a sulfur atom and the like. Preferred for use is a 5- to 9-membered non-aromatic nitrogen-containing heterocyclic ring (e.g., pyrrolidine, piperidine, hexamethyleneimine, heptamethyleneimine, piperazine, homopiperazine, tetrahydrooxazepine, morpholine, thiomorpholine).

More preferred examples of those groups where the "aryl group" in the "optionally substituted aryl group" represented by Ar is condensed with an optionally substituted bicyclic heterocyclic ring include a group of the following formula:

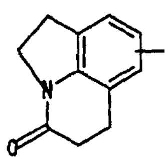,
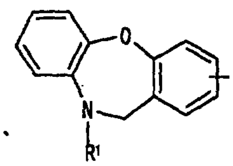,
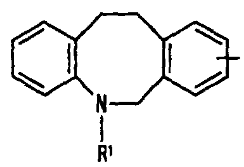,
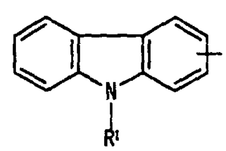,

or
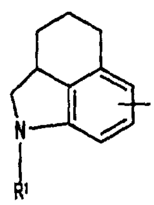

[wherein $R^1$ is as defined above].

Specific examples of those groups where the "phenyl group" in the "optionally substituted and condensed phenyl group" is condensed with an optionally substituted tricyclic heterocyclic ring include a group of the following formula:

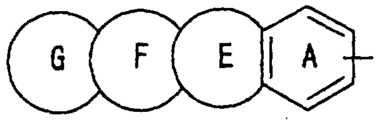

or

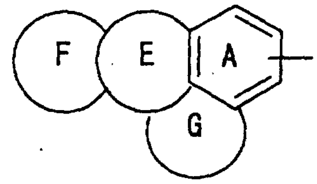

[wherein the ring A is as defined above, and at least one of the rings E, F and G represents an optionally substituted heterocyclic ring and the others represent an optionally substituted 5- to 9-membered ring which may contain a heteroatom(s)].

Examples of a "heterocyclic ring" used in and a "substituent" used for substitution on the "optionally substituted heterocyclic ring" represented by the rings E, F and G include the heterocyclic rings and substituents listed above for the "optionally substituted heterocyclic ring" represented by the rings C and D.

Examples of a "5- to 9-membered ring which may contain a heteroatom(s)" used in and a "substituent" used for substitution on the "optionally substituted 5- to 9-membered ring which may contain a heteroatom(s)" represented by the rings E, F and G include the 5- to 9-membered rings which may contain a heteroatom(s) and substituents listed above for the "optionally substituted 5- to 9-membered ring which may contain a heteroatom(s)" represented by the rings C and D.

More specific examples of those groups where the "phenyl group" in the "optionally substituted and condensed phenyl group" is condensed with an optionally substituted tricyclic heterocyclic ring include:

(1) a phenyl group condensed with a tricyclic heterocyclic ring, represented by the following formula:

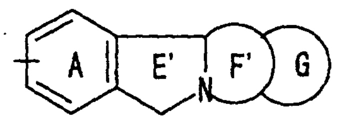

[wherein the rings E' and F' are as defined below], which may be formed by removing one hydrogen atom from a tetracyclic condensed benzene ring such as 2H-isoindolo[2,1-e]purine, 1H-pyrazolo[4',3':3,4]pyrido[2,1-a]isoindole, 1H-pyrido[2',3':4,5]imidazo[2,1-a]isoindole, 2H,6H-pyrido[1',2':3,4]imidazo[5,1-a]isoindole, 1H-isoindolo[2,1-a]benzimidazole, 1H-pyrido[3',4':4,5]pyrrolo[2,1-a]isoindole, 2H-pyrido[4',3':4,5]pyrrolo[2,1-a]isoindole, 1H-isoindolo[2,1-a]indole, 2H-isoindolo[1,2-a]isoindole, 1H-cyclopenta[4,5]pyrimido[2,1-a]isoindole, 2H,4H-pyrano[4',3':4,5][1,3]oxazino[2,3-a]isoindole, 2H-isoindolo[2,1-a][3,1]benzoxazine, 7H-isoindolo[1,2-b][1,3]benzoxazine, 2H-pyrido[2',1':3,4]pyrazino[2,1-a]isoindole, pyrido[2',3':4,5]pyrimido[2,1-a]isoindole, pyrido[3',2':5,6]pyrimido[2,1-a]isoindole, 1H-pyrido[1',2':3,4]pyrimido[2,1-a]isoindole, isoindolo[2,1-a]quinazoline, isoindolo[2,1-a]quinoxaline, isoindolo[1,2-a]isoquinoline, isoindolo[2,1-b]isoquinoline, isoindolo[2,1-a]quinoline, 6H-oxazino[3',4':3,4][1,4]diazepino[2,1-a]isoindole, azepino[Z',1':3,4]pyrazino[2,1-a]isoindole, 2H,6H-pyrido[2',1':3,4][1,4]diazepino[2,1-a]isoindole, 1H-isoindolo[1,2-b][1,3,4]benzotriazepine, 2H-isoindolo[2,1-a][1,3,4]benzotriazepine, isoindolo[2,1-d][1,4]benzoxazepine, 1H-isoindolo[2,1-b][2,4]benzodiazepine, 1H-isoindolo[2,1-c][2,3]benzodiazepine, 2H-isoindolo[1,2-a][2,4]benzodiazepine, 2H-isoindolo[2,1-d][1,4]benzodiazepine, 5H-indolo[2,1-b][3]benzazepine, 2H-isoindolo[1,2-a][2]benzazepine, 2H-isoindolo[1,2-b][3]benzazepine, 2H-isoindolo[2,1-b][2]benzazepine, 2H-isoindolo[1,2-b][1,3,4]benzooxadiazocine, isoindolo[2,1-b][1,2,6]benzotriazocine or 5H-4,8-methano-1H-[1,5]diazacycloundecino[1,11-a]indole;

(2) a phenyl group condensed with a tricyclic heterocyclic ring, represented by the following formula:

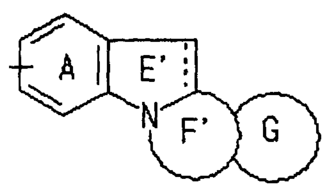

[wherein - - - represents a single bond or a double bond, and the rings E' and G' are as defined below], which may be formed by removing one hydrogen atom from a tetracyclic condensed benzene ring such as 1H,4H-pyrrolo[3',2':4,5]pyrrolo[3,2,1-ij]quinoline, pyrrolo[3,2,1-jk]carbazole,1H-furo[2',3':4,5]pyrrolo[3,2,1-ij]quinoline, 1H,4H-cyclopenta[4,5]pyrrolo[1,2,3-de]quinoxaline, 1H,4H-cyclopenta[4,5]pyrrolo[3,2,1-ij]quinoline, pyrido[3',4':4,5]pyrrolo[1,2,3-de]benzoxazine, [1,4]oxazino[2,3,4-jk]carbazole, 1H,3H-[1,3]oxazino(5,4,3-jk]carbazole, pyrido[3',4':4,5]pyrrolo[1,2,3-de][1,4]benzothiazine, 4H-pyrrolo[3,2,1-de]phenanthridine, 4H,5H-pyrido[3,2,1-de]phenanthridine, 1H,4H-3a,6a-diazafluoroanthene, 1-oxa-4,6a-diazafluoroanthene, 4-oxa-2,10b-diazafluoroanthene, 1-thia-4,6a-diazafluoroanthene, 1H-pyrazino[3,2,1-jk]carbazole, 1H-indolo[3,2,1-de][1,5]naphthylidine, benzo[b]pyrano[2,3,4-hi]indolizine, 1H,3H-benzo[b]pyrano[3,4,5-hi]indolizine, 1H,4H-pyrano[2',3':4,5]pyrrolo[3,2,1-ij]quinoline, 1H,3H-benzo[b]thiopyrano[3,4,5-hi]indolizine, 1H-pyrido[3,2,1-jk]carbazole, 4H-3-oxa-11b-azacyclohepta[jk]fluorene, 2H-azepino[1',2':1,2]pyrimidino[4,5-b]indole, 1H,4H-cyclohepta[4,5]pyrrolo[1,2,3-de]quinoxaline, 5H-pyrido[3',4':4,5]pyrrolo[1,2,3-ef][1,5]benzoxazepine, 4H-pyrido[3',4':4,5]pyrrolo[3,2,1 5H-pyrido[3',4':4,5]pyrrolo[1,2,3-ef][1,5]benzothiazepine, 5H-pyrido[4',3':4,5]pyrrolo[1,2,3-ef][1,5]benzothiazepine, [1,2,4]triazepino [6,5,4-jk]carbazole, [1,2,4]triazepino[6,7,1-jk]carbazole, [1,2,5]triazepino[3,4,5-jk]carbazole, 5H-[1,4]oxazepino [2,3,4-jk]carbazole, 5H-[1,4]thiazepino[2,3,4-jk]carbazole, [1,4]diazepino[3,2,1-jk]carbazole, [1,4]diazepino [6,7,1-jk]carbazole, azepino[3,2,1-jk]carbazole, 1H-cycloocta[4,5]pyrrolo[1,2,3-de]quinoxaline or 1H-cycloocta [4,5]pyrrolo[3,2,1-ij]quinoline;

(3) a phenyl group condensed with a tricyclic heterocyclic ring, represented by the following formula:

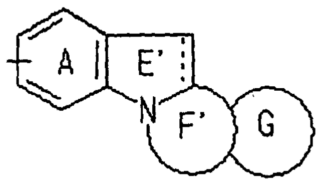

[wherein --- represents a single bond or a double bond, and the rings E' and F' are as defined below], which may be formed by removing one hydrogen atom from a tetracyclic condensed benzene ring such as 1H-indolo[1,2-a] benzimidazole, 1H-indolo[1,2-b]indazole, pyrrolo[2',1':3,4]pyrazino[1,2-a]indole, 1H,5H-pyrrolo[1',2':4,5]pyrazino [1,2-a]indole, 2H-pyrido[2',3':3,4]pyrrolo[1,2-a]indole, 1H-pyrrolo[2',3':3,4]pyrido[1,2-a]indole, 1H-indolo[1,2-a]indole, 6H-isoindolo[2,1-a]indole, 6H-indolo[1,2-c][1,3]benzoxazine, 1H-indolo[1,2-b][1,2]benzothiazine, pyrimido [4',5':4,5]pyrimido[1,6-a]indole, pyrazino[2',3':3,4]pyrido[1,2-a]indole, 6H-pyrido[1',2':3,4]pyrimido[1,6-a]indole, indolo[1,2-b]cinnoline, indolo[1,2-a]quinazoline, indolo[1,2-c]quinazoline, indolo[2,1-b]quinazoline, indolo[1,2-a]quinoxaline, indolo[1,2-a][1,8]naphthylidine, indolo[1,2-b]-2,6-naphthylidine, indolo[1,2-b] [2,7]naphthylidine, indolo [1,2-h]-1,7-naphthylidine, indolo[1,2-b]isoquinoline, indolo[2,1-a]isoquinoline, indolo[1,2-a]quinoline, 2H,6H-pyrido[2',1':3,4][1,4]diazepino[1,2-a]indole, 1H-indolo[2,1-c][1,4]benzodiazepine, 2H-indolo[1,2-d][1,4]benzodiazepine, 2H-indolo[2,1-a][2,3]benzodiazepine, 2H-indolo[2,1-b][1,3]benzodiazepine, 1H-indolo[1,2-b][2]benzazepine, 2H-indolo[1,2-a][1]benzazepine, 2H-indolo[2,1-a][2]benzazepine, indolo[1,2-e][1,5]benzodiazocine or indolo[2,1-b][3]benzazocine; and

(4) a phenyl group condensed with a tricyclic heterocyclic ring, represented by the following formula:

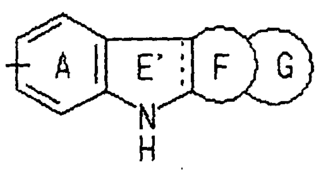

[wherein --- represents a single bond or a double bond, and the ring E' is as defined below], such as 1H-imidazo [1',2':1,2]pyrido[3,4-b]indole, 1H-imidazo[1',2':1,6]pyrido[4,3-b]indole, 1H-imidazo[1',5':1,2]pyrido[3,4-b]indole, 1H-imidazo[1',5':1,6]pyrido[4,3-b]indole, 1H-pyrido[2',1':2,3]imidazo[4,5-b]indole, imidazo[4,5-a]carbazole, imidazo[4,5-c]carbazole, pyrazolo[3,4-c]carbazole, 2H-pyrazino[1',2':1,5]pyrrolo[2,3-b]indole, 1H-pyrrolo[1',2':1,2]pyrimido[4,5-b]indole, 1H-indolizino[6,7-b]indole, 1H-indolizino[8,7-b]indole, indolo[2,3-b]indole, indolo[3,2-b]indole, pyrrolo[2,3-a]carbazole, pyrrolo[2,3-b]carbazole, pyrrolo[2,3-c]carbazole, pyrrolo[3,2-a]carbazole, pyrrolo[3,2-b] carbazole, pyrrolo[3,2-c]carbazole, pyrrolo[3,4-a]carbazole, pyrrolo[3,4-b]carbazole, pyrrolo[3,4-c]carbazole, 1H-pyrido[3',4':4,5]furo[3,2-b]indole, 1H-furo[3,4-a]carbazole, 1H-furo[3,4-b]carbazole, 1H-furo[3,4-c]carbazole, 2H-furo(2,3-a]carbazole, 2H-furo[2,3-c]carbazole, 2H-furo[3,2-a]carbazole, 2H-furo[3,2-c]carbazole, 1H-pyrido[3',4':4,5]thieno[2,3-b]indole, thieno[3',2':5,6]thiopyrano[4,3-b]indole, thieno[3',4':5,6]thiopyrano[4,3-b]indole, 1H-[1]benzothieno[2,3-b]indole, 1H-[1]benzothieno[3,2-b]indole, 1H-thieno[3,4-a]carbazole, 2H-thieno[2,3-b]carbazole, 2H-thieno[3,2-a]carbazole, 2H-thieno[3,2-b]carbazole, cyclopenta[4,5]pyrrolo[2,3-f]quinoxaline, cyclopenta[5,6] pyrido[2,3-b]indole, pyrido[2',3':3,4]cyclopenta[1,2-b]indole, pyrido[2',3':4,5]cyclopenta[1,2-b]indole, pyrido[3',4':3,4]cyclopenta[1,2-b]indole, pyrido[3',4':4,5]cyclopenta[1,2-b]indole, pyrido[4',3':4,5]cyclopenta[1,2-b]indole, 1H-cyclopenta[5,6]pyrano[2,3-b]indole, 1H-cyclopenta[5,6]thiopyrano[4,3-b]indole, cyclopenta[a]carbazole, cyclopenta[c]carbazole, indeno[1,2-b]indole, indeno[2,1-b]indole, [1,2,4]triazino[4',3':1,2]pyrido[3,4-b]indole, 1,3,5-triazino[1',2':1,1]pyrido[3,4-b]indole, 1H-[1,4]oxazino[4',3':1,2]pyrido[3,4-b]indole, 1H-[1,4]oxazino[4',3':1,6] pyrido[3,4-b]indole, 4H-[1,3]oxazino[3',4':1,2]pyrido[3,4-b]indole, indolo[3,2-b][1,4]benzoxazine, 1,3-oxazino [6,5-b]carbazole, 2H-pyrimido[2',1':2,3][1,3]thiazino[5,6-b]indole, 2H-[1,3]thiazino[3',2':1,2]pyrido[3,4-b]indole, 4H-[1,3]thiazino[3',4':1,2]pyrido[3,4-b]indole, indolo[2,3-b][1,4]benzothiazine, indolo[3,2-b][1,4]benzothiazine, indolo[3,2-c][2,1]benzothiazine, 1,4-thiazino[2,3-a]carbazole, [1,4]thiazino[2,3-b]carbazole, [1,4]thiazino[2,3-c]carbazole, 1,4-thiazino[3,2-b]carbazole, 1,4-thiazino[3,2-c]carbazole, 1H-indolo[2,3-g]pteridine, 1H-indolo[3,2-g]pteridine, pyrazino[1',2':1,2]pyrido[3,4-b]indole, pyrazino[1',2':1,2]pyrido[4,3-b]indole, 1H-pyrido[2',3':5,6]pyrazino[2,3-b]indole, 1H-pyrido[3',2':5,6]pyrazino(2,3-b]indole, 1H-pyrido[3',4':5,6]pyrazino[2,3-b]indole, pyrido[1',2':1,2]pyrimido[4,5-b]indole, pyrido[1',2':1,2]pyrimido[5,4-b]indole, pyrido[2',1':2,3]pyrimido[4,5-b]indole, pyrimido[1',2':1,2]pyrido[3,4-b]indole, pyrimido[1',2':1,6]pyrido[3,4-b]indole, pyrimido[5',4':5,6]pyrano[2,3-b]indole, pyridazino[4',5':5,6]thiopyrano[4,5-b]indole, 1H-indolo[3,2-c]cinnoline, 1H-indolo[2,3-b]quinoxaline, 1H-pyrazino[2,3-a]carbazole, 1H-pyrazino[2,3-b]carbazole, 1H-pyrazino[2,3-c]carbazole, 1H-pyridazino[3,4-c]carbazole, 1H-pyridazino[4,5-b]carbazole, 1H-pyrimido[4,5-a]carbazole, 1H-pyrimido[4,5-c]carbazole, 1H-pyrimido[5,4-a]carbazole, 1H-pyrimido[5,4-b]carbazole, 1H-pyrimido[5,4-c]carbazole, 7H-1,4-dioxino[2',3':5,6][1,2]dioxino[3,4-b]indole, 6H-[1,4]benzodioxino[2,3-b]indole, 6H-[1,4]benzodithiino[2,3-b]indole, 1H-indolo[2,3-b]-1,5-naphthylidine, 1H-indolo[2,3-b][1,6]naphthylidine, 1H-indolo[2,3-b][1,8]naphthylidine, 1H-indolo[2,3-c]-1,5-naphthylidine, 1H-indolo[2,3-c][1,6]naphthylidine, 1H-indolo[2,3-c][1,7]naphthylidine, 1H-indolo[2,3-c][1,8]naphthylidine, 1H-indolo[3,2-b]-1,5-naphthylidine, 1H-indolo[3,2-b][1,7]naphthylidine, 1H-indolo[3,2-b][1,8]naphthylidine, 1H-indolo[3,2-c][1,8]naphthylidine, indolo[2,3-a]quinolizine, indolo[2,3-b]quinolizine, indolo[3,2-a]quinolizine, indolo[3,2-b]quinolizine, pyrano[4',3':5,6]pyrido[3,4-b]indole, pyrido[4',3':4,5]pyrano[3,2-b]indole, pyrido[4',3':5,6]pyrano[2,3-b]indole, pyrido[4',3':5,6]pyrano[3,4-b]indole, 1H-indolo[2,3-c]isoquinoline, 1H-indolo[3,2-c]isoquinoline, 1H-indolo[2,3-c]quinoline, 1H-indolo[3,2-c]quinoline, 1H-pyrido[2,3-a]carbazole, 1H-pyrido[2,3-b]carbazole, 1H-pyrido[2,3-c]carbazole, 1H-pyrido[3,2-a]carbazole, 1H-pyrido[3,2-b]carbazole, 1H-pyrido[3,2-c]carbazole, 1H-pyrido[3,4-a]carbazole, 1H-pyrido[3,4-b]carbazole, 1H-pyrido[3,4-c]carbazole, 1H-pyrido[4,3-a]carbazole, 1H-pyrido[4,3-b]carbazole, 1H-pyrido[4,3-c]carbazole, 1H-quindoline, 1H-quinindoline, 1H-pyrano[3',4':5,6]pyrano[4,3-b]indole, [1]benzopyrano[2,3-b]indole, [1]benzopyrano[3,2-b]indole, [1]benzopyrano[3,4-b]indole, [1]benzopyrano[4,3-b]indole, [2]benzopyrano[4,3-b]indole, pyrano[2,3-a]carbazole, pyrano[2,3-b]carbazole, pyrano[2,3-c]carbazole, pyrano[3,2-a]carbazole, pyrano[3,2-c]carbazole, pyrano[3,4-a]carbazole, 1H-phosphinolino[4,3-b]indole, [1]benzothiopyrano[2,3-b]indole, [1]benzothiopyrano[3,2-b]indole, [1]benzothiopyrano[3,4-b]indole, [1]benzothiopyrano[4,3-b]indole, [2]benzothiopyrano[4,3-b]indole, 1H-benzo[a]carbazole, 1H-benzo[b]carbazole, 1H-benzo[c]carbazole, [1,6,2]oxathiazepino[2',3':1,2]pyrido[3,4-b]indole, 1H-azepino[1',2':1,2]pyrido[3,4-b]indole, 1H-pyrido[1',2':1,2]azepino[4,5-b]indole, 2H-pyrido[1',2':1,2]azepino[3,4-b]indole, 1H-pyrido[3',2':5,6]oxepino[3,2-b]indole, 1H-pyrido[4',3':5,6]oxepino[3,2-b]indole, 2H-pyrido[2',3':5,6]oxepino[2,3-b]indole, 2H-pyrido[2',3':5,6]oxepino[3,2-b]indole, 2H-pyrido[3',4':5,6]oxepino[3,2-b]indole, pyrido[2',3':4,5]cyclohepta[1,2-b]indole, pyrido[3',2':3,4]cyclohepta[1,2-b]indole, pyrido[3',4':4,5]cyclohepta[1,2-b]indole, pyrido[3',4':5,6]cyclohepta[1,2-b]indole, 2H-pyrano[3',2':2,3]azepino[4,5-b]indole, 1H-indolo[3,2-b][1,5]benzoxazepine, 1H-indolo[3,2-d][1,2]benzoxazepine, 1H-indolo[2,3-c][1,5]benzothiazepine, [1,4]diazepino[2,3-a]carbazole, indolo[2,3-b][1,5]benzodiazepine, indolo[2,3-d][1,3]benzodiazepine, indolo[3,2-b][1,4]benzodiazepine, indolo[3,2-b][1,5]benzodiazepine, indolo[3,2-d][1,3]benzodiazepine, indolo[3,2-d][2,3]benzodiazepine, indolo[2,3-a][3]benzazepine, indolo[2,3-c][1]benzazepine, indolo[2,3-d][1]benzazepine, indolo[2,3-d][2]benzazepine, indolo[3,2-b][1]benzazepine, indolo[3,2-c][1]benzazepine, indolo[3,2-d][1]benzazepine, 1H-indolo[2,1-b][3]benzazepine, 1H-[1]benzoxepino[5,4-b]indole, 1H-[2]benzoxepino[4,3-b]indole, 1H-[1]benzothiepino[4,5-b]indole, 1H-[1]benzothiepino[5,4-b]indole, benzo[3,4]cyclohepta[1,2-b]indole, benzo[4,5]cyclohepta[1,2-b]indole, benzo[5,6]cyclohepta[1,2-b]indole, benzo[6,7]cyclohepta[1,2-b]indole, cyclohepta[b]carbazole, 4H-[1,5]oxazocino[5',4':1,6]pyrido[3,4-b]indole, azocino[1',2':1,2]pyrido[3,4-b]indole, 2,6-methano-2H-azecino[4,3-b]indole, 3,7-methano-3H-azecino[5,4-b]indole, pyrido[1',2':1,8]azocino[5,4-b]indole, pyrido[4',3':6,7]oxocino[2,3-b]indole, pyrido[4',3':6,7]oxocino[4,3-b]indole; 1,5-methano-1H-azecino[3,4-b]indole, 2,6-methano-1H-azecino[5,4-b]indole, 1H-pyrido[3',4':5,6]cycloocta[1,2-b]indole, 1,4-ethanooxocino[3,4-b]indole, pyrano[3',4':5,6]cycloocta[1,2-b]indole, 1H-indolo[2,3-c][1,2,5,6]benzotetrazocine, 1H-indolo[2,3-c][1,6]benzodiazocine, 6,13b-methano-13bH-azecino[5,4-b]indole, oxocino[3,2-a]carbazole, 1H-benzo[g]cycloocta[b]indole, 6,3-(iminomethano)-2H-1,4-thiazonino[9,8-b]indole, 1H,3H-[1,4]oxazonino[4',3':1,2]pyrido[3,4-b]indole, 2H-3,6-ethanoazonino[5,4-b]indole, 2H-3,7-methanoazacycloundecino[5,4-b]indole, 1H-6,12b-ethanoazonino[5,4-b]indole, indolo[3,2-e][2]benzazonine, 5,9-methanoazacycloundecino[5,4-b]indole, 3,6-ethano-3H-azecino[5,4-b]indole, 3,7-methano-3H-azacycloundecino[5,4-b]indole, pyrano[4',3':8,9]azecino[5,4-b]indole, 1H-indolo[2,3-c][1,7]benzodiazecine or 1H-indolo[3,2-e][2]benzazecine.

Examples further include a group which may be formed by removing one hydrogen atom from a tetracyclic condensed benzene ring such as benzo[e]pyrrolo[3,2-b]indole, benzo[e]pyrrolo[3,2-g]indole, benzo[e]pyrrolo[3,2,1-hi]indole, benzo[e]pyrrolo[3,4-b]indole, benzo[g]pyrrolo[3,4-b]indole, 1H-benzo[f]pyrrolo[1,2-a]indole, 1H-benzo[g]pyrrolo[1,2-a]indole, 2H-benzo[e]pyrrolo[1,2-a]indole, 1H-benzo[f]pyrrolo[2,1-a]isoindole, 1H-benzo[g]pyrrolo[2,1-a]isoindole, 2H-benzo[e]pyrrolo[2,1-a]isoindole, isoindolo[6,7,1-cde]indole, spiro[cyclohexane-1,5'-[5H]pyrrolo[2,1-a]isoindole], isoindolo[7,1,2-hij]quinoline, 7,11-methanoazocino[1,2-a]indole, 7,11-methanoazocino[2,1-a]isoindole, dibenz[cd,f]indole, dibenz[cd,g]indole, dibenz[d,f]indole, 1H-dibenz[e,g]indole, 1H-dibenz[e,g]isoindole, naphtho[1,2,3-cd]indole, naphtho[1,8-ef]indole, naphtho[1,8-fg]indole, naphtho[3,2,1-cd]indole, 1H-naphtho[1,2-e]indole, 1H-naphtho[1,2-f]indole, 1H-naphtho[1,2-g]indole, 1H-naphtho[2,1-e]indole, 1H-naphtho[2,3-e]indole, 1H-naphtha[1,2-f]isoindole, 1H-naphtho[2,3-e]isoindole, spiro[1H-carbazole-1,1'-cyclohexane], spiro[2H-carbazole-2,1'-cyclohexane], spiro[3H-carbazole-3,1'-cyclohexane], cyclohepta[4,5]pyrrolo[3,2-f]quinoline, cyclohepta[4,5]pyrrolo[3,2-h]quinoline, azepino[4,5-b]benz[e]indole, 1H-azepino[1,2-a]benz[f]indole, 1H-azepino[2,1-a]benz[f]isoindole, benzo[e]cyclohepta[b]indole or benzo[g]cyclohepta[b]indole, and

(5) a phenyl group condensed with a tricyclic heterocyclic ring, represented by the following formula:

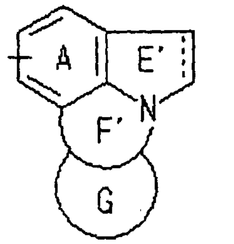

[wherein --- represents a single bond or a double bond, and the rings E' and F' are as defined below], which may be formed by removing one hydrogen atom from a tetracyclic condensed benzene ring such as 1H-dipyrrolo[2,3-b:3',2',1'-hi]indole, spiro[cyclopentane-1,2'(1'H)-pyrrolo[3,2,1-hi]indole], spiro[imidazolidine-4,1'(2'H)-[4H]pyrrolo[3,2,1-ij]quinoline], pyrido[2,3-b]pyrrolo[3,2,1-hi]indole, pyrido[4,3-b]pyrrolo[3,2,1-hi]indole, benzo[de]pyrrolo[3,2,1-ij]quinoline, 3H-pyrrolo[3,2,1-de]acridine, 1H-pyrrolo[3,2,1-de]phenanthridine, spiro[cyclohexane-1,6'-[6H]pyrrolo[3,2,1-ij]quinoline], 4,9-methanopyrrolo[3,2,1-1m][1]benzoazocine, spire[cycloheptane-1,6'-[6H]pyrrolo[3,2,1-ij]quinoline], 1H-pyrano[3,4-d]pyrrolo[3,2,1-jk][1]benzazepine, 3H-benzo[b]pyrrolo[3,2,1-jk][4,1]benzoxazepine, 7H-indolo[1,7-ab][4,1]benzaxazepine, benzo[b]pyrrolo[3,2,1-jk][1,4]benzodiazepine, indolo[1,7-ab][1,4]benzodiazepine, indolo[1,7-ab][1]benzazepine, indolo[7,1-ab][3]benzazepine, 1H-cyclohepta[d][3,2,1-jk][1]benzazepine, spiro[azepino[3,2,1-hi]indole-7(4H),1'-cycloheptane], 4H-5,11-methanopyrrolo[3,2,1-no][1]benzazacycloundecine or spiro[azepino[3,2,1-hi]indole-7(4H),1'-cyclooctane].

In addition, the "phenyl group condensed with a tricyclic heterocyclic ring" as used here encompasses, in addition to the above-listed phenyl groups condensed with tricyclic heterocyclic rings containing optionally hydrogenated indole or isoindole rings, the phenyl groups condensed with tricyclic heterocyclic rings listed below and their dihydro, tetrahydro, hexahydro, octahydro and decahydro forms. Specific examples include fluoranthene, acephenanthrylene, aceanthrylene, triphenylene, pyrene, chrysene, naphthacene, pleiadene, benzo[a]anthracene, indeno[1,2-a]indene, cyclopenta[a]phenanthrene, pyrido[1',2':1,2]imidazo[4,5-b]quinoxaline, 1H-2-oxapyrene and spiro[piperidine-4,9'-xanthene].

Preferred examples of those groups where the "phenyl group" in the "optionally substituted and condensed phenyl group" is condensed with an optionally substituted tricyclic heterocyclic ring include a group of the following formula:

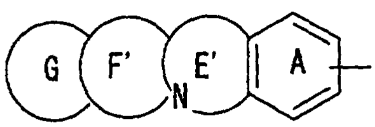

or

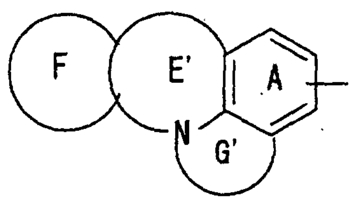

[wherein the rings E', F' and G' each represent a 5- to 9-membered nitrogen-containing heterocyclic ring which may be substituted with an oxo group, in addition to $R^1$, and the rings A, F and G and $R^1$ are as defined above].

Above all, a group of the following formula:

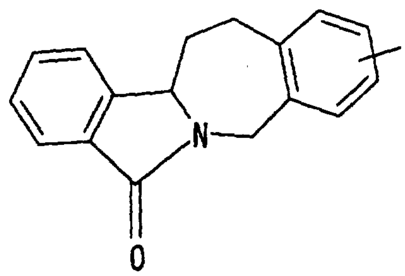

is particularly preferred, by way of example.

Examples of a "5- to 9-membered nitrogen-containing heterocyclic ring" used in the "5- to 9-membered nitrogen-containing heterocyclic ring which may be substituted with an oxo group" include the 5- to 9-membered nitrogen-containing heterocyclic rings listed above for the rings C' and D'.

Preferred examples of those groups where the "optionally substituted aryl group" represented by Ar is condensed with (2) an optionally substituted bicyclic heterocyclic ring or the same or different two monocyclic rings (at least one of which is a monocyclic heterocyclic ring) or with (3) an optionally substituted tricyclic heterocyclic ring include a group having the following formula as Ar:

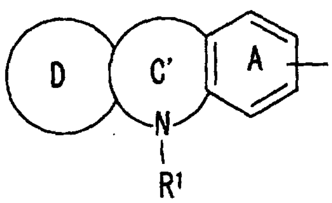 , 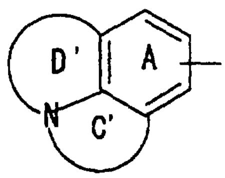 , 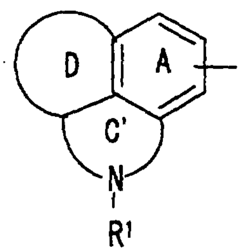 ,

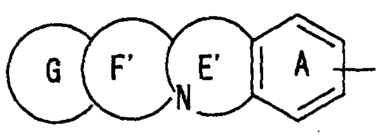 or 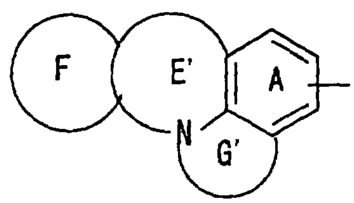

[wherein each symbol is as defined above].

Particularly preferred examples of the "optionally substituted aryl group" represented by Ar include a group of the following formula:

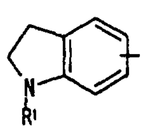 , 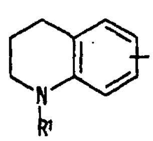 , 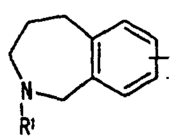 , 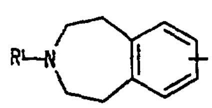 ,

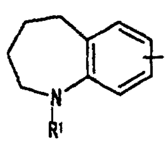 , 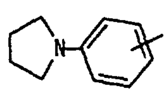 , 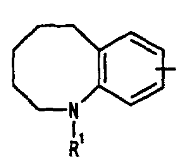 ,

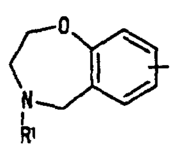 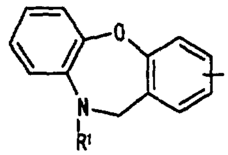, 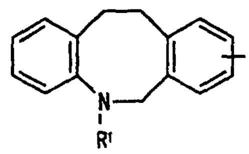, 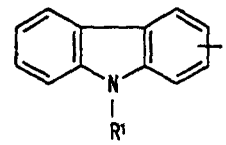,

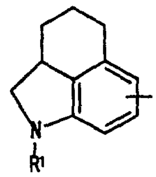, 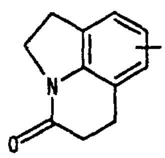 or 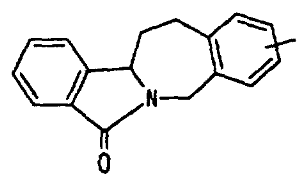

[wherein $R^1$ is as defined above]. Above all, a group of the following formula:

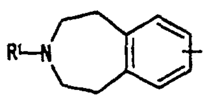, 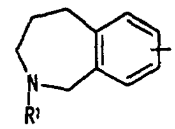 or : 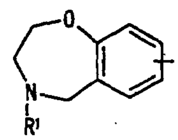

[wherein $R^1$ is as defined above] is most preferred.

In the above formula, n represents an integer of 1 to 10. Preferably, n represents an integer of 1 to 6, particularly preferably I to 5, more preferably 2 to 5, and most preferably 3, 4 or 5.

In the above formula, R represents a hydrogen atom or an optionally substituted hydrocarbon group which may be the same or different in n repeated units.

A "hydrocarbon group" in and a "substituent" on the "optionally substituted hydrocarbon group" represented by R have the same meanings as defined for the "hydrocarbon group" in and the "substituent" on the "optionally substituted hydrocarbon group" represented by $R^1$, respectively.

Alternatively, R may form a ring together with Ar or a subsdtuent on Ar.

Examples of a compound of Formula (Ib) wherein R forms a ring together with Ar or a substituent on Ar include a compound of the following formula:

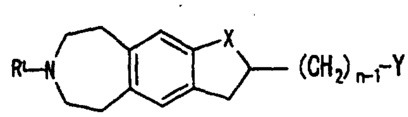

[wherein $R^1$, n, X and Y are as defined above], a compound of the following formula:

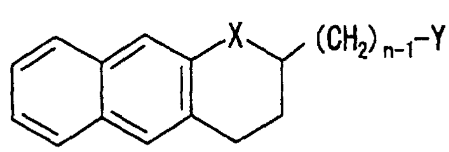

[wherein n, X and Y are as defined above] and a compound of the following formula:

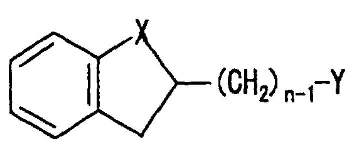

[wherein n, X and Y are as defined above].

R is preferably a hydrogen atom.

In the above formula, Y represents an optionally substituted amino group or an optionally substituted nitrogen-containing heterocyclic group (preferably, a nitrogen-containing saturated heterocyclic group) [Y preferably represents an optionally substituted amino group].

Examples of the "optionally substituted amino group" represented by Y include a group of the following formula:

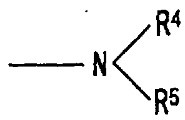

[wherein $R^4$ and $R^5$, which may be the same or different, each represent a hydrogen atom, an optionally substituted hydrocarbon group or an optionally substituted acyl group, or $R^4$ and $R^5$ may together form a ring].

Examples of a "substituent" used for substitution on and a "hydrocarbon group" used in the "optionally substituted hydrocarbon group" represented by $R^4$ and $R^5$ include the substituents and hydrocarbon groups listed above for the "optionally substituted hydrocarbon group" represented by $R^1$.

Preferred examples of the optionally substituted hydrocarbon group represented by $R^4$ and $R^5$ include (I) linear or branched lower alkyl groups (e.g., $C_{1-6}$ alkyl groups such as methyl, ethyl, propyl, isopropyl, butyl, isobutyl, tert-butyl, sec-butyl, pentyl and hexyl) which may have 1 to 3 substituents selected from (i) a halogen atom (e.g., fluoro, chloro, bromo, iodo), (ii) a lower alkoxy group (e.g., a $C_{1-6}$ alkoxy group such as methoxy, ethoxy, n-propyloxy, i-propyloxy or n-butyloxy), (iii) a hydroxyl group and the like, and (II) lower aralkyl groups (e.g., $C_{7-16}$ aralkyl groups such as phenyl-$C_{1-10}$ alkyls (e.g., benzyl, phenylethyl, phenylpropyl, phenylbutyl, phenylpentyl, phenylhexyl), naphthyl-$C_{1-6}$ alkyls (e.g., α-naphthylmethyl) or diphenyl-$C_{1-3}$ alkyls (e.g., diphenylmethyl, diphenylethyl)) which may have 1 to 3 substituents selected from (i) a halogen atom (e.g., fluoro, chloro, bromo, iodo), (ii) a lower alkoxy group (e.g., a $C_{1-6}$ alkoxy group such as methoxy, ethoxy, n-propyloxy, i-propyloxy or n-butyloxy), (iii) a hydroxyl group and the like.

More preferred examples include (I) unsubstituted linear or branched lower alkyl groups (e.g., $C_{1-6}$ alkyl groups such as methyl, ethyl, propyl, isopropyl, butyl, isobutyl, tert-butyl, sec-butyl, pentyl and hexyl) and (II) unsubstituted lower aralkyl groups (e.g., $C_{7-16}$ aralkyl groups such as phenyl-$C_{1-10}$ alkyls (e.g., benzyl, phenylethyl, phenylpropyl, phenylbutyl, phenylpentyl, phenylhexyl), naphthyl-$C_{1-6}$ alkyls (e.g., α-naphthylmethyl) or diphenyl-$C_{1-3}$ alkyls (e.g., diphenylmethyl, diphenylethyl)).

Examples of the "optionally substituted acyl group" represented by $R^4$ and $R^5$ include the optionally substituted acyl groups listed above for $R^1$.

In addition, specific examples of those groups where $R^4$ and $R^5$ together form a ring in the "optionally substituted amino group" represented by Y, i.e., where the "optionally substituted amino group" represented by Y is an "optionally substituted cyclic amino group" include a group of the following formula:

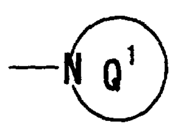

[wherein the ring $Q^1$ represents a 5- to 9-membered nitrogen-containing heterocyclic group (preferably, a 5- to 9-membered nitrogen-containing saturated heterocyclic group) which may contain, in addition to carbon atoms and one nitrogen atom, 1 or 2 heteroatoms selected from a nitrogen atom, an oxygen atom, a sulfur atom and the like]. More specifically, the following group:

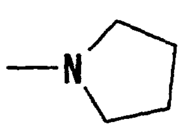 , 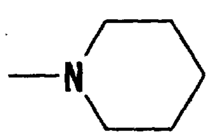 , 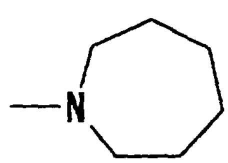 , 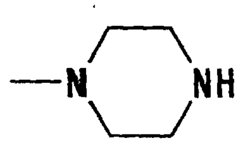 ,

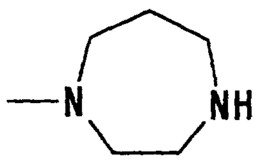,
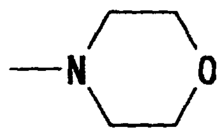 or 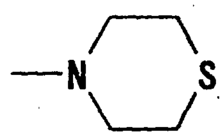

is frequently used, by way of example.

Examples of a "substituent" used for substitution on an "optionally substituted cyclic amino group" as the "optionally substituted amino group" represented by Y include the substituents listed above for the "optionally substituted nitrogen-containing heterocyclic ring" which may be formed by $R^{2b}$ and $R^{3b}$ together with their adjacent nitrogen atom, as well as the optionally substituted hydrocarbon, acyl or heterocyclic groups listed above for $R^1$.

Preferred examples of the "optionally substituted amino group" represented by Y include (1) a group of the following formula:

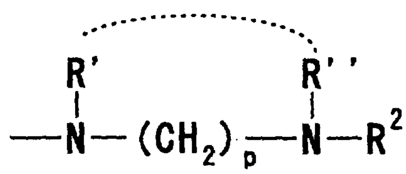

[wherein $R^2$ represents a hydrogen atom, an optionally substituted acyl group, an optionally substituted hydrocarbon group or an optionally substituted heterocyclic group, p represents an integer of 1 to 3, and R' and R" each represent a hydrogen atom or an optionally substituted alkyl group, or R' and R" may together form a ring] and (2) an optionally substituted piperidino group. Among these, preferred for use are (1a) a group of the following formula:

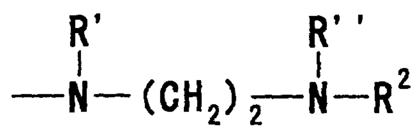

[wherein $R^2$ represents a hydrogen atom, an optionally substituted acyl group, an optionally substituted hydrocarbon group or an optionally substituted heterocyclic group, and R' and R" each represent a hydrogen atom or an optionally substituted alkyl group], (1b) a group of the following formula:

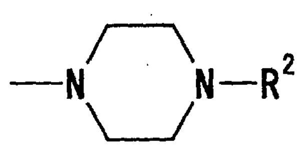

[wherein $R^2$ represents a hydrogen atom, an optionally substituted acyl group, an optionally substituted hydrocarbon group or an optionally substituted heterocyclic group], etc.

Examples of the "optionally substituted acyl group", "optionally substituted hydrocarbon group" and "optionally substituted heterocyclic group" represented by $R^2$ include the same groups as listed above for the "optionally substituted acyl group", "optionally substituted hydrocarbon group" and "optionally substituted heterocyclic group" represented by $R^1$.

Examples of an "alkyl group" in the "optionally substituted alkyl group" represented by R' and R" include $C_{1-6}$ alkyl groups, which may have the same substituent(s) as listed above for the "optionally substituted hydrocarbon group" represented by $R^1$.

In a case where R' and R" together form a ring, preferred examples include a 5-to 9-membered nitrogen-containing heterocyclic group (preferably, a 5- to 9-membered nitrogen-containing saturated heterocyclic group) which may contain, in addition to carbon atoms and two nitrogen atoms, one heteroatom selected from a nitrogen atom, an oxygen atom, a sulfur atom and the like, among the nitrogen-containing heterocyclic groups illustrated above for the

ring $Q^1$. Such a ring is preferably a 5- to 9-membered nitrogen-containing heterocyclic ring (preferably, a 5- to 9-membered nitrogen-containing saturated heterocyclic ring) composed of carbon atoms and two nitrogen atoms, which may further have the same substituent(s) as the ring $Q^1$.

The optionally substituted piperidino group as Y may be substituted with, for example, the optionally substituted acyl, hydrocarbon or heterocyclic group(s) listed above for $R^1$.

Examples of a "nitrogen-containing heterocyclic group" used in the "optionally substituted nitrogen-containing heterocyclic group" represented by Y include a 5- to 9-membered nitrogen-containing heterocyclic group (preferably, a 5- to 9-membered nitrogen-containing saturated heterocyclic group) which may contain, in addition to carbon atoms and one nitrogen atom, 1 to 3 heteroatoms selected from a nitrogen atom, an oxygen atom, a sulfur atom and the like. Such a nitrogen-containing heterocyclic group may have a binding hand on either a ring-constituting nitrogen atom or a ring-constituting carbon atom. Such a group having a binding hand on a ring-constituting nitrogen atom may be represented, for example, by the following formula:

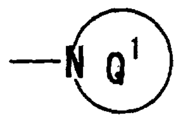

[wherein the ring $Q^1$ represents a 5- to 9-membered nitrogen-containing heterocyclic group (preferably, a 5- to 9-membered nitrogen-containing saturated heterocyclic group) which may contain, in addition to carbon atoms and one nitrogen atom, 1 or 2 heteroatoms selected from a nitrogen atom, an oxygen atom, a sulfur atom and the like]. More specifically, the following group:

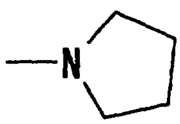 , 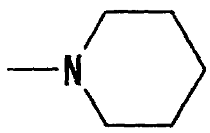 , 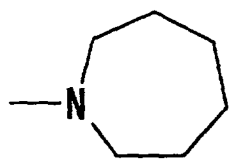 , 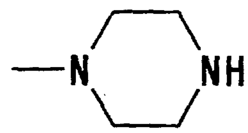 ,

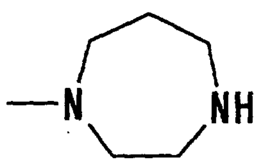 , 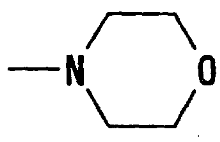 or 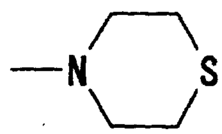 .

is frequently used, by way of example.

Such a group having a binding hand on a ring-constituting carbon atom may be represented, for example, by the following formula:

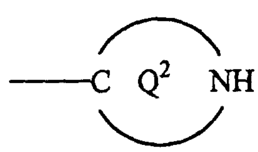

[wherein the ring $Q^2$ represents a 5- to 9-membered nitrogen-containing heterocyclic group (preferably, a 5- to 9-membered nitrogen-containing saturated heterocyclic group) which may contain, in addition to carbon atoms and one nitrogen atom, 1 or 2 heteroatoms selected from a nitrogen atom, an oxygen atom, a sulfur atom and the like]. More specifically, the following group:

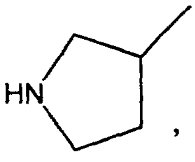, 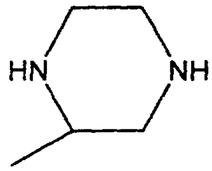, 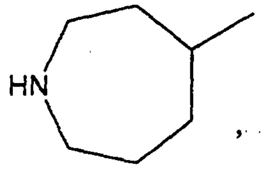, 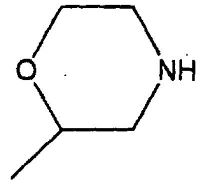,

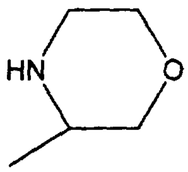 or 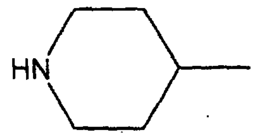

is frequently used, by way of example.

Examples of a "substituent" used for substitution on the "optionally substituted nitrogen-containing heterocyclic group (preferably, nitrogen-containing saturated heterocyclic group)" represented by Y include the substituents listed above for the "optionally substituted nitrogen-containing heterocyclic ring" which may be formed by $R^{2b}$ and $R^{3b}$ together with their adjacent nitrogen atom, as well as the optionally substituted hydrocarbon, acyl or heterocyclic groups listed above for $R^1$.

In a case where each of the "optionally substituted cyclic amino group" as the "optionally substituted amino group" represented by Y and the "optionally substituted nitrogen-containing heterocyclic group" represented by Y has two or more substituents, the substituents may together form a ring. Specific examples of such a ring include a benzene ring, a 5- to 8-membered (preferably, 5- to 6-membered) aromatic monocyclic heterocyclic ring (e.g., pyrrole, oxazole, isooxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine) and rings obtained by saturating part or all of unsaturated bonds in these rings.

In a case where each of the "optionally substituted cyclic amino group" as the "optionally substituted amino group" represented by Y and the "optionally substituted nitrogen-containing heterocyclic group" represented by Y has two or more substituents on one carbon atom, the substituents may together form a spiro ring. Specific examples of such a spiro ring include a spiro(1H-indene-1,4'-piperidinyl) ring.

Preferred examples of a "nitrogen-containing heterocyclic group" in the "optionally substituted nitrogen-containing heterocyclic group" represented by Y include a 4-piperidinyl group, a 1-piperidinyl group and a 1-piperazinyl group.

Namely, preferred examples of Y include a group of the following formula:

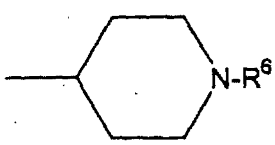, 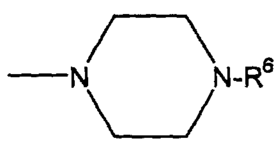 or 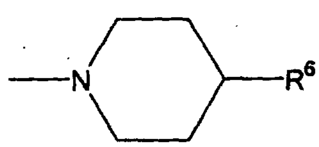

[wherein $R^6$ is as defined above for $R^1$].

More preferred examples of Y include a group of the following formula:

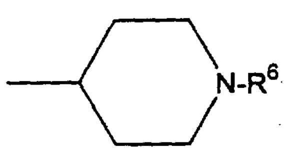, 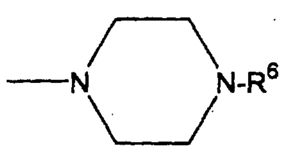 or 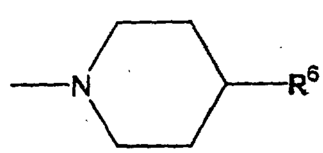

[wherein $R^6$ represents (i) a phenyl-$C_{1-6}$ alkyl group which may be substituted with a $C_{1-6}$ alkyl, a $C_{1-6}$ alkoxy, a halogen atom, nitro, a mono- or di-$C_{1-6}$ alkyl-carbamoyloxy, hydroxy, cyano, carboxyl, a $C_{1-6}$ alkoxycarbonyl, carbamoyl, cyclic aminocarbonyl, amino, a $C_{1-6}$ alkylcarbonylamino, phenylsulfonylamino, a $C_{1-6}$ alkylsulfonylamino, amidino, ureido or a heterocyclic ring (said $C_{1-6}$ alkyl, $C_{1-6}$ alkoxy, carbamoyl, cyclic aminocarbonyl, amino, phenylsulfonylamino, amidino, ureido or heterocyclic ring may further have a substituent(s) such as those listed above for the "optionally substituted hydrocarbon group" represented by $R^1$), (ii) a hydrogen atom, (iii) a $C_{1-6}$ alkyl group which may be substituted with a halogen atom, hydroxy, a $C_{1-6}$ alkoxy, amino, a mono-or di-$C_{1-6}$ alkylamino, carboxyl, cyano or a $C_{1-6}$ alkoxy-carbonyl, or (iv) a $C_{1-6}$ alkylcarbonyl group which may be substituted with a mono- or di-$C_{1-6}$ alkylamino or a $C_{1-6}$ alkoxy-carbonyl, preferably $R^6$ represents a benzyl group which may be substituted with a $C_{1-4}$ alkyl (e.g., methyl), a trihalogeno-$C_{1-4}$ alkyl (e.g., methyl), a halogen atom (e.g., fluoro, chloro), nitro, cyano, a $C_{1-4}$ alkoxy (e.g., methoxy), hydroxy, carbamoyl, a (4-$C_{1-4}$ alkyl (e.g., methyl)-1-piperazinyl)carbonyl, aminothiocarbonyl, morpholinocarbonyl, carboxyl, a $C_{1-4}$ alkoxy (e.g., methoxy)-carbonyl, a $C_{1-4}$ alkoxy (e.g., ethoxy)-carbonyl-$C_{1-4}$ alkoxy (e.g., methoxy), a carboxyl-$C_{1-4}$ alkoxy (e. g., methoxy), a $C_{1-4}$ alkoxy (e.g., ethoxy)-carbonyl-$C_{1-6}$ alkyl (e.g., isopropyl), a carboxyl-$C_{1-6}$ alkyl (e.g., isopropyl), amino, acetylamino, a $C_{1-4}$ alkyl (e.g., methyl)-sulfonylamino, a (4-$C_{1-4}$ alkyl (e.g., methyl)-phenyl)sulfonylamino, ureido, a 3-$C_{1-4}$ alkyl (e.g., methyl)-ureido, amidino, dihydrothiazolyl or dihydroimidazolyl].

Above all, preferred is a group wherein $R^6$ is a benzyl group which may be substituted with a $C_{1-4}$ alkyl (e.g., methyl), a trihalogeno (e.g., fluoro)-$C_{1-4}$ alkyl (e.g., methyl), a halogen atom (e.g., fluoro, chloro), nitro, hydroxy, carbamoyl, amino, amidino or dihydroimidazolyl.

Particularly preferred examples of Y include a 1-benzyl-4-piperidinyl group, a 4-benzyl-1-piperidinyl group or a 4-benzyl-1-piperazinyl group, a 1-acetyl-4-piperidinyl group, a 1-[(2-methylphenyl)methyl]-4-piperidinyl group, a 1-[(3-chlorophenyl)methyl]-4-piperidinyl group, a 1-[(2-chlorophenyl)methyl]-4-piperidinyl group, a 1-[(3-nitrophenyl) methyl]-4-piperidinyl group and a 1-[[3-(trifluoromethyl)phenyl]methyl]-4-piperidinyl group. Among these, commonly used are, for example, a 1-benzyl-4-piperidinyl group, a 1-acetyl-4-piperidinyl group, a 1-[(2-methylphenyl)methyl]-4-piperidinyl group, a 1-[(3-chlorophenyl)methyl]-4-piperidinyl group, a 1-[(2-chlorophenyl)methyl]-4-piperidinyl group, a 1-[(3-nitrophenyl)methyl]-4-piperidinyl group and a 1-[[3-(trifluoromethyl)phenyl]methyl]-4-piperidinyl group.

In the above formula, examples of the "spacer containing 1 to 4 atoms in its linear chain moiety" represented by X include saturated divalent groups such as:

(1) $-(CH_2)_{q1}-$ (wherein q1 represents an integer of 1 to 4);
(2) $-(CH_2)_{r1}-X^1-(CH_2)_{r2}-$ (wherein r1 and r2, which may be the same or different, each represent an integer of 0 to 3, provided that the sum of r1 and r2 is 1 to 3, and $X^1$ represents NH, O, S, SO or $SO_2$); and
(3) $-(CH_2)_{s1}-X^1-(CH_2)_{s2}-X^2-(CH_2)_{s3}-$ (wherein s1, s2 and s3, which may be the same or different, each represent an integer of 0 to 2, provided that the sum of s1, s2 and s3 is 0 to 2, and $X^1$ and $X^2$ each represent NH, O, S, SO or $SO_2$, provided that when s2 is 0, at least one of $X^1$ and $X^2$ preferably represents NH); as well as divalent groups obtained by partial unsaturation of these groups; or divalent groups containing I to 4 atoms in their linear chain moiety such as -CO-, -O-, $-NR^{3ba}-$, -S-, -SO-, $-SO_2-$, $-SO_2NR^{3ba}-$, $-SO_2NHCONR^{3ba}-$, $-SO_2NHC(=NH)NR^{3ba}-$, -CS-, $-CR^{3ba}(R^{3bb})-$, $-C(=CR^{3ba}(R^{3bb}))-$, $-C(=NR^{3ba})-$ and $-CONR^{3ba}-$ (wherein $R^{3ba}$ and $R^{3bb}$ each independently represent a hydrogen atom, a cyano group, a hydroxyl group, an amino group, a $C_{1-6}$ alkyl group or a $C_{1-6}$ alkoxy group).

More preferred examples of X include -CO-, -O-, $NR^{3ba}-$, -S-, -SO-, $-SO_2-$, $-SO_2NR^{3ba}-$, $-SO_2NHCONR^{3ba}-$, $-SO_2NHC(=NH)NR^{3ba}-$, -CS-, $-CR^{3ba}(R^{3bb})-$, $-C(=CR^{3ba}(R^{3bb}))-$, $-C(=NR^{3ba})-$ and $-CONR^{3ba}-$ (wherein $R^{3ba}$ and $R^{3bb}$ each independently represent a hydrogen atom, a cyano group, a hydroxyl group, an amino group, a $C_{1-6}$ alkyl group or a $C_{1-6}$ alkoxy group). Particularly preferred examples include -CO-, -O-, $-SO_2-$, $-SO_2NR^{3ba}-$, $-CR^{3ba}(R^{3bb})-$ and $-CONR^{3ba}-$. Above all, $-SO_2NR^{3ba}-$, $-CONR^{3ba}-$ or $-CR^{3ba}(R^{3bb})-$ is preferred for use, by way of example.

The divalent group represented by X may have a substituent(s) at any position (preferably, on its carbon atom (s)). Examples of such a substituent inchide a lower ($C_{1-6}$) alkyl (e.g., methyl, ethyl, propyl, isopropyl, butyl, isobutyl, sec-butyl, tert-butyl, pentyl, isopentyl, neopentyl, hexyl), a lower ($C_{3-7}$) cycloalkyl (e.g., cyclopropyl, cyclobutyl, cyclopentyl, cyclohexyl, cycloheptyl), formyl, a lower ($C_{2-7}$) alkanoyl (e.g., acetyl, propionyl, butyryl), a lower ($C_{1-6}$) alkoxycarbonyl, a lower ($C_{1-6}$) alkoxy, a hydroxyl group and oxo.

Among compounds of Formula (Ib) or salts thereof, preferred for use is a compound of Formula (IIb):

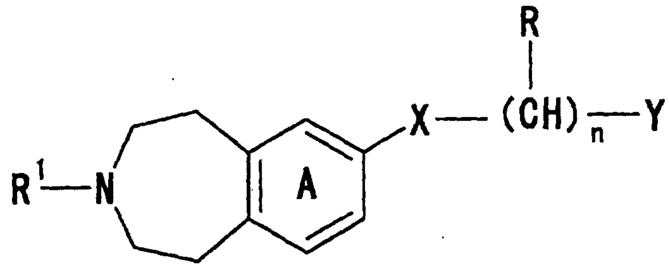

(IIb)

[wherein $R^1$ represents a hydrogen atom, an optionally substituted hydrocarbon group or an optionally substituted acyl group, the ring A represents a benzene ring which may have an additional substituent, X represents a spacer containing 1 to 4 atoms in its linear chain moiety, n represents an integer of 1 to 10, R represents a hydrogen atom or an optionally substituted hydrocarbon group which may be the same or different in n repeated units or R may form a ring together with the ring A or a substituent on the ring A, and Y represents an optionally substituted amino group or an optionally substituted nitrogen-containing heterocyclic group] or a salt thereof.

Compounds of Formulae (Ib) and (IIb) or salts thereof may be prepared, for example, according to the synthesis procedures shown below. Alternatively, they may be prepared according to the procedures as described in JP-A-6-166676, JP-A-11-310532, EP-A-487071, EP-A-560235, WO98/465 or WO00/23437 or equivalent procedures.

If compounds of Formulae (Ib) and (IIb) and compounds in the respective steps of their preparation (i.e., starting compounds or synthesis intermediates) are in free form, they may be converted into salts in a general manner. If they are in salt form, on the other hand, they may be converted into free forms or other salts in a general manner.

In addition, compounds of Formulae (Ib) and (IIb) and their respective starting compounds or synthesis intermediates may be optical isomers, stereoisomers, positional isomers, rotational isomers or mixtures thereof, which are also contemplated as being within the compounds of Formulae (Ib) and (IIb) of the present invention and their starting compounds or synthesis intermediates. For example, Compound (Ib) may be a racemate or an optical isomer resolved therefrom. These isomers may be isolated and purified according to well-known separation procedures.

An optical isomer may be prepared according to a well-known means. More specifically, an optical isomer may be prepared by using an optically active starting compound or synthesis intermediate or by optically resolving a racemate of the final compound in a general manner. For optical resolution, well-known techniques such as fractional recrystallization, an optically active column technique and a diastereomer technique may be applied. Likewise, stereoisomers, positional isomers and rotational isomers may be prepared by application of well-known techniques.

Each of the reactions shown below may be carried out without a solvent or, if necessary, with an appropriate solvent. Any solvent usually provided for chemical reactions may be used as long as it does not affect the reaction, including organic solvents such as hydrocarbon solvents (e.g., hexane, toluene), ether solvents (e.g., ethyl ether, tetrahydrofuran, dioxane, dimethoxyethane), amide solvents (e.g., formamide, N,N-dimethylformamide, N,N-dimethylacetamide, hexamethylghosphoric triamide), urea solvents (e.g., 1,3-dimethyl-2-imidazolidinone), sulfoxide solvents (e.g., dimethyl sulfoxide), alcohol solvents (e.g., methanol, ethanol, isopropanol, t-butanol), nitrile solvents (e.g., acetonitrile, propionitrile) and pyridine or water. Such a solvent is usually used in an amount of about 0.5 ml to about 100 ml; preferably about 3 ml to about 30 ml, relative to 1 mmol of a target compound. The reaction temperature will vary depending on the type of solvent to be used, but usually ranges from about -30°C to about 180°C, preferably about 0°C to about 120°C. The reaction time will vary depending on the reaction temperature, but usually ranges from about 0.5 hours to about 72 hours, preferably about 1 hour to about 24 hours. Each reaction is usually carried out under normal pressure, but if necessary, it may be carried out at an elevated pressure of about 1 atm to about 100 atms.

Each of compounds obtained in the respective steps shown below may be isolated and purified by known means including concentration, liquid conversion, phasic transfer, solvent extraction, fractional distillation, distillation, crystallization, recrystallization, chromatography and preparative high performance liquid chromatography, and then provided for use as a starting material in the subsequent reaction, but it may also be used as a starting material without any isolation and purification, i.e., still in the form of a reaction mixture.

In the explanation given below, "condensation" may be carried out in the presence of a base, if necessary. Examples of such a base include inorganic bases such as sodium carbonate, sodium bicarbonate, potassium carbonate, lithium carbonate, sodium hydroxide, potassium hydroxide, potassium hydride, sodium hydride, sodium methoxide and potassium t-butoxide as well as organic bases such as pyridine, lutidine, collidine and triethylamine. Such a base is usually used in an equimolar or excess amount, preferably about 1 molar equivalent to about 5-fold molar equivalents, relative to a target compound. Further, this reaction may be accelerated in the presence of a catalytic amount of, for example, an iodide compound (e.g., sodium iodide, potassium iodide) or 4-dimethylaminopyridine, if necessary.

Among compounds of Formula (IIb), a compound of Formula (IIba) (X = -O-) or a salt thereof may be prepared

according to the following Reaction Scheme 1-1.

**Reaction Scheme 1-1**

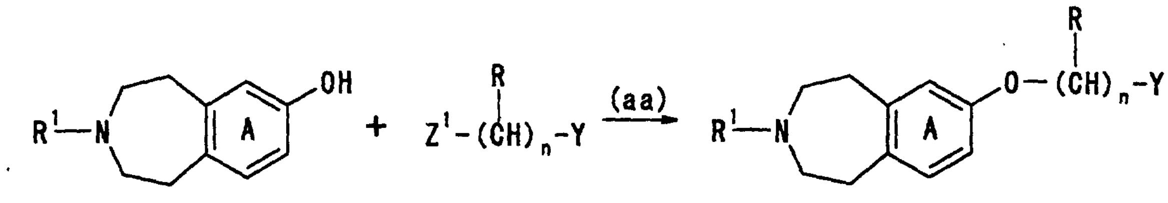


(IIIba) (IVba) (IIba)

Examples of the leaving group represented by $Z^1$ include a halogen atom (e.g., chloro, bromo, iodo), a $C_{1-6}$ alkylsulfonyloxy group (e.g., methanesulfonyloxy, ethanesulfonyloxy, trifluoromethanesulfonyloxy) and a $C_{6-10}$ arylsulfonyloxy group (e.g., benzenesulfonyloxy, p-toluenesulfonyloxy). In particular, a halogen atom (e.g., bromo, iodo) is preferred for use.

In Step (aa), a compound of Formula (IIIba) [wherein each symbol is as defined above] (hereinafter also simply referred to as Compound (IIIba)) and a compound of Formula (IVba) [wherein $Z^1$ represents a leaving group, and the other symbols are as defined above] (hereinafter also simply referred to as Compound (IVba)) can be condensed together to prepare Compound (IIba).

The condensation between Compound (IIIba) and Compound (IVba) is preferably carried out in a solvent such as an alcohol solvent (e.g., ethanol) or a nitrile solvent (e.g., acetonitrile). The reaction temperature will vary depending on the type of solvent to be used, but preferably ranges from about 0°C to about 120°C. The reaction time will vary depending on the reaction temperature, but preferably ranges from about 1 hour to about 24 hours. Preferred examples of abase available for use include sodium carbonate, potassium carbonate and triethylamine. Such a base is preferably used in an amount of about 1 equivalent to about 3 equivalents, relative to Compound (IVba). Further, this reaction may be accelerated in the presence of a catalytic amount of, for example, an iodide compound (e.g., sodium iodide, potassium iodide) or 4-dimethylaminopyridine, if necessary. More specifically, this reaction may be carried out in a solvent such as N,N-dimethylformamide and in the presence of a base such as potassium carbonate or sodium hydride. Such a base is preferably used in an amount of about 1 equivalent to about 3 equivalents, relative to Compound (IVba).

Compound (IVba) can be prepared according to well-known procedures or equivalents thereof.

Likewise, the starting compound (IIIba) in Step (aa) or a salt thereof can be prepared as described in, e.g., WO00/23437.

Among compounds of Formula (IIb), a compound of Formula (IIbb) (X = $-NR^{3ba}-$) or a salt thereof may be prepared according to the following Reaction Scheme 2-1.

**Reaction Scheme 2-1**

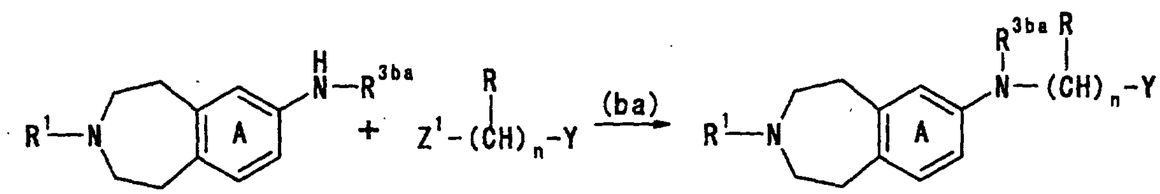


(IIIbb) (IVba) (IIbb)

In Step (ba), a compound of Formula (IIIbb) [wherein each symbol is as defined above] (hereinafter also simply referred to as Compound (IIIbb)) and Compound (IVba) can be condensed together to prepare Compound (IIbb).

The condensation between Compound (IIIbb) and Compound (IVba) may be carried out in a solvent such as N,N-dimethylformamide and in the presence of a base such as potassium carbonate or sodium hydride. Such a base is preferably used in an amount of about 1 equivalent to about 3 equivalents, relative to Compound (IVba).

Likewise, the starting compound (IIIbb) in Step (ba) or a salt thereof may be prepared according to the following Reaction Scheme 2-2. Namely, Compound (IIIbb) can be prepared by performing the following steps successively:

Step (bb): nitration of a compound of Formula (Vbb) [wherein each symbol is as defined above] (hereinafter also simply referred to as Compound (Vbb));

Step (bc): reduction of a compound of Formula (VIbb) [wherein each symbol is as defined above] (hereinafter also simply referred to as Compound (VIbb)); and

Step (bd): condensation between a compound of Formula (VIIbb) [wherein each symbol is as defined above] (hereinafter also simply referred to as Compound (VIIbb)) and a compound of Formula (IXbb) [wherein each symbol is as defined above] (hereinafter also simply referred to as Compound (IXbb)).

## Reaction Scheme 2-2

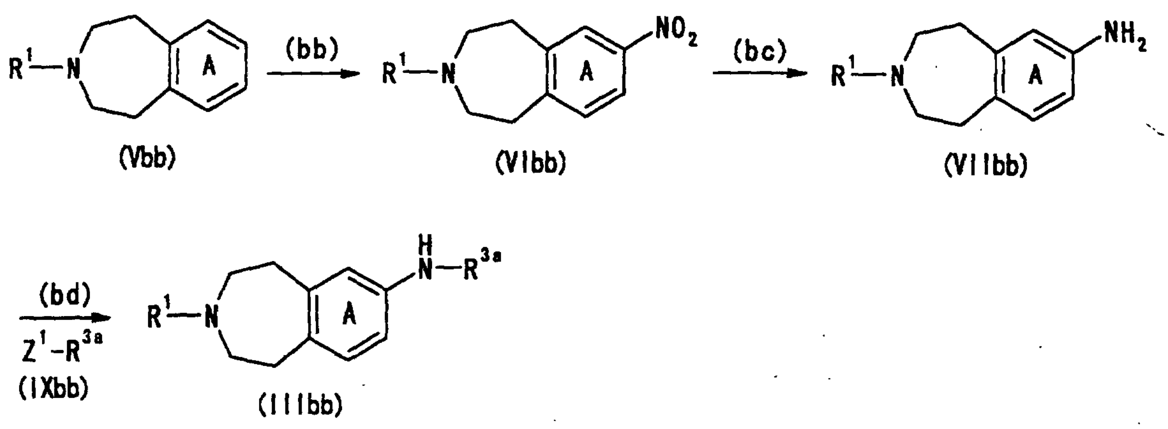


(Vbb) (VIbb) (VIIbb) (IXbb) (IIIbb)

In Step (bb), Compound (Vbb) can be nitrated to prepare Compound (VIbb).

This reaction may be carried out using an appropriate nitrating reagent (e.g., nitric acid, nitric acid-sulfuric acid, nitronium trifluoroborate) according to known procedures (see, e.g., Synthesis, 217-238 (1977), Chemistry of the Nitro and Nitroso Groups, pp. 1-48 Wiley (1970)) or equivalent procedures.

Compound (Vbb) can be prepared according to well-known procedures or equivalents thereof, such as those described in J. Org. Chem., 34, 2235 (1969), J. Org. Chem., 54, 5574 (1989), Tetrahedron Lett., 35, 3023 (1977), Bull. Chem. Soc. Jpn., 56, 2300 (1983), Indian. J. Chem. 2, 211 (1964), Indian. J. Chem., 12, 247 (1974), Bull. Chem. Soc., Jpn., 43, 1824 (1970), Chem. Pharm. Bull., 20, 1328 (1972), Chem. Pharm. Bull., 27, 1982 (1979), Helv. Chem. Acta, 46, 1696 (1963), Synthesis, 541 (1979), U.S. 3,682,962, U.S. 3,911,126, Ger. Offen. 2,314,392, Ger. 1,545,805, J. Chem. Soc., 1381 (1949), Can. J. Chem., 42, 2904 (1964), J. Org. Chem., 28, 3058 (1963), J. Am. Chem. Soc., 76, 3194 (1954), 87, 1397 (1965), 88, 4061 (1966) and JP-A-49-41539.

In Step (bc), Compound (VIbb) can be reduced to prepare Compound (VIIbb).

This reaction may be carried out using an appropriate reduction procedure (e.g., catalytic reduction in the presence of a transition metal catalyst, reduction in an acidic solvent and in the presence of a metal such as tin). More specifically, this reaction may be carried out according to known procedures (see, e.g., Organic Synthesis, Coll. Vol. 5, 829-833 (1973), Organic Synthesis, Coll. Vol. 1, 455 (1941), J. Am. Chem. Soc., 66, 1781 (1944)) or equivalent procedures.

In Step (bd), Compound (VIIbb) and Compound (IXbb) can be condensed together to prepare Compound (IIIbb).

The condensation between Compound (VIIbb) and Compound (IXbb) may be carried out, for example, in the same manner as described above for the condensation between Compound (IIIba) and Compound (IVba).

Alternatively, Compound (IIIbb) can also be prepared starting with Compound (VIIbb), for example, by reductive alkylation (see, e.g., J. Am. Chem. Soc., 87, 2767 (1965), Organic Synthesis, Coll. Vol. 4, 283-285 (1963)) or the Michael addition (see, e.g., Helv. Chem. Acta, 43, 1898 (1960), J. Org. Chem., 39, 2044 (1974), Synthesis, 5, 375 (1981)) or an equivalent reaction.

Among compounds of Formula (IIb), a compound of Formula (IIbc) (X = $-NR^{3ba}CO-$) or a salt thereof may be prepared according to the following Reaction Scheme 3.

**Reaction Scheme 3**

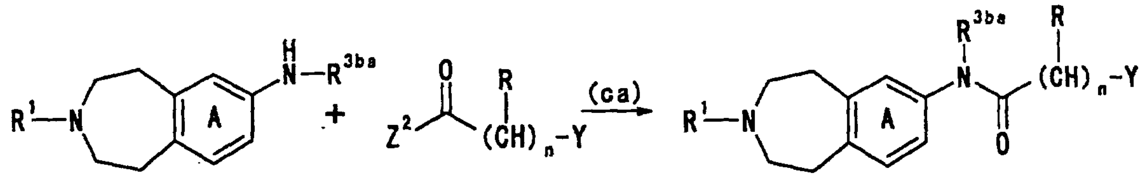


(IIIbb) (IVbc) (IIbc)

In Step (ca), Compound (IIIbb) can be amidated with a compound of Formula (IVbc) [wherein $Z^2$ represents a leaving group, and the other symbols are as defined above] (hereinafter also simply referred to as Compound (IVbc)) to prepare Compound (IIbc).

Examples of the leaving group represented by $Z^2$ include a halogen atom (e.g., chloro, bromo, iodo), a $C_{1-6}$ alkyloxy group (e.g., methoxy, ethoxy, benzyloxy), a $C_{6-10}$ aryloxy group (e.g., phenoxy, p-nitrophenoxy) and a hydroxyl group. In particular, a halogen atom (e.g., chloro) or a hydroxyl group is preferred for use.

The amidation of Compound (IIIbb) with Compound (IVbc) may also be carried out in the presence of an appropriate condensing agent or base. In a case where $Z^2$ is a hydroxyl group, for example, this amidation can be carried out in the presence of an appropriate condensing agent such as those commonly used in peptide chemistry, particularly carbodiimides such as dicyclohexylcarbodiimide and 1-ethyl-3-(3-dimethylaminopropyl)carbodiimide, phosphonates such as diphenylphosphorylazide and diethyl cyanophosphonate, or phosgene equivalents such as 1-1'-carbonylbis-1H-imidazole. Such a condensing agent is usually used in an amount of about 1 equivalent to about 5 equivalents, preferably about 1 equivalent to about 1.5 equivalents, relative to 1 mmol of Compound (IIIbb).

In a case where $Z^2$ is a halogen atom, for example, the reaction is preferably carried out in the presence of an appropriate base such as sodium carbonate, potassium carbonate or triethylamine. Such a base is usually used in an amount of about 1 equivalent to about 10 equivalents, preferably about 1 equivalent to about 2 equivalents, relative to Compound (IIIbb).

Among compounds of Formula (IIb), a compound of Formula (IIbd) (X = -S-, -SO- or $-SO_2-$) or a salt thereof may be prepared according to the following Reaction Scheme 4-1.

**Reaction Scheme 4-1**

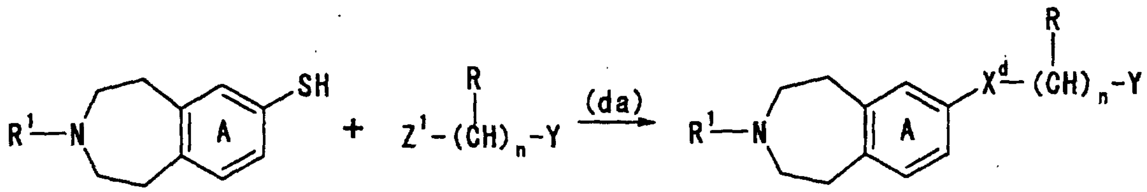


(IIIbd) (IVba) (IIbd)

In Step (da), Compound (IIIbd) and Compound (IVba) can be condensed together and, if necessary, followed by oxidation to prepare Compound (IIbd) [wherein $X^d$ represents -S-, -SO- or $-SO_2-$, and the other symbols are as defined above].

The condensation between Compound (IIIbd) and Compound (IVba) may be carried out in a solvent such as N,N-dimethylformamide and in the presence of a base such as potassium carbonate or sodium hydride. Such a base is preferably used in an amount of about 1 equivalent to about 3 equivalents, relative to Compound (IVba).

Compound (IIbd) wherein $X^d$ is -S- may further be subjected to oxidation, if necessary, leading to Compound (IIbd) wherein $X^d$ is -SO- or $-SO_2-$.

Any oxidizing agent may be used as long as it can be used as an oxidizing agent for sulfide. Preferred examples include methachloroperbenzoic acid, peracetic acid, hydrogen peroxide and alkali metal periodate, with methachloroperbenzoic acid and hydrogen peroxide begin particularly preferred for use. For oxidation of S into SO, such an oxidizing agent is particularly preferably used in an amount of about 1 equivalent to about 1.1 equivalents, relative to Compound (IIbd). For oxidation of S into $SO_2$, it is particularly preferably used in an amount of about 2 equivalents to 2.5 equivalents, relative to Compound (IVbd). Preferred examples of a solvent used in this reaction include dichloromethane, chloroform, acetic acid and ethyl acetate.

Likewise, the starting compound (IIIbd) in Step (da) or a salt thereof may be prepared according to the following Reaction Scheme 4-2. Namely, Compound (IIIbd) can be prepared through the following steps:

Step (db): chlorosulfonylation of Compound (Vbb); and
Step (dc): reduction of a compound of Formula (VIbd) [wherein each symbol is as defined above] (hereinafter also simply referred to as Compound (VIbd)).

**Reaction Scheme 4-2**

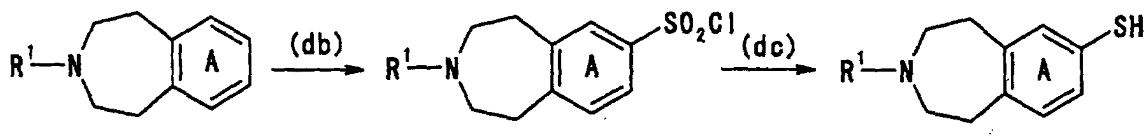


(Vbb) (VIbd) (IIIbd)

In Step (db), Compound (Vbb) can be chlorosulfonylated to prepare Compound (VIbd).
Examples of a chlorosulfonylating reagent used in this reaction include chlorosulfonic acid, sulfonylchloride and sulfur dioxide-copper chloride, with chlorosulfonic acid being particularly preferred. Such a chlorosulfonylation reagent is used in an amount of about 1 equivalent up to a large excess. This reaction may be carried out with or without a solvent. In the case of using a solvent, preferred examples include dichloromethane, 1,2-dichloroethane and carbon disulfide. The reaction is preferably carried out without a solvent and at a temperature of about -20°C to about 100°C.
A chlorosulfonyl group may be introduced at any reactable position. In a case where the ring A is unsubstituted, for example, chlorosulfonylation predominantly occurs at the 7-position. However, a compound chlorosulfonylted at the 6-position can also be yielded and isolated.
In Step (dc), Compound (VIbd) can be reduced to prepare Compound (IIIbd).
This reduction may be carried out under appropriate reductive conditions, for example, using a combination of a metal and an acid (e.g., zinc-acetic acid, tin-hydrochloric acid), catalytic reduction in the presence of a transition metal catalyst, or using a metal hydride (e.g., lithium aluminum hydride). Particularly preferred is reduction in the presence of zinc-acetic acid.
Among compounds of Formula (IIb), a compound of Formula (IIbe) (X = $-SO_2NR^{3ba}-$) or a salt thereof may be prepared according to the following Reaction Scheme 5.

**Reaction Scheme 5**

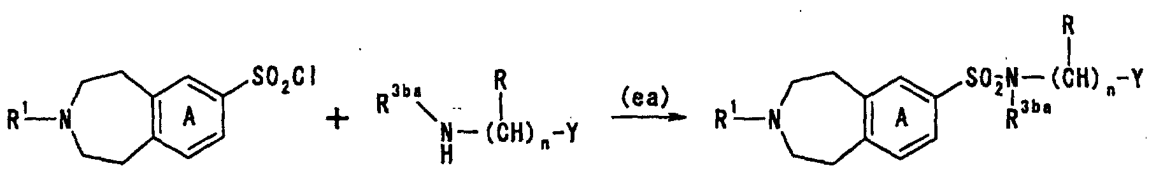


(VIbd) (IVbe) (IIbe)

In Step (ea), Compound (VIbd) and a compound of Formula (IVbe) [wherein each symbol is as defined above] (hereinafter also simply referred to as Compound (IVbe)) can be condensed together to prepare Compound (IIbe).
The condensation between Compound (VIbd) and Compound (IVbe) may be carried out, for example, in the same manner as described above for the amidation between Compound (IIIbb) and Compound (IVbc).
Compound (IVbe) or a salt thereof can be prepared according to well-known procedures or equivalents thereof, such as those described in J. Med. Chem., 33, 1880 (1990).
Among compounds of Formula (IIb), a compound of Formula (IIbf) (X = $-SO_2NHCONR^{3ba}-$) or a salt thereof may be prepared according to the followingReaction Scheme 6.

## Reaction Scheme 6

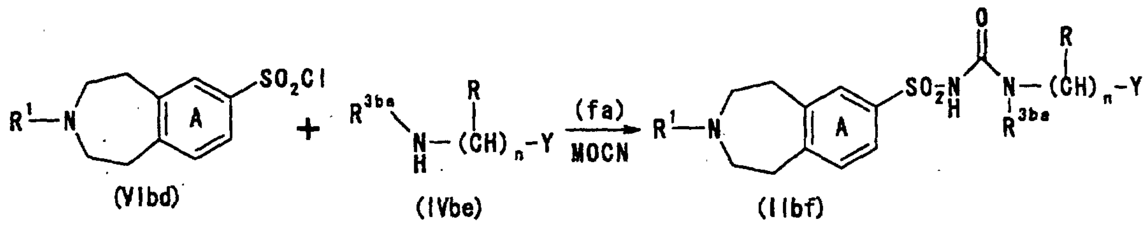


(VIbd) (IVbe) (IIbf)

In Step (fa), Compound (VIbd) can be treated with an alkali metal isocyanate (MOCN; wherein M represents an alkali metal) and then reacted with Compound (IVbe) to prepare Compound (IIbf). This reaction may be carried out according to the procedures as described in EP-759431 or JP-A-7-118267 or equivalent procedures.

The reaction between Compound (VIbd) and an alkali metal isocyanate is carried out in the presence of a base, if necessary. A base particularly preferred for use is pyridine, triethylamine, etc. Such a base is preferably used in an amount of about 1 equivalent to about 5 equivalents, relative to Compound (VIbd). As a reaction solvent, for example, acetonitrile is particularly preferred for use. As an alkali metal, for example, potassium is preferred for use.

Among compounds of Formula (IIb), a compound of Formula (IIbg) (X = $-SO_2NHC(=NH)NR^{3ba}-$) or a salt thereof may be prepared according to the followingReaction Scheme 7.

## Reaction Scheme 7

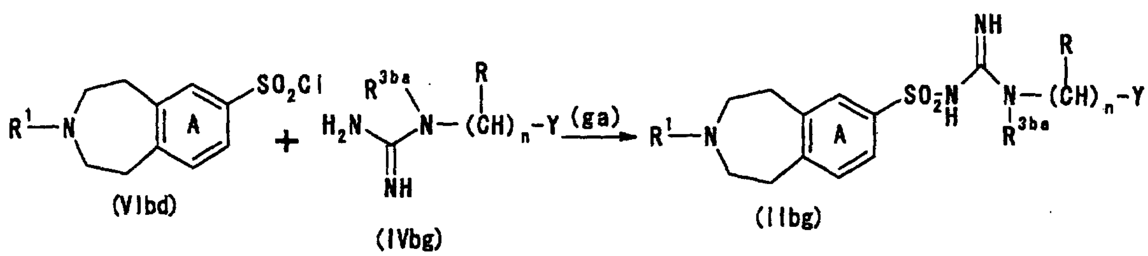


(VIbd) (IVbg) (IIbg)

In Step (ga), Compound (VIbd) and a compound of Formula (IVbg) [wherein each symbol is as defined above] (hereinafter also simply referred to as Compound (IVbg)) can be condensed together to prepare Compound (IIbg).

The condensation between Compound (VIbd) and Compound (IVbg) may be carried out, for example, in the same manner as described above for the amidation between Compound (IIIbb) and Compound (IVbc).

Compound (IVbg) can be prepared starting with Compound (IVbe) according to well-known procedures or equivalents thereof, for example, by treating Compound (IVbe) with S-methylisothiourea (see, e.g., J. Org. Chem., 13, 924 (1948)), with cyanamide (see, e.g., Helv. Chem. Acta., 29, 324 (1946)) or with 1,3-bis(tert-butoxycarbonyl)-2-methyl-2-thiopseudourea (see, e.g., Tetrahedron Lett., 33, 6541-6542 (1992), J. Org. Chem., 52, 1700-1703 (1987)).

Among compounds of Formula (IIb), a compound of Formula (IIbh) (X = $-CR^{3ba}(R^{3bb})-$) or a salt thereof may be prepared according to the following Reaction Scheme 8.

## Reaction Scheme 8

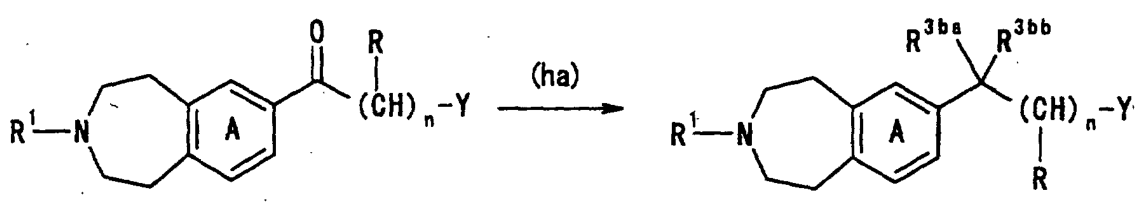


(IIIbh) (IIbh)

In Step (ha), a compound of Formula (IIIbh) [wherein each symbol is as defined above] (hereinafter also

simply referred to as Compound (IIIbh)) can be reacted with an appropriate reagent for conversion of the carbonyl group to prepare Compound (IIbh).

Examples of a reagent used for conversion of the carbonyl group include reducing agents such as sodium borohydride, lithium aluminum hydride and triethylsilane, organometallic reagents such as alkyllithium and alkylmagnesium halide, as well as nucleophilic reagents such as hydrogen cyanide.

More specifically, the carbonyl group can be converted into -CH(OH)- or $-CH_2-$, for example, using a reducing agent such as sodium borohydride, lithium aluminum hydride or triethylsilane under appropriate reductive conditions (e.g., a combination such as triethylsilane-trifluoroacetic acid, lithium aluminum hydride-aluminum chloride or zinc-hydrochloric acid).

This reaction may be carried out according to the procedures as described in Reduction with Complex Metal Hydrides, Interscience, New York (1956), Chem. Soc. Rev., 5, 23 (1976), Synthesis, 633 (1974), J. Am. Chem. Soc., 91, 2967 (1969), J. Org. Chem., 29, 121 (1964), Org. Reactions, 1, 155 (1942), Angew. Chem., 71, 726 (1956), Synthesis, 633 (1974), J. Am. Chem. Soc., 80, 2896 (1958), Org. Reactions, 4, 378 (1948) or J. Am. Chem. Soc., 108, 3385 (1986) or equivalent procedures.

Likewise, the carbonyl group can be converted into $-CR^{3bc}(OH)-$ (wherein $R^{3bc}$ represents a $C_{1-6}$ alkyl group), for example, using an organometallic reagent such as allryllithium or alkylinagnesium halide according to the procedures as described in Grignard Reactions of Nonmetallic Substances, Prentice-Hall: Englewood Cliffs, NJ, 1954, pp. 138-528 or Organolithium Methods, Academic Press: New York, 1988, pp. 67-75 or equivalent procedures.

Alternatively, the carbonyl group may also be converted according to the procedures as described in Advanced Organic Chemistry, 5th ed. Wiley-Interscience: New York, 1992, pp. 879-981 or equivalent procedures.

Compound (IIIbh) can be prepared according to well-known procedures or equivalents thereof, such as those described in JP-A-5-140149, JP-A-6-206875 and J. Med. Chem., 37, 2292 (1994).

Among compounds of Formula (IIb), a compound of Formula (IIbi) (X = $-C(=CR^{3ba}(R^{3bb}))-$) or a salt thereof may be prepared according to the following Reaction Scheme 9.

**Reaction Scheme 9**

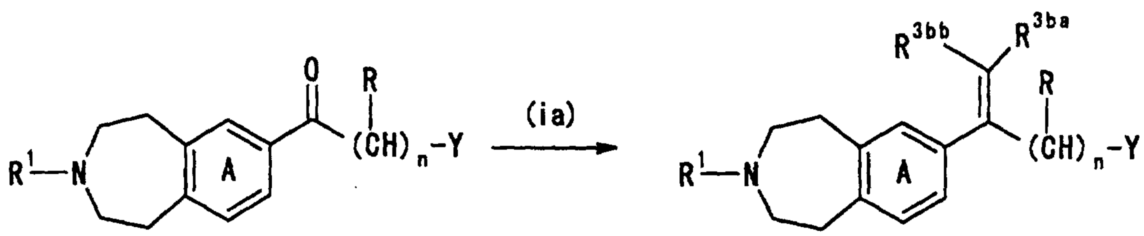


(IIIbh) (IIbi)

In Step (ia), Compound (IIIbh) can be reacted with an appropriate reagent for conversion of the carbonyl group to prepare Compound (IIbi).

Examples of a reaction used for conversion of the carbonyl group include the Wittig reaction, the Horner-Wadsworth-Emmons reaction, the Peterson olefination and the Knoevenagel reaction. As a reagent, any reagent commonly used for each reaction may be used.

This reaction may be carried out according to the procedures as described in Advanced Organic Chemistry, 5th ed. Wiley-Interscience: New York, 1992, pp. 879-981, Organic Synthesis, coll. vol. 5, 751 (1973), Organic Synthesis, coll. vol. 5, 509 (1973), Synthesis, 384 (1984) or Org. Reactions, 15, 204 (1967) or equivalent procedures.

Among compounds of Formula (IIb), a compound of Formula (IIbj) (X = $-C(=NR^{3ba})-$) or a salt thereof may be prepared according to the following Reaction Scheme 10.

## Reaction Scheme 10

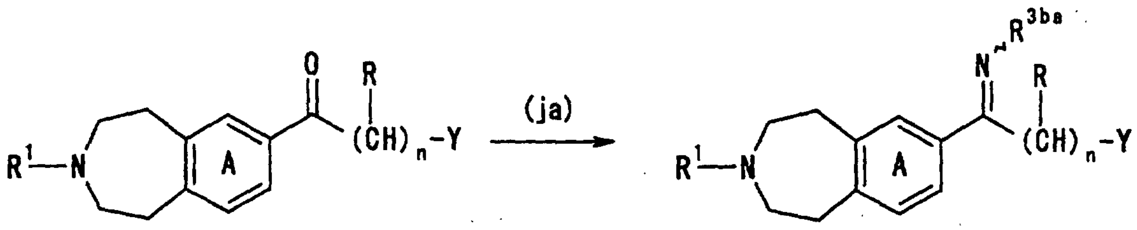


(IIIbh) (IIbj)

In Step (ja), Compound (IIIbh) can be reacted with an appropriate reagent for conversion of the carbonyl group to prepare Compound (IIbj).

Examples of a reagent used for conversion of the carbonyl group include an optionally substituted hydrazine and an optionally substituted hydroxylamine, each of which may have a $C_{1-6}$ alkyl group or the like as a substituent.

This reaction may be carried out according to the procedures as described in Advanced Organic Chemistry, 5th ed. Wiley-Interscience: New York, 1992, pp. 904-907, Organic Functional Group Preparations), vol. III, Academic (1983) or Rodd's Chemistry of Carbon Compounds, vol. 1, part C, Elsevier Publishing Co. (1965) or equivalent procedures.

Among compounds of Formula (IIb), a compound of Formula (IIbk) (X = -CS-) or a salt thereof may be prepared according to the following Reaction Scheme 11.

## Reaction Scheme 11

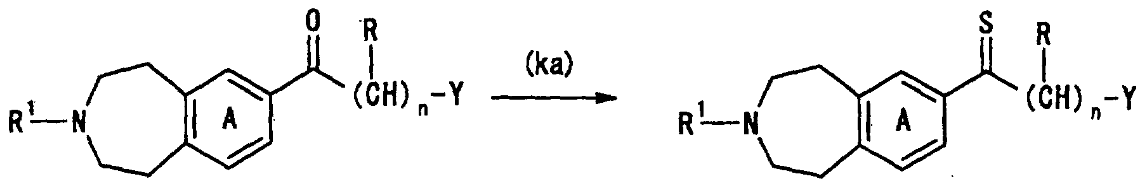


(IIIbh) (IIbk)

In Step (ka), Compound (IIIbh) can be reacted with an appropriate reagent for conversion of the carbonyl group into a thiocarbonyl group to prepare Compound (IIbk).

Examples of a reagent used for conversion of the carbonyl group into a thiocarbonyl group include commonly-used sulfurizing reagents such as Lawesson's reagent, diphosphorus pentasulfide and hydrogen sulfide-hydrochloric acid.

This reaction may be carried out according to the procedures as described in Synthesis, 7, 543 (1991), J. Am. Chem. Soc., 106, 934 (1984) or J. Am. Chem. Soc., 68, 769 (1946) or equivalent procedures.

Among compounds of Formula (IIb), a compound of Formula (IIbm) (X = $-CONR^{3ba}-$) or a salt thereof may be prepared according to the following Reaction Scheme 12-1.

## Reaction Scheme 12-1

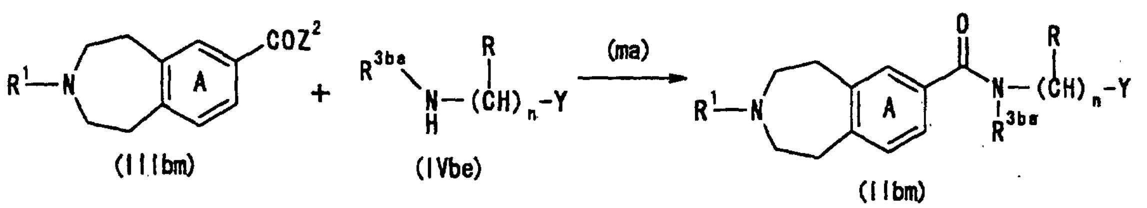


(IIIbm) (IVbe) (IIbm)

In Step (ma), a compound of Formula (IIIbm) [wherein each symbol is as defined above] (hereinafter also

simply referred to as Compound (IIIbm)) and Compound (IVbe) can be condensed together to prepare Compound (IIbm).

The condensation between Compound (IIIbm) and Compound (IVbe) may be carried out, for example, in the same manner as described above for the amidation between Compound (IIIbb) and Compound (IVbc).

Likewise, the starting compound (IIIbm) in Step (ma) may be prepared according to the following Reaction Scheme 12-2, Namely, Compound (IIIbm) can be prepared by performing the following steps successively:

Step (mb): acetylation of Compound (Vbb); and

Step (mc): oxidation of a compound of Formula (VIbm) [wherein each symbol is as defined above] (hereinafter also simply referred to as Compound (VIbm)) and, if necessary, followed by functional group conversion.

**Reaction Scheme 12-2**

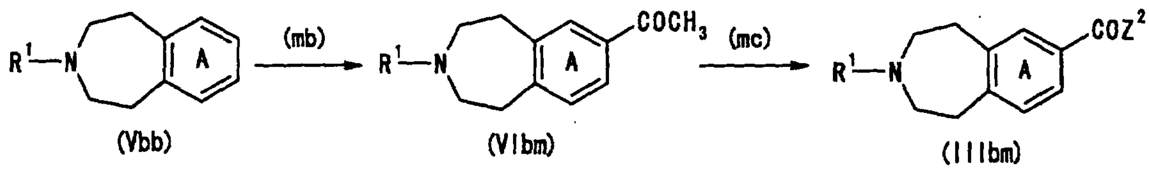


(Vbb) (VIbm) (IIIbm)

In Step (mb), Compound (Vbb) can be acetylated to prepare Compound (VIbm).

This reaction may be carried out under standard conditions for the Friedel-Crafts reaction. Examples of an acetylting reagent available for use include acetyl chloride and acetic anhydride. More specifically, Compound (VIbm) can be prepared according to the procedures as described in JP-A-5-140149, JP-A-6-206875 or J. Med. Chem., 37, 2292 (1994) or equivalent procedures.

In Step (mc), Compound (VIbm) can be oxidized to prepare Compound (IIIbm), in particular, wherein $Z^2$ is a hydroxyl group.

Examples of an oxidizing agent used in this reaction include hypochlorite, hypobromite, or a halogen element (e.g., bromine, iodine) in combination with an appropriate base (e.g., sodium hydroxide). More specifically, this reaction may be carried out according to the procedures as described in Org. Synthesis, Coll. Vol. 2, 428 (1943) or J. Am. Chem. Soc., 66, 894 (1944) or equivalent procedures.

If necessary, Compound (IIIbm) wherein $Z^2$ is a hydroxyl group may further be subjected to functional group conversion of the hydroxyl group to give Compound (IIIbm) wherein $Z^2$ is a halogen atom (e.g., chloro, bromo, iodo), a $C_{1-6}$ alkyloxy group (e.g., methoxy, ethoxy, benzyloxy) or a $C_{6-10}$ aryloxy group (e.g., phenoxy, p-nitrophenoxy).

The functional group conversion may be carried out according to the procedures as described in Advanced Organic Chemistry, 5th ed. Wiley-Interscience: New York, 1992, pp. 393-396 and 437-438 or Comprehensive Organic Transformations, VCH Publishers Inc. (1989) or equivalent procedures.

Compound (IIb) thus prepared may be isolated and purified by known isolation and purification means including concentration, vacuum concentration, solvent extraction, crystallization, recrystallization, phasic transfer and chromatography.

Still other compounds useful as GPR14 (SENR) antagonists, obtained by the screening method or screening kit of the present invention, include compounds of Formula (Ic):

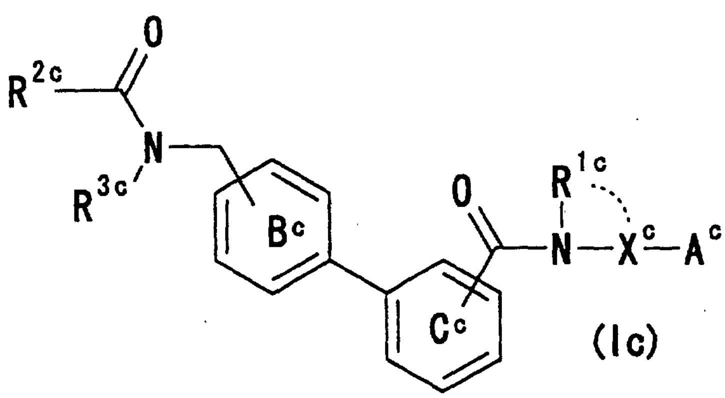

(Ic)

[wherein $R^{1c}$ represents a hydrogen atom or an optionally substituted hydrocarbon group, $X^c$ represents a spacer containing 1 to 12 atoms in its linear chain moiety, $R^{1c}$ and $X^c$ may together form a ring, $A^c$ represents an optionally substituted amino group or an optionally substituted nitrogen-containing heterocyclic group, $R^{2c}$ represents an optionally substituted hydrocarbon group or an optionally substituted amino group, $R^{3c}$ represents an optionally substituted hydrocarbon group, the rings $B^c$ and $C^c$ each represent a benzene ring which may further be substituted] or salts thereof.

In the above Formula (Ic), the "benzene ring which may further be substituted" represented by $B^c$ or $C^c$ refers to a benzene ring which may have a substituent(s) in addition to the substituent defined in Formula (Ic). Examples of such a substituent (other than the substituents defined in Formula (Ic)) include an optionally substituted hydrocarbon group, an optionally substituted heterocyclic group, a nitro group, a halogen atom, an optionally substituted amino group, a group of the formula $R^{6c}$-$Y^c$-(wherein $Y^c$ represents an oxygen atom or an optionally oxidized sulfur atom (e.g., S, S(O), $S(O)_2$), and $R^{6c}$ represents an optionally substituted hydrocarbon group or an optionally substituted heterocyclic group), a cyano group, an optionally substituted acyl group, and a carboxyl group which may be esterified or amidated.

Examples of a "hydrocarbon group" in an "optionally substituted hydrocarbon group" as a substituent which may be on the benzene ring in the "benzene ring which may further be substituted" represented by $B^c$ or $C^c$ and a "hydrocarbon group" in the "optionally substituted hydrocarbon group" represented by $R^{6c}$ include:

(1) alkyls (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(2) cycloalkyls (e.g., $C_{3-8}$ cycloalkyls such as cyclopropyl, cyclobutyl, cyclopentyl, cyclohexyl and cycloheptyl), in which the cycloalkyl may be condensed with the benzene ring to form indan (e.g., indan-1-yl, indan-2-yl), tetrahydronaphthalene (e.g., tetrahydronaphthalen-5-yl, tetrahydronaphthalen-6-yl) or the like (preferably, e.g., indan) or the cycloalkyls may be bridged together via a linear atom chain containing 1 or 2 carbon atoms to form a bridged cyclic hydrocarbon residue such as bicyclo[2.2.1]heptyl, bicyclo[2.2.2]octyl, bicyclo[3.2.1]octyl or bicyclo[3.2.2]nonyl (preferably, e.g., cyclohexyls bridged together via a linear atom chain containing 1 or 2 carbon atoms, more preferably, e.g., bicyclo[2.2.1]heptyl);
(3) alkenyls (e.g., $C_{2-10}$ alkenyls such as vinyl, allyl, crotyl, 2-pentenyl and 3-hexenyl, preferably lower ($C_{2-6}$) alkenyls);
(4) cycloalkenyls (e.g., $C_{3-8}$ cycloalkenyls such as 2-cyclopentenyl, 2-cyclohexenyl, 2-cyclopentenylmethyl and 2-cyclohexenylmethyl);
(5) alkynyls (e.g., $C_{2-10}$ alkynyls such as ethynyl, 1-propynyl, 2-propynyl, 1-butynyl, 2-pentynyl and 3-hexynyl, preferably lower ($C_{2-6}$) alkynyls);
(6) aryls (e.g., $C_{6-14}$ aryls such as phenyl and naphthyl, preferably $C_{6-10}$ aryls, more preferably phenyl); and
(7) aralkyls (e.g., $C_{1-6}$ alkyls having 1 to 3 $C_{6-14}$ aryls, preferably phenyl-$C_{1-4}$ alkyls such as benzyl and phenethyl).
Above all, alkyls are preferred, $C_{1-4}$ alkyls such as methyl and ethyl are more preferred, and methyl is particularly preferred for use.

The hydrocarbon group may have a substituent(s). Examples of such a substituent include a halogen atom (e.g., fluorine, chlorine, bromine, iodine), nitro, cyano, oxo, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a phenyl-lower ($C_{1-4}$) alkyl, a $C_{3-7}$ cycloalkyl, a carboxyl group which may be esterified or amidated (e.g., carboxyl, a $C_{1-4}$ alkoxy-carbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl) and a $C_{1-4}$ alkylsulfinyl (e.g., methanesulfinyl, ethanesulfinyl). The number of substituents is preferably 1 to 3.

Examples of a "heterocyclic group" in an "optionally substituted heterocyclic group" as a substituent which may be on the benzene ring in the "benzene ring which may further be substituted" represented by $B^c$ or $C^c$ and a "heterocyclic group" in the "optionally substituted heterocyclic group" represented by $R^{6c}$ include groups formed by removing one hydrogen atom from a 5- to 8-membered aromatic heterocyclic ring, a 5- to 8-membered saturated or unsaturated non-aromatic heterocyclic ring (aliphatic heterocyclic ring) or the like, each containing at least one heteroatom (preferably 1 to 4, more preferably 1 or 2 heteroatoms) selected from 1 to 3 kinds (preferably 1 or 2 kinds) of an oxygen atom, a sulfur atom, a nitrogen atom and the like.

Examples of the "aromatic heterocyclic ring" as used here include 5- to 8-membered (preferably 5- to 6-membered) aromatic monocyclic heterocyclic rings (e.g., furan, thiophene, pyrrole, oxazole, isooxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole,

1,3,4-thiadiazole, 1,2,3-triazole, 1,2,4-triazole, tetrazole, pyridine, pyridazine, pyrimidine, pyrazine, triazine). Examples of the "non-aromatic heterocyclic ring" as used here include 5- to 8-membered (preferably 5- to 6-membered) saturated or unsaturated monocyclic non-aromatic heterocyclic rings (aliphatic heterocyclic rings) such as pyrrolidine, tetrahydrofuran, tetrahydrothiophene, thiolane, dithiolane, oxathiolane, pyroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepine, thiepine and azepine, as well as 5- to 8-membered non-aromatic heterocyclic rings obtained by saturating part or all of double bonds in the above-mentioned aromatic monocyclic heterocyclic rings.

Alternatively, a "heterocyclic group" in an "optionally substituted heterocyclic group" as a substituent which may be on the benzene ring in the "benzene ring which may further be substituted" represented by $B^c$ or $C^c$ and a "heterocyclic group" in the "optionally substituted heterocyclic group" represented by $R^{6c}$ may each be a group formed by removing one hydrogen atom from a condensed ring formed by condensing 2 to 3 rings (preferably 2 rings) selected from the above-mentioned monocyclic heterocyclic rings (monocyclic aromatic heterocyclic rings and monocyclic non-aromatic heterocyclic rings) and 5- to 8-membered cyclic hydrocarbons (e.g., 5- to 8-membered (preferably 5- to 6-membered) saturated or unsaturated alicyclic hydrocarbons such as $C_{5-8}$ cycloalkanes, $C_{5-8}$ cycloalkenes and $C_{5-8}$ cycloalkadienes, 6-membered aromatic hydrocarbons such as benzene); such a condensed ring may be a saturated condensed ring, a partially unsaturated condensed ring or an aromatic condensed ring.

Preferred examples of such a condensed ring include a ring formed by condensing the same or different two heterocyclic rings (preferably one heterocyclic ring and one aromatic heterocyclic ring, more preferably the same or different two aromatic heterocyclic rings) and a ring formed by condensing one heterocyclic ring and one homocyclic ring (preferably one heterocyclic ring and one benzene ring, more preferably one aromatic heterocyclic ring and one benzene ring). Specific examples of such a condensed ring include indole, benzothiophene, benzofuran, benzimidazole, imidazo[1,2-a]pyridine, quinoline, isoquinoline and cinnoline.

A "heterocyclic group" in an "optionally substituted heterocyclic group" as a substituent which may be on the benzene ring in the "benzene ring which may further be substituted" represented by $B^c$ or $C^c$ and a "heterocyclic group" in the "optionally substituted heterocyclic group" represented by $R^{6c}$ may have a substituent(s). Examples of such a substituent include the same substituents as listed above for the "optionally substituted hydrocarbon group" as a substituent which may be on the benzene ring in the "benzene ring which may further be substituted" represented by $B^c$ or $C^c$.

Examples of a "halogen atom" as a substituent which may be on the benzene ring in the "benzene ring which may further be substituted" represented by $B^c$ or $C^c$ include fluorine, chlorine, bromine and iodine.

Examples of an "optionally substituted amino group" as a substituent which may be on the benzene ring in the "benzene ring which may further be substituted" represented by $B^c$ or $C^c$ include the same groups as listed below for the "optionally substituted amino group" represented by $A^c$. Above all, preferred are amino groups which may have 1 or 2 substituents selected from an "optionally substituted hydrocarbon group" (e.g., the same groups as listed above for the "optionally substituted hydrocarbon group" as a substituent which may be on the benzene ring in the "benzene ring which may further be substituted" represented by $B^c$ or $C^c$), an "optionally substituted heterocyclic group" (e.g., the same groups as listed above for the "optionally substituted heterocyclic group" as a substituent which may be on the benzene ring in the "benzene ring which may further be substituted" represented by $B^c$ or $C^c$) and an "optionally substituted acyl group" (e.g., the same groups as listed below for the "optionally substituted acyl group" as a substituent which may be on the benzene ring in the "benzene ring which may further be substituted" represented by $B^c$ or $C^c$). Particularly preferred are amino groups which may have 1 or 2 optionally substituted alkyls [e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls, each of which may have 1 to 3 substituents selected from a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a phenyl-lower ($C_{1-4}$) alkyl, a $C_{3-7}$ cycloalkyl, a carboxyl group which may be esterified or amidated (e.g., carboxyl, a $C_{1-4}$ alkoxycarbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl) and the like].

Alternatively, an "optionally substituted amino group" as a substituent which may be on the benzene ring in the "benzene ring which may further be substituted" represented by $B^c$ or $C^c$ may form a cyclic amino group through binding between substituents on the amino group (e.g., a cyclic amino group which is formed by removing one hydrogen atom from a ring-constituting nitrogen atom in a 5- to 6-membered ring such as tetrahydropyrrole, piperazine, piperidine, morpholine, thiomorpholine, pyrrole or imidazole and which has a binding hand on the nitrogen atom). The cyclic amino

group may have a substituent(s). Examples of such a substituent include a halogen atom (e.g., fluorine, chlorine, bromine, iodine), nitro, cyano, a hydroxyl group, a thiol group, an amino group, a carboxyl group, an optionally halogenated $C_{1-4}$ alkyl (e.g., trifluoromethyl, methyl, ethyl), an optionally halogenated $C_{1-4}$ alkoxy (e.g., methoxy, ethoxy, propoxy, butoxy, trifluoromethoxy, trifluoroethoxy), formyl, a $C_{2-4}$ alkanoyl (e.g., acetyl, propionyl) and a $C_{1-4}$ alkylsulfonyl (e.g., methanesulfonyl, ethanesulfonyl). The number of substituents is preferably 1 to 3.

Examples of an "optionally substituted acyl group" as a substituent which may be on the benzene ring in the "benzene ring which may further be substituted" represented by $B^c$ or $C^c$ include a carbonyl or sulfonyl group attached to a hydrogen atom, an "optionally substituted hydrocarbon group" (e.g., the same groups as listed above for the "optionally substituted hydrocarbon group" as a substituent which may be on the benzene ring in the "benzene ring which may further be substituted" represented by $B^c$ or $C^c$), an "optionally substituted heterocyclic group" (e.g., the same groups as listed above for the "optionally substituted heterocyclic group" as a substituent which may be on the benzene ring in the "benzene ring which may further be substituted" represented by $B^c$ or $C^c$) or the like. Preferred examples include a carbonyl or sulfonyl group attached to:

(1) a hydrogen atom;
(2) an optionally substituted alkyl (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(3) an optionally substituted cycloalkyl (e.g., $C_{3-7}$ cycloalkyls such as cyclopropyl, cyclobutyl, cyclopentyl, cyclohexyl and cycloheptyl);
(4) an optionally substituted alkenyl (e.g., $C_{2-10}$ alkenyls such as allyl, crotyl, 2-pentenyl and 3-hexenyl, preferably lower ($C_{2-6}$) alkenyls);
(5) an optionally substituted cycloalkenyl (e.g., $C_{3-7}$ cycloalkenyls such as 2-cyclopentenyl, 2-cyclohexenyl, 2-cyclopentenylmethyl and 2-cyclohexenylmethyl);
(6) an optionally substituted 5- to 6-membered monocyclic aromatic group (e.g., phenyl, pyridyl) or the like (e.g., acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl). Examples of a substituent which may be on the above-mentioned (2) optionally substituted alkyl, (3) optionally substituted cycloalkyl, (4) optionally substituted alkenyl, (5) optionally substituted cycloalkenyl or (6) optionally substituted 5- to 6-membered monocyclic aromatic group include a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group which may be esterified or amidated (e.g., carboxyl, a $C_{1-4}$ alkoxycarbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), formyl, a $C_{2-4}$ alkanoyl (e.g., acetyl, propionyl), a $C_{1-4}$ alkylsulfonyl (e.g., methanesulfonyl, ethanesulfonyl) and a $C_{1-4}$ alkylsulfinyl (e.g., methanesulfinyl, ethanesulfinyl). The number of substituents is preferably 1 to 3.

Examples of a "carboxyl group which may be esterified" as a substituent which may be on the benzene ring in the "benzene ring which may further be substituted" represented by $B^c$ or $C^c$ include a carbonyloxy group attached to a hydrogen atom, an "optionally substituted hydrocarbon group" (e.g., the same groups as listed above for the "optionally substituted hydrocarbon group" as a substituent which may be on the benzene ring in the "benzene ring which may further be substituted" represented by $B^c$ or $C^c$) or the like. Preferred examples include a carbonyloxy group attached to:

(1) a hydrogen atom;
(2) an optionally substituted alkyl (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(3) an optionally substituted cycloalkyl (e.g., $C_{3-7}$ cycloalkyls such as cyclopropyl, cyclobutyl, cyclopentyl, cyclohexyl and cycloheptyl);
(4) an optionally substituted alkenyl (e.g., $C_{2-10}$ alkenyls such as allyl, crotyl, 2-pentenyl and 3-hexenyl, preferably lower ($C_{2-6}$) alkenyls);
(5) an optionally substituted cycloalkenyl (e.g., $C_{3-7}$ cycloalkenyls such as 2-cyclopentenyl, 2-cyclohexenyl, 2-cyclopentenylmethyl and 2-cyclohexenylmethyl);
(6) an optionally substituted aryl (e.g., phenyl, naphthyl) or the like. More preferred examples include carboxyl, a lower ($C_{1-6}$) alkoxycarbonyl and an aryloxycarbonyl (e.g., methoxycarbonyl, ethoxycarbonyl, propoxycarbonyl, phenoxycarbonyl, naphthoxycarbonyl). Examples of a substituent which may be on the above-mentioned (2) optionally substituted alkyl, (3) optionally substituted cycloalkyl, (4) optionally substituted alkenyl, (5) optionally substituted cycloalkenyl or (6) optionally substituted aryl include a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group which may be esterified or amidated (e.g., carboxyl, a $C_{1-4}$ alkoxycarbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), formyl, a $C_{2-4}$ alkanoyl (e.g., acetyl, propionyl), a $C_{1-4}$ alkylsulfonyl (e.g., methanesulfonyl, ethanesulfonyl) and a $C_{1-4}$ alkylsulfinyl (e.g., methanesulfinyl, ethanesulfinyl). The number of substituents is preferably 1 to 3.

Examples of a "carboxyl group which may be amidated" as a substituent which may be on the benzene ring in the "benzene ring which may further be substituted" represented by $B^c$ or $C^c$ include a carbonyl group attached to:

(1) a hydroxyl group;
(2) an "optionally substituted amino group" (e.g., the same groups as listed above for the "optionally substituted amino group" as a substituent which may be on the benzene ring in the "benzene ring which may further be substituted" represented by $B^c$ or $C^c$) or the like.

The benzene ring in the "benzene ring which may further be substituted" represented by $B^c$ or $C^c$ may have the same or different 1 to 4 substituents (preferably 1 or 2 substituents) at any position on the ring. Also, in a case where the benzene ring in the "benzene ring which may further be substituted" represented by $B^c$ or $C^c$ has two or more substituents, two of these substituents may together form a lower ($C_{1-6}$) alkylene (e.g., trimethylene, tetramethylene), a lower ($C_{1-6}$) alkyleneoxy (e.g., $-CH_2-O-CH_2-$, $-O-CH_2-CH_2-$), a lower ($C_{1-6}$) alkylenedioxy (e.g., $-O-CH_2-O-$, $-O-CH_2-CH_2-O-$), a lower ($C_{2-6}$) alkenylene (e.g., $-CH_2-CH=CH-$, $-CH_2-CH_2-CH=CH-$, $-CH_2-CH=CH-CH_2-$), a lower ($C_{4-6}$) alkadienylene (e.g., $-CH=CH-CH=CH-$) or the like.

Preferred examples of a substituent which may be on the benzene ring in the "benzene ring which may further be substituted" represented by $B^c$ or $C^c$ include an optionally substituted hydrocarbon group, an optionally substituted heterocyclic group, a nitro group, a halogen atom, an optionally substituted amino group, a group of the formula $R^{6c}$-$Y^c$- (wherein $Y^c$ represents an oxygen atom or an optionally oxidized sulfur atom, and $R^{6c}$ represents an optionally substituted hydrocarbon group or an optionally substituted heterocyclic group). More preferred examples include an optionally substituted hydrocarbon group, an optionally substituted heterocyclic group, a halogen atom, an optionally substituted amino group, and a group of the formula $R^{6c}$-$Y^c$-(wherein $Y^c$ represents an oxygen atom or an optionally oxidized sulfur atom, and $R^{6c}$ represents an optionally substituted hydrocarbon group or an optionally substituted heterocyclic group), with a lower ($C_{1-4}$) alkyl, a halogen atom and the like being particularly preferred.

The "benzene ring which may further be substituted" represented by $B^c$ or $C^c$ is preferably free from any more substituent in addition to the defined substituent.

In the above Formula (Ic), examples of a "hydrocarbon group" in the "optionally substituted hydrocarbon group" represented by $R^{1c}$, $R^{2c}$ and $R^{3c}$ include:

(1) alkyls (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(2) cycloalkyls (e.g., $C_{3-8}$ cycloalkyls such as cyclopropyl, cyclobutyl, cyclopentyl, cyclohexyl and cycloheptyl), in which the cycloalkyl may be condensed with the benzene ring to form indan (e.g., indan-1-yl, indan-2-yl), tetrahydronaphthalene (e.g., tetrahydronaphthalen-5-yl, tetrahydronaphthalen-6-yl) or the like (preferably, e.g., indan) or the cycloalkyls may be bridged together via a linear atom chain containing 1 or 2 carbon atoms to form a bridged cyclic hydrocarbon residue such as bicyclo[2.2.1]heptyl, bicyclo[2.2.2]octyl, bicyclo[3.2.1]octyl or bicyclo[3.2.2]nonyl (preferably, e.g., cyclohexyls bridged together via a linear atom chain containing 1 or 2 carbon atoms, more preferably, e.g., bicyclo[2.2.1]heptyl);
(3) alkenyls (e.g., $C_{2-10}$ alkenyls such as vinyl, allyl, crotyl, 2-pentenyl and 3-hexenyl, preferably lower ($C_{2-6}$) alkenyls);
(4) cycloalkenyls (e.g., $C_{3-8}$ cycloalkenyls such as 2-cyclopentenyl, 2-cyclohexenyl, 2-cyclopentenylmethyl and 2-cyclohexenylmethyl);
(5) alkynyls (e.g., $C_{2-10}$ alkynyls such as ethynyl, 1-propynyl, 2-propynyl, 1-butynyl, 2-pentynyl and 3-hexynyl, preferably lower ($C_{2-6}$) alkynyls);
(6) aryls (e.g., $C_{6-14}$ aryls such as phenyl and naphthyl, preferably $C_{6-10}$ aryls, more preferably phenyl);
(7) aralkyls (e.g., $C_{1-6}$ alkyls having 1 to 3 $C_{6-14}$ aryls, preferably phenyl-$C_{1-4}$ alkyls such as benzyl and phenethyl);

(8) groups of the formula $-X^{c'''}-G^c-(CH_2)_n-J^c$
[wherein $X^{c'''}$ represents a $C_{1-4}$ alkylene group or a $C_{2-4}$ alkenylene group, $G^c$ represents a binding hand, -O-, -S-, -CO-NH- or -NH-CO-, n represents an integer of 0 to 3, and $J^c$ represents an optionally substituted aromatic ring group]; and
(9) groups of the formula $-X^{c''''}-L^c-(CH_2)_n-M^c$
[wherein $X^{c''''}$ represents a binding hand or a $C_{1-4}$ alkylene group, $L^c$ represents (a) a binding hand, (b) an optionally substituted aromatic ring group, (c) -O-, (d) -S-, (e) -CO-NH- or (f) -NH-CO-, n represents an integer of 0 to 3, and $M^c$ represents an amino group, a guanidino group, a sulfamoyl group, a carbamoyl group or a hydroxyl group].

In the above formulae, examples of the optionally substituted aromatic ring group represented by $J^c$ and $L^c$ include an optionally substituted aryl group and an optionally substituted aromatic heterocyclic group.
Examples of an "aryl group" in the "optionally substituted aryl group" represented by $J^c$ and $L^c$ include $C_{6-14}$ aryls such as phenyl and naphthyl, with $C_{6-10}$ aryls being preferred and with phenyl being more preferred.
Examples of an "aromatic heterocyclic group" in the "optionally substituted aromatic heterocyclic group" represented by $J^c$ and $L^c$ include the same groups as listed above for the optionally substituted aromatic heterocyclic group as the "optionally substituted heterocyclic group" represented by $R^{6c}$, with an optionally substituted 5- to 6-membered aromatic monocyclic heterocyclic group being particularly preferred. Examples of such a 5- to 6-membered aromatic monocyclic heterocyclic group include furan, thiophene, pyrrole, oxazole, isooxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine and triazine.
The "aromatic ring group" in the "optionally substituted aromatic ring group" represented by $J^c$ and $L^c$ may have a substituent(s). Examples of such a substituent include a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole, imidazole, 2-oxo-1-pyrrolidinyl or 2-oxo-1-piperidinyl), a phenyl-lower ($C_{1-4}$) alkyl, a $C_{3-7}$ cycloalkyl, a carboxyl group which may be esterified or amidated (e.g., carboxyl; a $C_{1-4}$ alkoxy-carbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl), an optionally substituted sulfamoyl group (e.g., sulfamoyl, a mono-$C_{1-4}$ alkylsulfamoyl, a di-$C_{1-4}$ alkylsulfamoyl), an optionally substituted aryl group and an optionally substituted heterocyclic group. The number of substituents is preferably 1 to 3.
The "hydrocarbon group" in the "optionally substituted hydrocarbon group" represented by $R^{1c}$, $R^{2c}$ and $R^{3c}$ may have a substituent(s). Examples of such a substituent include a halogen atom (e.g., fluorine, chlorine, bromine, iodine), nitro, cyano, oxo, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole, imidazole, 2-oxo-1-pyrrolidinyl or 2-oxo-1-piperidinyl), a phenyl-lower ($C_{1-4}$) alkyl, a $C_{3-7}$ cycloalkyl, a carboxyl group which may be esterified or amidated (e.g., carboxyl, a $C_{1-4}$ alkoxy-carbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl), an optionally substituted sulfamoyl group (e.g., sulfamoyl, a mono-$C_{1-4}$ alkylsulfamoyl, a di-$C_{1-4}$ alkylsulfamoyl), an optionally substituted aryl group and an optionally substituted heterocyclic group. The number of substituents is preferably 1 to 3.
Examples of an "aryl group" in the "optionally substituted aryl group" as a substituent on the "optionally substituted hydrocarbon group" represented by $R^{1c}$, $R^{2c}$ and $R^{3c}$ include $C_{6-14}$ aryls such as phenyl and naphthyl, with $C_{6-10}$ aryls being preferred and with phenyl being more preferred
Examples of a substituent which may be on the "aryl group" include a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a phenyl-lower ($C_{1-4}$) alkyl, a $C_{3-7}$ cycloalkyl, a carboxyl group which may be esterified or amidated (e.g., carboxyl, a $C_{1-4}$ alkoxy-carbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl,

ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl), an optionally substituted sulfamoyl group (e.g., sulfamoyl, a mono-$C_{1-4}$ alkylsulfamoyl, a di-$C_{1-4}$ alkylsulfamoyl) and a 5- to 6-membered aromatic monocyclic heterocyclic ring (e.g., furan, thiophene, pyrrole, oxazole, isooxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine). The number of substituents is preferably 1 to 3.

Examples of an "optionally substituted heterocyclic group" as a substituent on the "optionally substituted hydrocarbon group" represented by $R^{1c}$, $R^{2c}$ and $R^{3c}$ include the same groups as listed above for the "optionally substituted heterocyclic group" represented by $R^{6c}$.

In the above Formula (Ic), preferred examples of a substituent on the "amino group" in the "optionally substituted amino group" represented by $R^{2c}$ include an optionally substituted hydrocarbon group, an optionally substituted heterocyclic group and an optionally substituted acyl group. If the "amino group" is substituted, the number of substituents is 1 or 2.

Examples of a hydrocarbon group as a substituent on the "optionally substituted amino group" represented by $R^{2c}$ include:

(1) alkyls (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);

(2) cycloalkyls (e.g., $C_{3-8}$ cycloalkyls such as cyclopropyl, cyclobutyl, cyclopentyl, cyclohexyl and cycloheptyl), in which the cycloalkyl may be condensed with the benzene ring to form indan (e.g., indan-1-yl, indan-2-yl); tetrahydronaphthalene (e.g., terrahydronaphthalen-5-yl, tetrahydronaphthalen-6-yl) or the like (preferably, e.g., indan) or the cycloalkyls may be bridged together via a linear atom chain containing 1 or 2 carbon atoms to form a bridged cyclic hydrocarbon residue such as bicyclo[2.2.1]heptyl, bicyclo[2.2.2]octyl, bicyclo[3.2.1]octyl or bicyclo[3.2.2]nonyl (preferably, e.g., cyclohexyls bridged together via a linear atom chain containing 1 or 2 carbon atoms, more preferably, e.g., bicyclo[2.2.1]heptyl);

(3) alkenyls (e.g., $C_{2-10}$ alkenyls such as vinyl, allyl, crotyl, 2-pentenyl and 3-hexenyl, preferably lower ($C_{2-6}$)alkenyls);

(4) cycloalkenyls (e.g., $C_{3-8}$ cycloalkenyls such as 2-cyclopentenyl, 2-cyclohexenyl, 2-cyclopentenylmethyl and 2-cyclohexenylmethyl);

(5) alkynyls (e.g., $C_{2-10}$ alkynyls such as ethynyl, 1-propynyl, 2-propynyl, 1-butynyl, 2-pentynyl and 3-hexynyl, preferably lower ($C_{2-6}$) alkynyls);

(6) aryls (e.g., $C_{6-14}$ aryls such as phenyl and naphthyl, preferably $C_{6-10}$ aryls, more preferably phenyl); and

(7) aralkyls (e.g., $C_{1-6}$ alkyls having 1 to 3 $C_{6-14}$ aryls, preferably phenyl-$C_{1-4}$ alkyls such as benzyl and phenethyl).

Examples of a heterocyclic group as a substituent on the "optionally substituted amino group" represented by $R^{2c}$ include the same groups as listed above for the "heterocyclic group" in the "optionally substituted heterocyclic group" as a substituent which may be on the benzene ring in the "benzene ring which may further be substituted" represented by $B^c$ or $C^c$ and the same groups as listed above for the "heterocyclic group" in the "optionally substituted heterocyclic group" represented by $R^{6c}$.

Preferred examples of an acyl group as a substituent on the "optionally substituted amino group" represented by $R^{2c}$ include:

(I) a carbonyl or sulfonyl group attached to a hydrogen atom or a hydrocarbon group (e.g., the same groups as listed above for the hydrocarbon group as a substituent on the "optionally substituted amino group" represented by $R^{2c}$); and

(2) a carbonyl or sulfonyl group attached to a heterocyclic group (e.g., the same groups as listed above for the heterocyclic group as a substituent on the "optionally substituted heterocyclic group" represented by $R^{2c}$).

Examples of a substituent on the "optionally substituted hydrocarbon group", "optionally substituted heterocyclic group" or "optionally sbstituted acyl group" as a substituent on the "optionally substituted amino group" include the same substituents as listed above for the "hydrocarbon group" in the "optionally substituted hydrocarbon group" as a substituent which may be on the benzene ring in the "benzene ring which may further be substituted" represented by $B^c$ or $C^c$ and the same substituents as listed above for the "hydrocarbon group" in the "optionally substituted hydrocarbon group" represented by $R^{6c}$. The number of substituents is preferably 1 to 3.

In the above Formula (Ic), $R^{1c}$ is preferably a hydrogen atom or an optionally substituted $C_{1-6}$ alkyl, more preferably a hydrogen atom or a $C_{1-4}$ alkyl, and particularly preferably a hydrogen atom.

In the above Formula (Ic), the "optionally substituted hydrocarbon group" represented by $R^{2c}$ is preferably a group of the formula $-X^{c'''}-G^c-(CH_2)_m-J^c$ [wherein $X^{c'''}$ represents a $C_{1-4}$ alkylene group or a $C_{2-4}$ alkenylene group, $G^c$ represents a binding hand, -O-, -S-, -CO-NH- or -NH-CO-, n represents an integer of 0 to 3, and $J^c$ represents an optionally substituted aromatic ring group] or a group of the formula $-X^{c''''}-L^c-(CH_2)_n-M^c$ [wherein $X^{c''''}$ represents a binding hand or a $C_{1-4}$ alkylene group, $L^c$ represents (a) a binding hand, (b) an optionally substituted aromatic ring group, (c) -O-, (d) -S-, (e) -CO-NH- or (f) -NH-CO-, n represents an integer of 0 to 3, and $M^c$ represents an amino group, a guanidino group, a sulfamoyl group, a carbamoyl group or a hydroxyl group]. Preferred examples of the optionally substituted aromatic ring group represented by $J^c$ and $L^c$ include an optionally substituted phenyl and an optionally substituted 5- to 6-membered aromatic monocyclic heterocyclic group.

In the above Formula (Ic), the "optionally substituted hydrocarbon group" represented by $R^{3c}$ is preferably an optionally substituted $C_{1-6}$ alkyl, and particularly preferably a group of the formula $-(CH_2)_p-T^c$ [wherein p represents an integer of 1 to 6, and $T^c$ represents an optionally substituted aromatic ring group].

Examples of the "optionally substituted aromatic ring group" represented by $T^c$ include the same groups as listed above for the "optionally substituted aromatic ring group" represented by $J^c$, although a phenyl group is preferred for an "aromatic ring group" in the "optionally substituted aromatic ring group" represented by T. Preferred examples of a substituent which may be on the "aromatic ring group" in the "optionally substituted aromatic ring group" represented by $T^c$ include a hydroxyl group and an optionally substituted sulfamoyl group (e.g., sulfamoyl, a mono-$C_{1-4}$ alkylsulfamoyl, a di-$C_{1-4}$ alkylsulfamoyl).

In the above Formula (Ic), in a case where $R^{1c}$ and $X^c$ may together form a ring, such a "ring" may be either saturated or unsaturated as long as it is a nitrogen-containing heterocyclic ring. Although such a ring is of any size, a 3- to 8-membered nitrogen-containing heterocyclic ring is particularly preferred and a 3- to 8-membered nitrogen-containing saturated heterocyclic ring, i.e., a ring of the following formula:

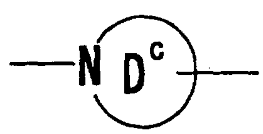

[wherein the ring $D^c$ represents a 3- to 8-membered nitrogen-containing saturated heterocyclic ring] is most particularly preferred.

Examples of such a "3- to 8-membered nitrogen-containing heterocyclic ring" include 3- to 8-membered nitrogen-containing heterocyclic rings which may contain, in addition to one nitrogen atom, 1 to 4 heteroatoms (preferably 1 or 2 heteroatoms) selected from 1 to 3 kinds (preferably 1 or 2 kinds) of an oxygen atom, a sulfur atom, a nitrogen atom and the like. More specific examples include 3- to 8-membered (preferably 5- to 6-membered) saturated or unsaturated (preferably saturated) monocyclic non-aromatic heterocyclic rings (aliphatic heterocyclic rings) such as pyrrolidine, pyroline, imidazolidine, imidazoline, pyrazolidine, pyrazoline, oxazine, oxadiazine, thiazine, thiadiazine, piperidine, morpholine, thiomorpholine, piperazine and azepine.

The "3- to 8-membered nitrogen-containing heterocyclic ring" may have a substituent(s). Examples of such a substituent include the same substituents as listed above for the "optionally substituted hydrocarbon group" as a substituent which may be on the benzene ring in the "benzene ring which may further be substituted" represented by $B^c$ or $C^c$.

Alternatively, in the above Formula (Ic), $R^{1c}$ may form a ring together with the "optionally substituted amino group" represented by $A^c$. Such a "ring" may be either saturated or unsaturated as long as it is a heterocyclic ring containing at least two nitrogen atoms. Although such a ring is of any size, a 3- to 8-membered nitrogen-containing heterocyclic ring is particularly preferred and a 3- to 8-membered nitrogen-containing saturated heterocyclic ring, i.e., a ring of the following formula:

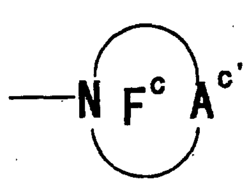

[wherein $A^{c'}$ represents an optionally substituted nitrogen atom, and the ring $F^c$ represents a 3- to 8-membered nitrogen-containing saturated heterocyclic ring] is most particularly preferred.

In the above formula, examples of a substituent which may be on the nitrogen atom in the "optionally substituted nitrogen atom" represented by $A^c$, include the same substituents as listed below for the "amino group" in the "optionally substituted amino group" represented by $A^c$.

Examples of such a "3- to 8-membered nitrogen-containing heterocyclic ring" include 3- to 8-membered nitrogen-containing heterocyclic rings which may contain, in addition to two nitrogen atoms, 1 to 4 heteroatoms (preferably 1 or 2 heteroatoms) selected from 1 to 3 kinds (preferably 1 or 2 kinds) of an oxygen atom, a sulfur atom, a nitrogen atom and the like. More specific examples include 3- to 8-membered (preferably 5- to 6-membered) saturated or unsaturated (preferably saturated) monocyclic non-aromatic heterocyclic rings (aliphatic heterocyclic rings) such as imidazolidine, imidazoline, pyrazolidine, pyrazoline, oxadiazine, thiadiazine, piperazine and diazepine.

The "3- to 8-membered nitrogen-containing heterocyclic ring" may have a substituent(s). Examples of such a substituent include the same substituents as listed above for the "optionally substituted hydrocarbon group" as a substituent which may be on the benzene ring in the "benzene ring which may further be substituted" represented by $B^c$ or $C^c$.

In the above formula, the "spacer containing 1 to 12 atoms in its linear chain moiety" represented by $X^c$ may be any "divalent group containing 1 to 12 atoms in its linear chain moiety." Examples include saturated divalent groups such as:

(1) $-(CH_2)_{t1}-$ (wherein t1 represents an integer of 1 to 12, preferably an integer of 1 to 8, more preferably an integer of 1 to 6, particularly preferably an integer of 1 to 4);
(2) $-(CH_2)_{u1}-X^{c1}-(CH_2)_{u2}-$ (wherein u1 and u2, which may be the same or different, each represent an integer of 0 to 11, provided that the sum of u1 and u2 is 0 to 11, and $X^{c1}$ represents NH, O, S, SO or $SO_2$);
(3) $-(CH_2)_{v1}-X^{c1}-(CH_2)_{v2}-X^{c2}-(CH_2)_{v3}-$ (wherein v1, v2 and v3, which may be the same or different, each represent an integer of 0 to 10, provided that the sum of v1, v2 and v3 is 0 to 10, and $X^{c1}$ and $X^{c2}$ each represent NH, O, S, SO or $SO_2$, provided that when v2 is 0, at least one of $X^1$ and $X^2$ preferably represents NH); as well as divalent groups obtained by partial unsaturation of these groups. Specific examples include divalent groups such as $-O-(CH_2)_{w3}-$ (wherein w3 is an integer of 0 to 11), $-(CH_2)_{w3}-O-$ (wherein w3 is an integer of 0 to 11), $-S-(CH_2)_{w3}-$ (wherein w3 is an integer of 0 to 11), $-(CH_2)_{w3}-S-$ (wherein w3 is an integer of 0 to 11), $-NH-(CH_2)_{w3}-$ (wherein w3 is an integer of 0 to 11), $-(CH_2)_{w3}-NH-$ (wherein w3 is an integer of 0 to 11), $-(CH_2)_{w4}-$(wherein w4 is an integer of 1 to 12), $-CH=CH-$, $-C\equiv C-$, $-CO-NH-$ and $-SO_2-NH-$.

$X^c$ is more preferably a divalent group containing 1 to 4 atoms in its linear chain moiety, and particularly preferably a $C_{1-4}$ alkylene, a $C_{2-4}$ alkenylene, etc. Above all, a $C_{1-4}$ alkylene is preferred for use.

The divalent group represented by $X^c$ may have a substituent(s) at any position (preferably, on its carbon atom(s)). Such a substituent may be any group as long as it can be attached to the divalent chain constituting the linear chain moiety, including the same substituents as listed above for the "benzene ring which may further be substituted" represented by $B^c$ or $C^c$ and an oxo group. The divalent group may have the same or different 1 to 4 substituents (preferably 1 or 2 substituents) at any position. Alternatively, substituents on the divalent group as $X^c$ may together form a ring. Examples of such a ring include $C_{5-7}$ cycloalkanes such as cyclopentane, cyclohexane and cycloheptane as well as benzene.

Examples of a preferred substituent which may be on the divalent group as $X^c$ include a lower ($C_{1-6}$) alkyl (e.g., methyl, ethyl, propyl, isopropyl, butyl, isobutyl, sec-butyl, tert-butyl, pentyl, isopentyl, neopentyl, hexyl), a lower ($C_{3-7}$) cycloalkyl (e.g., cyclopropyl, cyclobutyl, cyclopentyl, cyclohexyl, cycloheptyl), formyl, a lower ($C_{2-7}$) alkanoyl (e.g., acetyl, propionyl, butyryl), a lower ($C_{1-6}$) alkoxy-carbonyl, a lower ($C_{1-6}$) alkoxy, a hydroxyl group and oxo.

In the above formula, examples of the "optionally substituted amino group" represented by $A^c$ include amino groups which may have 1 or 2 substituents selected from an "optionally substituted hydrocarbon group" (e.g., the same groups as listed above for the "optionally substituted hydrocarbon group" as a substituent which may be on the benzene ring in the "benzene ring which may further be substituted" represented by $B^c$ or $C^c$), an "optionally substituted heterocyclic group" (e.g., the same groups as listed above for the "optionally substituted heterocyclic group" as a substituent which may be on the benzene ring in the "benzene ring which may further be substituted" represented by $B^c$ or $C^c$) and an "optionally substituted acyl group" (e.g., the same groups as listed above for the "optionally substituted acyl group" as a substituent which may be on the benzene ring in the "benzene ring which may further be substituted" represented by $B^c$ or $C^c$). Alternatively, the "optionally substituted amino group" represented by $A^c$ may form a cyclic amino group through binding between substituents on the amino group (e.g., a cyclic amino group which is formed by removing one hydrogen atom from a ring-constituting nitrogen atom in a 5- to 6-membered ring such as tetrahydropyrrole, piperazine, piperidine, morpholine, thiomorpholine, pyrrole or imidazole and which has a binding hand on the nitrogen atom). The cyclic amino group may have a substituent(s). Examples of such a substituent include a halogen atom (e.g., fluorine, chlorine, bromine, iodine), nitro, cyano, a hydroxyl group, a thiol group, an amino group, a carboxyl group, an optionally halogenated $C_{1-4}$ alkyl (e.g., trifluoromethyl, methyl, ethyl), an optionally halogenated $C_{1-4}$ alkoxy

(e.g., methoxy, ethoxy, propoxy, butoxy, trifluoromethoxy, trifluoroethoxy), formyl, a $C_{2-4}$ alkanoyl (e.g., acetyl, propionyl) and a $C_{1-4}$ alkylsulfonyl (e.g., methanesulfonyl, ethanesulfonyl). The number of substituents is preferably 1 to 3.

Preferred examples of a substituent on the amino group in the "optionally substituted amino group" represented by $A^c$ include:

(1) an optionally substituted alkyl (e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls);
(2) an optionally substituted cycloalkyl (e.g., $C_{3-8}$ cycloalkyls such as cyclopropyl, cyclobutyl, cyclopentyl, cyclohexyl, cycloheptyl and cyanooctyl), in which the cycloalkyl may be condensed with the benzene ring to form indan (e.g., indan-1-yl, indan-2-yl), tetrahydronaphthalene (e.g., tetrahydronaphthalen-5-yl, tetrahydronaphthalen-6-yl) or the like (preferably, e.g., indan) or the cycloalkyls may be bridged together via a linear atom chain containing 1 or 2 carbon atoms to form a bridged cyclic hydrocarbon residue such as bicyclo[2.2.1]heptyl, bicyclo[2.2.2]octyl, bicyclo[3.2.1]octyl or bicyclo[3.2.2]nonyl (preferably, e.g., cyclohexyls bridged together via a linear atom chain containing 1 or 2 carbon atoms, more preferably, e.g., bicyclo[2.2.1]heptyl);
(3) an optionally substituted alkenyl (e.g., $C_{2-10}$ alkenyls such as allyl, crotyl, 2-pentenyl and 3-hexenyl, preferably lower ($C_{2-6}$) alkenyls);
(4) an optionally substituted cycloalkenyl (e.g., $C_{3-7}$ cycloalkenyls such as 2-cyclopentenyl, 2-cyclohexenyl, 2-cyclopentenylmethyl and 2-cyclohexenylmethyl);
(5) an optionally substituted aralkyl (e.g., phenyl-$C_{1-4}$ alkyls such as benzyl and phenethyl);
(6) formyl or an optionally substituted acyl (e.g., $C_{2-4}$ alkanoyls such acetyl, propionyl, butyryl and isobutyryl, $C_{1-4}$ alkylsulfonyls such as methanesulfonyl and ethanesulfonyl);
(7) an optionally substituted aryl (e.g., phenyl, naphthyl); and
(8) an optionally substituted heterocyclic group (e.g., a group formed by removing one hydrogen atom from a 5- to 6-membered aromatic heterocyclic ring containing 1 to 4 heteroatoms selected from 1 or 2 kinds of a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine or triazole, a group formed by removing one hydrogen atom from a 5- to 6-membered non-aromatic heterocyclic ring containing 1 to 4 heteroatoms selected from 1 or 2 kinds of a nitrogen atom, a sulfur atom and an oxygen atom, such as tetrahydrofuran, tetrahydrothiophene, dithiolane, oxathiolane, pyrrolidine, pyroline, imidazolidine, imidazoline, pyrazolidine, pyrazoline, piperidine, piperazine, oxazine, oxadiazine, thiazine, thiadiazine, morpholine, thiomorpholine, pyran or tetrahydropyran).

Examples of a substituent which may be on the above-mentioned (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, (7) optionally substituted aryl or (8) optionally substituted heterocyclic group include a halogen atom (e.g., fluorine, chlorine, bromine, iodine), a $C_{1-4}$ alkyl which may be substituted with a halogen atom or a $C_{1-4}$ alkoxy, a $C_{1-4}$ alkoxy which may be substituted with a halogen atom or a $C_{1-4}$ alkoxy (e.g., methoxy, ethoxy, propoxy, butoxy, trifluoromethoxy, trifluoroethoxy), a $C_{1-4}$ alkylenedioxy (e.g., $-O-CH_2-O-$, $-O-CH_2-CH_2-O-$), formyl, a $C_{2-4}$ alkanoyl (e.g., acetyl, propionyl), a $C_{1-4}$ alkylsulfonyl (e.g., methanesulfonyl, ethanesulfonyl), a phenyl-lower ($C_{1-4}$) alkyl, a $C_{3-7}$ cycloalkyl, cyano, nitro, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group, a lower ($C_{1-4}$) alkoxy-carbonyl, a lower ($C_{7-10}$) aralkyloxy-carbonyl, carbamoyl, a mono-$C_{1-4}$ alkylcarbamoyl and a di-$C_{1-4}$ alkylcarbamoyl (preferably, e.g., a halogen atom, an optionally halogenated lower ($C_{1-4}$) alkyl, an optionally halogenated lower ($C_{1-4}$) alkoxy, a phenyl-lower ($C_{1-4}$) alkyl, a $C_{3-7}$ cycloalkyl, cyano, a hydroxyl group). The number of substituents is preferably 1 to 3.

Above all, the "optionally substituted amino group" represented by $A^c$ is preferably an amino group which may have 1 or 2 optionally substituted alkyls [e.g., $C_{1-10}$ alkyls such as methyl, ethyl, propyl, isopropyl, butyl, isobutyl, sec-butyl, tert-butyl, pentyl, isopentyl, neopentyl, hexyl, heptyl, octyl, nonyl and decyl, preferably lower ($C_{1-6}$) alkyls, each of which may have 1 to 3 substituents selected from a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group which may be esterified or amidated (e.g., carboxyl, a $C_{1-4}$ alkoxycarbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), a phenyl-lower ($C_{1-4}$) alkyl, a $C_{3-7}$ cycloalkyl, formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl) and the

like].

In the above formula, examples of a "nitrogen-containing heterocyclic group" in the "optionally substituted nitrogen-containing heterocyclic group" represented by $A^c$ include 5- to 8-membered aromatic monocyclic heterocyclic rings and 5- to 8-membered saturated or unsaturated non-aromatic monocyclic heterocyclic rings (aliphatic heterocyclic rings), each of which may contain, in addition to one nitrogen atom, 1 to 4 heteroatoms (preferably 1 or 2 heteroatoms) selected from 1 to 3 kinds (preferably 1 or 2 kinds) of an oxygen atom, a sulfur atom, a nitrogen atom and the like; as well as groups formed by removing one hydrogen atom from a condensed ring formed by condensing the same or different 2 to 3 rings selected from these monocyclic rings. The "optionally substituted nitrogen-containing heterocyclic group" represented by $A^c$ may be linked to $X^c$ through either a nitrogen atom or a carbon atom, preferably through a carbon atom.

Examples of the "aromatic monocyclic heterocyclic ring" as used here include 5- to 8-membered (preferably 5- to 6-membered) aromatic monocyclic heterocyclic rings (e.g., pyrrole, oxazole, isooxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine). Examples of the "non-aromatic monocyclic heterocyclic ring" as used here include 5- to 8-membered (preferably 5- to 6-membered) saturated or unsaturated monocyclic non-aromatic heterocyclic rings (aliphatic heterocyclic rings) such as pyrrolidine, pyroline, imidazolidine, imidazoline, pyrazolidine, pyrazoline, oxazine, oxadiazine, thiazine, thiadiazine, piperidine, morpholine, thiomorpholine, piperazine and azepine, as well as 5- to 8-membered non-aromatic heterocyclic rings obtained by saturating part or all of double bonds in the above-mentioned aromatic monocyclic heterocyclic rings.

Examples of a substituent which may be on the "nitrogen-containing heterocyclic group" in the "optionally substituted nitrogen-containing heterocyclic group" represented by $A^c$ include the same substituents as listed above for the "optionally substituted hydrocarbon group" as a substituent which may be on the benzene ring in the "benzene ring which may further be substituted" represented by $B^c$ or $C^c$.

The "nitrogen-containing heterocyclic group" in the "optionally substituted nitrogen-containing heterocyclic group" represented by $A^c$ is preferably a 5- to 6-membered nitrogen-containing heterocyclic group, more preferably a 5- to 6-membered nitrogen-containing saturated heterocyclic group, and particularly preferably pyrrolidine, piperidine, piperazine (preferably, a 5- to 6-membered nitrogen-containing saturated heterocyclic group containing one nitrogen atom) or the like.

In the above formula, preferred examples of a group represented by the following formula:

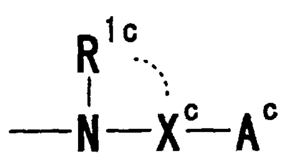

include a group of the following formula:

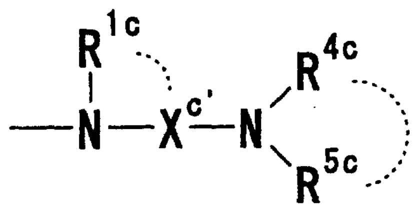

[wherein $R^{1c}$ is as defined above, $X^{c'}$ represents an optionally substituted $C_{1-6}$ alkylene group, and $R^{4c}$ and $R^{5c}$ each represent a hydrogen atom or an optionally substituted $C_{1-6}$ alkyl group, or $R^{4c}$ and $R^{5c}$ may together form a ring] and a group of the following formula:

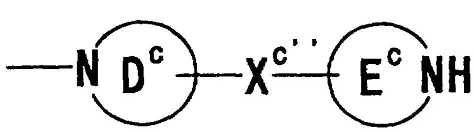

[wherein $X^{c'}$ represents a binding hand or an optionally substituted $C_{1-4}$ alkylene group, and the rings $D^c$ and $E^c$ each

represent a 3- to 8-membered nitrogen-containing saturated heterocyclic ring].

In the above formula, examples of a substituent which may be on a "$C_{1-6}$ alkylene group (preferably, $C_{1-4}$ alkylene group)" in the "optionally substituted $C_{1-6}$ alkylene group" represented by $X^{c,}$ include the same substituents as listed above for the divalent group as $X^c$.

In the above formula, examples of the "optionally substituted $C_{1-6}$ alkyl group" represented by $R^{4c}$ and $R^{5c}$ include lower ($C_{1-6}$) alkyls such as methyl, ethyl, propyl, isopropyl, butyl, isobutyl, sec-butyl, tert-butyl, pentyl, isopentyl, neopentyl and hexyl, each of which may have 1 to 3 substituents selected from a halogen atom (e.g., fluorine, chlorine, bromine, iodine), nitro, cyano, a hydroxyl group, an optionally substituted thiol group (e.g., thiol, a $C_{1-4}$ alkylthio), an optionally substituted amino group (e.g., amino, a mono-$C_{1-4}$ alkylamino, a di-$C_{1-4}$ alkylamino, a 5- to 6-membered cyclic amino such as tetrahydropyrrole, piperazine, piperidine, morpholine, thiomorpholine, pyrrole or imidazole), a carboxyl group which may be esterified or amidated (e.g., carboxyl, a $C_{1-4}$ alkoxycarbonyl, a lower ($C_{7-10}$) aralkyloxy-carbonyl, carbamoyl, a mono-$C_{1-4}$ alkylcarbamoyl, a di-$C_{1-4}$ alkylcarbamoyl), a $C_{1-4}$ alkyl which may be substituted with a halogen atom or a $C_{1-4}$ alkoxy (e.g., trifluoromethyl, methyl, ethyl), a $C_{1-4}$ alkoxy which may be substituted with a halogen atom or a $C_{1-4}$ alkoxy (e.g., methoxy, ethoxy, trifluoromethoxy, trifluoroethoxy), a $C_{1-4}$ alkylenedioxy (e.g., $-O-CH_2-O-$, $-O-CH_2-CH_2-O-$), a phenyl-lower ($C_{1-4}$) alkyl, a $C_{3-7}$ cycloalkyl, formyl, a $C_{2-4}$ alkanoyl (e.g., acetyl, propionyl), a $C_{1-4}$ alkylsulfonyl (e.g., methanesulfonyl, ethanesulfonyl), a $C_{1-4}$ alkylsulfinyl (e.g., methanesulfinyl, ethanesulfinyl) and the like.

In the above formula, $R^{4c}$ and $R^{5c}$ may be bridged together to form a cyclic amino group together with their adjacent nitrogen atom (e.g., a cyclic amino group which is formed by removing one hydrogen atom from a ring-constituting nitrogen atom in a 5- to 6-membered ring such as tetrahydropyrrole, piperazine, piperidine, morpholine, thiomorpholine, pyrrole or imidazole and which has a binding hand on the nitrogen atom; preferably, a 5- to 6-membered saturated cyclic amino group such as pyrrolidino, piperazino or piperidino; more preferably, pyrrolidino). The cyclic amino group may have a substituent(s). Examples of such a substituent include a halogen atom (e.g., fluorine, chlorine, bromine, iodine), nitro, cyano, a hydroxyl group, a thiol group, an amino group, a carboxyl group, an optionally halogenated $C_{1-4}$ alkyl (e.g., trifluoromethyl, methyl, ethyl), an optionally halogenated $C_{1-4}$ alkoxy (e.g., methoxy, ethoxy, propoxy, butoxy, trifluoromethoxy, trifluoroethoxy), formyl, a $C_{2-4}$ alkanoyl (e.g., acetyl, propionyl) and a $C_{1-4}$ alkylsulfonyl (e.g., methanesulfonyl, ethanesulfonyl). The number of substituents is preferably 1 to 3.

In the above formula, examples of a substituent which may be on a "$C_{1-4}$ alkylene group" in the "optionally substituted $C_{1-4}$ alkylene group" represented by $X^{c,,}$ include the same substituents as listed above for the divalent group as $X^c$.

In the above formula, examples of the "3- to 8-membered nitrogen-containing saturated heterocyclic ring" represented by the rings $D^c$ and $E^c$ include 3- to 8-membered nitrogen-containing heterocyclic rings which may contain, in addition to one nitrogen atom, 1 to 4 heteroatoms (preferably 1 or 2 heteroatoms) selected from 1 to 3 kinds (preferably 1 or 2 kinds) of an oxygen atom, a sulfur atom, a nitrogen atom and the like. More specific examples include 3- to 8-membered (preferably 5- to 6-membered) saturated or unsaturated (preferably saturated) monocyclic non-aromatic heterocyclic rings (aliphatic heterocyclic rings) such as pyrrolidine, pyroline, imidazolidine, imidazoline, pyrazolidine, pyrazoline, oxazine, oxadiazine, thiazine, thiadiazine, piperidine, morpholine, thiomorpholine, piperazine and azepine.

The "3- to 8-membered nitrogen-containing heterocyclic ring" may have a substituent(s). Examples of such a substituent include the same substituents as listed above for the "optionally substituted hydrocarbon group" as a substituent which may be on the benzene ring in the "benzene ring which may further be substituted" represented by $B^c$ or $C^c$.

The "3- to 8-membered nitrogen-containing heterocyclic group" represented by the rings $D^c$ and $E^c$ may be linked to $X^{c}$" through either a nitrogen atom or a carbon atom, preferably through a carbon atom.

In the above Formula (Ic), the groups defined as substituents on the rings $B^c$ and $C^c$ may each be located at any substitutable position. Preferably, a compound of Formula (Ic) or a salt thereof has any one of the following structures:

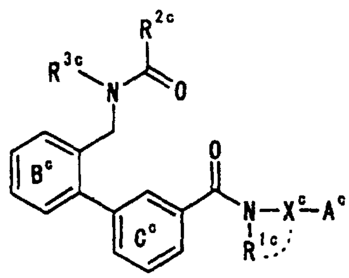

,

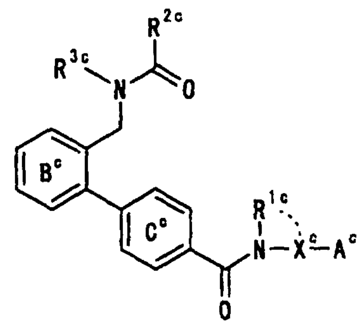

,

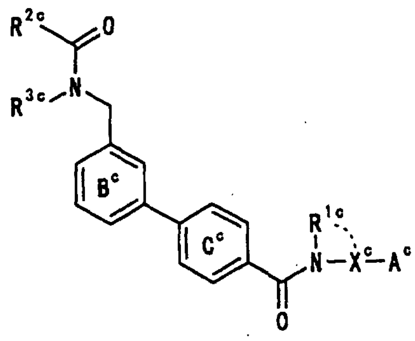

,

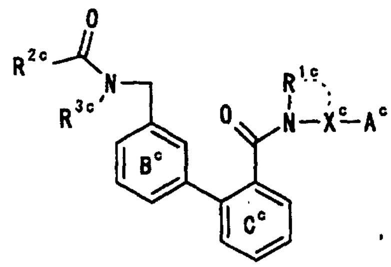

,

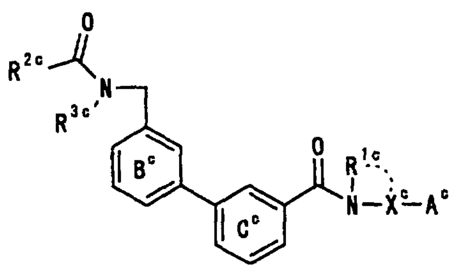

,

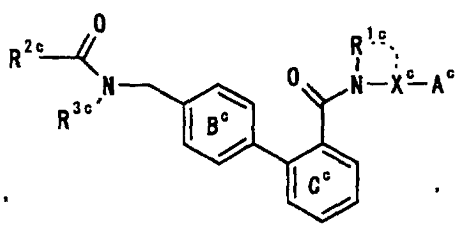

,

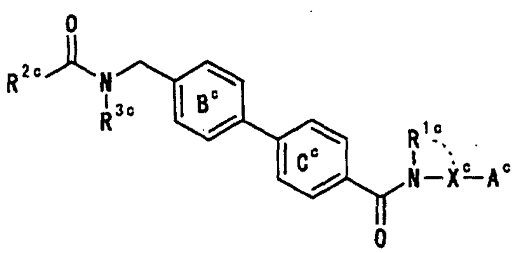

or

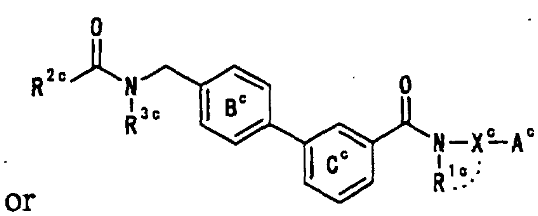

[wherein each symbol is as defined above].

Above all, it preferably has the following structure:

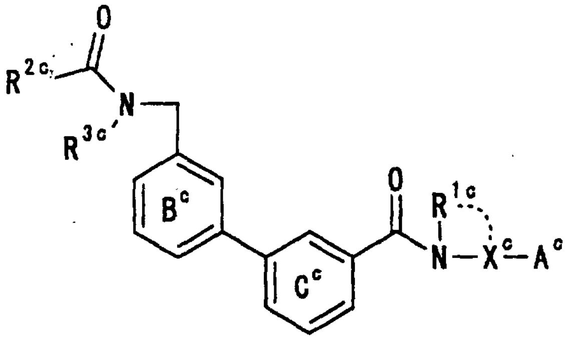

,

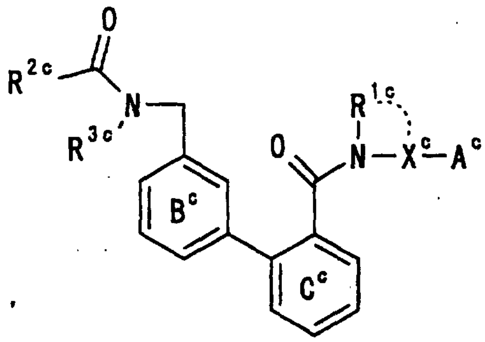

or

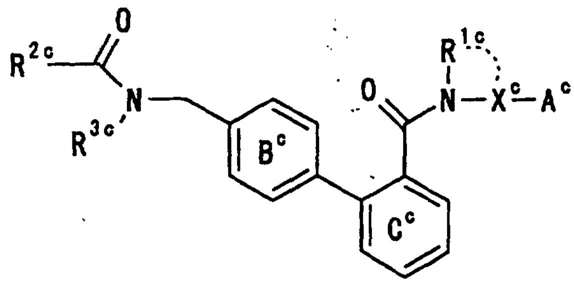

A compound of Formula (Ic) or a salt thereof may be prepared, for example, according to the following Scheme 1c:

**Scheme 1c**

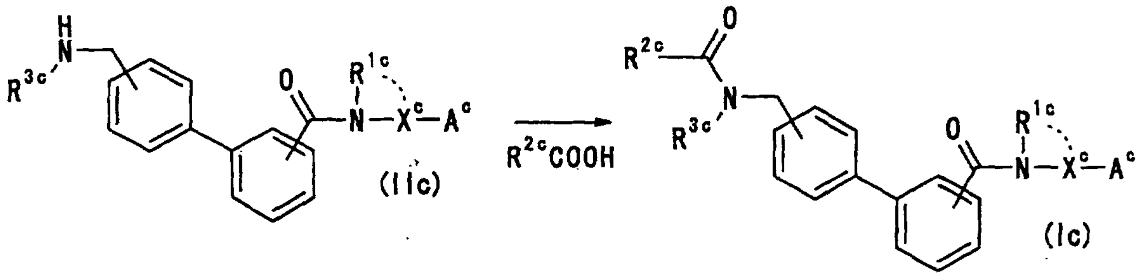


(IIc)

(Ic)

[wherein each symbol is as defined above].

The compound of Formula (Ic) or a salt thereof can be prepared from a compound of Formula (IIc) and a carboxylic acid of the formula $R^{2c}COOH$ or its reactive derivative or a salt thereof in a solvent and, if necessary, in the presence of a base by using a condensing agent. Examples of a reactive derivative of the carboxylic acid include acid anhydrides, active esters (e.g., p-nitrophenyl ester, N-hydroxysuccinimide ester, pentafluorophenyl ester, 1-hydroxybenzotriazole ester), acid halides (e.g., acid chloride, acid bromide), imidazolide or mixed acid anhydrides (e.g., anhydride with methyl carbonate, anhydride with ethyl carbonate). Specific examples include compounds in which a group of the formula -COOH is converted into a group of the formula $-COQ^c$ [wherein $Q^c$ represents a leaving group [e.g., a halogen atom (fluorine, chlorine, bromine, iodine), methanesulfonyloxy, benzenesulfonyloxy, p-toluenesulfonyloxy]]. Examples of a solvent available for use include ether solvents (e.g., diethyl ether, tetrahydrofuran, dioxane), hydrocarbon solvents (e.g., benzene, toluene, hexane, heptane), halogenated solvents (e.g., dichloromethane, dichloroethane, chloroform, carbon tetrachloride), acetonitrile and N,N-dimethylformamide. Examples of a base available for use include organic bases (e.g., triethylamine, 4-dimethylaminopyridine, N,N-diisopropylethylamine, triethylenediamine, 4-methylmorpholine), alkali metal or alkaline earth metal carbonates (e.g., sodium carbonate, potassium carbonate), alkali metal or alkaline earth metal bicarbonates (e.g., sodium bicarbonate, potassium bicarbonate) and alkali metal or alkaline earth metal hydroxides (e.g., sodium hydroxide, potassium hydroxide). Examples of a condensing agent available for use include those which are used in peptide synthesis. Specific examples include dicyclohexylcarbodiimide, diisopropylcarbodiimide, N-ethyl-N'-3-dimethylaminopropylcarbodiimide and a hydrochloride thereof, benzotriazol-1-yl-tris(dimethylamino)phosphonium hexafluorophosphide, benzotriazol-1-yl-trispyrrolidinophosphonium hexafluorophosphide, diethyl cyanophosphate, diphenylphosphorylazide, and N-hydroxy-5-norbornene-2,3-carboxyimide. These may be used alone or in combination with 1-hydroxybenzotriazole, 1-hydroxy-7-azabenzotriazole or the like. Relative to 1 mole of the compound of Formula (IIc) or a salt thereof, the carboxylic acid of the formula $R^{2c}COOH$ or a salt thereof is used in an amount of 0.5 to 10 molar equivalents, preferably 1 to 5 molar equivalents, while the condensing agent is used in an amount of 0.5 to 10 molar equivalents, preferably 1 to 6 molar equivalents. The reaction may be carried out at a temperature of -50°C to 200°C, preferably -20°C to 100°C, for 0.5 to 96 hours, preferably 0.5 to 72 hours, more preferably 1 to 24 hours.

The compound of Formula (Ic) or a salt thereof may also be prepared, for example, according to the following Scheme 2c:

**Scheme 2c**

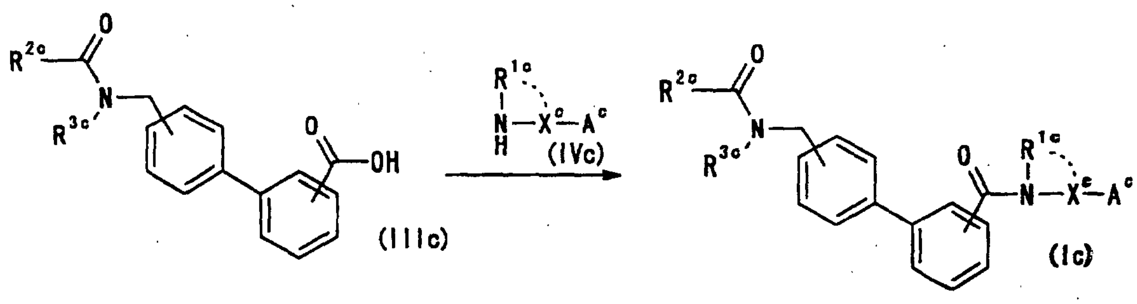

[wherein each symbol is as defined above].

The compound of Formula (Ic) or a salt thereof can be prepared from a compound of Formula (IIIc) or its reactive derivative or a salt thereof and a compound of Formula (IVc) or a salt thereof in a solvent and, if necessary, in the presence of a base by using a condensing agent. Examples of a reactive derivative of the compound of Formula (IIIc) include acid anhydrides, active esters (e.g., p-nitrophenyl ester, N-hydroxysuccinimide ester, pentafluorophenyl ester, 1-hydroxybenzotriazole ester), acid halides (e.g., acid chloride, acid bromide), imidazolide or mixed acid anhydrides (e.g., anhydride with methyl carbonate, anhydride with ethyl carbonate). Specific examples include compounds of Formula (III) in which a group of the formula -COOH is converted into a group of the formula $-COQ^c$ [wherein $Q^c$ represents a leaving group [e.g., a halogen atom (fluorine, chlorine, bromine, iodine), methanesulfonyloxy, benzenesulfonyloxy, p-toluenesulfonyloxy]]. Examples of a solvent available for use include ether solvents (e.g., diethyl ether, tetrahydrofuran, dioxane), hydrocarbon solvents (e.g., benzene, toluene, hexane, heptane), halogenated solvents (e. g., dichloromethane, dichloroethane, chloroform, carbon tetrachloride), acetonitrile and N,N-dimethylformamide. Examples of a base available for use include organic bases (e.g., triethylamine, 4-dimethylaminopyridine, N,N-diisopropylethylamine, triethylenediamine, 4-methylmorpholine), alkali metal or alkaline earth metal carbonates (e.g., sodium carbonate, potassium carbonate), alkali metal or alkaline earth metal bicarbonates (e.g., sodium bicarbonate, potassium bicarbonate) and alkali metal or alkaline earth metal hydroxides (e.g., sodium hydroxide, potassium hydroxide). Examples of a condensing agent available for use include those which are used in peptide synthesis. Specific examples include dicyclohexylcarbodiimide, diisopropylcarbodiimide, N-ethyl-N'-3-dimethylaminopropylcarbodiimide and a hydrochloride thereof, benzotriazol-1-yl-tris(dimethylamino)phosphonium hexafluorophosphide, benzotriazol-1-yl-trispyrrolidinophosphonium hexafluorophosphide, diethyl cyanophosphate and diphenylphosphorylazide. These may be used alone or in combination with 1-hydroxybenzotriazole, 1-hydroxy-7-azabenzotriazole or the like. Relative to 1 mole of the compound of Formula (IIIc) or a salt thereof, the compound of Formula (IVc) or a salt thereof is used in an amount of 0.5 to 10 molar equivalents, preferably 1 to 5 molar equivalents, while the condensing agent is used in an amount of 0.5 to 10 molar equivalents, preferably 1 to 6 molar equivalents. The reaction may be carried out at a temperature of -50°C to 200°C, preferably -20°C to 100°C, for 0.5 to 96 hours, preferably 0.5 to 72 hours, more preferably 1 to 24 hours.

The compound of Formula (IIc) or a salt thereof may be prepared, for example, according to the following Scheme 3c:

**Scheme 3c**

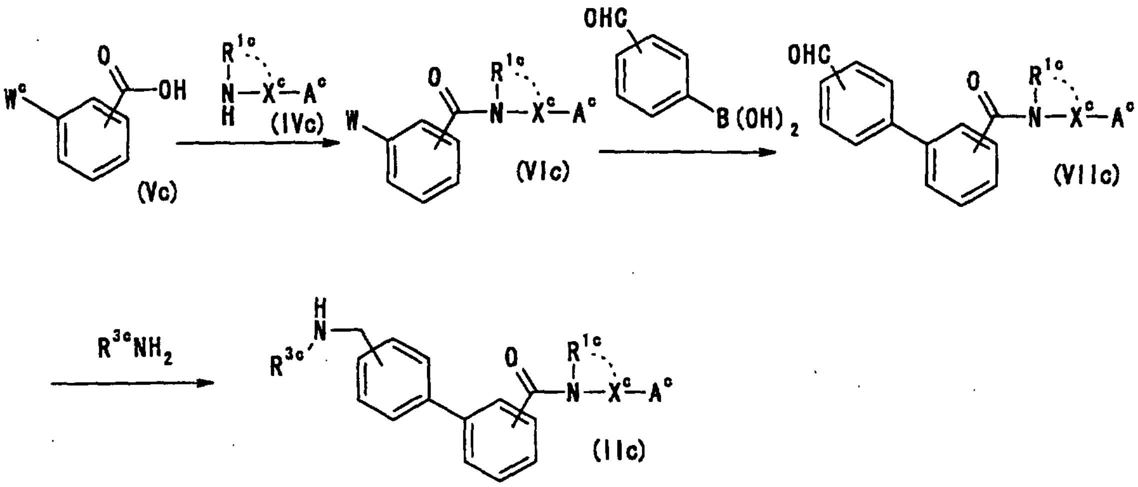


(Vc) (IVc) (VIc) (VIIc) (IIc)

[wherein $W^c$ represents a halogen atom (e.g., fluorine, chlorine, bromine, iodine) or a trifluoromethanesulfonyloxy group, and the other symbols are as defined above].

A compound of Formula (VIc) or a salt thereof can be prepared by reacting the compound of Formula (Vc) or its reactive derivative or a salt thereof with a compound of Formula (IVc) or a salt thereof. This reaction may be carried out, e.g., under the same conditions as shown above for the condensation in Scheme 2c.

A compound of Formula (VIIc) or a salt thereof can be prepared by reacting the compound of Formula (VIc) or a salt thereof with formylbenzeneboronic acid or an ester or anhydride thereof in a solvent under basic conditions and in the presence of a transition metal catalyst. Examples of a solvent available for use include water, alcohol solvents (e.g., methanol, ethanol, n-propanol, isopropanol), ether solvents (e.g., diethyl ether, tetrahydrofuran, 1,2-dimethoxyethane), hydrocarbon solvents (e.g., benzene, toluene, hexane, heptane) and N,N-dimethylformamide. These solvents may be used alone or, if necessary, in combination at an appropriate mixing ratio. Examples of a base available for use include alkali metal or alkaline earth metal carbonates (e.g., sodium carbonate, potassium carbonate), alkali metal or alkaline earth metal bicarbonates (e.g., sodium bicarbonate, potassium bicarbonate), alkali metal or alkaline earth metal hydroxides (e.g., sodium hydroxide, potassium hydroxide), triethylamine, 4-dimethylaminopyridine, N,N-diisopropylethylamine, triethylenediamine and 4-methylmorpholine. Examples of a transition metal catalyst available for use include palladium catalysts [e.g., tetrakis(triphenylphosphine)palladium, 1,1-bis(diphenylphosphino)ferrocene-dichloropalladium, dichlorobis(triphenylphosphine)palladium]. Relative to 1 mole of the compound of Formula (VIc) or a salt thereof, formylbenzeneboronic acid or an ester or anhydride thereof is used in an amount of 0.5 to 10 molar equivalents, preferably 1 to 5 molar equivalents, while the transition metal catalyst is used in an amount of 0.01 to 1 molar equivalent, preferably 0.05 to 0.2 molar equivalents. The reaction may be carried out at a temperature of 0°C to 200°C, preferably 50°C to 100°C, for 0.5 to 48 hours, preferably 1 to 24 hours.

The compound of Formula (IIc) or a salt thereof can be prepared from the compound of Formula (VIIc) or a salt thereof and an amine of the formula $R^{3c}NH_2$ or a salt thereof under reductive amination conditions. Reductive amination may be accomplished, for example, by reacting the compound of Formula (VIIc) or a salt thereof with the amine of the formula $R^{3c}NH_2$ or a salt thereof in the presence of a metal-hydrogen complex compound (e.g., sodium borohydride, sodium cyanoborohydride, sodium triacetoxyborohydride) in a solvent such as an ether solvent (e.g., diethyl ether, tetrahydrofuran, dioxane), a hydrocarbon solvent (e.g., benzene, toluene, hexane, heptane), a halogenated solvent (e.g., dichloromethane, dichloroethane, chloroform, carbon tetrachloride), an alcohol solvent (e.g., methanol, ethanol, n-propanol, isopropanol), acetonitrile, N,N-dimethylformamide or acetic acid or a mixture thereof. Relative to 1 mole of the compound of Formula (VIIc) or a salt thereof, the amine of the formula $R^{3c}NH_2$ or a salt thereof is used in an amount of 0.5 to 10 molar equivalents, preferably 1 to 5 molar equivalents, while the metal-hydrogen complex compound is used in an amount of 0.5 to 10 molar equivalents, preferably 1 to 5 molar equivalents. The reaction may be carried out at a temperature of 0°C to 200°C, preferably 20°C to 100°C, for 0.5 to 96 hours, preferably 1 to 24 hours.

The compound of Formula (IIc) or a salt thereof may also be prepared, for example, according to the following Scheme 4c:

**Scheme 4c**

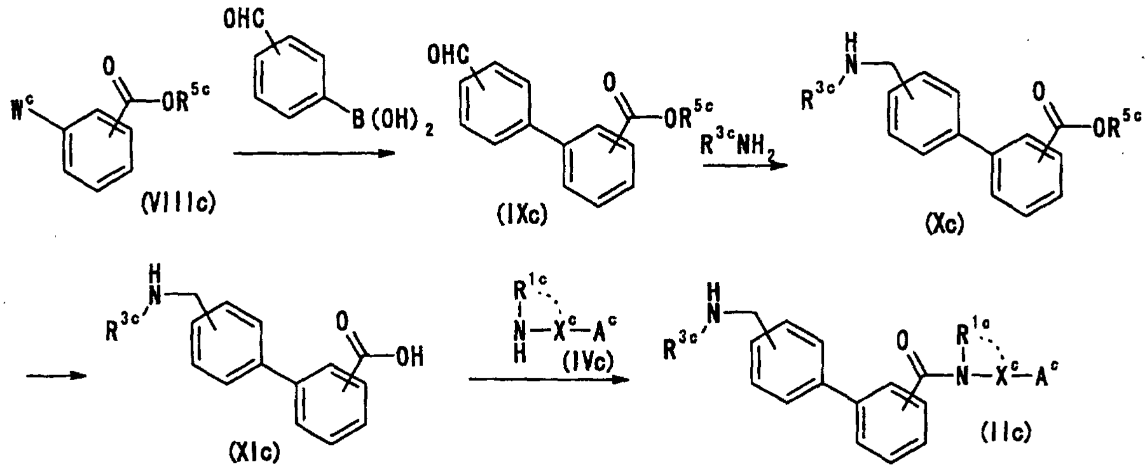


(VIIIc) (IXc) (Xc) (XIc) (IVc) (IIc)

[wherein $R^{5c}$ represents an optionally substituted $C_{1-6}$ alkyl (e.g., methyl, ethyl, propyl, isopropyl, butyl, tert-butyl), phenyl, trityl or silyl, and the other symbols are as defined above].

A compound of Formula (IXc) or a salt thereof can be prepared by reacting a compound of Formula (VIIIc) or a salt thereof with formylbenzeneboronic acid or an ester or anhydride thereof in a solvent under basic conditions and in the presence of a transition metal catalyst. This reaction may be carried out, e.g., under the same conditions as shown above for the reaction in Scheme 3c where the compound of Formula (VIc) or a salt thereof is converted into the compound of Formula (VIII) or a salt thereof.

A compound of Formula (Xc) or a salt thereof can be prepared from the compound of Formula (IXc) or a salt thereof and an amine of the formula $R^{3c}NH_2$ or a salt thereof under reductive amination conditions. This reaction may be carried out, e.g., under the same conditions as shown above for the reaction in Scheme 3c where the compound of Formula (VIIc) or a salt thereof is converted into the compound of Formula (IIc) or a salt thereof.

A compound of Formula (XIc) or a salt thereof can be prepared by treating the compound of Formula (Xc) or a salt thereof with an acid or a base. Namely, the compound of Formula (Xc) or a salt thereof is reacted using a mineral acid (e.g., nitric acid, hydrochloric acid, hydrobromic acid, iodic acid, sulfuric acid) or an alkali metal hydroxide (e.g., sodium hydroxide, potassium hydroxide, lithium hydroxide) at 0°C to 150°C, preferably at 20°C to 50°C, in a solvent such as water, an ether solvent (e.g., diethyl ether, tetrahydrofuran, dioxane) or an alcohol solvent (e.g., methanol, ethanol, n-propanol, isopropanol) or a mixture thereof. The acid and base are both desirably used at a strength of around 0.1 to 10 N and reacted for 1 to 72 hours.

The compound of Formula (IIc) or a salt thereof can be prepared by reacting the compound of Formula (XIc) or its reactive derivative or a salt thereof with a compound of Formula (IVc) or a salt thereof. This reaction may be carried out, e.g., under the same conditions as shown above for the condensation in Scheme 2c.

The compound of Formula (IIIc) or a salt thereof may be prepared, for example, according to the following Scheme 5c:

**Scheme 5c**

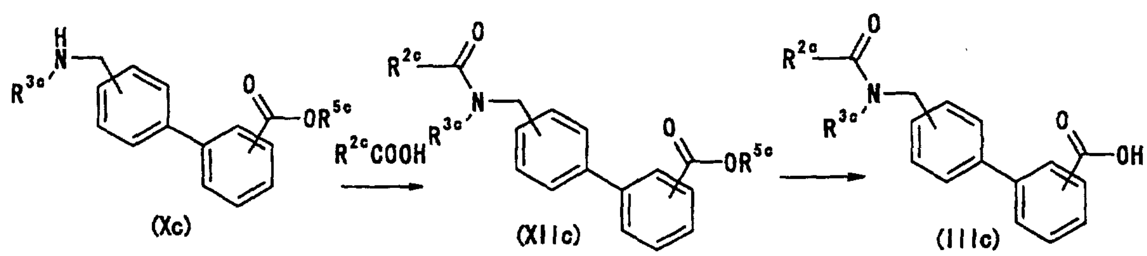


(Xc) (XIIc) (IIIc)

[wherein each symbol is as defined above].

A compound of Formula (XIIc) or a salt thereof can be prepared from the compound of Formula (Xc), whose preparation was shown above in Scheme 4c, and a carboxylic acid of the formula $R^{2c}COOH$ or its reactive derivative

or a salt thereof in a solvent and, if necessary, in the presence of a base by using a condensing agent. This reaction may be carried out, e.g., under the same conditions as shown above for the condensation in Scheme 1c.

The compound of Formula (IIIc) or a salt thereof can be prepared by treating the compound of Formula (XIIc) or a salt thereof with an acid or a base. This reaction may be carried out, e.g., under the same conditions as shown above for the reaction in Scheme 4c where the compound of Formula (Xc) or a salt thereof is converted into the compound of Formula (XIc) or a salt thereof.

Compound (Ic) thus prepared may be isolated and purified by known isolation and purification means including concentration, vacuum concentration, solvent extraction, crystallization, recrystallization, phasic transfer and chromatography.

Compounds used in the respective schemes shown above may be in the same salt form as Compound (Ic) as long as they do not adversely affect the reaction.

Likewise, in each reaction shown above, when a starting compound has an amino group, a carboxyl group or a hydroxyl group as a substituent, it may have an introduced protecting group(s) such as those commonly used for these substituents in peptide chemistry or elsewhere. Such a protecting group(s) may be removed after the reaction, when needed, to give a compound of interest.

Protecting groups used for an amino group include, for example, an optionally substituted $C_{1-6}$ alkylcarbonyl (e.g., acetyl, propionyl), formyl, phenylcarbonyl, a $C_{1-6}$ alkyloxycarbonyl (e.g., methoxycarbonyl, ethoxycarbonyl, t-butoxycarbonyl), phenyloxycarbonyl (e.g., benzoxycarbonyl), a $C_{7-10}$ aralkyloxycarbonyl (e.g., benzyloxycarbonyl), trityl and phthaloyl. These protecting groups may have around 1 to 3 substituents such as a halogen atom (e.g., fluorine, chlorine, bromine, iodine), a $C_{1-6}$ alkylcarbonyl (e.g., acetyl, propionyl, butyryl) or a nitro group.

Protecting groups used for a carboxyl group include, for example, an optionally substituted $C_{1-6}$ alkyl (e.g., methyl, ethyl, propyl, isopropyl, butyl, tert-butyl), phenyl, trityl and silyl. These protecting groups may have around 1 to 3 substituents such as a halogen atom (e.g., fluorine, chlorine, bromine, iodine), a $C_{1-6}$ alkylcarbonyl (e.g., acetyl, propionyl, butyryl), formyl or a nitro group.

Protecting groups used for a hydroxyl group include, for example, an optionally substituted $C_{1-6}$ alkyl (e.g., methyl, ethyl, propyl, isopropyl, butyl, tert-butyl), phenyl, a $C_{7-10}$ aralkyl (e.g., benzyl), a $C_{1-6}$ alkylcarbonyl (e.g., acetyl, propionyl), formyl, phenyloxycarbonyl, a $C_{7-10}$ aralkyloxycarbonyl (e.g., benzyloxycarbonyl), pyranyl, furanyl and silyl. These protecting groups may have around 1 to 4 substituents such as a halogen atom (e.g., fluorine, chlorine, bromine, iodine), a $C_{1-6}$ alkyl, phenyl, a $C_{7-10}$ aralkyl or a nitro group.

Although introduction and removal of protecting groups may be carried out according to well-known procedures or equivalents thereof [see, e.g., Protective Groups in Organic Chemistry (J.F.W. McOmie et al., Prenum Press)], protecting groups may be removed by treatment with, e.g., an acid, a base, reduction, ultraviolet radiation, hydrazine, phenylhydrazine, sodium N-methyldithiocarbamate, tetrabutylammonium fluoride and/or palladium acetate.

Salts of the compounds obtained by the screening method or screening kit mentioned above may be, for example, pharmaceutically acceptable salts. Examples include salts with inorganic bases, salts with organic bases, salts with inorganic acids, salts with organic acids, and salts with basic or acidic amino acids.

Preferred examples of salts with inorganic bases include alkali metal salts such as sodium salt and potassium salt, alkaline earth metal salts such as calcium salt and magnesium salt, as well as aluminum salt and ammonium salt.

Preferred examples of salts with organic bases include those which are formed with trimethylamine, triethylamine, pyridine, picoline, 2,6-lutidine, ethanolamine, diethanolamine, triethanolamine, cyclohexylamine, dicyclohexylamine and N,N'-dibenzylethylenediamine.

Preferred examples of salts with inorganic acids include those which are formed with hydrochloric acid, hydrobromic acid, sulfuric acid and phosphoric acid.

Preferred examples of salts with organic acids include those which are formed with formic acid, acetic acid, propionic acid, fumaric acid, oxalic acid, tartaric acid, maleic acid, citric acid, succinic acid, malic acid, methanesulfonic acid, benzenesulfonic acid and benzoic acid.

Preferred examples of salts with basic amino acids include those which are formed with arginine, lysine and ornithine. Preferred examples of salts with acidic amino acids include those which are formed with aspartic acid and glutamic acid.

In a case where the above GPR14 (SENR) antagonist is used as an anti-anxiety, anti-depression, anti-insomnia, anti-schizophrenia or anti-fear agent, it may be formulated in a routine manner. For example, it may be used for oral administration in dosage forms of optionally sugar-coated or enteric-coated tablets, capsules, elixirs, microcapsules and the like or may be used for parenteral administration in dosage forms of injections such as sterile solutions or suspensions in water or other pharmaceutically acceptable fluids. For example, these formulations may be prepared by incorporating the compound or a salt thereof in unit dosage forms required in generally-accepted pharmaceutical practice in combination with physiologically acceptable carriers, flavors, excipients, vehicles, antiseptics, stabilizers, binders and the like. The amount of an active ingredient in these formulations is intended to give an appropriate dose within the indicated range.

Examples of additives, which can be incorporated into tablets, capsules and the like, include binders such as gelatin, corn starch, gum tragacanth or gum arabic; excipients such as crystalline cellulose; swelling agents such as corn starch, gelatin or alginic acid; lubricants such as magnesium stearate; edulcorants such as sucrose, lactose or saccharin; and flavors such as peppermint, *Gaultheria adenothrix* oil or cherry. In a case where the unit dosage form is a capsule, it may further contain liquid carriers such as fats and oils, in addition to the above types of materials. Sterile compositions for injection may be formulated according to general pharmaceutical practice, for example, by dissolving or suspending an active substance, a naturally-occurring vegetable oil(s) (e.g., sesame oil, coconut oil) and other ingredients, if any, into a vehicle such as water for injection.

Examples of aqueous fluids for injection include physiological saline and isotonic solutions containing glucose and/or other auxiliaries (e.g., D-sorbitol, D-mannitol, sodium chloride), which may be used in combination with appropriate solubilizers such as alcohols (e.g., ethanol), polyalcohols (e.g., propylene glycol, polyethylene glycol) and nonionic surfactants (e.g., Polysorbate 80™, HCO-50). Examples of oil fluids include sesame oil and soy bean oil, which may be used in combination with solubilizers such as benzyl benzoate and benzyl alcohol.

In addition, these fluids may be blended with buffers (e.g., phosphate buffer, sodium acetate buffer), soothing agents (e.g., benzalkonium chloride, procaine hydrochloride), stabilizers (e.g., human serum albumin, polyethylene glycol), preservatives (e.g., benzyl alcohol, phenol), antioxidants, etc. The prepared solutions for injection are usually filled into appropriate ampules.

Since the formulations thus prepared are safer and less toxic, they can be administered to, for example, mammals (e.g., human, mouse, rat, guinea pig, rabbit, sheep, pig, cattle, cat, dog, monkey).

The dose of the GPR14 (SENR) antagonist of the present invention will *vary* depending on symptoms etc. For oral administration, it is usually administered to an adult (body weight: 60 kg) in an amount of about 0.1 to 100 mg/day, preferably about 1.0 to 50 mg/day, more preferably about 1.0 to 20 mg/day. For parenteral administration, the single dose will vary depending on subjects to be administered, target organs, symptoms, the intended route of administration, etc. In a dosage form of injection, for example, it is advantageously administered to an adult (body weight: 60 kg) in an amount of about 0.01 to 30 mg/day, preferably about 0.1 to 20 mg/day, more preferably about 0.1 to 10 mg/day, by intravenous injection. Other animals may be administered with the GPR14 (SENR) antagonist in an amount calculated per 60 kg.

<u>(2) Diagnostic method using a polynucleotide containing part or all of a nucleotide sequence complementary to a DNA containing the nucleotide sequence encoding the polypeptide of the present invention</u>

A polynucleotide containing part or all of a nucleotide sequence complementary to a DNA containing the nucleotide sequence encoding the polypeptide of the present invention can inhibit *in vivo* functions of a DNA containing the nucleotide sequence encoding the polypeptide of the present invention or a protein encoded by the same; it can therefore be used, for example, as a therapeutic and/or prophylactic agent for attention deficit disorder or narcolepsy or for anxiety, depression, insomnia, schizophrenia or fear.

For example, the polynucleotide may be used alone or inserted into an appropriate vector such as a retrovirus vector, an adenovirus vector or an adenovirus-associated virus vector, and then administered in a routine manner. The antisense DNA may be used alone or formulated together with physiologically acceptable carriers such as auxiliaries for enhancement of uptake, and then administered with a gene gun or through a catheter such as a hydrogel catheter. Alternatively, it may be aerosolized to give an inhalant for intratracheal administration.

Further, the antisense DNA can also be used as a diagnostic oligonucleotide probe for examining the presence and/or expression patterns of the DNA according to the present invention in tissues and cells, i.e., as a diagnostic agent for attention deficit disorder or narcolepsy or for anxiety, depression, insomnia, schizophrenia or fear.

<u>(3) Diagnostic method using an antibody against the polypeptide of the present invention or a precursor protein thereof or an amide or ester of the polypeptide or precursor protein or a salt thereof</u>

An antibody against the polypeptide of the present invention or a precursor protein thereof or an amide or ester of the polypeptide or precursor protein or a salt thereof can inhibit *in vivo* functions of the polypeptide of the present invention or a precursor protein thereof or an amide or ester of the polypeptide or precursor protein or a salt thereof; it can therefore be used, for example, as a therapeutic and/or prophylactic agent for anxiety, depression, insomnia, schizophrenia or fear.

Also, an antibody against the polypeptide of the present invention or a precursor protein thereof or an amide or ester of the polypeptide or precursor protein or a salt thereof can be used for diagnosis of diseases such as attention deficit disorder, narcolepsy, anxiety, depression, insomnia or schizophrenia. In addition, such an antibody may be used not only for quantification of the polypeptide of the present invention or a precursor protein thereof or an amide or ester of the polypeptide or precursor protein or a salt thereof, but also for their detection by tissue staining etc. For these

purposes, either the whole antibody molecule or a $F(ab')_2$, Fab' or Fab fraction thereof may be used. Such an antibody may be a monoclonal antibody, a polyclonal antibody, a human-mouse chimeric antibody, human antibody or a genetically-engineered human antibody. A human antibody may be prepared by cell fusion using human myeloma cells or by immunization of mice carrying the introduced human immunoglobulin gene and the subsequent cell fusion between immunocompetent cells from the mice and myeloma cells (K. Tomizuka et. al., Proc. Natl. Acad. Sci. 97, 722-727, 2000). A genetically-engineered human antibody also encompasses a single-chain antibody comprising $V_H$ and $V_L$ regions crosslinked together.

Any assay may be used for quantification of the polypeptide of the present invention or a precursor protein thereof or an amide or ester of the polypeptide or precursor protein or a salt thereof using an antibody against the polypeptide of the present invention or a precursor protein thereof or an amide or ester of the polypeptide or precursor protein or a salt thereof, as long as the amount of an antigen (e.g., the polypeptide of the present invention or a precursor protein thereof or an amide or ester of the polypeptide or precursor protein or a salt thereof) in a fluid to be assayed is chemically or physically detected as the corresponding amount of the antibody, antigen or antibody-antigen complex, and then calculated from a calibration curve prepared using standard solutions containing known amounts of the antigen. For example, nephelometry, a competitive assay, an immunometric assay and a sandwich assay are preferred for use. In adapting these individual immunoassays to the quantification method of the present invention, there is no need to establish particular conditions, manipulations, etc. Standard conditions and manipulations used in the individual assays may be modified by technical considerations common to those skilled in the art so as to construct an assay system for the polypeptide of the present invention. Details about these common techniques can be found in reviews, established textbooks, etc.

For example, reference may be made to Hiroshi Irie ed., "Radioimmunoassay" (Kodansha Ltd., published in 1974), Hiroshi Irie ed., "Continued Radioimmunoassay" (Kodansha Ltd., published in 1979), Eiji Ishikawa ed., "Enzyme Immunoassay" (Igaku-Shoin Ltd., published in 1978), Eiji Ishikawa ed., "Enzyme Immunoassay" (2nd edition) (Igaku-Shoin Ltd., published in 1982), Eiji Ishikawa ed., "Enzyme Immunoassay" (3rd edition) (Igaku-Shoin Ltd., published in 1987), "Methods in ENZYMOLOGY" Vol. 70, Academic Press (Immunochemical Techniques (Part A)), ibidem Vol. 73 (Immunochemical Techniques (Part B)), ibidem Vol. 74 (Immunochemical Techniques (Part C)), ibidem Vol. 84 (Immunochemical Techniques (Part D: Selected Immunoassays)), ibidem Vol. 92 (Immunochemical Techniques (Part E: Monoclonal Antibodies and General Immunoassay Methods)), ibidem Vol. 121 (Immunochemical Techniques (Part I: Hybridoma Technology and Monoclonal Antibodies)), etc.

(4) Diagnostic method using an antibody against GPR14 (SENR) or its amide or ester or a salt thereof

An antibody against GPR14 (SENR) or its amide or ester or a salt thereof can inhibit *in vivo* functions of GPR14 (SENR) or its amide or ester or a salt thereof; it can therefore be used, for example, as a therapeutic and/or prophylactic agent for attention deficit disorder or narcolepsy or for anxiety, depression, insomnia, schizophrenia or fear.

Also, an antibody against GPR14 (SENR) or its amide or ester or a salt thereof can be used for diagnosis of diseases such as attention deficit disorder, narcolepsy, anxiety, depression, insomnia or schizophrenia. In addition, such an antibody may be used not only for quantification of GPR14 (SENR) or its amide or ester or a salt thereof, but also for their detection by tissue staining etc. For these purposes, either the whole antibody molecule or a $F(ab')_2$, Fab' or Fab fraction thereof may be used. Such an antibody may be a monoclonal antibody, a polyclonal antibody, a human-mouse chimeric antibody, human antibody or a genetically-engineered human antibody. A human antibody may be prepared by cell fusion using human myeloma cells or by immunization of mice carrying the introduced human immunoglobulin gene and the subsequent cell fusion between immunocompetent cells from the mice and myeloma cells (K. Tomizuka et. al., Proc. Natl. Acad. Sci. 97, 722-727, 2000). A genetically-engineered human antibody also encompasses a single-chain antibody comprising $V_H$ and $V_L$ regions crosslinked together.

Any assay may be used for quantification of GPR14 (SENR) or its amide or ester or a salt thereof using an antibody against GPR14 (SENR) or its amide or ester or a salt thereof, as long as the amount of an antigen (e.g., GPR14 (SENR) or its amide or ester or a salt thereof) in a fluid to be assayed is chemically or physically detected as the corresponding amount of the antibody, antigen or antibody-antigen complex, and then calculated from a calibration curve prepared using standard solutions containing known amounts of the antigen. For example, nephelometry, a competitive assay, an immunometric assay and a sandwich assay are preferred for use. In adapting these individual immunoassays to the quantification method of the present invention, there is no need to establish particular conditions, manipulations, etc. Standard conditions and manipulations used in the individual assays may be modified by technical considerations common to those skilled in the art so as to construct an assay system for the polypeptide of the present invention. Details about these common techniques can be found in reviews, established textbooks, etc.

For example, reference may be made to Hiroshi Irie ed., "Radioimmunoassay" (Kodansha Ltd., published in 1974), Hiroshi Irie ed., "Continued Radioimmunoassay" (Kodansha Ltd., published in 1979), Eiji Ishikawa ed., "Enzyme Immunoassay" (Igaku-Shoin Ltd., published in 1978), Eiji Ishikawa ed., "Enzyme Immunoassay" (2nd edition) (Igaku-Shoin Ltd., published in 1982), Eiji Ishikawa ed., "Enzyme Immunoassay" (3rd edition) (Igaku-Shoin Ltd., published in 1987), "Methods in ENZYMOLOGY" Vol. 70, Academic Press (Immunochemical Techniques (Part A)), ibidem Vol. 73 (Immunochemical Techniques (Part B)), ibidem Vol. 74 (Immunochemical Techniques (Part C)), ibidem Vol. 84 (Immunochemical Techniques (Part D: Selected Immunoassays)), ibidem Vol. 92 (Immunochemical Techniques (Part E: Monoclonal Antibodies and General Immunoassay Methods)), ibidem Vol. 121 (Immunochemical Techniques (Part I: Hybridoma Technology and Monoclonal Antibodies)), etc.

(5) Gene diagnostic method associated with the polypeptide of the present invention

If certain abnormalities (gene abnormalities) are found in the DNA (including the promoter region, exons and introns) or mRNA encoding the polypeptide of the present invention, information on the properties of the DNA or mRNA will enable detection of abnormalities such as damage, mutation, underexpression, increased copy number and overexpressoin of the DNA or mRNA related to attention deficit disorder, narcolepsy, anxiety, depression, insomnia, schizophrenia or fear. Thus, the DNA or mRNA encoding the polypeptide of the present invention is useful in conducting gene diagnosis. In the case of mRNA, over- or under-expression of splice variants or mRNA editing-induced mutagenesis (C. M. Niswender et. al. Ann. N. Y Acad. Sci. 861, 38-48, 1998) is also taken into account. Likewise, information on chromosomal loci is used for studies of hereditary diseases associated with the DNA of the present invention. The gene diagnosis using the DNA encoding the polypeptide of the present invention may be accomplished, for example, by well-known Northern hybridization or PCR-SSCP (Genomics, vol. 5, pp. 874-879 (1989), Proceedings of the National Academy of Sciences of the United States of America, vol. 86, pp. 2766-2770 (1989)), a DNA microarray technique (Science, vol. 270, pp. 467-470 (1995)), or other techniques (Experimental Medicine, vol. 18, no. 14, pp. 1894-1906, 2000). If over- or under-expression or DNA mutation of the gene is detected by any of the above techniques, for example, it is possible to make a diagnosis that a subject is susceptible to various diseases, in particular, attention deficit disorder, narcolepsy, anxiety, depression, insomnia, schizophrenia or fear or a diagnosis that a subject is suspected to suffer from attention deficit disorder, narcolepsy, anxiety, depression, insomnia, schizophrenia or fear.

Recently, in particular, polymorphic markers called SNPs (single nucleotide polymorphism). have been developed as very important tools for screening disease-related genes and have received increasing attentions because they would define the susceptibility (or resistance) to diseases and would contribute to differences in drug sensitivity and/or side effects. Examples of a typing method for SNPs include direct sequencing, the Invader method, the Sniper method, the MALDI-TOF/MS method and olig-SNP chips (Experimental Medicine, vol. 18, no. 12, 2000), depending on the intended purposes.

The SNPs thus found on the DNA (including the promoter region, exons and introns) encoding the protein of the present invention are useful in determining the susceptibility to attention deficit disorder, narcolepsy, anxiety, depression, insomnia, schizophrenia or fear and in estimating the onset of these diseases, or useful for diagnosis of attention deficit disorder, narcolepsy, anxiety, depression, insomnia, schizophrenia or fear, when such SNPs are analyzed alone or in combination with SNPs on other genes or the DNA of the present invention.

(6) Gene diagnostic method associated with GPR14 (SENR)

If certain abnormalities (gene abnormalities) are found in the DNA (including the promoter region, exons and introns) or mRNA encoding a protein containing GPR14 (SENR) (e.g., the amino acid sequence shown in SEQ ID NO: 3 or SEQ ID NO: 11 of the present invention), information on the properties of the DNA or mRNA will enable detection of abnormalities such as damage, mutation, underexpression, increased copy number and overexpressoin of the DNA or mRNA related to attention deficit disorder, narcolepsy, anxiety, depression, insomnia, schizophrenia or fear. Thus, the DNA or mRNA encoding a protein containing GPR14 (SENR) is useful in conducting gene diagnosis. In the case of mRNA, over- or under-expression of splice variants or mRNA editing-induced mutagenesis (C. M. Niswender et. al. Ann. N. Y. Acad. Sci. 861, 38-48, 1998) is also taken into account. Likewise, information on chromosomal loci is used for studies of hereditary diseases associated with the DNA of the present invention. The gene diagnosis using the DNA encoding a protein containing GPR14 (SENR) (e.g., the amino acid sequence shown in SEQ ID NO: 3 or SEQ ID NO: 11 of the present invention) may be accomplished, for example, by well-known Northern hybridization or PCR-SSCP (Genomics, vol. 5, pp. 874-879 (1989), Proceedings of the National Academy of Sciences of the United States of America, vol. 86, pp. 2766-2770 (1989)), a DNA microarray technique (Science, vol. 270, pp. 467-470 (1995)), or other techniques (Experimental Medicine, vol. 18, no. 14, pp. 1894-1906, 2000). If over- or under-expression or DNA mutation of the gene is detected by any of the above techniques, for example, it is possible to make a diagnosis that a subject is susceptible to various diseases, in particular, attention deficit disorder, narcolepsy, anxiety, depression, insomnia, schizophrenia or fear or a diagnosis that a subject is suspected to suffer from attention deficit disorder, narcolepsy, anxiety, depression, insomnia, schizophrenia or fear.

In addition, SNPs present on the DNA (including the promoter region, exons and introns) encoding a protein

containing GPR14 (SENR) (e.g., the amino acid sequence shown in SEQ ID NO: 3 or SEQ ID NO: 11 of the present invention) are useful in determining the susceptibility to attention deficit disorder, narcolepsy, anxiety, depression, insomnia, schizophrenia or fear and in estimating the onset of these diseases, or useful for diagnosis of attention deficit disorder, narcolepsy, anxiety, depression, insomnia, schizophrenia or fear, when such SNPs are analyzed alone or in combination with SNPs on other genes or the DNA of the present invention.

In the specification and drawings, abbreviations for nucleotides, amino acids and others are based on IUPAC-IUB Commission on Biochemical Nomenclature or the notation commonly used in the art, as shown below. In the case of amino acids having optical isomers, L-amino acids are intended unless otherwise specified.

| | |
|---|---|
| DNA | deoxyribonucleic acid |
| cDNA | complementary deoxyribonucleic acid |
| A | adenine |
| T | thymine |
| G | guanine |
| C | cytosine |
| Y | thymine or cytosine |
| N | thymine, cytosine, adenine or guanine |
| R | adenine or guanine |
| M | cytosine or adenine |
| W | thymine or adenine |
| S | cytosine or guanine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| EDTA | ethylenediamine tetraacetate |
| SDS | sodium dodecyl sulfate |
| TFA | trifluoroacetic acid |
| EIA | enzyme immunoassay |
| Gly or G | glycine |
| Ala or A | alanine |
| Val or V | valine |
| Leu or L | leucine |
| Ile or I | isoleucine |
| Ser or S | serine |
| Thr or T | threonine |
| Cys or C | cysteine |
| Met or M | methionine |
| Glu or E | glutamic acid |
| Asp or D | aspartic acid |
| Lys or K | lysine |
| Arg or R | arginine |
| His or H | histidine |
| Phe or F | phenylalanine |
| Tyr or Y | tyrosine |
| Trp or W | tryptophan |
| Pro or P | proline |
| Asn or N | asparagine |
| Gln or Q | glutamine |
| pGlu | pyroglutamic acid |

(continued)

| | |
|---|---|
| Me | a methyl group |
| Et | an ethyl group |
| Bu | a butyl group |
| Ph | a phenyl group |
| TC | a thiazolidine-4(R)-carboxamide group |
| Bom | benzyloxymethyl |
| NMP | N-methylpyrrolidone |
| PAM | phenylacetamidemethyl |

Likewise, substituents, protecting groups and reagents frequently used herein are abbreviated as follows.
Tos: p-toluenesulfonyl
HONB: N-hydroxy-5-norbornene-2,3-dicarboxyimide
Bzl: benzyl
Z: benzyloxycarbonyl
Br-Z: 2-bromobenzyloxycarbonyl
Cl-Z: 2-chlorobenzyloxycarbonyl
Boc: t-butyloxycarbonyl
HOBt: 1-hydroxybenztriazole
DCC: N,N'-dicyclohexylcarbodiimide
TFA: trifluoroacetic acid
Fmoc: N-9-fluorenylmethoxycarbonyl
DNP: dinitrophenyl
Bum: tert-butoxymethyl
Trt: trityl
MeBzl: 4-methylbenzyl
CHO: formyl
NMP: N-methylpyrrolidone
OcHex: cyclohexyl ester

SEQ ID NOs in the Sequence Listing attached to the specification show the following sequences, respectively.

[SEQ ID NO: 1]

SEQ ID NO: 1 shows the amino acid sequence of porcine SENR ligand peptide (porcine ligand 1).

[SEQ ID NO: 2]

SEQ ID NO: 2 shows the amino acid sequence of porcine SENR ligand peptide (porcine ligand 2).

[SEQ ID NO: 3]

SEQ ID NO: 3 shows the amino acid sequence of rat SENR protein.

[SEQ ID NO: 4]

SEQ ID NO: 4 shows the entire nucleotide sequence of porcine SENR ligand precursor protein cDNA.

[SEQ ID NO: 5]

SEQ ID NO: 5 shows the entire nucleotide sequence of porcine SENR ligand precursor protein cDNA.

[SEQ ID NO: 6]

SEQ ID NO: 6 shows the entire nucleotide sequence of porcine SENR ligand precursor protein cDNA.

[SEQ ID NO: 7]

SEQ ID NO: 7 shows the entire amino acid sequence of porcine SENR ligand precursor protein.

[SEQ ID NO: 8]

SEQ ID NO: 8 shows the entire amino acid sequence of porcine SENR ligand precursor protein.

[SEQ ID NO: 9]

SEQ ID NO: 9 shows the amino acid sequence of bovine SENR ligand peptide.

[SEQ ID NO: 10]

SEQ ID NO: 10 shows the amino acid sequence of human SENR ligand polypeptide (human urotensin II).

[SEQ ID NO: 11]

SEQ ID NO: 11 shows the entire amino acid sequence of human SENR protein.

[SEQ ID NO: 12]

SEQ ID NO: 12 shows the DNA sequence for SEQ ID NO: 2 (porcine ligand 2).

[SEQ ID NO: 13]

SEQ ID NO: 13 shows the DNA sequence for SEQ ID NO: 9 (bovine ligand).

[SEQ ID NO: 14]

SEQ ID NO: 14 shows the entire amino acid sequence of bovine SENR ligand precursor protein.

[SEQ ID NO: 15]

SEQ ID NO: 15 shows the entire nucleotide sequence of bovine SENR ligand precursor protein cDNA.

[SEQ ID NO: 16]

SEQ ID NO: 16 shows the entire nucleotide sequence of rat urotensin II like peptide precursor protein cDNA.

[SEQ ID NO: 17]

SEQ ID NO: 17 shows the entire amino acid sequence of rat urotensin II like peptide precursor protein.

[SEQ ID NO: 18]

SEQ ID NO: 18 shows the amino acid sequence of rat urotensin II like peptide-1.

[SEQ ID NO: 19]

SEQ ID NO: 19 shows the amino acid sequence of rat urotensin II like peptide-2.

[SEQ ID NO: 20]

SEQ ID NO: 20 shows the DNA sequence for SEQ ID NO: 18 (rat urotensin II like peptide-1).

[SEQ ID NO: 21]

SEQ ID NO: 21 shows the DNA sequence for SEQ ID NO: 19 (rat urotensin II like peptide-2).

[SEQ ID NO: 22]

SEQ ID NO: 22 shows the entire nucleotide sequence of mouse urotensin II like peptide precursor protein cDNA.

[SEQ ID NO: 23]

SEQ ID NO: 23 shows the entire amino acid sequence of mouse urotensin II like peptide precursor protein.

[SEQ ID NO: 24]

SEQ ID NO: 24 shows the amino acid sequence of mouse urotensin II like peptide.

[SEQ ID NO: 25]

SEQ ID NO: 25 shows the DNA sequence for SEQ ID NO: 24 (mouse urotensin II like peptide).

[SEQ ID NO: 26]

SEQ ID NO: 26 shows the amino acid sequence of a mature form of rat urotensin II like peptide, predicted from the amino acid sequence of its precursor protein.

[SEQ ID NO: 27]

SEQ ID NO: 27 shows the amino acid sequence of a mature form of rat urotensin II like peptide, predicted from the amino acid sequence of its precursor protein.

[SEQ ID NO: 28]

SEQ ID NO: 28 shows the amino acid sequence of a mature form of mouse urotensin II like peptide, predicted from the amino acid sequence of its precursor protein.

[SEQ ID NO: 29]

SEQ ID NO: 29 shows the amino acid sequence of a mature form of mouse urotensin II like peptide, predicted from the amino acid sequence of its precursor protein.

[SEQ ID NO: 30]

SEQ ID NO: 30 shows the DNA sequence for SEQ ID NO: 26 (a mature form of rat urotensin II like peptide).

[SEQ ID NO: 31]

SEQ ID NO: 31 shows the DNA sequence for SEQ ID NO: 27 (a mature form of rat urotensin II like peptide).

[SEQ ID NO: 32]

SEQ ID NO: 32 shows the DNA sequence for SEQ ID NO: 28 (a mature form of mouse urotensin II like peptide).

[SEQ ID NO: 33]

SEQ ID NO: 33 shows the DNA sequence for SEQ ID NO: 29 (a mature form of mouse urotensin II like peptide).

[SEQ ID NO: 34]

SEQ ID NO: 34 shows the DNA sequence of human SENR ligand (SEQ ID NO: 10).

## EXAMPLES

The present invention will be further described in more detail in the following Reference Examples and Examples, which are not intended to limit the scope of the invention.

In the following Reference Examples, each mass spectrum (MS) was measured under the conditions shown below:

Instrument for measurement: a Micromass Platform II system
Ionization: atmospheric pressure chemical ionization (APCI) or electron spray ionization (ESI).

### Reference Example 1

1-Benzyl-6-bromo-2,3-dihydro-1H-pyrrolo[2,3-b]quinolin-4-ylamine

N-Benzylpyrrolidone (1.8 g, 10.4 mmol) was dissolved in chloroform (4 ml) and stirred at room temperature for 30 minutes in the presence of phosphorus oxychloride (1.8 g, 11.7 mmol). 4-Bromo-2-cyanoaniline (2.0 g, 10 mmol) was added, followed by heating at reflux for 3 hours. The reaction mixture was poured into ice-cold water and neutralized with 20% aqueous sodium hydroxide. After extraction with chloroform, the organic layer was dried over anhydrous sodium sulfate. After concentration under reduced pressure, the residue was dissolved in nitrobenzene (10 ml) and heated at 160°C for 3 hours in the presence of zinc chloride (2 g). Aqueous sodium hydroxide (20%) was added to the reaction mixture, which was then extracted with ethyl acetate. After the organic layer was dried over anhydrous sodium sulfate and concentrated under reduced pressure, the residue was applied to silica gel column chromatography (silica gel 50 g, ethyl acetate/hexane = 1/2). Fractions of interest were concentrated under reduced pressure and ethanol was added to the residue to collect a precipitated product by filtration. The precipitated product was washed with ethanol and dried under reduced pressure to give the titled compound (1.2 g, 3.4 mmol). $^1$H-NMR (DMSO-$d_6$) δ: 2.86 (2H, t, J=8.0Hz), 3.41 (2H, t, J=8.0Hz), 4.59 (2H, s), 7.24-7.33 (6H, m), 7.42 (1H, dd, J=9.2, 2.2Hz), 8.12 (1H, d, J=2.2Hz).
Mass (ESI+); 354 (M+H), 356

### Reference Example 2

1-Benzyl-6-(4-methylphenyl)-2,3-dihydro-1H-pyrrolo[2,3-b]quinolin-4-ylamine

1-Benzyl-6-bromo-2,3-dihydro-1H-pyrrolo[2,3-b]quinolin-4-ylamine (70 mg, 0.2 mmol) was suspended in toluene (0.5 ml), to which $Pd(Ph_3P)_4$ (6 mg), 2M aqueous sodium carbonate (0.2 ml) and a solution of 4-methylphenylboronic acid (30 mg) in ethanol (0.25 ml) were then added and reacted at 90°C for 16 hours. After the reaction mixture was partitioned between water and ethyl acetate, the organic layer was washed with water, dried over anhydrous sodium sulfate and then concentrated under reduced pressure. The residue was applied to silica gel column chromatography (silica gel 2 g, ethyl acetate/hexane = 1/2). Fractions of interest were concentrated, followed by addition of hydrochloric acid in ethanol to collect a precipitated product by filtration. The precipitated product was washed with ethanol and dried under reduced pressure to give the titled compound (15 mg).
$^1$H-NMR ($CDCl_3$) δ: 2.41(3H, s), 2.92 (2H, t, J=8.0Hz), 3.50 (2H, t, J=8.0Hz), 4.75 (2H, s), 7.24-7.38 (7H, m), 7.57 (2H, d), 7.69 (3H, m).
Mass (ESI+); 366 (M+H)

### Reference Example 3

1-Benzyl-6-(3-thienyl)-2,3-dihydro-1H-pyrrolo[2,3-b]quinolin-4-ylamine hydrochloride

1-Benzyl-6-bromo-2,3-dihydro-1H-pyrrolo[2,3-b]quinolin-4-ylamine (140 mg, 0.4 mmol) was suspended in toluene (1 ml), to which $Pd(Ph_3P)_4$ (12 mg), 2M aqueous sodium carbonate (0.4 ml) and a solution of 3-thiopheneboronic acid (56 mg) in ethanol (0.5 ml) were then added and reacted at 90°C for 16 hours. After the reaction mixture was partitioned between water and ethyl acetate, the organic layer was washed with water, dried over anhydrous sodium sulfate and then concentrated under reduced pressure. The residue was applied to silica gel column chromatography (silica gel 2 g, ethyl acetate/hexane = 1/2). Fractions of interest were concentrated, followed by addition of hydrochloric

acid in ethanol to collect a precipitated product by filtration. The precipitated product was washed with ethanol and dried under reduced pressure to give the titled compound (52 mg).
$^1$H-NMR (DMSO-$d_6$) δ: 3.01(2H, t, J=8.0Hz), 3.73 (2H, t, J=8.0Hz), 4.92 (2H, s), 7.40 (5H, bs), 7.70-7:88 (3H, m), 8.02-8.09 (2H, m), 8.54 (1H, s).
Mass (ESI+); 358 (M+H)

**Reference Example 4**

N-Benzyl-1-benzyl-6-bromo-2,3-dihydro-1H-pyrrolo[2,3-b]quinolin-4-ylamine

1-Benzyl-6-bromo-2,3-dihydro-1H-pyrrolo[2,3-b]quinolin-4-ylamine (70 mg, 0.2 mmol) was dissolved in dimethylformamide (1 ml), to which 2-tert-butylimino-2-diethylamino-1,3-dimethylperhydro-1,3,2-diazaphospholine (0.24 ml) and benzyl bromide (0.095 ml) were then added and reacted at 80°C for I hour. After the reaction mixture was partitioned between water and ethyl acetate, the organic layer was washed with water, dried over anhydrous sodium sulfate and then concentrated under reduced pressure. The residue was purified by preparative HPLC (YMC CombiPrep ODS, 20 x 50 mm). Fractions of interest were dried under reduced pressure to give the titled compound (44 mg).
$^1$H-NMR ($CDCl_3$) δ: 3.13(2H, t, J=8.0Hz), 3.54 (2H, t, J=8.0Hz), 4.72 (2H, s), 4.82 (2H, s), 7.22-7.37 (10H, m), 7.50 (1H, dd, J=9.0, 2.0Hz), 7.69 (1H, d, J=9.0Hz), 8.00 (1H, d, J=2.0Hz).
Mass (ESI+); 444 (M+H), 446

**Reference Example 5**

N,N-Dibenzyl-1-benzyl-6-bromo-2,3-dihydro-1H-pyrrolo[2,3-b]quinolin-4-ylamine

1-Benzyl-6-bromo-2,3-dihydro-1H-pyrrolo[2,3-b]quinolin-4-ylamine (70 mg, 0.2 mmol) was dissolved in dimethylformamide (1 ml), to which 2-tert-butylimino-2-diethylamino-1,3-dimethylperhydro-1,3,2-diazaphospholine (0.24 ml) and benzyl bromide (0.095 ml) were then added and reacted at 80°C 1 hour. After the reaction mixture was partitioned between water and ethyl acetate, the organic layer was washed with water, dried over anhydrous sodium sulfate and then concentrated under reduced pressure. The residue was purified by preparative HPLC (YMC CombiPrep ODS, 20 x 50 mm). Fractions of interest were dried under reduced pressure to give the titled compound (32 mg).
$^1$H-NMR ($CDCl_3$) δ: 2.61(2H, t, J=8.0Hz), 3.49 (2H, t, J=8.0Hz), 4.27 (4H, s), 4.99 (2H, s), 7.17-7.38 (15H, m), 7.74 (1H, dd, J=8.8, 2.0Hz), 7.97 (1H, d, J=8.8Hz), 8.16 (1H, d, J=2.0Hz).
Mass (ESI+); 534 (M+H), 536

**Reference Example 6**

N-Allyl-1-benzyl-6-bromo-2,3-dihydro-1H-pyrrolo[2,3-b]quinolin-4-ylamine

1-Benzyl-6-bromo-2,3-dihydro-1H-pyrrolo[2,3-b]quinolin-4-ylamine (70 mg, 0.2 mmol) was dissolved in dimethylformamide (1 ml), to which 2-tert-butylimino-2-diethylamino-1,3-dimethylperhydro-1,3,2-diazaphospholine (0.24 ml) and allyl bromide (0.07 ml) were then added and reacted at 80°C for 1 hour. After the reaction mixture was partitioned between water and ethyl acetate, the organic layer was washed with water, dried over anhydrous sodium sulfate and then concentrated under reduced pressure. The residue was purified by preparative HPLC (YMC CombiPrep ODS, 20 × 50 mm). Fractions of interest were dried under reduced pressure to give the titled compound (26 mg).
$^1$H-NMR ($CDCl_3$) δ: 3.23(2H, t, J=8.0Hz), 3.62 (2H, t, J=8.0Hz), 4.07 (2H, bs), 4.83 (2H, s), 5.12-5.24 (2H, m), 5.91-6.00 (1H, m), 7.35 (5H, bs), 7.44 (1H, dd, J=8.8, 2.0Hz), 7.61 (1H, d, J=8.8Hz), 8.03 (1H, d, J=2.0Hz).
Mass (ESI+); 394 (M+H), 396

**Reference Example 7**

N,N-Diallyl-1-benzyl-6-bromo-2,3-dihydro-1H-pyrrolo[2,3-b]quinolin-4-ylamine

1-Benzyl-6-bromo-2,3-dihydro-1H-pyrrolo[2,3-b]quinolin-4-ylamine (70 mg, 0.2 mmol) was dissolved in dimethylformamide (1 ml), to which 2-tert-butylimino-2-diethylamino-1,3-dimethylperhydro-1,3,2-diazaphospholine (0.24 ml) and allyl bromide (0.07 ml) were then added and reacted at 80°C for 1 hour. After the reaction mixture was partitioned between water and ethyl acetate, the organic layer was washed with water, dried over anhydrous sodium sulfate and then concentrated under reduced pressure. The residue was purified by preparative HPLC (YMC Combi-

Prep ODS, 20 × 50 mm). Fractions of interest were dried under reduced pressure to give the titled compound (12 mg).
$^1$H-NMR ($CDCl_3$) δ: 3.20 (2H, t, J=8.0Hz), 3.70 (2H, t, J=8.0Hz), 3.88 (4H, d), 5.01 (2H, s), 5.20-5.29 (4H, m), 5.68-5.89 (2H, m), 7.36 (5H, bs), 7.67 (1H, dd, J=9.0, 2.0Hz), 7.90 (1H, d, J=9.0Hz), 7.97 (1H, d, J=2.0Hz).
Mass (ESI+); 434 (M+H), 436

**Reference Example 8**

N-Methyl-1-benzyl-6-bromo-2,3-dihydro-1H-pyrrolo[2,3-b]quinolin-4-ylamine

1-Benzyl-6-bromo-2,3-dihydro-1H-pyrrolo[2,3-b]quinolin-4-ylamine (50 mg) was dissolved in dimethylformamide (0.5 ml), to which diisopropylethylamine (0.05 ml) and methyl iodide (0.5 ml) were then added and reacted at room temperature for 40 hours. The reaction mixture was concentrated under reduced pressure and the residue was purified by preparative HPLC (YMC CombiPrep ODS, 20 × 50 mm). Fractions of interest were dried under reduced pressure to give the titled compound (8 mg).
$^1$H-NMR ($CDCl_3$) δ: 3.19 (3H, s), 3.39 (2H, t, J=8.0Hz), 3.66 (2H, t, J=8.0Hz), 4.81 (2H, s), 7.28-7.57 (7H, m), 7.97 (1H, s).
Mass (ESI+); 368 (M+H), 370

**Reference Example 9**

N,N-Dimethyl-1-benzyl-6-bromo-2,3-dihydro-1H-pyrrolo[2,3-b]quinolin-4-ylamine

1-Benzyl-6-bromo-2,3-dihydro-1H-pyrrolo[2,3-b]quinolin-4-ylamine (50 mg) was dissolved in dimethylformamide (0.5 ml), to which diisopropylethylamine (0.05 ml) and methyl iodide (0.5 ml) were then added and reacted at room temperature for 40 hours. The reaction mixture was concentrated under reduced pressure and the residue was purified by preparative HPLC (YMC CombiPrep ODS, 20 × 50 mm). Fractions of interest were dried under reduced pressure to give the titled compound (5 mg).
$^1$H-NMR ($CDCl_3$) δ: 3.14 (6H, s), 3.37 (2H, t, J=8.0Hz), 3.74 (2H, t, J=8.0Hz), 4.94 (2H, s), 7.38 (5H, bs), 7.67 (1H, dd, J=9.0, 2.0Hz), 7.77 (1H, d, J=9.0Hz), 7.95 (1H, d, J=2.0Hz).
Mass (ESI+); 382 (M+H), 384

**Reference Example 10**

6-Bromo-1-(4-fluorobenzyl)-2,3-dihydro-1H-pyrrolo[2,3-b]quinolin-4-ylamine

To a suspension of sodium hydride (60% in mineral oil; 440 mg) in N,N-dimethylformamide (10 ml), 2-pyrrolidone (0.76 ml) was added and stirred at room temperature for 15 minutes, followed by addition of 4-fluorobenzyl bromide (1.37 ml). After stirring at room temperature for 15 hours, water was added to the reaction mixture, which was then extracted with diethyl ether. The extracted solution was washed with saturated brine, dried over anhydrous magnesium sulfate and then concentrated under reduced pressure. The resulting residue was purified by silica gel chromatography (n-hexane/ethyl acetate = 1/2) to give 1-(4-fluorobenzyl)-2-pyrrolidone (1.28 g).
To a solution of 1-(4-fluorobenzyl)-2-pyrrolidone (600 mg) in chloroform (3 ml), phosphorus oxychloride (0.30 ml) was added and stirred at room temperature for 30 minutes, followed by addition of 2-amino-5-bromobenzonitrile (583 mg). After heating at reflux for 3 hours, ice-cold water was added to the reaction mixture. Further, the reaction mixture was neutralized with 20% aqueous sodium hydroxide and extracted with chloroform. The extracted solution was washed with saturated brine, dried over anhydrous magnesium sulfate and then concentrated under reduced pressure to give 5-bromo-2-{(1-(4-fluorobenzyl)-2-pyrrolidinylidene)amino}benzonitrile (1.01 g).
Under nitrogen atmosphere, a solution of 5-bromo-2-{(1-(4-fluorobenzyl)-2-pyrrolidinylidene)amino}benzonitrile (1.01 g) in tetrahydrofuran (8 ml) was cooled to -40°C. Under stirring at the same temperature, lithium diisopropylamide (2.0 M in heptane/tetrahydrofuran/ethylbenzene; 1.63 ml) was added dropwise. The reaction mixture was slowly warmed to room temperature and stirred for 1 hour. Ice-cold water was added to the reaction mixture, which was then extracted with ethyl acetate. The extracted solution was washed with saturated brine, dried over anhydrous magnesium sulfate and then concentrated under reduced pressure. The resulting residue was purified by silica gel chromatography (n-hexane/ethyl acetate = 2/1) to give the titled compound (347 mg).
$^1$H-NMR (DMSO-$d_6$) δ: 2.86 (2H, t, J=8.0Hz), 3.42 (2H, t, J=8.0Hz), 4.58 (2H, s), 7.14 (2H, d, J=8.8Hz), 7.29-7.46 (4H, m), 8.13 (1H, d, J=2.2Hz)
Mass (APCI+); 372 (M+H), 374

**Reference Example 11**

6-Bromo-1-(2-phenethyl)-2,3-dihydro-1H-pyrrolo[2,3-b]quinolin-4-ylamine hydrochloride

To a suspension of sodium hydride (60% in mineral oil; 440 mg) in N,N-dimethylformamide (10 ml), 2-pyrrolidone (0.76 ml) was added and stirred at room temperature for 15 minutes, followed by addition of (2-bromoethyl) benzene (1.50 ml). After stirring at room temperature for 15 hours, water was added to the reaction mixture, which was then extracted with diethyl ether. The extracted solution was washed with saturated brine, dried over anhydrous magnesium sulfate and then concentrated under reduced pressure. The resulting residue was purified by silica gel chromatography (n-hexane/ethyl acetate = 1/2) to give 1-(2-phenethyl)-2-pyrrolidone (0.438 g).
To a solution of 1-(2-phenethyl)-2-pyrrolidone (438 mg) in chloroform (3 ml), phosphorus oxychloride (0.23 ml) was added and stirred at room temperature for 30 minutes, followed by addition of 2-amino-5-bromobenzonitrile (435 mg). After heating at reflux for 3 hours, ice-cold water was added to the reaction mixture. Further, the reaction mixture was neutralized with 20% aqueous sodium hydroxide and extracted with chloroform. The extracted solution was washed with saturated brine, dried over anhydrous magnesium sulfate and then concentrated under reduced pressure to give 5-bromo-2-{(1-(2-phenethyl)-2-pyrrolidinylidene)amino}benzonitrile (736 mg).
Under nitrogen atmosphere, a solution of 5-bromo-2-{(1-(2-phenethyl)-2-pyrrolidinylidene)amino}benzonitrile (736 mg) in tetrahydrofuran (6 ml) was cooled to -40°C. Under stirring at the same temperature, lithium diisopropylamide (2.0 M in heptane/tetrahydrofuran/ethylbenzene; 1.20 ml) was added dropwise. The reaction mixture was slowly warmed to room temperature and stirred for 1 hour. Ice-cold water was added to the reaction mixture, which was then extracted with ethyl acetate. The extracted solution was washed with saturated brine, dried over anhydrous magnesium sulfate and then concentrated under reduced pressure. The resulting residue was purified by silica gel chromatography (n-hexane/ethyl acetate = 2/1) and further treated with a 4N ethyl acetate solution of hydrochloric acid to give the titled compound (254 mg).
$^1$H-NMR (DMSO-$d_6$) δ: 2.80-3.00 (2H, m), 3.15-3.30 (2H, m), 3.83 (2H, t, J=8.0Hz), 3.93 (2H, t, J=8.0Hz), 7.15-7.45 (5H, m), 7.75-7.90 (2H, m), 8.39 (1H, s)

**Reference Example 12**

6-Bromo-1-(3-pyridinylmethyl)-2,3-dihydro-1H-pyrrolo[2,3-b]quinolin-4-ylamine dihydrochloride

To a suspension of sodium hydride (60% in mineral oil; 880 mg) in N,N-dimethylformamide (10 ml), 2-pyrrolidone (0.76 ml) was added and stirred at room temperature for 15 minutes, followed by addition of 3-(chloromethyl) pyridine hydrochloride (1.80 g). After stirring at room temperature for 15 hours, water was added to the reaction mixture, which was then extracted with chloroform. The extracted solution was washed with saturated brine, dried over anhydrous magnesium sulfate and then concentrated under reduced pressure. The resulting residue was treated with a 4 N ethyl acetate solution of hydrochloric acid to give 1-(3-pyridinylmethyl)-2-pyrrolidone hydrochloride (2.02 g).
To a solution of 1-(3-pyridinylmethyl)-2-pyrrolidone hydrochloride (600 mg) in chloroform (3 ml), phosphorus oxychloride (0.31 ml) was added and stirred at room temperature for 30 minutes, followed by addition of 2-amino-5-bromobenzonitrile (530 mg). After heating at reflux for 3 hours, ice-cold water was added to the reaction mixture. Further, the reaction mixture was neutralized with 20% aqueous sodium hydroxide and extracted with chloroform. The extracted solution was washed with saturated brine, dried over anhydrous magnesium sulfate and then concentrated under reduced pressure to give 5-bromo-2-{(1-(3-pyridinylmethyl)-2-pyrrolidinylidene)-amino}benzonitrile (736 mg).
Under nitrogen atmosphere, a solution of 5-bromo-2-{(1-(3-pyridinylmethyl)-2-pyrrolidinylidene)amino}benzonitrile (940 mg) in tetrahydrofuran (10 ml) was cooled to -40°C. Under stirring at the same temperature, lithium diisopropylamide (2.0 M in heptane/tetrahydrofuran/ethylbenzene; 1.99 ml) was added dropwise. The reaction mixture was slowly warmed to room temperature and stirred for 1 hour. Ice-cold water was added to the reaction mixture, which was then extracted with ethyl acetate. The extracted solution was washed with saturated brine, dried over anhydrous magnesium sulfate and then concentrated under reduced pressure. The resulting residue was purified by silica gel chromatography (n-hexane/ethyl acetate = 2/1) and further treated with a 4N ethyl acetate solution of hydrochloric acid to give the titled compound (198 mg).
$^1$H-NMR (DMSO-$d_6$) δ: 3.00 (2H, t, J=8.0Hz), 3.79 (2H, t, J=8.0Hz), 5.28 (2H,s), 7.56 (2H, m), 7.83 (1H, d, J=8.0Hz), 7.90-8.10 (2H, m), 8.46 (1H, s), 8.54 (1H, d, J=8.0Hz), 8.85 (1H, d, J=5.8Hz), 9.05 (1H, s)
Mass (APCI+); 355 (M+H), 357

**Reference Example 13**

1-Benzyl-6-fluoro-2,3-dihydro-1H-pyrrolo[2,3-b]quinolin-4-ylamine hydrochloride

To a solution of 1-benzyl-2-pyrrolidone (0.84 ml) in chloroform (3 ml), phosphorus oxychloride (0.51 ml) was added and stirred at room temperature for 30 minutes, followed by addition of 2-amino-5-fluorobenzonitrile (0.65 ml). After heating at reflux for 3 hours, ice-cold water was added to the reaction mixture. Further, the reaction mixture was neutralized with 20% aqueous sodium hydroxide and extracted with chloroform. The extracted solution was washed with saturated brine, dried over anhydrous magnesium sulfate and then concentrated under reduced pressure to give 5-fluoro-2-{(1-benzyl-2-pyrrolidinylidene)amino}benzonitrile (1.68 g).

A solution of 5-fluoro-2-{(1-benzyl-2-pyrrolidinylidene)amino}benzonitrile (500 mg) in tetrahydrofuran (2 ml) was added to a solution of hexamethyldisilazane sodium salt (3.91 ml) in tetrahydrofuran (3 ml) which had been cooled to -78°C. After stirring at the same temperature for 15 minutes, the reaction mixture was slowly warmed to -20°C and stirred for 2 hours. The reaction mixture was further warmed to 40°C and stirred for 15 hours, followed by addition of saturated brine and extraction with ethyl acetate. The extracted solution was washed with saturated brine, dried over anhydrous magnesium sulfate and then concentrated under reduced pressure. The resulting residue was purified by silica gel chromatography (n-hexane/ethyl acetate = 2/1) and further treated with a 4N ethyl acetate solution of hydrochloric acid to give the titled compound (60 mg).
$^1$H-NMR (DMSO-$d_6$) δ: 2.99 (2H, t, J=8.0Hz), 3.72 (2H, t, J=8.0Hz), 4.95 (2H,s), 7.25-7.65 (6H, m), 7.95-8.15 (2H, m)
Mass (APCI+); 294 (M+H)

**Reference Example 14**

1-Benzyl-7-bromo-1,2,3,4-tetrahydrobenzo[b][1,8]naphthylidin-5-ylamine hydrochloride

To a suspension of sodium hydride (60% in mineral oil; 440 mg) in N,N-dimethylformamide (10 ml), 2-piperidone (991 mg) was added and stirred at room temperature for 15 minutes, followed by addition of benzyl bromide (1.31 ml). After stirring at room temperature for 4 hours, water was added to the reaction mixture, which was then extracted with diethyl ether. The extracted solution was washed with saturated brine, dried over anhydrous magnesium sulfate and then concentrated under reduced pressure. The resulting residue was purified by silica gel chromatography (n-hexane/ethyl acetate = 1/1) to give 1-benzyl-2-piperidone (1.30 g).

To a solution of 1-benzyl-2-piperidone (567 mg) in chloroform (3 ml), phosphorus oxychloride (0.29 ml) was added. After heating at reflux for 3 hours, ice-cold water was added to the reaction mixture. Further, the reaction mixture was neutralized with 20% aqueous sodium hydroxide and extracted with chloroform. The extracted solution was washed with saturated brine, dried over anhydrous magnesium sulfate and then concentrated under reduced pressure to give 5-bromo-2-{(1-benzyl-2-piperidinylidene)amino}benzonitrile (1.05 g).

5-Bromo-2-{(1-benzyl-2-piperidinylidene)amino}benzonitrile (1.01 g) was dissolved in nitrobenzene (5 ml) and stirred at 155°C for 1 hour in the presence of zinc chloride (466 mg). After cooling, 20% aqueous sodium hydroxide was added to adjust the reaction mixture to pH10, followed by extraction with chloroform. The extracted solution was washed with saturated brine, dried over anhydrous magnesium sulfate and then concentrated under reduced pressure. The resulting residue was purified by silica gel chromatography (n-hexane/ethyl acetate = 9/1 → 3/2) and further treated with a 4N ethyl acetate solution of hydrochloric acid to give the titled compound (551 mg).
$^1$H-NMR (DMSO-$d_6$) δ: 1.85-2.00 (2H, m), 2.59 (2H, t, J=6.0Hz), 3.10-3.50 (2H, m), 5.12 (2H,s), 7.25-7.45 (5H, m), 7.80 (1H, dd, J=1.8Hz, 8.8Hz), 8.03 (1H, d, J=8.8Hz), 8.54 (1H, d, J=1.8Hz)
Mass (APCI+); 368 (M+H), 370

**Reference Example 15**

1-Benzyl-7-bromo-2,2,4,5-tetrahydro-1H-azepino[2,3-b]quinolin-6-ylamine hydrochloride

To a suspension of sodium hydride (60% in mineral oil; 440 mg) in N,N-dimethylformamide (10 ml), ε-caprolactam (1.13 g) was added and stirred at room temperature for 15 minutes, followed by addition of benzyl bromide (1.31 ml). After stirring at room temperature for 4 hours, water was added to the reaction mixture, which was then extracted with diethyl ether. The extracted solution was washed with saturated brine, dried over anhydrous magnesium sulfate and then concentrated under reduced pressure. The resulting residue was purified by silica gel chromatography (n-hexane/ethyl acetate = 1/1) to give N-benzyl-ε-caprolactam (1.72 g).

To a solution of N-benzyl-ε-caprolactam (607 mg) in chloroform (3 ml), phosphorus oxychloride (0.29 ml) was added. After heating at reflux for 3 hours, ice-cold water was added to the reaction mixture. Further, the reaction mixture

was neutralized with 20% aqueous sodium hydroxide and extracted with chloroform. The extracted solution was washed with saturated brine, dried over anhydrous magnesium sulfate and then concentrated under reduced pressure to give 1-benzyl-2-{(4-bromo-2-cyanophenyl)imino}hexahydro-1H-azepine (1.08 g).

1-Benzyl-2-{(4-bromo-2-cyanophenyl)imino}hexahydro-1H-azepine (1.08 g) was dissolved in nitrobenzene (5 ml) and stirred at 155°C for 1 hour in the presence of zinc chloride (463 mg). After cooling, aqueous ammonia was added to adjust the reaction mixture to pH10, followed by extraction with chloroform. The extracted solution was washed with saturated brine, dried over anhydrous magnesium sulfate and then concentrated under reduced pressure. The resulting residue was purified by silica gel chromatography (n-hexane/ethyl acetate = 9/1 → 3/2) and further treated with a 4N ethyl acetate solution of hydrochloric acid to give the titled compound (475 mg).
$^1$H-NMR (DMSO-$d_6$) δ: 1.55-1.85 (4H, m), 2.71 (2H, m), 3.51 (2H, m),4.94 (2H,s), 7.30-7.50 (5H, m), 7.84 (1H, dd, J=2.0Hz, 8.8Hz), 8.07 (1H, d, J=8.8Hz), 8.60 (1H, d, J=2.0Hz)
Mass (APCI+); 382 (M+H), 384

## Reference Example 16

1-Benzyl-6-(benzofuran-2-yl)-2,3-dihydro-1H-pyrrolo[2,3-b]quinolin-4-ylamine hydrochloride

1-Benzyl-6-bromo-2,3-dihydro-1H-pyrrolo[2,3-b]quinolin-4-ylamine (140 mg, 0.4 mmol) was suspended in toluene (1 ml), to which $Pd(Ph_3P)_4$ (12 mg), 2M aqueous sodium carbonate (0.4 ml) and a solution of benzofuran-2-ylboronic acid (72 mg) in ethanol (0.5 ml) were then added and reacted at 90°C for 16 hours. After the reaction mixture was partitioned between water and ethyl acetate, the organic layer was washed with water, dried over anhydrous sodium sulfate and then concentrated under reduced pressure. The residue was applied to silica gel column chromatography (silica gel 2 g, ethyl acetate/hexane = 1/2). Fractions of interest were concentrated, followed by addition of hydrochloric acid in ethanol to collect a precipitated product by filtration. The precipitated product was washed with ethanol and dried under reduced pressure to give the titled compound (63 mg).
$^1$H-NMR (DMSO-$d_6$) δ: 3.01(2H, t, J=8.0Hz), 3.74 (2H, t, J=8.0Hz), 4.95 (2H, s), 7.26-7.74 (12H, m), 7.97 (1H, d, J=8.4Hz), 8.19 (1H, d, J=8.4Hz), 8.75 (1H, s).
Mass (ESI+); 392 (M+H)

## Reference Example 17

1-Benzyl-6-(3-acetoaminophenyl)-2,3-dihydro-1H-pyrrolo[2,3-b]quinolin-4-ylamine hydrochloride

1-Benzyl-6-bromo-2,3-dihydro-1H-pyrrolo[2,3-b]quinolin-4-ylamine (140 mg, 0.4 mmol) was suspended in toluene (1 ml), to which $Pd(Ph_3P)_4$ (12 mg), 2M aqueous sodium carbonate (0.4 ml) and a solution of 3-acetoaminophenylboronic acid (79 mg) in ethanol (0.5 ml) were then added and reacted at 90°C for 16 hours. After the reaction mixture was partitioned between water and ethyl acetate, the organic layer was washed with water, dried over anhydrous sodium sulfate and then concentrated under reduced pressure. The residue was applied to silica gel column chromatography (silica gel 2 g, ethyl acetate/hexane = 1/2). Fractions of interest were concentrated, followed by addition of hydrochloric acid in ethanol to collect a precipitated product by filtration. The precipitated product was washed with ethanol and dried under reduced pressure to give the titled compound (17 mg).
$^1$H-NMR (DMSO-$d_6$) δ: 2.09 (3H, s), 3.00 (2H, t, J=8.0Hz), 3.73 (2H, t, J=8.0Hz), 4.98 (2H, s), 7.35-7.64 (9H, m), 7.88 (1H, d, J=8.4Hz), 7.99 (2H, bs), 8.01 (1H, d, J=8.4Hz), 8.44 (1H, s).
Mass (ESI+); 409 (M+H)

## Reference Example 18

1-(4-tert-Butylbenzyl)-6-bromo-2,3-dihydro-1H-pyrrolo[2,3-b]quinolin-4-ylamine hydrochloride

1-Benzyl-6-bromo-2,3-dihydro-1H-pyrrolo[2,3-b]quinolin-4-ylamine (350 mg, 1 mmol) was suspended in dichloromethane (2 ml), followed by dropwise addition of $BBr_3$ (1.4 ml). After stirring at room temperature for 8 hours, ice-cold water was added to the reaction mixture, which was then alkalized with 30% aqueous sodium hydroxide and extracted with ethanol-containing dichloromethane. After concentration under reduced pressure, the residue was crystallized from dichloromethane to give 6-bromo-2,3-dihydro-1H-pyrrolo[2,3-b]quinolin-4-ylamine (50 mg). This compound was dissolved in N,N-dimethylformamide (1 ml), to which 2-tert-butylimino-2-diethylamino-1,3-dimethylperhydro-1,3,2-diazaphospholine (0.088 ml) and 4-tert-butylbenzyl bromide (0.060 ml) were then added and reacted at room temperature for 40 hours. After the reaction mixture was partitioned between ice-cold water and dichloromethane, the organic layer was washed with water, dried over anhydrous sodium sulfate and then concentrated under reduced

pressure. The residue was applied to silica gel column chromatography (silica gel 2 g, ethyl acetate/hexane = 1/2). Fractions of interest were concentrated, followed by addition of hydrochloric acid in ethanol to collect a precipitated product by filtration. The precipitated product was washed with ethanol and dried under reduced pressure to give the titled compound (20 mg).
$^1$H-NMR (DMSO-$d_6$) δ: 1.27 (9H, s), 2.98 (2H, t, J=8.0Hz), 3.73 (2H, t, J=8.0Hz), 4.87 (2H, s), 7.29 (1H, d, J=8.2Hz), 7.42 (1H, d, J=8.2Hz), 7.48 (2H, bs), 7.81 (2H, s), 8.43 (1H, s).
Mass (ESI+); 410 (M+H)

## Reference Example 19

1-(4-Cyanobenzyl)-6-bromo-2,3-dihydro-1H-pyrrolo[2,3-b]quinolin-4-ylamine hydrochloride

1-Benzyl-6-bromo-2,3-dihydro-1H-pyrrolo[2,3-b]quinolin-4-ylamine (350 mg, 1 mmol) was suspended in dichloromethane (2 ml), followed by dropwise addition of $BBr_3$ (1.4 ml). After stirring at room temperature for 8 hours, ice-cold water was added to the reaction mixture, which was then alkalized with 30% aqueous sodium hydroxide and extracted with ethanol-containing dichloromethane. After concentration under reduced pressure, the residue was crystallized from dichloromethane to give 6-bromo-2,3-dihydro-1H-pyrrolo[2,3-b]quinolin-4-ylamine (50 mg). This compound was dissolved in N,N-dimethylformamide (1 ml), to which 2-tert-butylimino-2-diethylamino-1,3-dimethylperhydro-1,3,2-diazaphospholine (0.088 ml) and 4-cyanobenzyl bromide (66 mg) were then added and reacted at room temperature for 40 hours. After the reaction mixture was partitioned between ice-cold water and dichloromethane, the organic layer was washed with water, dried over anhydrous sodium sulfate and then concentrated under reduced pressure. The residue was applied to silica gel column chromatography (silica gel 2 g, ethyl acetate/hexane = 1/2). Fractions of interest were concentrated, followed by addition of hydrochloric acid in ethanol to collect a precipitated product by filtration. The precipitated product was washed with ethanol and dried under reduced pressure to give the titled compound (20 mg).
$^1$H-NMR (DMSO-$d_6$) δ: 3.00 (2H, t, J=8.0Hz), 3.74 (2H, t, J=8.0Hz), 5.06 (2H, s), 7.50-7.62 (4H, m), 7.82-7.90 (6H, m), 8.45 (1H, s).
Mass (ESI+); 379 (M+H)

## Reference Example 20

1-(3,5-Dimethoxybenzyl)-6-bromo-2,3-dihydro-1H-pyrrolo[2,3-b]quinolin-4-ylamine hydrochloride.

1-Benzyl-6-bromo-2,3-dihydro-1H-pyrrolo[2,3-b]quinolin-4-ylamine (350 mg, 1 mmol) was suspended in dichloromethane (2 ml), followed by dropwise addition of $BBr_3$ (1.4 ml). After stirring at room temperature for 8 hours, ice-cold water was added to the reaction mixture, which was then alkalized with 30% aqueous sodium hydroxide and extracted with ethanol-containing dichloromethane. After concentration under reduced pressure, the residue was crystallized from dichloromethane to give 6-bromo-2,3-dihydro-1H-pyrrolo[2,3-b]quinolin-4-ylamine (50 mg). This compound was dissolved in N,N-dimethylformamide (1 ml), to which 2-tert-butylimino-2-diethylamino-1,3-dimethylperhydro-1,3,2-diazaphospholine (0.088 ml) and 3,5-dimethoxybenzyl bromide (75 mg) were then added and reacted at room temperature for 40 hours. After the reaction mixture was partitioned between ice-cold water and dichloromethane, the organic layer was washed with water, dried over anhydrous sodium sulfate and then concentrated under reduced pressure. The residue was applied to silica gel column chromatography (silica gel 2 g, ethyl acetate/hexane = 1/2). Fractions of interest were concentrated, followed by addition of hydrochloric acid in ethanol to collect a precipitated product by filtration. The precipitated product was washed with ethanol and dried under reduced pressure to give the titled compound (17 mg).
$^1$H-NMR (DMSO-$d_6$) δ: 2.98 (2H, t, J=8.0Hz), 3.70-3.74 (8H, m), 6.42-6.54 (3H, m), 7.48 (2H, bs), 7.80 (2H, s), 8.43 (1H, s).
Mass (ESI+); 414 (M+H)

## Reference Example 21

1-(4-Methoxybenzyl)-6-bromo-2,3-dihydro-1H-pyrrolo[2,3-b]quiolin-4-ylamine hydrochloride

1-Benzyl-6-bromo-2,3-dihydro-1H-pyrrolo[2,3-b]quinolin-4-ylamine (350 mg, 1 mmol) was suspended in dichloromethane (2 ml), followed by dropwise addition of $BBr_3$ (1.4 ml). After stirring at room temperature for 8 hours, ice-cold water was added to the reaction mixture, which was then alkalized with 30% aqueous sodium hydroxide and extracted with ethanol-containing dichloromethane. After concentration under reduced pressure, the residue was crystallized from dichloromethane to give 6-bromo-2,3-dihydro-1H-pyrrolo[2,3-b]quinolin-4-ylamine (50 mg). This compound was dissolved in N,N-dimethylformamide (1 ml), to which 2-tert-butylimino-2-diethylamino-1,3-dimethylperhydro-1,3,2-diazaphospholine (0.088 ml) and 4-methoxybenzyl bromide (0.045 ml) were then added and reacted at room temperature for 40 hours. After the reaction mixture was partitioned between ice-cold water and dichloromethane, the organic layer was washed with water, dried over anhydrous sodium sulfate and then concentrated under reduced pressure. The residue was applied to silica gel column chromatography (silica gel 2 g, ethyl acetate/hexane = 1/2). Fractions of interest were concentrated, followed by addition of hydrochloric acid in ethanol to collect a precipitated product by filtration. The precipitated product was washed with ethanol and dried under reduced pressure to give the titled compound (17 mg).
$^1$H-NMR (DMSO-$d_6$) δ: 2.95 (2H, t, J=8.0Hz), 3.69 (2H, t, J=8.0Hz), 3.75 (3H, s), 4.85 (2H, s), 6.95 (2H, d, J=8.8Hz), 7.33 (2H, d, J=8.8Hz), 7.45 (2H, bs), 7.84 (2H, bs), 8.43 (1H, s).

## Reference Example 22

4-(4-Phenyl-1-piperazinyl)-1-(2,3,4,5-tetrahydro-1H-3-benzazepin-7-yl)-1-butanone trihydrochloride

1) 2,2,2-Trifluoro-1-(1,2,4,5-tetrahydro-3H-3-benzazepin-3-yl)-1-ethanone

To a solution of 2,3,4,5-tetrahydro-1H-3-benzazepine(15 g) and triethylamine (51 ml) in tetrahydrofuran (THF; 100 ml), trifluoroacetic acid anhydride (31 g) was added on ice. After stirring at room temperature for 15 hours, 1N hydrochloric acid was added to stop the reaction and the reaction mixture was extracted with ethyl acetate. The extracted solution was washed with water, dried over anhydrous magnesium sulfate and then evaporated under reduced pressure to remove the solvent. The residue was purified by silica gel column chromatography (n-hexane/ethyl acetate = 4/1) to give the titled compound (25 g). $^1$H-NMR ($CDCl_3$) δ: 2.95-3.05 (4H, m), 3.65-3.85 (4H, m), 7.10-7.30 (4H, m)

2) 4-Bromo-1-[3-(2,2,2-trifluoroacetyl)-2,3,4,5-tetrahydro-1H-3-benzazepin-7-yl]-1-butanone

To a solution of 2,2,2-trifluoro-1-(1,2,4,5-tetrahydro-3H-3-benzazepin-3-yl)-1-ethanone (10 g) in dichloromethane (70 ml), 4-bromobutyryl chloride (4.8 ml) and aluminum chloride (8.2 g) were added and stirred at room temperature for 3 hours. The reaction mixture was poured into ice-cold water and extracted with dichloromethane. The extracted solution was washed with saturated brine, dried over anhydrous magnesium sulfate and then evaporated under reduced pressure to remove the solvent. The residue was purified by silica gel column chromatography (n-hexane/ethyl acetate = 4/1) to give the titled compound (5.9 g).
$^1$H-NMR ($CDCl_3$) δ: 2.20-2.40 (2H, m), 2.95-3.10 (4H, m), 3.17 (2H, t, J=7.0 Hz), 3.56 (2H, t, J=6.4 Hz), 3.65-3.85 (4H, m), 7.20-7.30 (1H, m), 7.75-7.85 (2H, m)

3) 4-(4-Phenyl-1-piperazinyl)-1-[3-(2,2,2-trifluoroacetyl)-2,3,4,5-tetrahydro-1H-3-benzazepin-7-yl]-1-butanone

4-Bromo-1-[3-(2,2,2-trifluoroacetyl)-2,3,4,5-tetrahydro-1H-3-benzazepin-7-yl] -1-butanone (100 mg), 1-phenylpiperazine (0.043 ml), potassium carbonate (35 mg) and N,N-dimethylformamide (DMF; 3 ml) were mixed and stirred at 80°C for 2 hours. The reaction mixture was diluted with water and then extracted with ethyl acetate. The extracted solution was washed with saturated brine, dried over anhydrous magnesium sulfate and then evaporated under reduced pressure to remove the solvent. The residue was purified by silica gel column chromatography (n-hexane/ethyl acetate = 1/3) to give the titled compound (72 mg).
$^1$H-NMR ($CDCl_3$) δ: 1.91-2.05 (2H, m), 2.47 (2H, t, J=6.8 Hz), 2.55-2.65 (4H, m), 2.95-3.05 (6H, m), 3.10-3.20 (4H, m), 3.60-3.80 (4H, m), 6.80-6.95 (3H, m), 7.20-7.30 (3H, m), 7.75-7.85 (2H, m)
MS (APCI+): 474 (M+H)

4) 4-(4-Phenyl-1-piperazinyl)-1-(2,3,4,5-tetrahydro-1H-3-benzazepin-7-yl)-1-butanone trihydrochloride

To a solution of 4-(4-phenyl-1-piperazinyl)-1-[3-(2,2,2-trifluoroacetyl)-2,3,4,5-tetrahydro-1H-3-benzazepin-7-yl]-1-butanone (58 mg) in methanol (1 ml), 1M aqueous potassium carbonate (0.24 ml) was added and stirred at room temperature for 1.5 hours. After distilling off methanol under reduced pressure, the residue was extracted with ethyl acetate. The extracted solution was washed with saturated brine, dried over anhydrous magnesium sulfate and then evaporated under reduced pressure to remove the solvent, thereby giving 4-(4-phenyl-1-piperazinyl)-1-(2,3,4,5-tetrahydro-1H-3-benzazepin-7-yl)-1-butanone. This compound was treated with a 1N ethyl acetate solution of hydrogen chloride to give the compound of interest (22 mg).
$^1$H-NMR (DMSO-$d_6$) δ: 2.00-2.20 (2H, m), 3.10-3.40 (16H, m), 3.50-3.65 (2H, m), 3.70-3.90 (2H, m), 6.87 (1H, t, J=8.0

Hz), 7.00 (2H, d, J=8.0 Hz), 7.27 (2H, t, J=8.0 Hz), 7.38 (1H, d, J=8.4 Hz), 7.80-7.85 (2H, m)
MS (APCI+): 378 (M+H)
The following compounds were prepared in the same manner as shown in Reference Example 22.

## Reference Example 23

4-[4-(1,3-Benzodioxol-5-ylmethyl)-1-piperazinyl]-1-(2,3,4,5-tetrahydro-1H-3-benzazepin-7-yl)-1-butanone trihydrochloride

$^1$H-NMR (DMSO-$d_6$) δ: 2.00-2.15 (2H, m), 3.00-3.20 (12H, m), 3.25-3.80 (10H, m), 6.07 (2H, s), 6.98 (1H,d, J=8.0 Hz), 7.05-7.15 (1H, m), 7.27 (1H, m), 7.37 (1H, d, J=8.0 Hz), 7.75-7.85 (2H, m)
MS (ESI+): 436 (M+H)

## Reference Example 24

4-(4-Benzhydryl-1-piperazinyl)-1-(2,3,4,5-tetrahydro-1H-3-benzazepin-7-yl)-1-butanone

$^1$H-NMR ($CDCl_3$) δ: 1.55 (4H, m), 2.20-2.60 (12H, m), 2.80-2.30 (8H, m), 4.21 (1H, s), 6.85-7.60 (13H, m)
MS (ESI+): 454 (M+H)

## Reference Example 25

4-(4-Benzhydryl-1-piperazinyl)-1-(2,3,4,5-tetrahydro-1H-3-benzazepin-7-yl)-1-butanone trihydrochloride

$^1$H-NMR (DMSO-$d_6$) δ: 1.40-1.80 (4H, m), 3.00-3.40 (12H, m), 3.50-4.00 (9H, m), 7.00-7.80 (13H, m)
MS (ESI+): 454 (M+H)

## Reference Example 26

4-{4-[Bis(4-fluorophenyl)methyl]-1-piperazinyl}-1-(2,3,4,5-tetrahydro-1H-3-benzazepin-7-yl)-1-butanone

$^1$H-NMR ($CDCl_3$) δ: 1.80-2.00 (2H, m), 2.25-2.55 (10H, m), 3.90-4.00 (10H, m), 4.18 (1H, s), 6.90-7.00 (4H, m), 7.15 (1H, d, J=8.2 Hz), 7.25-7.50 (4H, m), 7.50-7.80 (2H, m)
MS (ESI+): 504 (M+H)

## Reference Example 27

4-{4-[Bis(4-fluorophenyl)methyl]-1-piperazinyl}-1-(2,3,4,5-tetrahydro-1H-3-benzazepin-7-yl)-1-butanone trihydrochloride

$^1$H-NMR (DMSO-$d_6$) δ: 1.90-2.15 (2H, m), 2.60-3.80 (21H, m), 7.10-7.30 (4H, m), 7.37 (1H, d, J=8.4 z), 7.40-7.95 (6H, m)
MS (ESI+): 504 (M+H)

## Reference Example 28

4-{4-(4-Chlorobenzyl)-1-piperazinyl}-1-(2,3,4,5-tetrahydro-1H-3-benzazepin-7-yl)-1-butanone

$^1$H-NMR (DMSO-$d_6$) δ: 1.80-2.00 (2H, m), 2.30-2.55 (10H, m), 2.85-3.00 (10H, m), 3.45 (2H, s), 7.10-7.30 (5H, m), 7.65-7.75 (2H, m)
MS (ESI+): 426 (M+H)

## Reference Example 29

4-{4-(4-Chlorobenzyl)-1-piperazinyl}-1-(2,3,4,5-tetrahydro-1H-3-benzazepin-7-yl)-1-butanone trihydrochloride

$^1$H-NMR (DMSO-$d_6$) δ: 1.95-2.10 (2H, m), 3.00-3.95 (20H, m), 4.20-4.40 (2H, m), 7.38 (1H, d, J=8.4Hz), 7.53

(2H, d, J=8.4 Hz), 7.68 (2H, d, J=8.4 Hz), 7.75-7.85 (2H, m)
MS (APCI+): 426 (M+H)

**Reference Example 30**

4-{4-(1-Naphthylmethyl)-1-piperazinyl}-1-(2,3,4,5-tetrahydro-1H-3-benzazepin-7-yl)-1 -butanone

$^1$H-NMR (DMSO-$d_6$) δ: 1.85-2.00 (2H, m), 2.21 (2H, m), 2.35-2.60 (8H, m), 2:80-3.00 (10H, m), 3.88 (2H, s), 7.14-7.19 (1H, m), 7.40-7.55 (4H, m), 7.65-7.90 (4H, m), 8.25-8.35 (1H, m)
MS (APCI+): 442 (M+H)

**Reference Example 31**

4-{4-(1-Naphthylmethyl)-1-piperazinyl}-1-(2,3,4,5-terrahydro-1H-3-benzazepin-7-yl)-1-butanone trihydrochloride

$^1$H-NMR (DMSO-$d_6$) δ: 1.90-2.10 (2H, m), 3.00-4.00 (22H, m), 7.30-7.40 (1H, m), 7.50-7.70 (2H, m), 7.75-8.15 (6H, m), 8.35-8.45 (1H, m)
MS (APCI+): 442 (M+H)

**Reference Example 32**

N-[2-(4-Benzylpiperazin-1-yl)ethyl]-2,3,4,5-tetrahydro-1H-3-benzazepine-7-carboxamide trihydrochloride

1) 1,2,4,5-Tetrahydro-3H-3-benzazepine-3-carboaldehyde

Acetic anhydride (18 ml) was added to formic acid (54 ml) and stirred at room temperature for 1 hour. To this mixture, 2,3,4,5-tetrahydro-1H-3-benzazepine (9.5 g) in ethyl acetate (5 ml) was injected dropwise on ice. After stirring at room temperature for 30 minutes, the solvent was concentrated under reduced pressure. Ethyl acetate and saturated aqueous sodium bicarbonate were added to the residue, which was then extracted with ethyl acetate. The extracted solution was washed with saturated brine and dried over anhydrous magnesium sulfate, followed by concentration of the solvent under reduced pressure to give the titled compound (9.37 g).
$^1$H-NMR ($CDCl_3$) δ: 2.85-3.00 (4H, m), 3.45-3.50 (2H, m), 3.64-3.70 (2H, m), 7.10-7.20 (4H, m), 8.15 (1H, s)

2) 7-Acetyl-1,2,4,5-tetrahydro-3H-3-benzazepine-3-carboaldehyde

To a solution of 1,2,4,5-terrahydro-3H-3-benzazepine-3-carboaldehyde (4.50 g) and acetyl chloride (2.01 ml) in dichloroethane (25 ml), aluminum chloride (12.0 g) was added. The reaction mixture was stirred at room temperature for 15 hours, poured into ice-cold water and then extracted with ethyl acetate. The extracted solution was washed with saturated brine and dried over anhydrous magnesium sulfate, followed by concentration of the solvent under reduced pressure. The residue was purified by silica gel column chromatography (ethyl acetate) to give the titled compound (3.26 g).
$^1$H-NMR ($CDCl_3$) δ: 2.60 (3H, s), 2.90-3.05 (4H, m), 3.45-3.55 (2H, m), 3.65-3.75 (2H, m), 7.20-7.30 (1H, m), 7.50-7.80 (2H, m), 8.16 (1H, s)

3) 3-Formyl-2,3,4,5-tetrahydro-1H-3-benzazepine-7-carboxylic acid

A solution of sodium hydroxide (4.78 g) in water (70 ml) was added to a solution of 7-acetyl-1,2,4,5-tetrahydro-3H-3-benzazepine-3-carboaldehyde (3.24 g) in dioxane (50 ml), followed by dropwise addition of bromine (2.31 ml) on ice. After the reaction mixture was stirred on ice for 30 minutes, acetone was added to stop the reaction. After the solvent was concentrated under reduced pressure, the aqueous layer was extracted with ethyl acetate and 5N hydrochloric acid was added to the extracted solution. Precipitated crystals were collected by filtration and washed sequentially with water and ether to give the titled compound (2.11 g).
$^1$H-NMR (DMSO-$d_6$) δ: 2.85-3.00 (4H, m), 3.45-3.60 (4H, m), 7.32 (1H, dd, J=2.2, 7.6Hz), 7.72-7.80 (2H, m), 8.12 (1H, s)

4)2,3,4,5-Tetrahydro-1H-3-benzazepine-7-carboxylic acid

A solution of 3-formyl-2,3,4,5-terrahydro-1H-3-benzazepine-7-carboxylic acid (1.0 g) in concentrated hydrochloric acid (50 ml) was stirred at 100°C for 12 hours. After the solvent was concentrated under reduced pressure, the

resulting solid was collected by filtration and washed sequentially with water and ether to give the titled compound (990 mg).
$^1$H-NMR ($CDCl_3$) δ: 3.18 (4H, m), 3.46 (4H, m), 7.33 (1H, d, J=7.8Hz), 7.76 (1H, d, J=7.8Hz), 7.78 (1H, s)

5) 3-(tert-Butoxycarbonyl)-2,3,4,5-tetrahydro-1H-3-benzazepine-7-carboxylic acid

2,3,4,5-Tetrahydro-1H-3-benzazepine-7-carboxylic acid (300 mg) was dissolved in 1N aqueous sodium hydroxide (2.64 ml), water (2.5 ml) and tetrahydrofuran (2.5 ml), to which di-tert-butyl dicarbonate (0.33 ml) was then added and stirred at room temperature for 2 hours. After tetrahydrofuran was concentrated under reduced pressure, the aqueous layer was acidified with 5% aqueous potassium bisulfate and extracted with ethyl acetate. The extracted solution was washed with saturated brine and dried over anhydrous magnesium sulfate, followed by concentration of the solvent under reduced pressure to give the titled compound (344 mg).
$^1$H-NMR ($CDCl_3$) δ: 1.49 (9H, s), 2.95-3.00 (4H, m), 3.55-3.60 (4H, m), 7.23 (1H, d, J=8.4Hz), 7.86 (1H, s), 7.89 (1H, d, J=8.4Hz)

6) tert-Butyl 7-({[2-(4-benzylpiperazin-1-yl)ethyl]amino}carbonyl)-1,2,4,5-tetrahydro-3H-3-benzazepine-3-carboxylate

Diethyl cyanophosphate (0.086 ml) was added to a solution of 3-(tert-butoxycarbonyl)-2,3,4,5-tetrahydro-1H-3-benzazepine-7-carboxylic acid (150 mg), 2-(4-benzylpiperazin-1-yl)ethylamine (124 mg) and triethylamine (0.079 ml) in DMF (5 ml). The reaction mixture was stirred at room temperature for 15 hours and then diluted with water. After extraction with ethyl acetate, the extracted solution was washed with saturated brine and dried over anhydrous magnesium sulfate, followed by concentration of the solvent under reduced pressure. The residue was purified by silica gel column chromatography (n-hexane/ethyl acetate = 1/2) to give the titled compound (199 mg).
$^1$H-NMR ($CDCl_3$) δ: 1.49 (9H, s), 2.50-2.65 (8H, m), 2.59 (2H, t, J=6.0Hz), 2.90-3.00 (4H, m), 3.53 (2H, s), 3.45-3.60 (6H, m), 6.81 (1H, m), 7.15-7.35 (6H, m), 7.45-7.60 (2H, m)
MS (ESI+): 493 (M+H)

7) N-[2-(4-Benzylpiperazin-1-yl)ethyl]-2,3,4,5-tetrahydro-1H-3-benzazepine-7-carboxamide trihydrochloride

tert-Butyl 7-({[2-(4-benzylpiperazin-1-yl)ethyl]amino}carbonyl)-1,2,4,5-tetrahydro-3H-3-benzazepine-3-carboxylate (199 mg) was treated with a 1N ethyl acetate solution of hydrogen chloride to give the compound of interest (126 mg).
$^1$H-NMR (DMSO-$d_6$) δ: 3.00-4.00 (20H, m), 4.35 (2H, m), 7.30 (1H, d, J=7.8Hz), 7.40-7.50 (3H, m), 7.60-7.70 (2H, m), 7.70-7.80 (2H, m), 8.84 (1H, m)
MS (ESI+): 393 (M+H)
Compounds of Reference Examples 33 to 39 were prepared in the same manner as shown in Reference Example 32.

## Reference Example 33

N-[2-(4-Benzhydrylpiperazin-1-yl)ethyl]-2,3,4,5-tetrahydro-1H-3-benzazepine-7-carboxamide trihydrochloride

Yield: 238 mg
$^1$H-NMR (DMSO-$d_6$) δ: 3.00-4.00 (21H, m), 7.25-7.40 (8H, m), 7.60-7.90 (5H, m), 8.89 (1H, m)
MS (APCI +): 469 (M+H)

## Reference Example 34

N-[2-[4-(4-chlorobenzyl)piperazin-1-yl]ethyl]-2,3,4,5-tetrahydro-1H-3-benzazepine-7-c arboxamide trihydrochloride

Yield: 198 mg
$^1$H-NMR (DMSO-$d_6$) δ: 3.00-4.00 (20H, m), 4.31 (2H, m), 7.30 (1H, d, J=7.8Hz), 7.45-7.80 (6H, m), 8.85 (1H, m)
MS (APCII+): 427 (M+H)

**Reference Example 35**

N-(2-{4-[Bis(4-fluorophenyl)methyl]piperazin-1-yl}ethyl)-2,3,4,5-tetrahydro-1H-3-benzazepine-7-carboxamide trihydrochloride

Yield: 148 mg
$^1$H-NMR (DMSO-$d_6$) δ: 3.00-3.45 (16H, m), 3.50-3.80 (5H, m), 7.15-7.40 (5H, m), 7.50-8.00 (6H, m), 8.90 (1H, m)
MS (APCI±): 505 (M+H)

**Reference Example 36**

N-[2-(4-Benzylpiperazin-1-yl)ethyl]-2,3,4,5-tetrahydro-1H-2-benzazepine-8-carboxamide trihydrochloride

Yield: 139 mg
$^1$H-NMR (DMSO-$d_6$) δ: 1.80-2.00 (2H, m), 3.00-4.20 (18H, m), 4.37 (2H, m), 7.30-7.80 (6H, m), 7.80-8.05 (2H, m), 8.95 (1H, m)
MS (ESI+): 393 (M+H)

**Reference Example 37**

N-[2-(4-Benzhydrylpiperazin-1-yl)ethyl]-2,3,4,5-tetrahydro-1H-2-benzazepine-8-carboxamide trihydrochloride

Yield: 201 mg
$^1$H-NMR (DMSO-$d_6$) δ: 1.75-1.95 (2H, m), 2.95-4.20 (18H, m), 4.35 (1H, s), 7.30-7.45 (7H, m), 7.60-8.00 (6H, m), 8.97 (1H, m)
MS (ESI +): 469 (M+H)

**Reference Example 38**

N-[2-[4-(4-Chlorobenzyl)piperazin-1-yl]ethyl]-2,3,4,5-tetrahydro-1H-2-benzazepine-8-carboxamide trihydrochloride

Yield: 205 mg
$^1$H-NMR (DMSO-$d_6$) δ: 1.80-2.00 (2H, m), 3.00-4.00 (18H, m), 4.36 (2H, s), 7.36 (1H, d, J=8.0Hz), 7.52 (1H, d, J=8.4Hz), 7.69 (1H, d, J=8.4Hz), 7.89 (1H, d, J=8.0Hz), 8.00 (1H, s), 8.94 (1H, m)
MS (ESI+): 427 (M+H)

**Reference Example 39**

N-(2-{4-[Bis(4-fluorophenyl)methyl]piperazin-1-yl}ethyl)-2,3,4,5-tetrahydro-1H-2-benzazepine-8-carboxamide trihydrochloride

Yield: 325 mg
$^1$H-NMR (DMSO-$d_6$) δ: 1.80-2.00 (2H, m), 3.00-4.50 (19H, m), 7.20-7.40 (5H, m),
7.60-8.10 (5H, m), 8.97 (1H, m)
MS (ESI+): 505 (M+H)

**Reference Example 40**

2-Benzyl-N-(2-{4-[bis(4-fluorophenyl)methyl]piperazin-1-yl}ethyl)-2,3,4,5-tetrahydro-1H-2-benzazepine-8-carboxamide trihydrochloride

2,3,4,5-Tetrahydro-1H-2-benzazepine-8-carboxylic acid (200 mg), synthesized in the same manner as shown in Reference Example 32, Steps 1) to 4), was mixed with benzyl bromide (0.23 ml) and potassium carbonate (267 mg) in DMF (10 ml), stirred at room temperature for 24 hours and then diluted with water. The aqueous layer was washed with ethyl acetate, acidified with 1N hydrochloric acid and then extracted with dichloromethane. The extracted solution was washed with saturated brine and dried over anhydrous magnesium sulfate, followed by concentration of the solvent under reduced pressure to give 2-benzyl-2,3,4,5-tetrahydro-1H-2-benzazepine-8-carboxylic acid (89 mg). This compound was treated in the same manner as shown in Reference Example 32, Steps 6) to 7) to synthesize the titled

compound (104 mg).
$^1H$-NMR (DMSO-$d_6$) δ: 1.85-2.05 (2H, m), 3.00-4.70 (21H, m), 7.23 (4H, m), 7.35-7.50 (4H, m), 7.60-7.80 (6H, m), 7.90-8.00 (2H, m), 8.97 (1H, m)
MS (ESI+): 595 (M+H)

**Reference Example 41**

N-[2-(4-Benzhydrylpiperazin-1-yl)ethyl]-N-benzyl-2,3,4,5-tetrahydro-1H-3-benzazepine-7-carboxamide trihydrochloride

A mixture of 2-(4-benzhydrylpiperazin-1-yl)ethylamine (275 mg), benzaldehyde (0.15 ml), molecular sieves (1 g) and methanol (5 ml) was stirred at room temperature for 2 hours. After the molecular sieves were filtered off, the filtrate was concentrated under reduced pressure. The resulting residue was dissolved in methanol-THF (3:2; 5 ml), to which sodium tetrahydroborate (56 mg) was then added and stirred at room temperature for 17 hours. The solvent was concentrated under reduced pressure and brine was added to the residue. After extraction with ethyl acetate, the extracted solution was washed with saturated brine and then dried over anhydrous magnesium sulfate. The solvent was concentrated under reduced pressure to give N-[2-(4-benzhydrylpiperazin-1-yl)ethyl]-N-benzylamine (245 mg). This compound was treated in the same manner as shown in Reference Example 32, Steps 6) to 7) to synthesize the titled compound (154 mg).
$^1H$-NMR (DMSO-$d_6$) δ: 2.90-4.00 (21H, m), 4.58 (2H, m), 7.10-7.50 (12H, m), 7.50-7.90 (3H, m),
MS (ESI+): 559(M+H)

**Reference Example 42**

N-Benzyl-N-{2-[4-(4-chlorobenzyl)piperazin-1-yl]ethyl}-2,3,4,5-terrahydro-1H-3-benzazepine-7-carboxamide trihydrochloride

This compound was prepared in the same manner as shown in Reference Example 41.
$^1H$-NMR (DMSO-$d_6$) δ: 3.00-3.80 (20H, m), 4.37 (2H, m), 4.59(2H, m), 7.10-7.50 (5H, m), 7.53 (2H, d, J=8.0Hz), 7.70 (2H, d, J=8.0Hz)
MS (ESI+): 517(M+H)

**Reference Example 43**

3-(4-Benzylpiperazin-1-yl)-N-(2,3,4,5-tetrahydro-1H-3-benzazepin-7-yl)propionamide trihydrochloride

1) 7-Nitro-3-(trifluoroacetyl)-2,3,4,5-tetrahydro-1H-3-benzazepine

To a solution of 3-(trifluoroacetyl)-2,3,4,5-tetrahydro-1H-3-benzazepine (500 mg) in sulfuric acid (3 ml), potassium nitrate (229 mg) was added on ice. After stirring on ice for 3 hours, the reaction mixture was poured into ice-cold water and extracted with ethyl acetate. The extracted solution was washed with saturated aqueous sodium bicarbonate and saturated brine and then dried over anhydrous magnesium sulfate, followed by concentration of the solvent under reduced pressure. The residue was purified by silica gel column chromatography (n-hexane/ethyl acetate = 4/1) to give the titled compound (295 mg).
$^1H$-NMR ($CDCl_3$) δ: 3.05-3.15 (4H, m), 3.70-3.86 (4H, m), 7.30-7.38 (1H, m), 8.02-8.10 (2H, m)
MS (APCI-): 287(M-H)

2) 3-(Trifluoroacetyl)- 2,3,4,5-tetrahydro-1H-3-benzazepine-7-amine

A mixture of 7-nitro-3-(trifluoroacetyl)-2,3,4,5-tetrahydro-1H-3-benzazepine (100 mg), tin(II) chloride dihydrate (391 mg) and DMF (2 ml) was stirred at room temperature for 5 hours. The reaction mixture was diluted with water and extracted with ethyl acetate. The extracted solution was washed with saturated aqueous sodium bicarbonate and saturated brine and then dried over anhydrous magnesium sulfate, followed by concentration of the solvent under reduced pressure to give the titled compound (85 mg).
$^1H$-NMR ($CDCl_3$) δ: 2.80-3.00 (4H, m), 3.60-3.80 (6H, m), 6.45-6.52 (2H, m), 6.85-6.98 (1H, m)
MS (APCI+): 259(M+H)

3) 3-(4-Benzylpiperazin-1-yl)-N-[3-(trifluoroacetyl)-2,3,4,5-tetrahydro-1H-3-benzazepin-7-yl]propionamide

Diethyl cyanophosphate (0.050 ml) was added to a solution of 3-(trifluoroacetyl)-2,3,4,5-tetrahydro-1H-3-benzazepine-7-amine (77 mg), 3-(4-benzylpiperazin-1-yl)propionic acid (105 mg) and triethylamine (0.137 ml) in DMF (3 ml). The reaction mixture was stirred at room temperature for 15 hours and then diluted with water. After extraction with ethyl acetate, the extracted solution was washed with saturated brine and dried over anhydrous magnesium sulfate, followed by concentration of the solvent under reduced pressure. The residue was purified by silica gel column chromatography (n-hexane/ethyl acetate = 2/3) to give the titled compound (71 mg).
$^1$H-NMR ($CDCl_3$) δ: 2.40-2.80 (12H, m), 2.90-3.00 (4H, m), 3.95 (2H, s), 3.65-3.85 (4H, m), 7.00-7.50 (8H, m)
MS (APCI+): 489 (M+H)

4) 3-(4-Benzylpiperazin-1-yl)-N-(2,3,4,5-terrahydro-1H-3-benzazepin-7-yl)-propionamide

To a solution of 3-(4-benzylpiperazin-1-yl)-N-[3-(trifluoroacetyl)-2,3,4,5-tetrahydro-1H-3-benzazepin-7-yl]propionamide (64 mg) in methanol (1 ml), 1M aqueous potassium carbonate (0.39 ml) was added and stirred at room temperature for 1.5 hours. After distilling off methanol under reduced pressure, the residue was extracted with ethyl acetate. The extracted solution was washed with saturated brine, dried over anhydrous magnesium sulfate and then evaporated under reduced pressure to remove the solvent, thereby giving the titled compound (31 mg).
$^1$H-NMR ($CDCl_3$) δ: 2.35-2.80 (12H, m), 2.85-3.00 (8H, m), 3.51 (2H, s), 7.03 (1H, d, J=8.0Hz), 7.15-7.35 (7H, m)
MS (APCI+): 393 (M+H)

5) 3-(4-benzylpiperazin-1-yl)-N-(2,3,4,5-tetrahydro-1H-3-benzazepin-7-yl)-propionamide trihydrochloride

3-(4-Benzylpiperazin-1-yl)-N-(2,3,4,5-tetrahydro-1H-3-benzazepin-7-yl)-propionamide (27 mg) was treated with a 1N ethyl acetate solution of hydrogen chloride to give the compound of interest (4.0 mg).
MS (APCI+): 393 (M+H)
Compounds of Reference Examples 44 and 45 were prepared in the same manner as shown in Reference Example 43.

## Reference Example 44

3-(4-Benzhydrylpiperazin-1-yl)-N-(2,3,4,5-tetrahydro-1H-3-benzazepin-7-yl)-propionamide trihydrochloride

Yield: 24 mg
$^1$H-NMR (DMSO-$d_6$) δ: 2.80-3.80 (21H, m), 7.10-7.70 (13H, m), 10.30 (1H, m)
MS (ESI+): 469 (M+H)

## Reference Example 45

3-[4-(4-Chlorobenzyl)piperazin-1-yl]-N-(2,3,4,5-tetrahydro-1H-3-benzazepin-7-yl)-propionamide trihydrochloride

Yield: 73 mg
$^1$H-NMR (DMSO-$d_6$) δ: 2.80-4.00 (20H, m), 4.33 (2H, m), 7.12 (1H, d, J=8.0Hz),
7.35-7.60 (4H, m), 7.60-7.75 (2H, m), 10.36 (1H, m)
MS (ESI+): 427 (M+H)

## Reference Example 46

3'-{({2-[4-(Aminosulfonyl)phenyl]ethyl}[(E)-3-phenyl-2-propenoyl]amino)methyl}-N-[ 2-(1-pyrrolidinyl)ethyl][1,1'-biphenyl]-3-carboxamide

1) 3-Bromo-N-[2-(1-pyrrolidinyl)ethyl]phenylcarboxamide

To a solution of 3-bromobenzoic acid (5.00 g) in N,N-dimethylformamide (DMF; 60 ml), 1-(2-aminoethyl)pyrrolidine (4.34 g), diethyl cyanophosphate (5.57 ml) and triethylamine (10.4 ml) were added and stirred at room temperature for 16 hours. The reaction mixture was diluted with water and then extracted with diethyl ether. The extracted solution was dried over anhydrous magnesium sulfate and then evaporated under reduced pressure to remove the solvent. The residue was crystallized by addition of hexane to give the titled compound (6.31 g).

$^1$H-NMR ($CDCl_3$) δ: 1.70-1.90 (4H, m), 2.50-2.60 (4H, m), 2.70 (2H, t, J=6.0 Hz), 3.45-3.60 (2H, m), 6.86 (1H, s), 7.30 (1H, t, J=8.0 Hz), 7.60 (1H, dm, J=8.0 Hz), 7.70 (1H, dm, 8.0 Hz), 7.93 (1H, t, J=1.6 Hz).

2) 3'-Formyl-N-[2-(1-pyrrolidinyl)ethyl][1,1'-biphenyl]-3-carboxamide

To a solution of 3-bromo-N-[2-(1-pyrrolidinyl)ethyl]phenylcarboxamide (6.31 g) in toluene (50 ml), palladium tetrakistriphenylphosphine (735 mg) and 2M aqueous sodium carbonate (21.2 ml) were added and a solution of 3-formylboronic acid (3.49 g) in ethanol (15 ml) was further added, followed by stirring at 90°C for 15 hours. The reaction mixture was diluted with water and then extracted with diethyl ether. The extracted solution was washed with saturated brine and then dried over anhydrous magnesium sulfate. The solvent was distilled off under reduced pressure to give the titled compound (6.83 g).
$^1$H-NMR ($CDCl_3$) δ: 1.95-2.35 (4H, m), 2.95 (2H, m), 3.30-3.50 (2H, m), 3.80-3.40 (4H, m), 7.40-7.60 (2H, m), 7.76 (1H, dm, J=8.0Hz), 7.85 (1H, dm, J=8.0Hz), 8.00 (1H, dm, 8.0Hz), 8.09 (1H, dm, J=8.0Hz), 8.25 (1H, bs), 8.40 (1H, bs), 8.41 (1H, m), 10.10 (1H, s).

3) 3'-[{2-[4-(Aminosulfonyl)phenyl]ethyl}aminomethyl]-N-[2-(1-pyrrolidinyl)ethyl]-[1,1'-biphenyl]-3-carboxamide

To a solution of 3'-formyl-N-[2-(1-pyrrolidinyl)ethyl][1,1'-biphenyl]-3-carboxamide (3.81 g) in methanol (50 ml), 4-(2-aminoethyl)benzenesulfonamide (2.37 g) and Molecular Sieves 3A (4.0 g) were added and then stirred at room temperature for 1.5 hours. After the reaction mixture was diluted with tetrahydrofuran (THF), the molecular sieves were filtered off and the filtrate was concentrated under reduced pressure. The residue was dissolved in a mixed methanol/THF solvent (1:1; 100 ml), to which sodium borohydride (0.89 g) was then added. After stirring at room temperature for 5 hours, the reaction mixture was evaporated under reduced pressure to remove the solvent. The residue was diluted with water and then extracted with ethyl acetate. The extracted solution was washed with saturated brine, dried over anhydrous magnesium sulfate and then evaporated under reduced pressure to remove the solvent. The residue was crystallized by addition of hexane to give the compound of interest (3.71 g).
$^1$H-NMR ($CDCl_3$) δ: 1.75-1.85 (4H, m), 2.55-2.65 (4H, m), 2.78 (2H, t, J=6.0Hz), 2.85-3.00 (4H, m), 3.60-3.65 (2H, m), 3.87 (2H, s), 7.05-7.15 (1H, m), 7.20-7.60 (6H, m), 7.65-7.85 3H, m), 7.84 (2H, d, J=8.4Hz), 8.05 (1H, s).

4) 3'-{({2-[4-(aminosulfonyl)phenyl]ethyl}[(E)-3-phenyl-2-propenoyl]amino)methyl}-N-[2-(1-pyrrolidinyl)ethyl][1,1'-biphenyl]-3-carboxamide

3'-[{2-[4-(Aminosulfonyl)phenyl]ethyl}aminomethyl]-N-[2-(1-pyrrolidinyl)-ethyl]-[1,1'-biphenyl]-3-carboxamide (506 mg), *trans*-cinnamic acid (163 mg), 1-ethyl-3-(3-dimethylaminopropyl)carbodiimide hydrochloride (EDCI·HCl; 211 mg) and 1-hydroxybenzotriazole (HOBT; 149 mg) were dissolved in a mixed solvent of dichloromethane (15 ml) and DMF (7 ml) and then stirred at room temperature for 18 hours. After distilling off the solvent under reduced pressure, water was added to the residue, which was then extracted with ethyl acetate. The extracted solution was washed with saturated brine, dried over anhydrous magnesium sulfate and then evaporated under reduced pressure to remove the solvent. The residue was purified by silica gel column chromatography (dichloromethane/methanol = 98/2) to give the compound of interest (284 mg).
$^1$H-NMR ($CDCl_3$) δ: 1.73 (4H, m), 2.52 (4H, m), 2.69 (2H, t, J=6.0Hz), 2.85-3.00 (2H, m), 3.50-3.60 (2H, m), 3.66 (2H, t, J=7.0Hz), 4.60 (2H,s), 6.57 (1H, d, J=15.6Hz), 6.85 (1H, d, J=15.6Hz), 7.10-7.90 (16H, m), 8.05 (1H, s).
MS (APCI+): 637 (M+H)

## Reference Example 47

3'-{({2-[4-(Aminosulfonyl)phenyl]ethyl}[(E)-3-phenyl-2-propenoyl]amino)methyl}-N-[ 2-(1-pyrrolidinyl)ethyl][1,1'-biphenyl]-3-carboxamide hydrochloride

3'-{({2-[4-(Aminosulfonyl)phenyl]ethyl}[(E)-3-phenyl-2-propenoyl]amino)-methyl}-N-[2-(1-pyrrolidinyl)ethyl][1,1'-biphenyl]-3-carboxamide (200 mg) was treated with a 4N ethyl acetate solution of hydrogen chloride to give the compound of interest (198 mg).
$^1$H-NMR (DMSO-$d_6$) δ: 1.80-2.10 (4H, m), 2.90-3.10 (4H, m), 3.30-3.50 (2H, m), 3.55-3.90 (6H, m), 4.73 (2H,s), 7.05-8.00 (18H, m), 8.25 (1H, s), 9.03 (1H, m).
Elementary Analysis (molecular formula $C_{37}H_{40}N_4O_4S \cdot HCl \cdot 1.5H_2O$):
Calculated: C: 63.46; H: 6.33; N: 8.00; C1: 5.08
Found: C: 63.65; H: 6.51; N: 7.86; C1: 5.25

**Reference Example 48**

3'-{({2-[4-(Aminosulfonyl)phenyl]ethyl}[4-phenylbutanoyl]amino)methyl}-N-[2-(1-pyrrolidinyl)ethyl][1,1'-biphenyl]-3-carboxamide

The same procedure as shown in Reference Example 46 was repeated to give the compound of interest (277 mg).
$^1$H-NMR (DMSO-$d_6$) δ: 1.75-1.85 (8H, m), 2.20-2.40 (2H, m), 2.45-2.60 (2H, m), 2.60-2.95 (4H, m), 3.20-3.60 (6H, m), 4.62 (2H,s), 7.05-7.95 (18H, m), 8.13 (1H, s), 8.71 (1H, m).
MS (ESI+): 653 (M+H)

**Reference Example 49**

3'-{({2-[4-(Aminosulfonyl)phenyl]ethyl}[4-phenylbutanoyl]amino)methyl}-N-[2-(1-pyrrolidinyl)ethyl][1,1'-biphenyl]-3-carboxamide hydrochloride

The same procedure as shown in Reference Example 47 was repeated to give the compound of interest (185 mg).
$^1$H-NMR (DMSO-$d_6$) δ: 1.75-2.10 (8H, m), 2.25-2.45 (2H, m), 2.45-2.60 (2H, m), 2.80-2.90 (2H, m), 2.95-3.10 (2H, m), 3.20-3.50 (2H, m), 3.50-3.75 (4H, m), 4.61 (2H,s), 7.05-8.00 (18H, m), 8.23 (1H, s), 9.02 (1H, m).
Elementary Analysis (molecular formula $C_{38}H_{44}N_4O_4S \cdot HCl \cdot H_2O$):
Calculated: C: 64.53; H: 6.70; N: 7.92; C1: 5.01
Found: C: 64.39; H: 6.82; N: 7.86; C1: 5.20

**Reference Example 50**

3'-{({2-[4-(Aminosulfonyl)phenyl]ethyl}[(benzyloxy)acetyl]amino)methyl}-N-[2-(1-pyrrolidinyl)ethyl][1,1'-biphenyl]-3-carboxamide

To a solution of 3'-[{2-[4-(aminosulfonyl)phenyl]ethyl}aminomethyl]-N-[2-(1-pyrrolidinyl)ethyl]-[1,1'-biphenyl]-3-carboxamide (506 mg) in DMF (10 ml), pyridine (0.16 ml) and benzyloxyacetyl chloride (0.16 ml) were added. After stirring at room temperature for 16 hours, the reaction mixture was diluted with water and extracted with ethyl acetate. The extracted solution was washed with saturated brine, dried over anhydrous magnesium sulfate and then evaporated under reduced pressure to remove the solvent. The residue was purified by silica gel column chromatography (dichloromethane/methanol = 98/2) to give the compound of interest (257 mg).
$^1$H-NMR ($CDCl_3$) δ: 1.74 (4H, m), 2.60-2.80 (4H, m), 2.88 (2H, m), 3.20-3.40 (8H, m), 4.17 (2H,s), 4.47 (2H, s), 4.62 (2H, s), 6.57 (1H, d, J=15.6Hz), 7.20-7.90 (18H, m), 8.11 (1H, s), 8.65 (1H, m).
MS (ESI+): 655 (M+H)

**Reference Example 51**

3'-{({2-[4-(Aminosulfonyl)phenyl]ethyl}[(benzyloxy)acetyl]amino)methyl}-N-[2-(1-pyrrolidinyl)ethyl][1,1'-biphenyl]-3-carboxamide hydrochloride

The same procedure as shown in Reference Example 47 was repeated to give the compound of interest (155 mg).
$^1$H-NMR (DMSO-$d_6$) δ: 1.80-2.10 (4H, m), 2.80-3.15 (6H, m), 3.20-3.50 (2H, m), 3.60-3.75 (4H, m), 4.19 (2H, s), 4.48 (2H, s), 4.62 (2H, s), 7.20-7.90 (18H, m), 8.11 (1H, s), 8.65 (1H, m).
Elementary Analysis (molecular formula $C_{37}H_{42}N_4O_5S \cdot HCl \cdot 1.5H_2O$):
Calculated: C: 61.87; H: 6.45; N: 7.80; C1: 4.94
Found: C: 61.76; H: 6.31; N: 7.73; C1: 5.25

**Example 1**

Locomotion measurement

Wistar male rats (9 weeks old) were anesthetized with pentobarbital and a guide cannula (AG-8, Eicom) was inserted into the lateral ventricle of each rat (AP: +8.1 mm, L: 1.8 mm, H: +7.1 mm). The rats were then allowed to

recover for at least one week before the experiment. During the recovery period, handling was made every day to reduce stresses resulting from intraventricular administration.

On the day before the experiment, the rats were transferred to a locomotion testing system for acclimation purposes. Under unanesthetized and unrestrained conditions, a microinjection cannula was attached to the guide cannula through which each rat was administered with the polypeptide (SEQ ID NO: 9; 10 nmol) dissolved in phosphate buffered saline (PBS) or PBS alone at a rate of 5 μl/min for 2 minutes. Immediately after the administration, the rats were returned to the locomotion testing system (Supermex, Muromachi Kikai Co., Ltd.) and tested for their spontaneous locomotion and the number of rearings (Figure 1).

When the rats are acclimatized in the locomotion testing system before intraventricular administration, they will show a transient increase in their spontaneous locomotion under the stimulus of handling during the administration, but in turn they will show a rapid decrease in their spontaneous locomotion. Under such conditions, the rats administered with the polypeptide shown in SEQ ID NO: 9 (10 nmol) tended to decrease their spontaneous locomotion immediately after the administration, as compared with the control rats, and then they showed an increase in their locomotion. The same effects as found in spontaneous locomotion were observed for the number of rearings.

## Example 2

Locomotion measurement

Wistar male rats (9 weeks old) were anesthetized with pentobarbital and a guide cannula (AG-8, Eicom) was inserted into the lateral ventricle of each rat (AP: 8.1 mm, L: 1.8 mm, H: 7.1 mm). The rats were then allowed to recover for at least one week before the experiment. During the recovery period, handling was made every day to reduce stresses resulting from intraventricular administration.

On the day before the experiment, the rats were transferred to a locomotion testing system for acclimation purposes. Under unanesthetized and unrestrained conditions, a microinjection cannula was attached to the guide cannula through which each rat was administered with the polypeptide (SEQ ID NO: 9; 1 nmol) dissolved in PBS or PBS alone in a volume of 10 μl (5 μl/min for 2 minutes). Immediately after the administration, the rats were returned to the locomotion testing system (Supermex, Muromachi Kikai Co., Ltd.) and tested for their spontaneous locomotion and the number of rearings (Figure 2).

The rats administered with the polypeptide shown in SEQ ID NO: 9 (1 nmol) showed an increase in their locomotion from immediately after the administration. However, they did not show such an inhibition against a transient increase in locomotion as observed in 10 nmol administration. This indicated that the polypeptide shown in SEQ ID NO: 9 exhibited an inhibitory effect on locomotion at high dose.

## Example 3

Locomotion measurement

Each of Wistar male rats (8 weeks old) was anesthetized with pentobarbital and fixed on a stereotaxic apparatus. A guide cannula (AG-8, Eicom) was inserted into the lateral ventricle of each rat (AP: +8.1 mm, L: 1.8 mm, H: +7.1 mm). The rats were then allowed to recover for at least one week before the experiment. During the recovery period, handling was made every day to reduce stresses resulting from intraventricular administration.

The cannulated rats were transferred to a locomotion testing system (Supermex, Muromachi Kikai Co., Ltd.) and acclimatized overnight. The rats were transferred from the locomotion testing system and intraventricularly administered with the polypeptide (SEQ ID NO: 9; 1 nmol or 10 nmol) dissolved in phosphate buffered saline (PBS) or PBS alone at a flow rate of 5 μl/min for 4 minutes. Immediately after the administration, the rats were returned to the locomotion testing system and tested for their spontaneous locomotion and the number of rearings.

The rats intraventricularly administered with 10 nmol of the polypeptide (SEQ ID NO: 9) showed decreases in both locomotion and the number of rearings immediately after the administration (Figure 3). These changes in behavior were continued for about 10 minutes. In addition, this transient decrease in locomotion was followed by an increase in locomotion (Figure 3). The increase in locomotion was continued for about 1 hour. There was a significant increase in cumulative locomotion over 90 minutes after the administration (PBS: 1612.7 ± 130.4 counts/90 min, n=27; polypeptide (SEQ ID NO: 9) 10 nmol: 2759.3* ± 422.5 counts/90 min, n=10, *$p < 0.05$, Dunnett). The number of rearings showed a less significant tendency to increase (PBS: 35.1 ± 3.8 times/90 min, polypeptide (SEQ ID NO: 9) 10 nmol: 51.0 ± 13.4 times/90 min).

In the rats administered with 1 nmol of the polypeptide (SEQ ID NO: 9), the decrease in locomotion observed immediately after 10 nmol administration disappeared and, instead, there were observed increases in both locomotion and the number of rearings from immediately after the administration (Figure 3). There was a significant increase in

cumulative locomotion over 90 minutes immediately after the administration (PBS: 1612.7 ± 130.4 counts/90 min, polypeptide (SEQ ID NO: 9) 1 nmol: 3741.0** ± 378.5 counts/90 min, n=9, **p < 0.01, Dunnett). There was also a significant increase in the number of rearings (PBS: 35.1 ± 3.8 times/90 min, polypeptide (SEQ ID NO: 9) 1 nmol: 75.3** ± 12.5 times/90 min, **p < 0.01, Dunnett).

**Example 4**

Locomotion measurement

Each of Wistar male rats (8 weeks old) was anesthetized with pentobarbital and fixed on a stereotaxic apparatus. A guide cannula (AG-8, Eicom) was inserted into the lateral ventricle of each rat (AP: +8.1 mm, L: 1.8 mm, H: +7.1 mm). The rats were then allowed to recover for at least one week before the experiment. During the recovery period, handling was made every day to reduce stresses resulting from intraventricular administration.

The cannulated rats were transferred to a locomotion testing system (Supermex, Muromachi Kikai Co., Ltd.) and acclimatized overnight. The rats were transferred from the locomotion testing system and subcutaneously administered with diazepam (1 mg/kg). An hour later, the rats were intraventricularly administered with the polypeptide (SEQ ID NO: 9; 10 nmol) or PACAP38 (3 nmol) dissolved in phosphate buffered saline (PBS) at a flow rate of 2.5 µl/min for 4 minutes. Immediately after the administration, the rats were returned to the locomotion testing system and tested for their spontaneous locomotion (Figure 4).

Diazepam significantly inhibits the increase in locomotion induced by the polypeptide (SEQ ID NO: 9), whereas it dose not affect the increase in locomotion induced by PACAP38 known to have the ability to increase locomotion (Figure 4). These results suggest that the polypeptide (SEQ ID NO: 9) may cause stress enhancement.

**Example 5**

Elevated plus maze test

Wistar male rats (9 weeks old) were anesthetized with pentobarbital and a guide cannula was inserted into the lateral ventricle of each rat. The rats were then allowed to recover for at least one week before the experiment. The polypeptide shown in SEQ ID NO: 9 (10 nmol) or PBS was administered into the lateral ventricle of each rat. Thirty minutes after the administration, each rat was placed on an elevated plus maze [an acrylic maze having 4 arms (25 cm long, 8 cm wide) placed in the shape of a cross, of which two opposite arms were enclosed by walls (closed arms) and the other two arms were wall-less (open arms); see Figure 5] and tested over 5 minutes for the number of entries into open and closed arms and the time spent on open arms. The results are shown in Figure 6.

**Example 6**

Elevated plus maze test

An acrylic maze having 4 arms (25 cm long, 8 cm wide) placed in the shape of a cross (elevated plus maze) was positioned in a sound-proof chamber under low illumination (0.5 lux). Two opposite arms of the elevated plus maze were enclosed by walls (closed arms) and the other two arms were wall-less (open arms). The maze was elevated 25 cm above the floor (Figure 5).

After C57BL/6N mice were acclimatized in the laboratory, PBS containing the polypeptide (SEQ ID NO: 9; 1 nmol or 3 nmol) or PBS alone was administered in a volume of 5 µl into the lateral ventricle of each mouse using a double-needle (Matsumoto Seisakusho) under ether anesthesia. Thirty minutes after the administration, each mouse was placed on the elevated plus maze and tested over 5 minutes for the number of entries into each arm and the time spent on open arms. The results are shown in Table 1.

Table 1

| Polypeptide (SEQ ID NO: 9) (nmol) | (Open arm entries)/(open arm entries + closed arm entries) | n (animals) |
|---|---|---|
| 0 | 0.134 ± 0.021 | 20 |
| 1 | 0.131 ± 0.009 | 20 |
| 3 | 4.065 ± 0.018* | 20 |

*p<0.05, Dunnett

The administration of the polypeptide (SEQ ID NO: 9) reduced the probability to select open arms.

## Example 7

Hole board test

A polyvinyl chloride box having 4 holes (diameter: 3.8 cm) at its bottom was used as a hole board. Locomotion on the hole board was measured with a Supermex sensor (Muromachi Kikai Co., Ltd.). To measure the number of head dippings, MRS-110RX infrared-scanning sensors (Muromachi Kikai Co., Ltd.) were provided immediately below the respective holes.

After C57BL/6N mice were acclimatized in the laboratory, PBS containing the polypeptide (SEQ ID NO: 9; 0.1 nmol, 0.3 nmol or 3 nmol) or PBS alone was administered in a volume of 5 μl into the lateral ventricle of each mouse using a double-needle (Matsumoto Seisakusho) under ether anesthesia. Thirty minutes after the administration, each mouse was placed in the center of the hole board and tested over 5 minutes for locomotion and the number of head dippings. The results are shown in Figure 7.

The administration of the polypeptide (SEQ ID NO: 9) caused no change in locomotion, but provided a significant decrease in the number of head dippings. These results suggest that the polypeptide (SEQ ID NO: 9) may contribute to anxiety in the brain.

## Example 8

Locomotion measurement

Each of Wistar male rats (8 weeks old) was anesthetized with pentobarbital and fixed on a stereotaxic apparatus. A guide cannula (AG-8, Eicom) was inserted into the lateral ventricle of each rat (AP: +8.1 mm, L: 1.8 mm, H: +7.1 mm). The rats were then allowed to recover for at least one week before the experiment. During the recovery period, handling was made every day to reduce stresses resulting from intraventricular administration. The rats were intraventricularly administered with the polypeptide (SEQ ID NO: 9; 10 nmol) dissolved in phosphate buffered saline (PBS), CRF known to have the ability to induce anxiety and stresses (1 nmol in PBS), or PBS alone at a flow rate of 2.5 μl/min for 4 minutes. Fifteen minutes after the administration, the rats were decapitated and blood was collected. The level of plasma ACTH was assayed using a radioimmunoassay system (Yuka Medias Co., Ltd.). The results are shown in Figure 8.

## INDUSTRIAL APPLICABILITY

The DNA encoding the polypeptide of the present invention, the polypeptide of the present invention or a precursor protein thereof can be used for development of medicaments including an anti-attention-deficit-disorder or anti-narcolepsy agent or an anti-anxiety, anti-depression, anti-insomnia, anti-schizophrenia or anti-fear agent, development of a receptor-binding assay system using a recombinant receptor protein expression system, screening of candidate compounds for medicaments, gene therapy, etc.

Since the polypeptide of the present invention or a precursor protein thereof is associated with an effect resembling anxiety stimulation, compounds capable of changing the binding property between SENR and the polypeptide of the present invention, which are obtained by a screening method using the polypeptide, are useful as medicaments. A SENR agonist can be used, for example, as a therapeutic and/or prophylactic agent for attention deficit disorder or narcolepsy, whereas a SENR antagonist can be used, for example, as a therapeutic and/or prophylactic agent for anxiety, depression, insomnia, schizophrenia or fear.

Sequence Listing Free Text

SEQ ID NO: 1

Other information: The 6th cysteine residue binds with the 11th cysteine residue to form an intramolecular disulfide bond.

SEQ ID NO: 2

Other information: The 6th cysteine residue binds with the 11th cysteine residue to form an intramolecular disulfide bond.

SEQ ID NO: 9

Other information: The 6th cysteine residue binds with the 11th cysteine residue to form an intramolecular disulfide bond.

SEQ ID NO: 10

Other information: The 5th cysteine residue binds with the 10th cysteine residue to form an intramolecular disulfide bond.

SEQ ID NO: 18

Other information: The 11th cysteine residue binds with the 16th cysteine residue to form an intramolecular disulfide bond.

SEQ ID NO: 19

Other information: The 8th cysteine residue binds with the 13th cysteine residue to form an intramolecular disulfide bond.

SEQ ID NO: 24

Other information: The 11th cysteine residue binds with the 16th cysteine residue to form an intramolecular disulfide bond.

SEQ ID NO: 26

Other information: The 14th cysteine residue binds with the 19th cysteine residue to form an intramolecular disulfide bond.

SEQ ID NO: 27

Other information: The 18th cysteine residue binds with the 23rd cysteine residue to form an intramolecular disulfide bond.

SEQ ID NO: 28

Other information: The 14th cysteine residue binds with the 19th cysteine residue to form an intramolecular disulfide bond.

SEQ ID NO: 29

Other information: The 18th cysteine residue binds with the 23rd cysteine residue to form an intramolecular disulfide bond.

## Claims

1. An anti-attention-deficit-disorder or anti-narcolepsy agent, which comprises a polypeptide containing the same or substantially the same amino acid sequence as shown in SEQ ID NO: 1 or an amide or ester of the polypeptide or a salt thereof.

2. The anti-attention-deficit-disorder or anti-narcolepsy agent of claim 1, wherein substantially the same amino acid sequence is the amino acid sequence shown in SEQ ID NO: 2, SEQ ID NO: 9, SEQ ID NO: 10, SEQ ID NO: 18, SEQ ID NO: 19, SEQ ID NO: 24, SEQ ID NO: 26, SEQ ID NO: 27, SEQ ID NO: 28 or SEQ ID NO: 29.

3. An anti-attention-deficit-disorder or anti-narcolepsy agent, which comprises a DNA containing the nucleotide sequence encoding the polypeptide of claim 1.

4. The anti-attention-deficit-disorder or anti-narcolepsy agent of claim 3, wherein the DNA contains the nucleotide sequence shown in SEQ ID NO: 12, SEQ ID NO: 13, SEQ ID NO: 34, SEQ ID NO: 20, SEQ ID NO: 21, SEQ ID NO: 25, SEQ ID NO: 30, SEQ ID NO: 31, SEQ ID NO: 32 or SEQ ID NO: 33.

5. An anti-attention-deficit-disorder or anti-narcolepsy agent, which comprises a precursor protein of the polypeptide of claim 1 or an amide or ester of the precursor protein or a salt thereof.

6. The anti-attention-deficit-disorder or anti-narcolepsy agent of claim 5, wherein the agent comprises the same or substantially the same amino acid sequence as shown in SEQ ID NO: 7, SEQ ID NO: 8, SEQ ID NO: 14, SEQ ID NO: 17 or SEQ ID NO: 23,

**7.** A method for screening compounds having an anti-attention-deficit-disorder or anti-narcolepsy activity or compounds having an anti-anxiety, anti-depression, anti-insomnia, anti-schizophrenia or anti-fear activity or salts of thereof, which comprises using a polypeptide containing the same or substantially the same amino acid sequence as shown in SEQ ID NO: 1 or an amide or ester of the polypeptide or a salt thereof or using a precursor protein of the polypeptide or a salt thereof.

**8.** A kit for screening compounds having an anti-attention-deficit-disorder or anti-narcolepsy activity or compounds having an anti-anxiety, anti-depression, anti-insomnia, anti-schizophrenia or anti-fear activity or salts thereof, which comprises a polypeptide containing the same or substantially the same amino acid sequence as shown in SEQ ID NO: 1 or an amide or ester of the polypeptide or a salt thereof or comprises a precursor protein of the polypeptide or a salt thereof.

**9.** A compound having an anti-attention-deficit-disorder or anti-narcolepsy activity or a compound having an anti-anxiety, anti-depression, anti-insomnia, anti-schizophrenia or anti-fear activity or a salt thereof, which is obtainable using the method of claim 7 or the kit of claim 8.

**10.** An anti-attention-deficit-disorder or anti-narcolepsy agent or an anti-anxiety, anti-depression, anti-insomnia, anti-schizophrenia or anti-fear agent, which comprises the compound of claim 9 or a salt thereof.

**11.** A diagnostic method for attention deficit disorder or narcolepsy or for anxiety, depression, insomnia, schizophrenia or fear, which comprises using a polynucleotide containing part or all of a nucleotide sequence complementary to a DNA containing the nucleotide sequence encoding the polypeptide of claim 1.

**12.** An anti-anxiety, anti-depression, anti-insomnia, anti-schizophrenia or anti-fear agent, which comprises an antibody against the polypeptide of claim 1 or the precursor protein of claim 5 or an amide or ester of the polypeptide or precursor protein or a salt thereof.

**13.** An anti-attention-deficit-disorder or anti-narcolepsy agent or an anti-anxiety, anti-depression, anti-insomnia, anti-schizophrenia or anti-fear agent, which comprises an antibody against a protein containing the amino acid sequence shown in SEQ ID NO: 3 or SEQ ID NO: 11 or an amide or ester of the protein or a salt thereof.

**14.** A diagnostic agent for anxiety, depression, insomnia, schizophrenia or fear, which comprises an antibody against the polypeptide of claim 1 or the precursor protein of claim 5 or an amide or ester of the polypeptide or precursor protein or a salt thereof.

**15.** A diagnostic agent for attention deficit disorder or narcolepsy or for anxiety, depression, insomnia, schizophrenia or fear, which comprises an antibody against a protein containing the amino acid sequence shown in SEQ ID NO: 3 or SEQ ID NO: 11 or an amide or ester of the protein or a salt thereof.

**16.** A diagnostic agent, which comprises single nucleotide polymorphisms (SNPs) of a DNA containing the nucleotide sequence shown in SEQ ID NO: 34.

**17.** The diagnostic agent of claim 16, wherein the diagnostic agent is provided for diagnosis of attention deficit disorder or narcolepsy or diagnosis of anxiety, depression, insomnia, schizophrenia or fear.

**18.** A diagnostic method for attention deficit disorder or narcolepsy or for anxiety, depression, insomnia, schizophrenia or fear, which comprises analyzing single nucleotide polymorphisms (SNPs) of a DNA containing the nucleotide sequence shown in SEQ ID NO: 34.

**19.** An anti-attention-deficit-disorder or anti-narcolepsy agent, which comprises a GPR14 agonist.

**20.** An anti-anxiety, anti-depression, anti-insomnia, anti-schizophrenia or anti-fear agent, which comprises a GPR14 antagonist.

**21.** The anti-anxiety, anti-depression, anti-insomnia, anti-schizophrenia or anti-fear agent of claim 20, wherein the GPR14 antagonist is a compound of Formula (Ia):

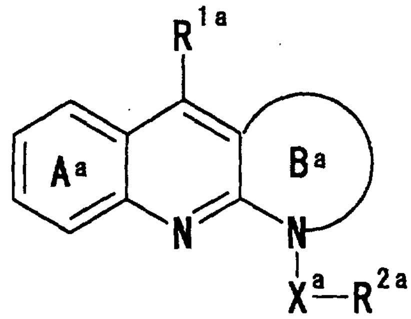

(Ia)

[wherein $A^a$ represents an optionally substituted benzene ring, $B^a$ represents an optionally substituted 5- to 8-membered ring, $X^a$ represents a divalent group containing 1 to 4 atoms in its linear chain moiety, $R^{1a}$ represents an optionally substituted amino group, and $R^{2a}$ represents an optionally substituted cyclic group] or a salt thereof.

**22.** The anti-anxiety, anti-depression, anti-insomnia, anti-schizophrenia or anti-fear agent of claim 20, wherein the GPR14 antagonist is a compound of Formula (IIa):

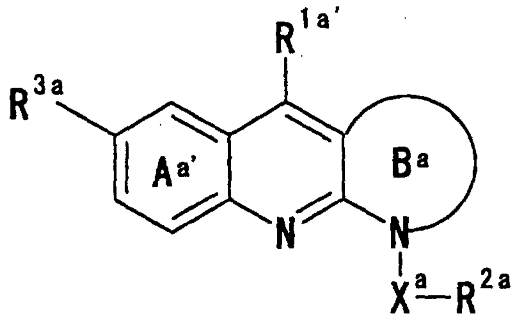

(IIa)

[wherein $A^{a'}$ represents a benzene ring which may have a substituent in addition to the substituent $R^{3a}$, $B^a$ represents an optionally substituted 5- to 8-membered ring, $X^a$ represents a divalent group containing 1 to 4 atoms in its linear chain moiety, $R^{1a'}$ represents a substituted amino group, $R^{2a}$ represents an optionally substituted cyclic group, and $R^{3a}$ represents an optionally substituted hydrocarbon group, an optionally substituted heterocyclic group, a nitro group, a halogen atom, an optionally substituted amino group or a group of the formula $R^{4a}$-$Y^a$- (wherein $Y^a$ represents an oxygen atom or an optionally oxidized sulfur atom, and $R^{4a}$ represents an optionally substituted hydrocarbon group or an optionally substituted heterocyclic group)] or a salt thereof.

**23.** The anti-anxiety, anti-depression, anti-insomnia, anti-schizophrenia or anti-fear agent of claim 20, wherein the GPR14 antagonist is a compound of Formula (Ib):

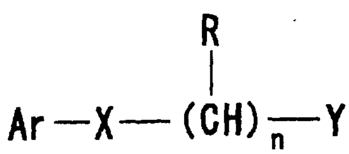

(Ib)

[wherein Ar represents an optionally substituted aryl group, X represents a spacer containing 1 to 4 atoms in its linear chain moiety, n represents an integer of I to 10, R represents a hydrogen atom or an optionally substituted hydrocarbon group which may be the same or different in n repeated units or R may form a ring together with Ar or a substituent on Ar, and Y represents an optionally substituted amino group or an optionally substituted nitrogen-containing heterocyclic group] or a salt thereof.

**24.** The anti-anxiety, anti-depression, anti-insomnia, anti-schizophrenia or anti-fear agent of claim 20, wherein the GPR14 antagonist is a compound of Formula (IIb):

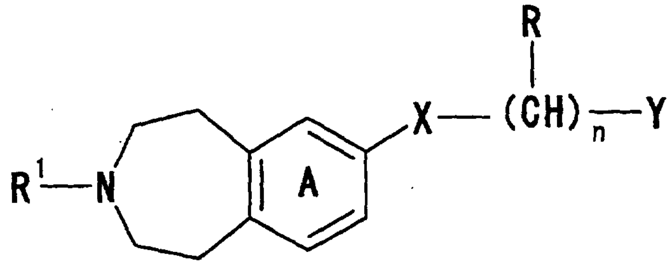

(IIb)

[wherein $R^1$ represents a hydrogen atom, an optionally substituted hydrocarbon group or an optionally substituted acyl group, the ring A represents a benzene ring which may have an additional substituent, X represents a spacer containing 1 to 4 atoms in its linear chain moiety, n represents an integer of 1 to 10, R represents a hydrogen atom or an optionally substituted hydrocarbon group which may be the same or different in n repeated units or R may form a ring together with the ring A or a substituent on the ring A, and Y represents an optionally substituted amino group or an optionally substituted nitrogen-containing heterocyclic group] or a salt thereof.

**25.** The anti-anxiety, anti-depression, anti-insomnia, anti-schizophrenia or anti-fear agent of claim 20, wherein the GPR14 antagonist is a compound of Formula (Ic):

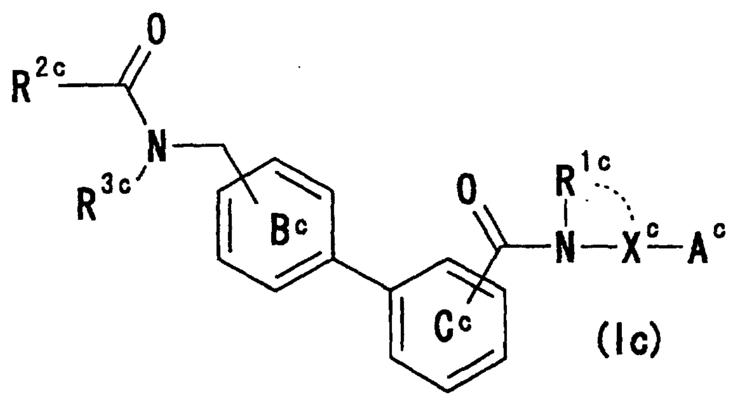

(Ic)

[wherein $R^{1c}$ represents a hydrogen atom or an optionally substituted hydrocarbon group, $X^c$ represents a spacer containing 1 to 12 atoms in its linear chain moiety, $R^{1c}$ and $X^c$ may together form a ring, $A^c$ represents an optionally substituted amino group or an optionally substituted nitrogen-containing heterocyclic group, $R^{2c}$ represents an optionally substituted hydrocarbon group or an optionally substituted amino group, $R^{3c}$ represents an optionally substituted hydrocarbon group, the rings $B^c$ and $C^c$ each represent a benzene ring which may further be substituted] or a salt thereof.

**26.** A prophylactic or therapeutic method for attention deficit disorder or narcolepsy, which comprises administering an effective amount of a GPR14 agonist to a mammal.

**27.** Use of a GPR14 agonist for the preparation of a prophylactic or therapeutic agent for attention deficit disorder or narcolepsy.

**28.** A prophylactic or therapeutic method for anxiety, depression, insomnia, schizophrenia or fear, which comprises administering an effective amount of a GPR14 antagonist to a mammal.

**29.** Use of a GPR14 antagonist for the preparation of a prophylactic or therapeutic agent for anxiety, depression, insomnia, schizophrenia or fear.

Fig. 1(A)

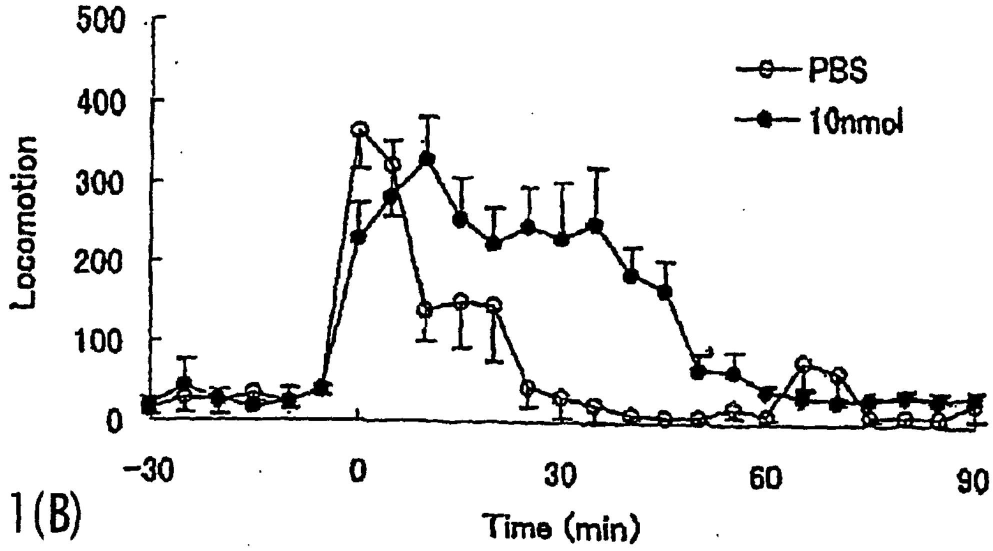

500
400
300
200
100
0
Locomotion
-30
0
30
60
90
Time (min)
PBS
10nmol


Fig. 1(B)

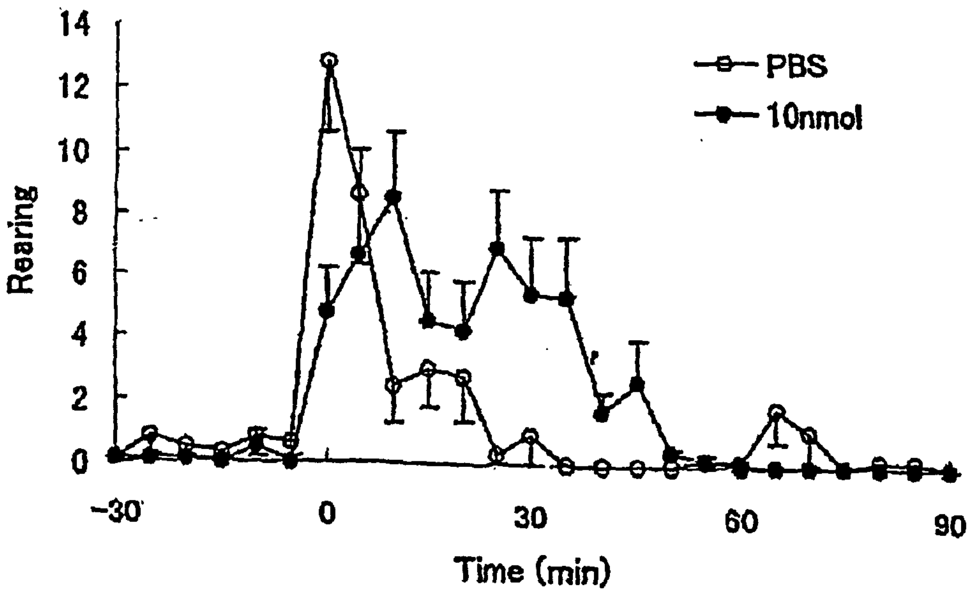

14
12
10
8
6
4
2
0
Rearing
-30
0
30
60
90
Time (min)
PBS
10nmol

Fig. 2(A)

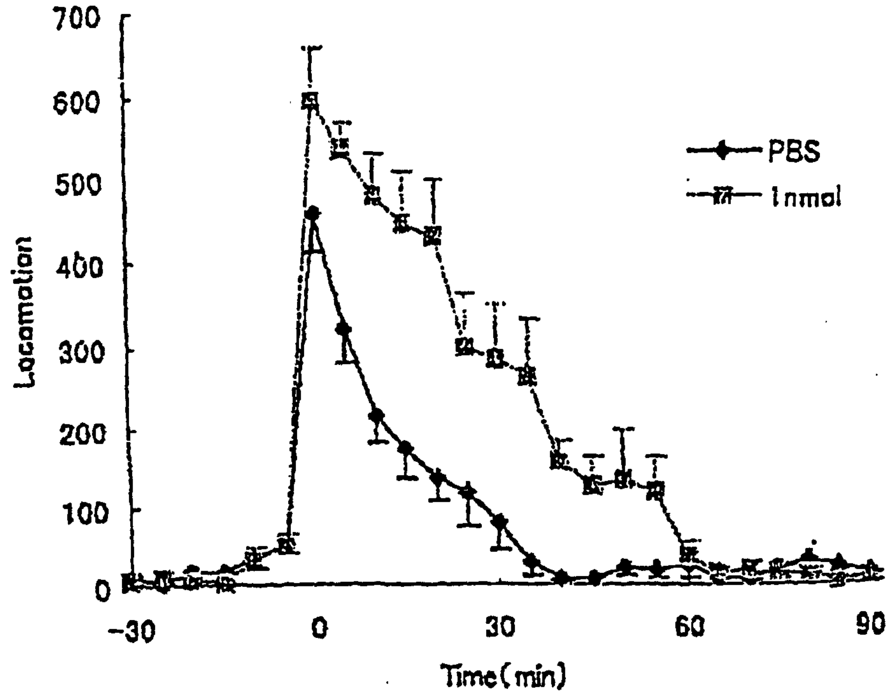

700
600
500
400
300
200
100
0
Locomotion
PBS
1nmol
-30
0
30
60
90
Time(min)


Fig. 2(B)

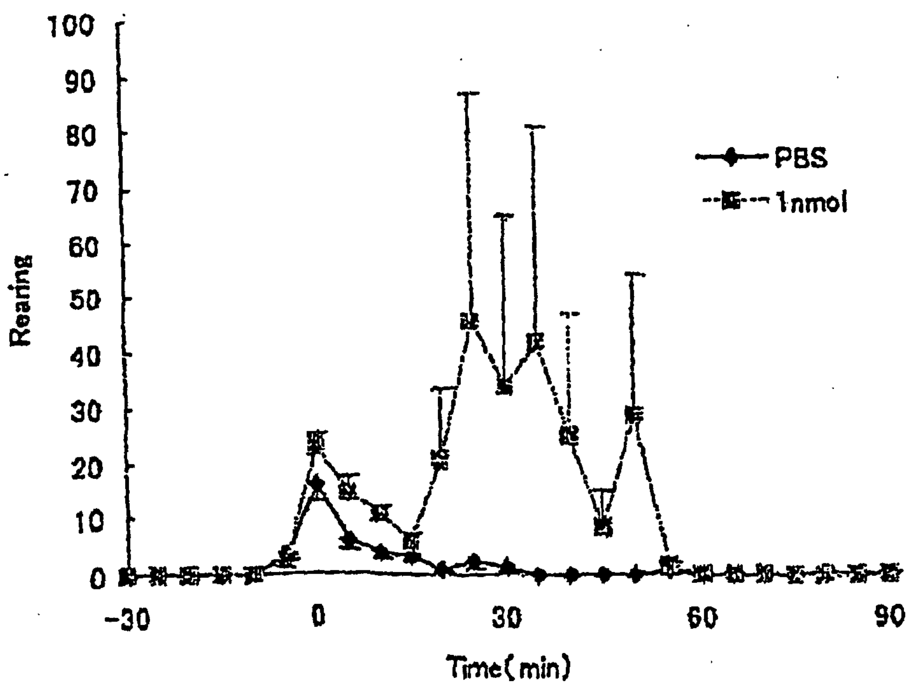

100
90
80
70
60
50
40
30
20
10
0
Rearing
PBS
1nmol
-30
0
30
60
90
Time(min)

Fig. 3(A)

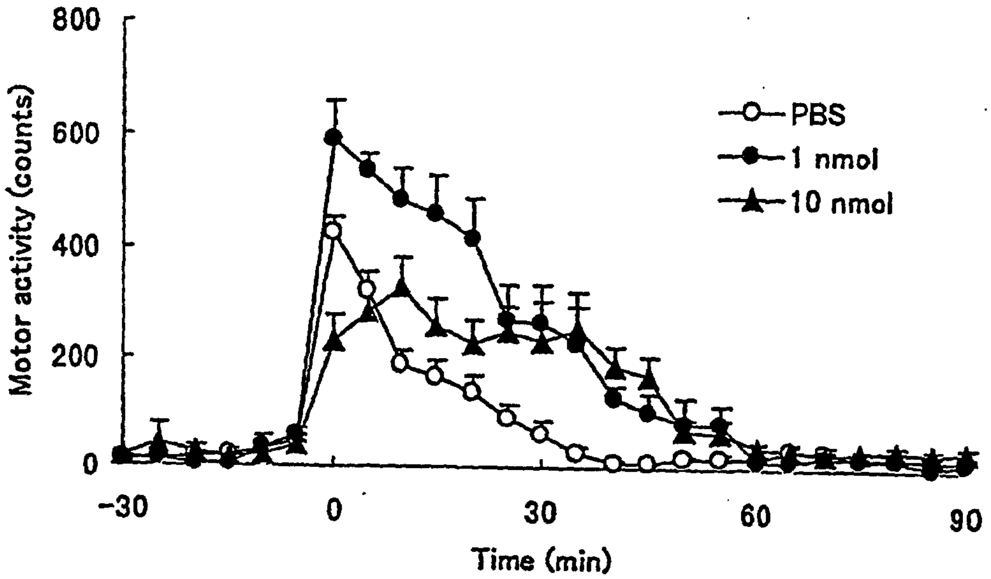
800
600
400
200
0
Motor activity (counts)
PBS
1 nmol
10 nmol
-30
0
30
60
90
Time (min)

Fig. 3(B)

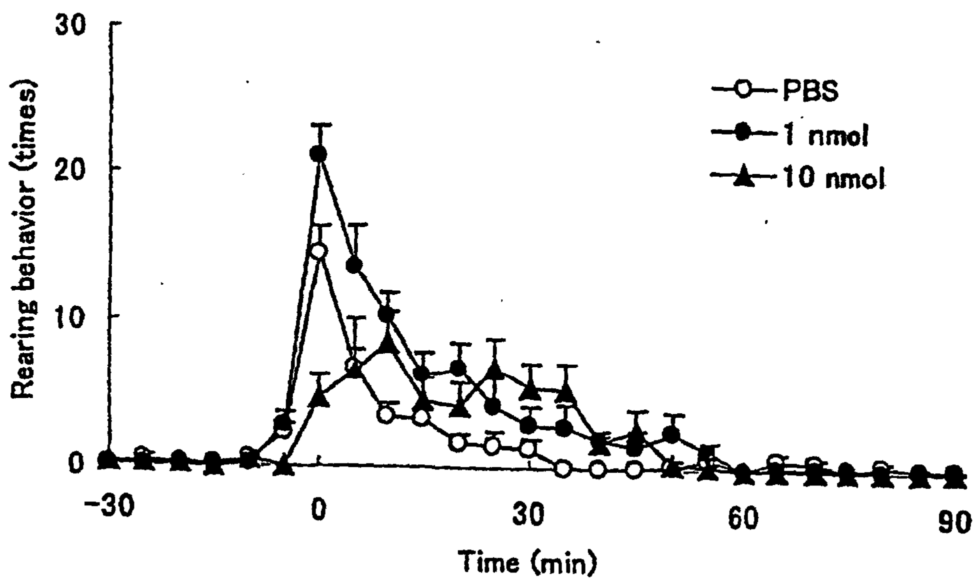
30
20
10
0
Rearing behavior (times)
PBS
1 nmol
10 nmol
-30
0
30
60
90
Time (min)

Fig. 4(A)

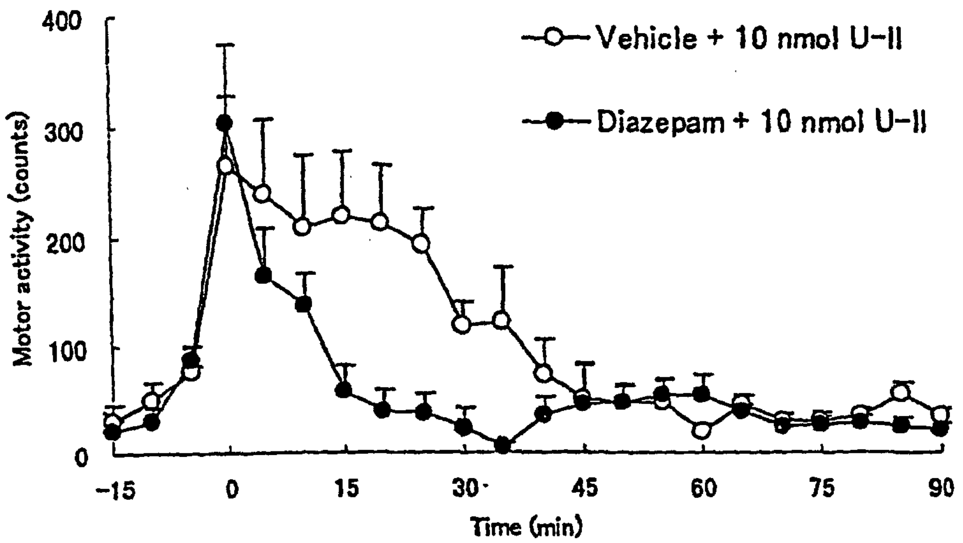

400
300
200
100
0
Motor activity (counts)
Vehicle + 10 nmol U-II
Diazepam + 10 nmol U-II
-15
0
15
30
45
60
75
90
Time (min)


Fig. 4(B)

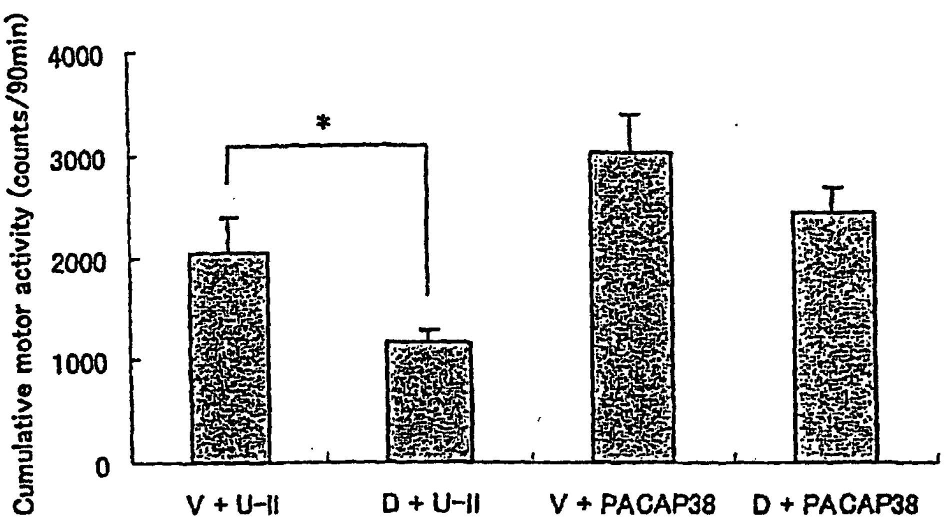

Cumulative motor activity (counts/90min)
4000
3000
2000
1000
0
*
V + U-II
D + U-II
V + PACAP38
D + PACAP38

Fig. 5

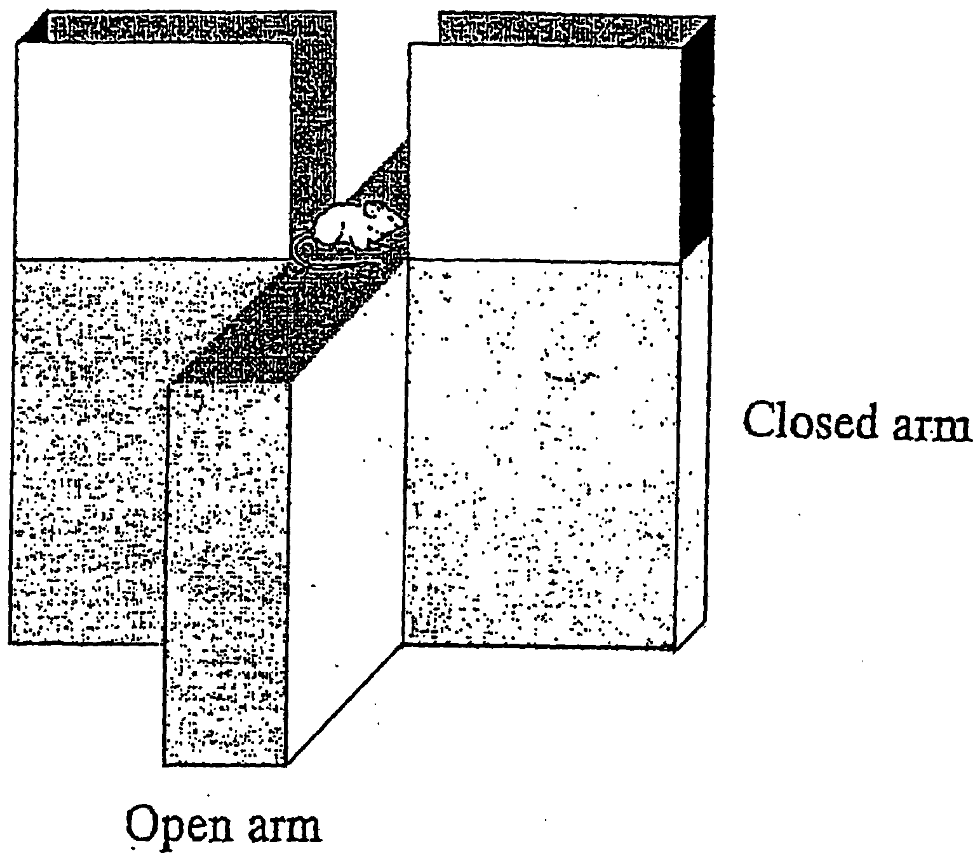

Fig. 6(A)

Number of closed arm visits

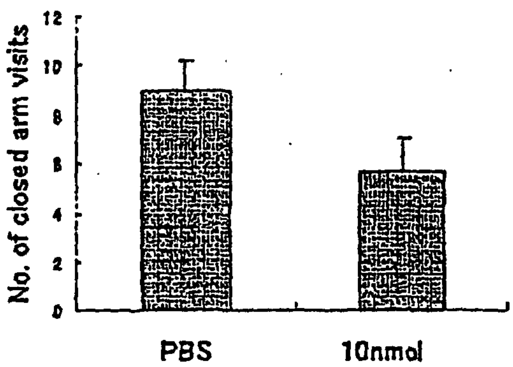


Fig. 6(B)

Time on open arms

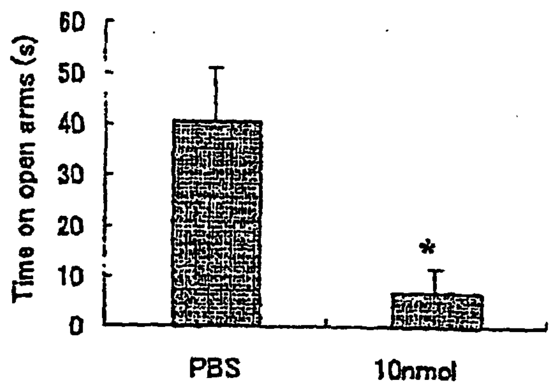


Fig. 6(C)

Number of open arm visits

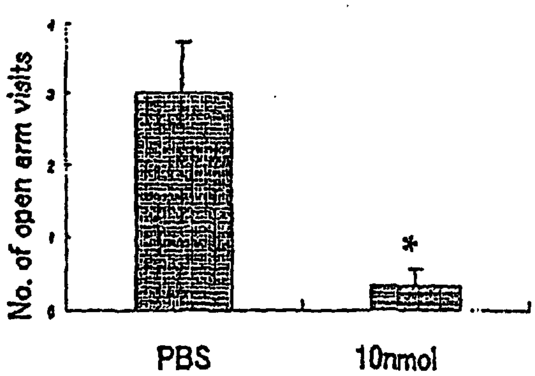

Fig. 7(A)

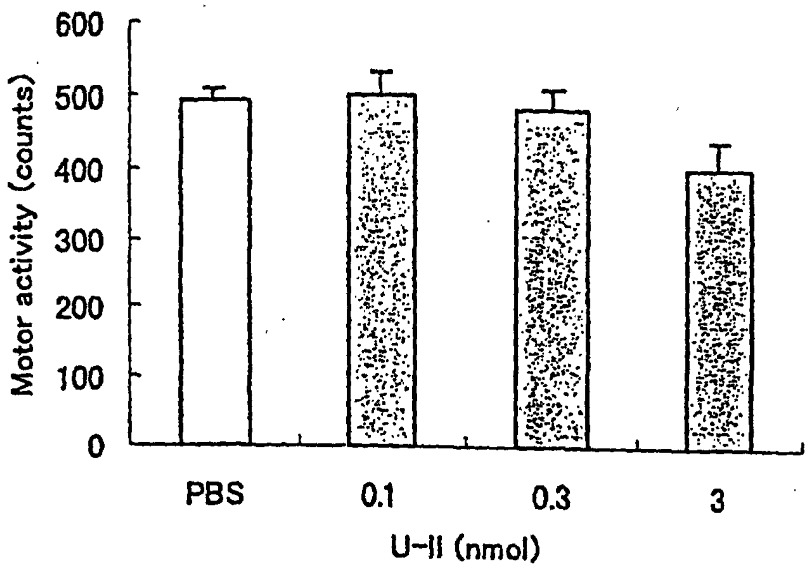
600
500
400
300
200
100
0
Motor activity (counts)
PBS
0.1
0.3
3
U-II (nmol)

Fig. 7(B)

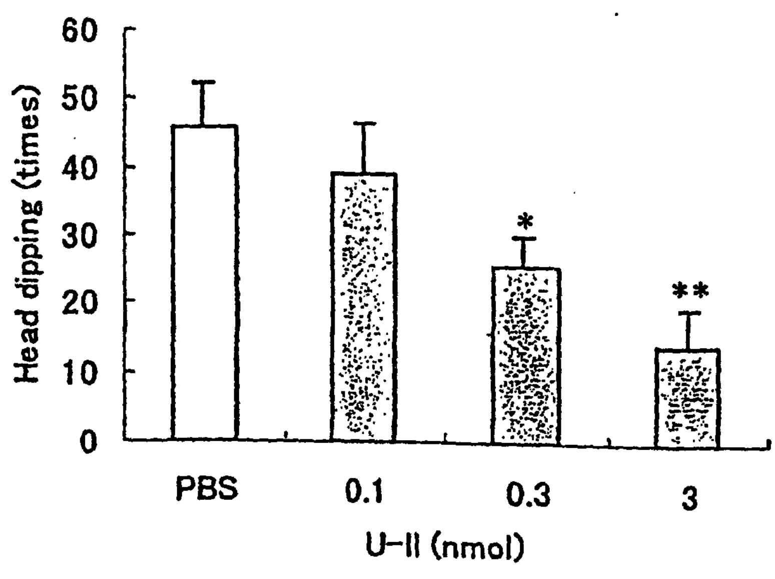
60
50
40
30
20
10
0
Head dipping (times)
*
**
PBS
0.1
0.3
3
U-II (nmol)

Fig. 8

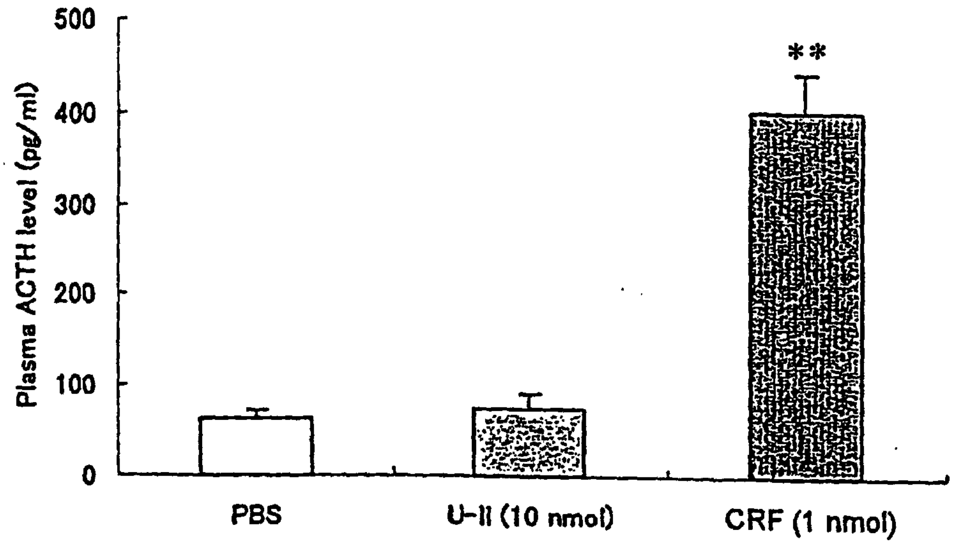
Plasma ACTH level (pg/ml)
500
400
300
200
100
0
**
PBS
U-II (10 nmol)
CRF (1 nmol)

# INTERNATIONAL SEARCH REPORT

International application No.
PCT/JP01/06899

A. CLASSIFICATION OF SUBJECT MATTER
Int.Cl$^7$ C12N15/12, C07K14/47, G01N33/50, G01N33/15, A61K31/711, A61K38/00, A61K45/00, A61K48/00, A61P25/00, A61P25/18, A61P25/20, A61P25/22, A61P25/24, A61P43/00, A61K31/4745, C07D471/04, A61K31/4375, A61K31/55, C07D223/16, A61K31/4025, C07D207/32

According to International Patent Classification (IPC) or to both national classification and IPC

B. FIELDS SEARCHED

Minimum documentation searched (classification system followed by classification symbols)
Int.Cl$^7$ C12N15/12, C07K14/47, G01N33/50, G01N33/15, A61K31/711, A61K38/00, A61K45/00, A61K48/00, A61K31/4745, C07D471/04, A61K31/4375, A61K31/55, C07D223/16, A61K31/4025, C07D207/32

Documentation searched other than minimum documentation to the extent that such documents are included in the fields searched

Electronic data base consulted during the international search (name of data base and, where practicable, search terms used)
JICST FILE (JOIS), WPI (DIALOG), BIOSIS (DIALOG), MEDLINE (STN), CAPLUS (STN), REGISTRY (STN), EMBL/DDBJ/Genebank/PIR/Swissprot/Geneseq

C. DOCUMENTS CONSIDERED TO BE RELEVANT

| Category* | Citation of document, with indication, where appropriate, of the relevant passages | Relevant to claim No. |
|---|---|---|
| X | WO 99/40192 A1 (SmithKline Beecham Corporation), 12 August, 1999 (12.08.99), & US 6159700 A & EP 1056844 A & US 6133420 A Claims; pages 30 to 32 | 1-10,12-17, 19-20,27,29 |
| X | EP 859052 A1 (SmithKline Beecham Corporation), 19 August, 1998 (19.08.98), & US 5851798 A & US 6005074 A & JP 10-295376 A Claims; page 9, lines 28 to 40 | 1-10,12-17, 19-20,27,29 |
| X | EP 430485 A2 (Ube Ind. Ltd.), 05 June, 1991 (05.06.91), & JP 3-220189 A page 2, lines 4 to 6 | 21-22 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 October, 2001 (25.10.01) | 06 November, 2001 (06.11.01) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP01/06899

C (Continuation). DOCUMENTS CONSIDERED TO BE RELEVANT

| Category* | Citation of document, with indication, where appropriate, of the relevant passages | Relevant to claim No. |
|---|---|---|
| X | EP 487071 A1 (Takeda Chem. Ind., Ltd.), 27 May, 1992 (27.05.92), & US 5273974 A & JP 5-140149 A & AU 9188045 A & CA 2055947 A & CN 1062143 A & RU 2095361 B page 50, lines 50 to 57 | 23-24 |
| X | EP 560235 A1 (Takeda Chem. Ind., Ltd.), 15 September, 1993 (15.09.93), & US 5462934 A & JP 6-166676 A & AU 9333803 A & CA 2091216 A & CN 1078969 A page 54, lines 19 to 23 | 23-24 |
| X | EP 607864 A2 (Takeda Chem. Ind., Ltd.), 27 July, 1994 (27.07.94), & US 5527800 A & JP 7-206854 A & AU 9453861 A & CA 2113603 A & CN 1104211 A & US 5686466 A page 71, lines 23 to 30 | 23 |
| X | EP 533266 A1 (Glaxo Group Ltd.), 24 March, 1993 (24.03.93), & US 5356893 A & JP 6-107649 A & AU 9224529 A & CA 2078506 A & CN 1071922 A page 5, lines 23 to 30 | 25 |
| P,A | WO 00/32627 A1 (Takeda Chem. Ind., Ltd.), 08 June, 2000 (08.06.00), & AU 200014112 A & EP 1136503 A & JP 2001-128688 A | 1-10,12-17, 19-20,27,29 |
| P,A | WO 01/04298 A1 (Takeda Chem. Ind., Ltd.), 18 January, 2001 (18.01.01), & AU 200058484 A & JP 2001-69996 A | 1-10,12-17, 19-20,27,29 |
| P,A | WO 01/66143 A1 (Takeda Chem. Ind., Ltd.), 13 September, 2001 (13.09.01), (Family: none) | 1-10,12-17, 19-20,27,29 |

Form PCT/ISA/210 (continuation of second sheet) (July 1992)

## INTERNATIONAL SEARCH REPORT

International application No.
PCT/JP01/06899

**Box I Observations where certain claims were found unsearchable (Continuation of item 1 of first sheet)**

This international search report has not been established in respect of certain claims under Article 17(2)(a) for the following reasons:

1. ☒ Claims Nos.: 11,18,26,28
because they relate to subject matter not required to be searched by this Authority, namely:

The inventions as set forth in claims 11, 18, 26 and 28 pertain to methods for treatment and diagnosis of diseases and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)